# EUROPÄISCHE PATENTSCHRIFT

(11) **EP 0 958 292 B1**
(45) Veröffentlichungstag und Bekanntmachung des Hinweises auf die Patenterteilung: **03.01.2007**
(21) Anmeldenummer: 98907930.6
(22) Anmeldetag: 08.01.1998
(51) Int. Cl.: C07D 413/10, A01N 43/72, A01N 43/48, C07D 417/10, C07D 409/10, C07D 405/10, C07D 401/10, C07D 403/10, C07D 411/10, C07D 231/20, C07D 231/24

(54) **4-(3-HETEROCYCLYL-1-BENZOYL)PYRAZOLE UND IHRE VERWENDUNG ALS HERBIZIDE**
4-(3-HETEROCYCLYL-1-BENZOYL)PYRAZOLES AND THEIR USE AS HERBICIDES
4-(3-HETEROCYCLYLE-1-BENZOYLE)PYRAZOLES ET LEUR UTILISATION COMME HERBICIDES

(30) Priorität: 17.01.1997 DE 19701446; 15.09.1997 DE 19740494
(43) Veröffentlichungstag der Anmeldung: 24.11.1999
(73) Patentinhaber: BASF Aktiengesellschaft, 67056 Ludwigshafen (DE)
(72) Erfinder: VON DEYN, Wolfgang, D-67435 Neustadt (DE); HILL, Regina, Luise, D-67346 Speyer (DE); BAUMANN, Ernst, D-67373 Dudenhofen (DE); ENGEL, Stefan, D-65510 Idstein (DE); MAYER, Guido, D-67433 Neustadt (DE); RHEINHEIMER, Joachim, D-67063 Ludwigshafen (DE); WITSCHEL, Matthias, D-67061 Ludwigshafen (DE); MISSLITZ, Ulf, D-67433 Neustadt (DE); WAGNER, Oliver, D-67061 Ludwigshafen (DE); OTTEN, Martina, D-67069 Ludwigshafen (DE); WALTER, Helmut, D-67283 Obrigheim (DE); WESTPHALEN, Karl-Otto, D-67346 Speyer (DE)
(86) Internationale Anmeldenummer: PCT/EP1998/000070
(87) Internationale Veröffentlichungsnummer: WO 1998/031682

(56) Entgegenhaltungen:
- EP-A- 0 203 428
- WO-A-96/26206
- GB-A- 2 122 188
- US-A- 4 460 597

## Beschreibung

Die vorliegende Erfindung betrifft 4-(3-Heterocyclyl-1-benzoyl)-pyrazole der Formel I 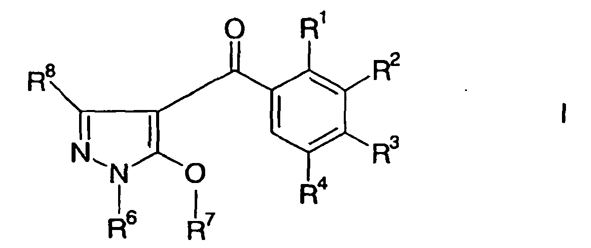 in der die Variablen folgende Bedeutungen haben:
- R¹, R³: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
- R²: für einen unsubstituierten oder durch einen oder zwei Reste aus folgender Gruppe:
C₁-C₄-Alkyl, C₃-C₆-Cycloalkyl, C₁-C₄-Halogenalkyl,
C₁-C₄-Alkoxy-C₁-C₄-alkyl, C₁-C₄-Alkoxycarbonyl,
C₁-C₄-Alkoxy oder C₃-C₆-Spirocycloalkan;
substituierten 5-gliedrigen, C-verknüpften Heterocyclyl-Rest, enthaltend zwei gleiche oder verschiedene Hetero-atome, ausgewählt aus folgender Gruppe:
Sauerstoff, Schwefel oder Stickstoff;
- R⁴: Wasserstoff, Halogen oder C₁-C₆-Alkyl;
- R⁶: C₁-C₆-Alkyl;
- R⁷: C₁-C₆-Alkyl, C₃-C₆-Alkenyl, C₃-C₆-Alkinyl, C₁-C₆-Alkyl-carbonyl, C₂-C₆-Alkenylcarbonyl, C₁-C₆-Alkoxycarbonyl, Di-(C₁-C₆-alkyl)-aminocarbonyl, N-(C₁-C₆-Alkoxy)-N-(C₁-C₆-alkyl)-aminocarbonyl oderDi-(C₁-C₆-alkyl)-aminothiocarbonyl, wobei die genannten Alkyl- und Alkoxyreste partiell oder vollständig halogeniert sein können und/oder eine bis drei der folgenden Gruppen tragen können:
Cyano, C₁-C₄-Alkoxy, C₁-C₄-Alkoxycarbonyl, C₁-C₄-Alko-xy-C₁-C₄-alkoxycarbonyl, Di-(C₁-C₄-alkyl)-amino-C₁-C₄-alkoxycarbonyl, C₁-C₄-Alkylaminocarbonyl, Di-(C₁-C₄-alkyl)-aminocarbonyl oder C₁-C₄-Alkylcarbonyloxy;
Phenyl-C₁-C₆-alkyl, Phenylcarbonyl-C₁-C₆-alkyl, Phenyl-carbonyl oder Phenyl-C₂-C₆-alkenylcarbonyl wobei der Phenyl-Rest der 4 letztgenannten Substituenten partiell oder vollständig halogeniert sein kann und/oder einen bis drei der folgenden Reste tragen kann:
   Nitro, Cyano, C₁-C₄-Alkyl, C₁-C₄-Halogenalkyl, C₁-C₄-Alkoxy oder C₁-C₄-Halogenalkoxy;
- R⁸: Wasserstoff, C₁-C₆-Alkyl oder C₁-C₆-Halogenalkyl;
sowie deren landwirtschaftlich brauchbaren Salze.

Außerdem betrifft die Erfindung Verfahren zur Herstellung von Verbindungen der Formel I, Mittel welche diese enthalten sowie die Verwendung dieser Derivate oder diese enthaltende Mittel zur Schadpflanzenbekämpfung.

Aus der Literatur, beispielsweise aus EP-A 282 944,
EP-A 203 428, US-A 4,460,597, GB-A-2 122 188, WO 96/26206 und der älteren deutschen Patentanmeldung DE-A 19 701 446 sind pyrazol-4-yl-benzoylderivate bekannt. Die herbiziden Eigenschaften der bisher bekannten Verbindungen sowie die Verträglichkeiten gegenüber Kulturpflanzen können jedoch nur bedingt befriedigen.
Es lag daher dieser Erfindung die Aufgabe zugrunde, neue, insbesondere herbizid wirksame, Verbindungen mit verbesserten Eigenschaften zu finden.

Demgemäß wurden die 4-(3-Heterocyclyl-1-benzoyl)pyrazole der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Di-(2-hydroxyeth-1-yl)ammonium, [2-(2-Hydroxy-eth-1-oxy)-eth-1-yl]-ammonium, Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R⁸ oder als Reste an Phenyl- und Heterocyclyl-Resten genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cyanoalkyl-, Alkoxy-, Halogenalkoxy-, Alkylthio-, Halogenalkylthio-, Alkylsulfinyl-, Halogenalkylsulfinyl-, Alkylsulfonyl-, Halogenalkylsulfonyl-, N-Alkylaminosulfonyl, N,N-Dialkylaminosulfonyl, Dialkylamino-, N-Alkylsulfonylamino, N-Halogenalkylsulfonylamino, N-Alkyl-N-alkylsulfonylamino, N-Alkyl-N-halogenalkylsulfonylamino, Alkylcarbonyl-, Halogenalkylcarbonyl-, Alkoxycarbonyl-, Halogenalkoxycarbonyl, Alkylcarbonyloxy-, Alkylaminocarbonyl-, Dialkylaminocarbonyl-, Dialkylaminothiocarbonyl, Alkoxyalkyl-, Dialkoxyalkyl-, Alkylthioalkyl-, Dialkylaminoalkyl-, Dialkylhydrazinoalkyl-, Alkyliminooxyalkyl-, Alkylcarbonylalkyl, Alkoxyiminoalkyl, N-(Alkyl-amino)-iminoalkyl, N-(Dialkylamino)-iminoalkyl, Alkoxycarbonylalkyl-, Dialkylaminocarbonylalkyl-, Phenylalkenylcarbonyl, Heterocyclylalkenylcarbonyl, N-Alkoxy-N-alkylaminocarbonyl-, N-Alkyl-N-phenylaminocarbonyl-, N-Alkyl-N-heterocyclylaminocarbonyl-, Phenylalkyl-, Heterocyclylalkyl-, Phenylcarbonylalkyl-, Heterocyclylcarbonylalkyl-, Dialkylaminoalkoxycarbonyl-, Alkoxyalkoxycarbonyl-, Alkenylcarbonyl-, Alkenyloxycarbonyl-, Alkenylaminocarbonyl-, N-Alkenyl-N-alkylaminocarbonyl-, N-Alkenyl-N-alkoxyaminocarbonyl-, Alkinylcarbonyl-, Alkinyloxycarbonyl-, Alkinylaminocarbonyl-, N-Alkinyl-N-alkylaminocarbonyl-, N-Alkinyl-N-alkoxyaminocarbonyl-, Alkenyl-, Alkinyl-, Halogenalkenyl-, Halogenalkinyl- und Alkoxyalkoxy-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl: z.B. Methyl, Ethyl, Propyl, 1-Methylethyl, Butyl, 1-Methylpropyl, 2-Methylpropyl oder 1,1-Dimethylethyl;
- C₁-C₆-Alkyl, sowie die Alkylteile von N(C₁-C₆-Alkoxy)-N-(C₁-C₆-alkyl)-aminocarbonyl, Phenyl-C₁-C₆-alkyl, Phenylcarbonyl-C₁-C₆-alkyl : C₁-C₄-Alkyl, wie voranstehend genannt, sowie z.B. Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl: C₁-C₄-Halogenalkyl, wie voranstehend genannt, sowie z.B. 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Cyanoalkyl: z.B. Cyanomethyl, 1-Cyanoeth-1-yl, 2-Cyano-eth-1-yl, 1-Cyanoprop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyano-but-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl oder 2-Cyanomethyl-prop-2-yl;
- C₁-C₄-Alkoxy, z.B. Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy, sowie die Alkoxyteile von N- (C₁-C₆-Alkoxy) -N-(C₁-C₆-alkyl) -aminocarbonyl C₁-C₄-Alkoxy, wie voranstehend genannt, sowie z.B. Pentoxy, 1-Methylbutoxy, 2-Methylbutoxy, 3-Methoxylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₄-Halogenalkoxy: einen C₁-C₄-Alkoxyrest, wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethexy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;
- C₁-C₆-Halogenalkoxy: C₁-C₄-Halogenalkoxy, wie voranstehend genannt, sowie z.B. 5-Fluorpentoxy, 5-Chlorpentoxy, 5-Brompentoxy, 5-Iodpentoxy, Undecafluorpentoxy, 6-Fluorhexoxy, 6-Chlorhexoxy, 6-Bromhexoxy, 6-Iodhexoxy oder Dodecafluorhexoxy;
- C₁-C₄-Alkylthio: z.B. Methylthio, Ethylthio, Propylthio, 1-Methylethylthio, Butylthio, 1-Methylpropylthio, 2-Methylpropylthio oder 1,1-Dimethylethylthio;
- C₁-C₆-Alkylthio: C₁-C₄-Alkylthio, wie voranstehend genannt, sowie z.B. Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₄-Halogenalkylthio: einen C₁-C₄-Alkylthiorest, wie voranstehend genannt, der partiell oder vollständig durch Fluor, Chlor, Brom und/oder Iod substituiert ist, also z.B. Fluormethylthio, Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2-Dichlor-2-fluorethylthio, Pentafluorethylthio, 2-Fluorpropylthio, 3-Fluorpropylthio, 2-Chlorpropylthio, 3-Chlorpropylthio, 2-Brompropylthio, 3-Brompropylthio, 2,2-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;
- C₁-C₆-Halogenalkylthio: C₁-C₄-Halogenalkylthio, wie voranstehend genannt, sowie z.B. 5-Fluorpentylthio, 5-Chlorpentylthio, 5-Brompentylthio, 5-Iodpentylthio, Undecafluorpentylthio, 6-Fluorhexylthio, 6-Chlorhexylthio, 6-Bromhexylthio, 6-Iodhexylthio oder Dodecafluorhexylthio;
- C₁-C₆-Alkylsulfinyl (C₁-C₆-Alkyl-S(=O)-): z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropylsulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl oder 1-Ethyl-2-methylpropylsulfinyl;
- C₁-C₆-Halogenalkylsulfinyl: C₁-C₆-Alkylsulfinylrest, wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentylsulfinyl, 5-Brompentylsulfinyl, 5-Iodpentylsulfinyl, Undecafluorpentylsulfinyl, 6-Fluorhexylsulfinyl, 6-Chlorhexylsulfinyl, 6-Bromhexylsulfinyl, 6-Iodhexylsulfinyl oder Dodecafluorhexylsulfinyl;
- C₁-C₆-Alkylsulfonyl (C₁-C₆-Alkyl-S(=O)₂-) : z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Dimethylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl;
- C₁-C₆-Halogenalkylsulfonyl : einen C₁-C₆-Alkylsulfonylrest, wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl oder Dodecafluorhexylsulfonyl;
- C₁-C₆-Alkylamino. , also z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino oder 1-Ethyl-2-methylpropylamino;
- Di-(C₁-C₄-alkyl)amino, sowie die Dialkylaminoreste von Di-(C₁-C₄-alkyl)amino-C₁-C₄-alkoxycarbonyl und N-(Di-C₁-C₆-alkylamino)-imino-C₁-C₆-alkyl, also z.B. N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, H-Dutyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)-amino, N-Butyl-N-(2-methylpropyl)amino, N-Butyl-N-(1,1-dimethylethyl)amino, N-(1-Methylpropyl)-N-(2-methylpropyl)-amino, N-(1,1-Dimethylethyl)-N-(1-methylpropyl)-amino oder N-(1,1-Dimethylethyl)-N-(2-methylpropyl)amino;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcarbonyl oder 1,1-Dimethylethylcarbonyl;
- C₁-C₆-Alkylcarbonyl, sowie die Alkylcarbonylreste von C₁-C₆-Alkylcarbonyl-C₁-C₆-alkyl: C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2,-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl; C₁-C₄-Halogenalkylcarbonyl: einen C₁-C₄-Alkylcarbonylrest, wie vorstehend genannt, der partiell oder vollständig durch Fluor, Chlor, Brom und/oder Iod substituiert ist, also z.B. Chloracetyl, Dichloracetyl, Trichloracetyl, Fluoracetyl, Difluoracetyl, Trifluoracetyl, Chlorfluoracetyl, Dichlorfluoracetyl, Chlordifluoracetyl, 2-Fluorethylcarbonyl, 2-Chlorethylcarbonyl, 2-Bromethylcarbonyl, 2-Iodethylcarbonyl, 2,2-Difluorethylcarbonyl, 2,2,2-Trifluorethylcarbonyl, 2-Chlor-2-fluorethylcarbonyl, 2-Chlor-2,2-difluorethylcarbonyl, 2,2-Dichlor-2-fluorethylcarbonyl, 2,2,2-Trichlorethylcarbonyl, Pentafluorethylcarbonyl, 2-Fluorpropylcarbonyl, 3-Fluorpropylcarbonyl, 2,2-Difluorpropylcarbonyl, 2,3-Difluorpropylcarbonyl, 2-Chlorpropylcarbonyl, 3-Chlorpropylcarbonyl, 2,3-Dichlorpropylcarbonyl, 2-Brompropylcarbonyl, 3-Brompropylcarbonyl, 3,3,3-Trifluorpropylcarbonyl, 3,3,3-Trichlorpropylcarbonyl, 2,2,3,3,3-Pentafluorpropylcarbonyl, Heptafluorpropylcarbonyl, 1-(Fluormethyl)-2-fluorethylcarbonyl, 1-(Chlormethyl)-2-chlorethylcarbonyl, 1-(Brommethyl)-2-bromethylcarbonyl, 4-Fluorbutylcarbonyl, 4-Chlorbutylcarbonyl, 4-Brombutylcarbonyl oder Nonafluorbutylcarbonyl;
- C₁-C₄-Alkoxycarbonyl, sowie die Alkoxycarbonylteile von Di-(C₁-C₄-alkyl)amino-C₁-C₄-alkoxycarbonyl, also z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₆-Alkoxy)carbonyl: (C₁-C₄-Alkoxy)carbonyl, wie vorstehend genannt, sowie z.B. Pentoxycarbonyl, 1-Methylbutoxycarbonyl, 2-Methylbutoxycarbonyl, 3-Methylbutoxycarbonyl, 2,2-Dimethylpropoxycarbonyl, 1-Ethylpropoxycarbonyl, Hexoxycarbonyl, 1,1-Dimethylpropoxycarbonyl, 1,2-Dimethylpropoxycarbonyl, 1-Methylpentoxycarbonyl, 2-Methylpentoxycarbonyl, 3-Methylpentoxycarbonyl, 4-Methylpentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl, 1,1,2-Trimethylpropoxycarbonyl, 1,2,2-Trimethylpropoxycarbonyl, 1-Ethyl-1-methyl-propoxycarbonyl oder 1-Ethyl-2-methyl-propoxycarbonyl;
- C₁-C₄-Halogenalkoxycarbonyl: einen C₁-C₄-Alkoxycarbonylrest, wie voranstehend genannt, der partiell oder vollständig durch Fluor, Chlor, Brom und/oder Iod substituiert ist, also z.B. Fluormethoxycarbonyl, Difluormethoxycarbonyl, Trifluormethoxycarbonyl, Chlordifluormethoxycarbonyl, Bromdifluormethoxycarbonyl, 2-Fluorethoxycarbonyl, 2-Chlorethoxycarbonyl, 2-Bromethoxycarbonyl, 2-Iodethoxycarbonyl. 2,2-Difluorethoxycarbonyl, 2,2,2-Trifluorethoxycarbonyl, 2-Chlor-2-fluorethoxycarbonyl, 2-Chlor-2,2-difluorethoxycarbonyl, 2,2-Dichlor-2-fluorethoxycarbonyl, 2,2,2-Trichlorethoxycarbonyl, Pentafluorethoxycarbonyl, 2-Fluorpropoxycarbonyl, 3-Fluorpropoxycarbonyl, 2-Chlorpropoxycarbonyl, 3-Chlorpropoxycarbonyl, 2-Brompropoxycarbonyl, 3-Brompropoxycarbonyl, 2,2-Difluorpropoxycarbonyl, 2,3-Difluorpropoxycarbonyl, 2,3-Dichlorpropoxycarbonyl, 3,3,3-Trifluorpropoxycarbonyl, 3,3,3-Trichlorpropoxycarbonyl, 2,2,3,3,3-Pentafluorpropoxycarbonyl, Heptafluorpropoxycarbonyl, 1-(Fluormethyl)-2-fluorethoxycarbonyl, 1-(Chlormethyl)-2-chlorethoxycarbonyl, 1-(Brommethyl)-2-bromethoxycarbonyl, 4-Fluorbutoxycarbonyl, 4-Chlorbutoxycarbonyl, 4-Brombutoxycarbonyl oder 4-Iodbutoxycarbonyl;
- (C₁-C₄-Alkyl)carbonyloxy: Acetyloxy, Ethylcarbonyloxy, Propylcarbonyloxy, 1-Methylethylcarbonyloxy, Butylcarbonyloxy, 1-Methylpropylcarbonyloxy, 2-Methylpropylcarbonyloxy oder 1,1-Dimethylethylcarbonyloxy;
- (C₁-C₄-Alkylamino)carbonyl: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- Di-(C₁-C₄-alkyl)-aminocarbonyl: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)-aminocarbonyl, N,N-Di-(2-methylpropyl)-aminocarbonyl, N,N-Di-(1,1-dimethylethyl)-aminocarbonyl, N-Ethyl-N-methylaminocarbonyl, N-Methyl-N-propylaminocarbonyl, N-Methyl-N-(1-methylethyl)-aminocarbonyl, N-Butyl-N-methylaminbcarbonyl, N-Methyl-N-(1-methylpropyl)-aminocarbonyl, N-Methyl-N-(2-methylpropyl)-aminocarbonyl, N-(1,1-Dimethylethyl)-N-methylaminocarbonyl, N-Ethyl-N-propylaminocarbonyl, N-Ethyl-N-(1-methylethyl)-aminocarbonyl, N-Butyl-N-ethylaminocarbonyl, N-Ethyl-N-(1-methylpropyl)-aminocarbonyl, N-Ethyl-N-(2-methylpropyl)-aminocarbonyl, N-Ethyl-N-(1,1-dimethylethyl)-aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)-aminocarbonyl, N-(1-Methylethyl)-N-(1-methylpropyl)-aminocarbonyl, N-(1-Methylethyl)-N-(2-methylpropyl)-aminocarbonyl, N-(1,1-Dimethylethyl)-N-(1-methylethyl)-aminocarbonyl, N-Butyl-N-(1-methylpropyl)-aminocarbonyl, N-Butyl-N-(2-methylpropyl)-aminocarbonyl, N-Butyl-N-(1,1-dimethylethyl)-aminocarbonyl, N-(1-Methylpropyl)-N-(2-methylpropyl)-aminocarbonyl, N-(1,1-Dimethylethyl)-N-(1-methylpropyl)-aminocarbonyl oder N-(1,1-Dimethylethyl)-N-(2-methylpropyl)-aminocarbonyl;
- Di-(C₁-C₆-alkyl)-aminocarbonyl: Di-(C₁-C₄-alkyl)-aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-aminocarbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-aminocarbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)-aminocarbonyl, N-Methyl-N-(3,3-dimethylbutyl)-aminocarbonyl, N-Methyl-N-(1-ethylbutyl)-aminocarbonyl, N-Methyl-N-(2-ethylbutyl)-aminocarbonyl, N-Methyl-N-(1,1,2-trimethylpropyl)-aminocarbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-aminocarbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)-aminocarbonyl, N-Ethyl-N-(2,3-dimethylbutyl)-aminocarbonyl, N-Ethyl-N-(3.3-dimethylbutyl)-aminocarbonyl, N-Ethyl-N-(1-ethylbutyl)-aminocarbonyl, N-Ethyl-N-(2-ethylbutyl)-aminocarbonyl, N-Ethyl-N-(1.1,2-trimethylpropyl)-aminocarbonyl, N-Ethyl-N-(1,2,2-trimethylpropyl)-aminocarbonyl, N-Ethyl-N-(1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexylaminocarbonyl, N-Butyl-N-hexylaminocarbonyl, N-Pentyl-N-hexylaminocarbonyl oder N,N-Dihexyl-aminocarbonyl;
- Di-(C₁-C₆-alkyl)-aminothiocarbonyl: z.B. N,N-Dimethylamino-thiocarbonyl, N,N-Diethylaminothiocarbonyl, N,N-Di-(1-methylethyl) aminothiocarbonyl, N,-N-Dipropylaminothiocarbonyl, N,N-Dibutylaminothiocarbonyl, N,N-Di-(1-methylpropyl)-amino-thiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)-aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1.1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylaminothiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N-(1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N-(1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl; N-Ethyl-N-pentylaminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)-aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethylpropyl)-aminothiocarbonyl, N-Ethyl-N-(1,2-dimethylpropyl)-aminothiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-aminothiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothiocarbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexylaminothiocarbonyl, N-Butyl-N-hexylaminothiocarbonyl, N-Pentyl-N-hexylaminothiocarbonyl oder N,N-Dihexylaminothiocarbonyl;
- C₁-C₄-Alkoxy-C₁-C₄-alkyl, sowie die Alkoxyalkoxyteile von Di-(C₁-C₄-alkoxy)-C₁-C₄-alkyl: durch C₁-C₄-Alkoxy, wie vorstehend genannt, substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methylpropoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(Propoxy)propyl, 2-(1-Methylethoxy)-propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)propyl, 3-(Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(Propoxy)butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methylpropoxy)butyl, 3-(2-Methylpropoxy)butyl, 3-(1,1-Dimethylethoxy)butyl, 4-(Methoxy)butyl, 4-(Ethoxy)-butyl, 4-(Propoxy)butyl, 4-(1-Methylethoxy)butyl, 4-(Butoxy)-butyl, 4-(1-Methylpropoxy)butyl, 4-(2-Methylpropoxy)butyl oder 4-(1,1-Dimethylethoxy)butyl;
- C₁-C₄-Alkoxycarbonyl-C₁-C₄-alkyl: durch C₁-C₄-Alkoxycarbonyl, wie vorstehend genannt, substituiertes C₁-C₄-Alkyl, also z.B. für Methoxycarbonylmethyl, Ethoxycarbonylmethyl, Propoxycarbonylmethyl, (1-Methylethoxycarbonyl)methyl, Butoxycarbonylmethyl, (1-Methylpropoxycarbonyl)methyl, (2-Methylpropoxycarbonyl)methyl, (1,1-Dimethylethoxycarbonyl)methyl, 1-(Methoxycarbonyl)ethyl, 1-(Ethoxycarbonyl)ethyl, 1-(Propyloxycarbonyl)ethyl, 1-(1-Methylethoxycarbonyl)ethyl, 1-(Butoxycarbonyl)ethyl, 1-(1-Methylpropoxycarbonyl)ethyl, 1-(2-Methylpropoxycarbonyl)ethyl, 1-(1,1-Dimethylethoxycarbonyl)ethyl, 2-(Methoxycarbonyl)ethyl, 2-(Ethoxycarbonyl)ethyl, 2-(Propoxycarbonyl)ethyl, 2-(1-Methylethoxycarbonyl)ethyl, 2-(Butoxycarbonyl)ethyl, 2-(1-Methylpropoxycarbonyl)ethyl, 2-(2-Methylpropoxycarbonyl)ethyl, 2-(1,1-Dimethylethoxycarbonyl)ethyl, 1-(Methoxycarbonyl)propyl, 1-(Ethoxycarbonyl)propyl, 1-(Propoxycarbonyl)propyl, 1-(1-Methylethoxycarbonyl)propyl, 1-(Butoxycarbonyl)propyl, 1-(1-Methylpropoxycarbonyl)propyl, 1-(2-Methylpropoxycarbonyl)propyl, 1-(1,1-Dimethylethoxycarbonyl)pxopyl, 2-(Methoxycarbonyl)propyl, 2-(Ethoxycarbonyl)propyl, 2-(Propoxycarbonyl)propyl, 2-(1-Methylethoxycarbonyl)propyl, 2-(Butoxycarbonyl)propyl, 2-(1-Methylpropoxycarbonyl)propyl, 2-(2-Methylpropoxycarbonyl)propyl, 2-(1,1-Dimethylethoxycarbonyl)propyl, 3-(Methoxycarbonyl)- propyl, 3-(Ethoxycarbonyl)propyl, 3-(Propoxycarbonyl)propyl, 3-(1-Methylethoxycarbonyl)propyl, 3-(Butoxycarbonyl)propyl, 3-(1-Methylpropoxycarbonyl)propyl, 3-(2-Methylpropoxycarbonyl)propyl, 3-(1,1-Dimethylethoxycarbonyl)propyl, 1-(Methoxycarbonyl)butyl, 1-(Ethoxycarbonyl)butyl, 1-(Propyloxycarbonyl)-butyl, 1-(1-Methylethoxycarbonyl)butyl, 1-(Butoxycabonyl)-butyl, 1-(1-Methylpropoxycarbonyl)butyl, 1-(2-Methylpropoxycarbonyl)butyl, 1-(1,1-Dimethylethoxycarbonyl)butyl, 2-(Methoxycarbonyl)butyl, 2-(Ethoxycarbonyl)butyl, 2-(Propoxycarbonyl)butyl, 2-(1-Methylethoxycarbonyl)butyl, 2-(Butoxycarbonyl)butyl, 2-(1-Methylpropoxycarbonyl)butyl, 2-(2-Methylpropoxycarbonyl)butyl, 2-(1,1-Dimethylethoxycarbonyl)-butyl, 3-(Methoxycarbonyl)butyl, 3-(Ethoxycarbonyl)butyl, 3-(Propoxycarbonyl)butyl, 3-(1-Methylethoxycarbonyl)butyl 3-(Butoxycarbonyl)butyl, 3-(1-Methylpropoxycarbonyl)butyl, 3-(2-Methylpropoxycarbonyl)butyl, 3-(1,1-Dimethylethoxycarbonyl)butyl, 4-(Methoxycarbonyl)-butyl, 4-(Ethoxycarbonyl)butyl, 4-(Propoxycarbonyl)butyl, 4-(1-Methylethoxycarbonyl)butyl, 4-(Butoxycarbonyl)butyl, 4-(1-Methylpropoxy)-butoxy, 4-(2-Methylpropoxy)butoxy oder 4-(1,1-Dimethylethoxycarbonyl)butyl;
- Di-(C₁-C₄-alkyl)-aminocarbonyl-C₁-C₄-alkyl: durch Di-(C₁-C₄-alkyl)amino, wie voranstehend genannt, substituiertes C₁-C₄-Alkyl, also z.B. N,N-Dimethylaminocarbonylmethyl, N,N-Diethylaminocarbonylmethyl, N,N-Dipropylaminocarbonylmethyl, N,N-Di-(1-methylethyl)-aminocarbonylmethyl, N,N-Dibutylamino-carbonylmethyl, N,N-Di-(1-methylpropyl)-aminocarbonylmethyl, N,N-Di-(2-methylpropyl)-aminocarbonylmethyl, N,N-Di-(1,1-dimethylethyl)-aminocarbonylmethyl, N-Ethyl-N-methylaminocarbonylmethyl, N-Methyl-N-propylaminocarbonylmethyl, N-Methyl-N-(1-methylethyl)-aminocarbonylmethyl, N-Butyl-N-methylaminocarbonylmethyl, N-Methyl-N-(1-methylpropyl)-aminocarbonylmethyl, N-Methyl-N-(2-methylpropyl)-aminocarbonylmethyl, N-(1,1-Dimethylethyl)-N-methylaminocarbonylmethyl, N-Ethyl-N-propylaminocarbonylmethyl, N-Ethyl-N-(1-methylethyl)-aminocarbonylmethyl, N-Butyl-N-ethylaminocarbonylmethyl, N-Ethyl-N-(1-methylpropyl)-aminocarbonylmethyl, N-Ethyl-N-(2-methylpropyl)-aminocarbonylmethyl, N-Ethyl-N-(1,1-dimethylethyl)-aminocarbonylmethyl, N-(1-Methylethyl)-N-propylaminocarbonylmethyl, N-Butyl-N-propylaminocarbonylmethyl, N-(1-Methylpropyl)-N-propylaminocarbonylmethyl, N-(2-Methylpropyl)-N-propylaminocarbonylmethyl, N-(1,1-Dimethylethyl)-N-propylaminocarbonylmethyl, N-Butyl-N-(1-methylethyl)-aminocarbonylmethyl, N-(1-Methylethyl)-N-(1-methylpropyl)-aminocarbonylmethyl, N-(1-Methylethyl)-N-(2-methylpropyl)-aminocarbonylmethyl, N-(1,1-Dimethylethyl)-N-(1-methylethyl)-aminocarbonylmethyl, N-Butyl-N-(1-methylpropyl) -aminocarbonylmethyl, N-Butyl-N-(2-methylpropyl)-aminocarbonylmethyl, N-Butyl-N-(1,1-di-methylethyl)-aminocarbonylmethyl, N-(1-Methylpropyl)-N-(2-methylpropyl)-aminocarbonylmethyl, N-(1,1-Dimethylethyl)-N-(1-methylpropyl)-aminocarbonylmethyl. N-(1,1-Dimethylethyl)-N-(2-methylpropyl)-aminocarbonylmethyl, N,N-Dimethylaminocarbonylethyl, N,N-Diethylaminocarbonylethyl, N,N-Dipropylaminocarbonylethyl, N,N-Di-(1-methylethyl)-aminocarbonylethyl, N,N-Dibutylaminocarbonylethyl, N,N-Di-(1-methylpropyl)-aminocarbonylethyl, N,N-Di-(2-methylpropyl)-aminocarbonylethyl, N,N-Di-(1,1-dimethylethyl)-aminocarbonylethyl, N-Ethyl-N-methylaminocarbonylethyl, N-Methyl-N-propylaminocarbonylethyl, N-Methyl-N-(1-methylethyl)-aminocarbonylethyl, N-Butyl-N-methylaminocarbonylethyl, N-Methyl-N-(1-methylpropyl)-aminocarbonylethyl, N-Methyl-N-(2-methylpropyl)-aminocarbonylethyl, N-(1,1-Dimethylethyl)-N-methylaminocarbonylethyl, N-Ethyl-N-propylaminocarbonylethyl, N-Ethyl-N-(1-methylethyl)-aminocarbonylethyl, N-Butyl-N-ethylaminocarbonylethyl, N-Ethyl-N-(1-methylpropyl)-aminocarbonylethyl, N-Ethyl-N-(2-methylpropyl)-aminocarbonylethyl, N-Ethyl-N-(1,1-dimethylethyl)-aminocarbonylethyl, N-(1-Methylethyl)-N-propylaminocarbonylethyl, N-Butyl-N-propylaminocarbonylethyl, N-(1-Methylpropyl)-N-propylaminocarbonylethyl, N-(2-Methylpropyl)-N-propylaminocarbonylethyl, N-(1,1-Dimethylethyl)-N-propylaminocarbonylethyl, N-Butyl-N-(1-methylethyl)-aminocarbonylethyl, N-(1-Methylethyl)-N-(1-methylpropyl)-aminocarbonylethyl, N-(1-Methylethyl)-N-(2-methylpropyl)-aminocarbonylethyl, N-(1,1-Dimethylethyl)-N-(1-methylethyl)-aminocarbonylethyl, N-Butyl-N-(1-methylpropyl)-aminocarbonylethyl, N-Butyl-N-(2-methylpropyl)-aminocarbonylethyl, N-Butyl-N-(1,1-dimethylethyl)-aminocarbonylethyl, N-(1-Methylpropyl)-N-(2-methylpropyl)-aminocarbonylethyl, N-(1,1-Dimethylethyl)-N-(1-methylpropyl)-aminocarbonylethyl, N-(1,1-Dimethylethyl)-N-(2-methylpropyl)-aminocarbonylethyl, N,N-Dimethylaminocarbonylpropyl, N,N-Diethylaminocarbonylpropyl, N,N-Dipropylaminocarbonylpropyl, N,N-Di-(1-methylethyl)-aminocarbonylpropyl, N,N-Dibutylaminocarbonylpropyl, N,N-Di-(1-methylpropyl)-aminocarbonylpropyl, N.N-Di-(2-methylpropyl)-aminocarbonylpropyl, N,N-Di-(1,1-dimethylethyl)-aminocarbonylpropyl, N-Ethyl-N-methylaminocarbonylpropyl, N-Methyl-N-propylaminocarbonylpropyl, N-Methyl-N-(1-methylethyl)-aminocarbonylpropyl, N-Butyl-N-methylaminocarbonylpropyl, N-Methyl-N-(1-methylpropyl)-aminocarbonylpropyl, N-Methyl-N-(2-methylpropyl)-aminocarbonylpropyl, N-(1,1-Dimethylethyl)-N-methylaminocarbonylpropyl, N-Ethyl-N-propylaminocarbonylpropyl, N-Ethyl-N-(1-methylethyl)-aminocarbonylpropyl, N-Butyl-N-ethylaminocarbonylpropyl, N-Ethyl-N-(1-methylpropyl)-aminocarbonylpropyl, N-Ethyl-N-(2-methylpropyl)-aminocarbonylpropyl, N-Ethyl-N-(1,1-dimethylethyl)-aminocarbonylpropyl, N-(1-Methylethyl)-N-propylaminocarbonylpropyl, N-Butyl-N-propylaminocarbonylpropyl, N-(1-Methylpropyl)-N-propylaminocarbonylpropyl, N-(2-Methylpropyl)-N-propylaminocarbonylpropyl, N-(1,1-Dimethylethyl)-N-propylaminocarbonylpropyl, N-Butyl-N-(1-methylethyl)-aminocarbonylpropyl, N-(1-Methylethyl)-N-(1-methylpropyl)-aminocarbonylpropyl, N-(1-Methylethyl)-N-(2-methylpropyl)-aminocarbonylpropyl, N-(1,1-Dimethylethyl)-N-(1-methylethyl)-aminocarbonylpropyl, N-Butyl-N-(1-methylpropyl)-aminocarbonylpropyl, N-Butyl-N-(2-methylpropyl)-aminocarbonylpropyl, N-Butyl-N-(1,1-dimethylethyl)-aminocarbonylpropyl, N-(1-Methylpropyl)-N-(2-methylpropyl)-aminocarbonylpropyl, N-(1,1-Dimethylethyl)-N-(1-methylpropyl)-aminocarbonylpropyl, N-(1,1-Dimethylethyl)-N-(2-methylpropyl)-aminocarbonylpropyl, N,N-Dimethylaminocarbonylbutyl, N,N-Diethylaminocarbonylbutyl, N,N-Dipropylaminocarbonylbutyl, N,N-Di-(1-methylethyl)-aminocarbonylbutyl, N,N-Dibutylaminocarbonylbutyl, N,N-Di-(1-methylpropyl)-aminocarbonylbutyl, N,N-Di-(2-methylpropyl)-aminocarbonylbutyl, N,N-Di-(1,1-dimethylethyl)-aminocarbonylbutyl, N-Ethyl-N-methylaminocarbonylbutyl, N-Methyl-N-propylaminocarbonylbutyl, N-Methyl-N-(1-methylethyl)-aminocarbonylbutyl, N-Butyl-N-methylaminocarbonylbutyl, N-Methyl-N-(1-methylpropyl)-aminocarbonylbutyl, N-Methyl-N-(2-methylpropyl)-aminocarbonylbutyl, N-(1,1-Dimethylethyl)-N-methylaminocarbonylbutyl, N-Ethyl-N-propylaminocarbonylbutyl, N-Ethyl-N-(1-methylethyl)-aminocarbonylbutyl, N-Butyl-N-ethylaminocarbonylbutyl, N-Ethyl-N-(1-methylpropyl)-aminocarbonylbutyl, N-Ethyl-N-(2-methylpropyl)-aminocarbonylbutyl, N-Ethyl-N-(1,1-dimethylethyl)-aminocarbonylbutyl, N-(1-Methylethyl)-N-propylaminocarbonylbutyl, N-Butyl-N-propylaminocarbonylbutyl, N-(1-Methylpropyl)-N-propylaminocarbonylbutyl, N-(2-Methylpropyl)-N-propylaminocarbonylbutyl, N-(1,1-Dimethylethyl)-N-propylaminocarbonylbutyl, N-Butyl-N-(1-methylethyl)-aminocarbonylbutyl, N-(1-Methylethyl)-N-(1-methylpropyl)-aminocarbonylbutyl, N-(1-Methylethyl)-N-(2-methylpropyl)-aminocarbonylbutyl, N-(1,1-Dimethylethyl)-N-(1-methylethyl)-aminocarbonylbutyl, N-Butyl-N-(1-methylpropyl)-aminocarbonylbutyl, N-Butyl-N-(2-methylpropyl)-aminocarbonylbutyl, N-Butyl-N-(1,1-dimethylethyl)-aminocarbonylbutyl, N-(1-Methylpropyl)-N-(2-methylpropyl)-aminocarbonylbutyl, N-(1,1-Dimethylethyl)-N-(1-methylpropyl)-aminocarbonylbutyl oder N-(1,1-Dimethylethyl)-N-(2-methylpropyl)-aminocarbonylbutyl;
- C₁-C₄-Alkoxy-C₁-C₄-alkoxy, sowie die Alkoxyalkoxyteile von C₁-C₄-Alkoxy-C₁-C₄-alkoxycarbonyl: durch C₁-C₄-Alkoxy, wie vorstehend genannt, substituiertes C₁-C₄-Alkoxy, also z.B. für Methoxymethoxy, Ethoxymethoxy, Propoxymethoxy, (1-Methylethoxy)methoxy, Butoxymethoxy, (1-Methylpropoxy)methoxy, (2-Methylpropoxy)methoxy, (1,1-Dimethylethoxy)methoxy, 2-(Methoxy)ethoxy, 2-(Ethoxy)ethoxy, 2-(Propoxy)ethoxy, 2-(1-Methylethoxy)ethoxy, 2-(Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(Propoxy)propoxy, 2-(1-Methylethoxy)propoxy, 2-(Butoxy)-propoxy, 2-(1-Methylpropoxy)propoxy, 2-(2-Methylpropoxy)propoxy, 2-(1,1-Dimethylethoxy)propoxy, 3-(Methoxy)-propoxy, 3-(Ethoxy)propoxy, 3-(Propoxy)propoxy, 3-(1-Methylethoxy)propoxy, 3-(Butoxy)propoxy, 3-(1-Methylpropoxy)-propoxy, 3-(2-Methylpropoxy)propoxy, 3-(1,1-Dimethylethoxy)propoxy, 2-(Methoxy)butoxy, 2-(Ethoxy)butoxy, 2-(Propoxy)butoxy, 2-(1-Methylethoxy)butoxy, 2-(Butoxy)-butoxy, 2-(1-Methylpropoxy)butoxy, 2-(2-Methylpropoxy)butoxy, 2-(1,1-Dimethylethoxy)butoxy, 3-(Methoxy)butoxy, 3-(Ethoxy) - butoxy, 3-(Propoxy)butoxy, 3-(1-Methylethoxy)butoxy, 3-(Butoxy)butoxy, 3-(1-Methylpropoxy)butoxy, 3-(2-Methylpropoxy)butoxy, 3-(1,1-Dimethylethoxy)butoxy, 4-(Methoxy) - butoxy, 4-(Ethoxy)butoxy, 4-(Propoxy)butoxy, 4-(1-Methylethoxy)butoxy, 4-(Butoxy)butoxy, 4-(1-Methylpropoxy)butoxy, 4-(2-Methylpropoxy)butoxy oder 4-(1,1-Dimethylethoxy)butoxy;
- C₃-C₆-Alkenyl : z.B. Prop-2-en-1-yl, But-1-en-4-yl, 1-Methyl-prop-2-en-1-yl, 2-Methyl-prop-2-en-1-yl, 2-Buten-1-yl, 1-Penten-3-yl, 1-Penten-4-yl, 2-Penten-4-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-2-en-1-yl, 1-Ethyl-prop-2-en-l-yl, Hex-3-en-1-yl, Hex-4-en-1-yl, Hex-5-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-l-yl, 1,2-Dimethyl-but-2-en-1-yl, 1,2-Dimethyl-but-3-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-2-en-1-yl, 2,3-Dimethyl-but-3-en-1-yl, 3,3-Dimethyl-but-2-en-1-yl, 1-Ethyl-but-2-en-1-yl, 1-Ethyl-but-3-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl oder 1-Ethyl-2-methyl-prop-2-en-1-yl; C₂-C₆-Alkenyl, sowie die Alkenylteile von C₂-C₆-Alkenylcarbonyl, Phenyl-C₂-C₆-alkenylcarbonyl : C₃-C₆-Alkenyl, wie voranstehend genannt, sowie Ethenyl;
- C₃-C₆-Halogenalkenyl: einen C₃-C₆-Alkenylrest, wie vorstehend genannt, der partiell oder vollständig durch Fluor, Chlor, Brom und/oder Iod substituiert ist, also z.B. 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromallyl, 3-Bromallyl, 2,3-Dibromallyl, 3,3-Dibromallyl, 2,3,3-Tribromallyl oder 2,3-Dibrombut-2-enyl;
- C₃-C₆-Alkinyl : z.B. Propargyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl;
- C₃-C₆-Cycloalkyl : z.B. Cyclopropyl, Cyclobutyl, Cyclopentyl oder Cyclohexyl;
- C₃-C₆-Spirocycloalkan (C₃-C₆-Cycloalkylrest, wobei ein Ringglied - das sogenannte Spiroatom - sowohl zum C₃-C₆-Cycloalkylrest als auch zu dem Rest, mit dem der cyclische Rest verbunden ist, gehörig ist): z.B. Spirocyclopropyl, Spirocyclobutyl, Spirocyclopentyl oder Spirocyclohexyl;
- Heterocyclyl : ein gesättigter, partiell gesättigter oder ungesättiger 5-gliedriger heterocyclischer Ring, der ein bis vier gleiche oder verschiedene Heteroatome, ausgewählt aus folgender Gruppe: Sauerstoff, Schwefel oder Stickstoff, enthält, und über C gebunden ist. also z.B. C-gebundene 5-gliedrige, gesättigte Ringe wie:
   Tetrahydropyrazol-3-yl, Tetrahydropyrazol-4-yl, Tetra-hydroisoxazol-3-yl, Tetrahydroisoxazol-4-yl, Tetrahydroisoxazol-5-yl, 1,2-Oxathiolan-3-yl, 1,2-Oxathiolan-4-yl, 1,2-Oxathiolan-5-yl, Tetrahydroisothiazol-3-yl, Tetrahydroisothiazol-4-yl, Tetrahydroisothiazol-5-yl, 1,2-Dithiolan-3-yl, 1,2-Dithiolan-4-yl, Tetrahydroimidazol-2-yl, Tetrahydroimidazol-4-yl, Tetrahydrooxazol-2-yl, Tetrahydrooxazol-4-yl, Tetrahydrooxazol-5-yl, Tetrahydrothiazol-2-yl, Tetrahydrothiazol-4-yl, Tetrahydrothiazol-5-yl, 1,3-Dioxolan-2-yl, 1,3-Dioxolan-4-yl, 1,3-Oxathiolan-2-yl, 1,3-Oxathiolan-4-yl, 1,3-Oxathiolan-5-yl, 1,3-Dithiolan-2-yl, 1,3-Dithiolan-4-yl;
   C-gebundene, 5-gliedrige, partiell gesättigte Ringe wie:
   4,5-Dihydro-1H-pyrazol-3-yl, 4,5-Dihydro-1H-pyrazol-4-yl, 4,5-Dihydro-1H-pyrazol-5-yl, 2,5-Dihydro-1H-pyrazol-3-yl, 2,5-Dihydro-1H-pyrazol-4-yl, 2,5-Dihydro-1H-pyrazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroisoxazol-5-yl, 2,3-Dihydroisoxazol-3-yl, 2,3-Dihydroisoxazol-4-yl, 2,3-Dihydroisoxazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydroisothiazol-3-yl, 2,3-Dihydroisothiazol-4-yl, 2,3-Dihydroisothiazol-5-yl, Δ³-1,2-Dithiol-3-yl, Δ³-1,2-Dithiol-4-yl, Δ³-1,2-Dithiol-5-yl, 4,5-Dihydro-1H-imidazol-2-yl, 4,5-Dihydro-1H-imidazol-4-yl, 4,5-Dihydro-1H-imidazol-5-yl, 2,5-Dihydro-1H-imidazol-2-yl, 2,5-Dihydro-1H-imidazol-4-yl, 2,5-Dihydro-1H-imidazol-5-yl, 2,3-Dihydro-1H-imidazol-2-yl, 2,3-Dihydro-1H-imidazol-4-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrooxazol-2-yl, 2,3-Dihydrooxazol-4-yl, 2,3-Dihydrooxazol-5-yl, 4,5-Dihydrothiazol-2-yl, 4.5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydrothiazol-2-yl, 2,3-Dihydrothiazol-4-yl, 2,3-Dihydrothiazol-5-yl, 1,3-Dioxol-2-yl, 1,3-Dioxol-4-yl, 1,3-Dithiol-2-yl, 1.3-Dithiol-4-yl, 1,3-Oxathiol-2-yl, 1,3-Oxathiol-4-yl, 1,3-Oxathiol-5-yl;
   C-gebundene, 5-gliedrige, ungesättigte Ringe wie:
   Pyrazol-3-yl, Pyrazol-4-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, Oxazol-2-yl, Oxazol-4-yl, Oxazol-5-yl, Thiazol-2-yl, Thiazol-4-yl, Thiazol-5-yl;
wobei ggf. der Schwefel der genannten Heterocyclen zu S=O oder S(=O)₂ oxidiert sein kann.

Alle Phenylringe bzw. alle Phenylkomponenten in Phenyl-C₁-C₆-alkyl, Phenylcarbonyl-C₁-C₆-alkyl, Phenylcarbonyl, Phenylalkenylcarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl; besonders bevorzugt Nitro, Halogen, wie Chlor oder Brom, C₁-C₆-Alkyl, wie Methyl oder Ethyl, C₁-C₆-Alkoxy, wie Methoxy oder Ethoxy, C₁-C₆-Halogenalkyl, wie Tri-fluormethyl, C₁-C₆-Alkylsulfonyl, wie Methylsulfonyl oder Ethylsulfonyl, oder C₁-C₆-Halogenalkylsulfonyl, wie Trifluormethylsulfonyl;
- R₂: gegebenenfalls substituierter 5-gliedriger, C-verknüpfter Heterocyclyl-Rest, enthaltend zwei gleiche oder verschiedene Heteroatome, ausgewählt aus folgender Gruppe: Sauerstoff, Schwefel oder Stickstoff; besonders bevorzugt 5-gliedriger, C-verknüpfter Heterocyclyl-Rest, enthaltend zwei gleiche oder verschiedene Heteroatome, ausgewählt aus folgender Gruppe: Sauerstoff, Schwefel oder Stickstoff, wobei der Heterocyclyl-Rest unsubstituiert ist oder einen oder zwei Substituenten aus folgender Gruppe trägt:
- C₁-C₄-Alkyl wie Methyl, Ethyl, Propyl, 1-Methylethyl oder 2-Methylpropyl, C₁-C₄-Alkoxy-C₁-C₄-alkyl wie 2-Methoxyethyl oder 2-Ethoxyethyl, C₁-C₄-Halogenalkyl wie z.B. Chlormethyl, Brommethyl, Fluormethyl, Difluormethyl oder Trifluormethyl, C₃-C₆-Cycloalkyl wie Cyclopropyl, Cyclopentyl oder Cyclohexyl, C₁-C₄-Alkoxy wie Methoxy oder Ethoxy, C₁-C₄-Alkoxycarbonyl wie Methoxycarbonyl, Ethoxycarbonyl, Propoxycarbonyl oder 1-Methylethoxycarbonyl,
- C₃-C₆-Spiro-cycloalkan, wie Spiro-cyclopentan, Spiro-cyclohexan.
- R³: Nitro, Halogen, Cyano, C₁-C₆-Alkyl, C₁-C₆-Halogenalkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
besonders bevorzugt Nitro, Halogen, wie Chlor oder Brom, C₁-C₆-Halogenalkyl, wie Trifluormethyl, C₁-C₆-Alkylsulfonyl, wie Methylsulfonyl, Ethylsulfonyl oder Propylsulfonyl, oder C₁-C₆-Halogenalkylsulfonyl, wie Trifluormethylsulfonyl;
- _{R}4: Wasserstoff;
- R⁶: C₁-C₆-Alkyl;
besonders bevorzugt C₁-C₄-Alkyl; insbesondere bevorzugt Methyl, Ethyl, Propyl, 2-Methyl-prop-1-yl oder Butyl;
- _{R}7: C₁-C₆-Alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, Di-(C₁-C₆-alkyl)-aminocarbonyl, N-(C₁-C₆-Alkoxy)-N-(C₁-C₆-alkyl)-aminocarbonyl oder Di-(C₁-C₆-alkyl)-aminothiocarbonyl, wobei die genannten Alkyl- oder Alkoxyreste partiell oder vollständig halogeniert sein können und/oder eine bis drei der folgenden Gruppen tragen können: Cyano, C₁-C₄-Alkoxy, C₁-C₄-Alkoxycarbonyl, C₁-C₄-Alkoxy-C₁-C₄-alkoxycarbonyl, Di-(C₁-C₄-alkyl)amino-C₁-C₄-alkoxycarbonyl, C₁-C₄-Alkylaminocarbonyl, Di-(C₁-C₄-alkyl)-aminocarbonyl oder C₁-C₄-Alkylcarbonyloxy;
Phenyl-C₁-C₆-alkyl, Phenylcarbonyl-C₁-C₆-alkyl, Phenyl-C₂-C₆-alkenylcarbonyl oder Phenylcarbonyl, wobei der Phenylring der 4 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenoxy;
insbesondere bevorzugt C₁-C₆-Alkyl, C₁-C₆-Cyanoalkyl, C₁-C₄-Alkoxy-C₁-C₆-alkyl, C₁-C₄-Alkylcarbonyloxy-C₁-C₆-C₁-C₄-Alkoxycarbonyl-C₁-C₆-alkyl, C₁-C₄-Alkoxy-C₁-C₄-alkoxycarbonyl-C₁-C₆-alkyl, Di-(C₁-C₄-alkyl)-amino-C₁-C₄-alkoxycarbonyl-C₁-C₆-alkyl, C₁-C₄-Alkyl-aminocarbonyl-C₁-C₆-alkyl, Di-(C₁-C₄-alkyl)-amino-carbonyl-C₁-C₆-alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothiocarbonyl, Phenyl-C₁-C₆-alkyl, Phenylcarbonyl-C₁-C₆-alkyl, Phenyl-C₂-C₆-alkenyl-carbonyl oder Phenylcarbonyl, wobei der Phenylring der 4 letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy oder C₁-C₄-Halogenalkoxy;
- R^{B}: Wasserstoff oder C₁-C₆-Alkyl;
insbesondere Wasserstoff oder Methyl.

Insbesondere bevorzugt sind Verbindungen der Formel I, wobei
- R²: gegebenenfalls substituiertes Tetrahydrooxazol-2-yl, Tetrahydrothiazol-2-yl, 1,3-Dioxolan-2-yl, 1,3-Oxathiolan-2-yl, 1,3-Dithiolan-2-yl, 4,5-Dihydroisoxazol-3-yl, 4,5-Dihydro-1H-imidazol-2-yl, 4,5-Dihydrooxazol-2-yl, 4,5-Dihydro-thiazol-2-yl, Pyrazol-3-yl, Pyrazol-4-yl, Isoxazol-3-yl, Isoxazol-5-yl, Isothiazol-3-yl, Oxazol-2-yl, oxazol-5-yl, Thiazol-2-yl, Thiazol-4-yl, Thiazol-5-yl,
bedeutet.

Außerordentlich bevorzugt sind Verbindungen der Formel I, wobei
- _{R}²: gegebenenfalls substituiertes 1,3-Dioxolan-2-yl, 1,3-Dithiolan-2-yl, 4,5-Dihydrothiazol-2-yl, Isoxazol-3-yl, Oxazol-2-yl, Thiazol-2-yl, 4,5-Dihydroisoxazol-3-yl, 4,5-Dihydrooxazol-2-yl, insbesondere bevorzugt gegebenenfalls substituiertes Thiazol-2-yl, 1,3-Dithiolan-2-yl, 4,5-Dihydrothiazol-2-yl, 4,5-Dihydroisoxazol-3-yl oder Isoxazol-3-yl;
bedeutet.

Ebenso insbesondere bevorzugt sind die Verbindungen der Formel I, wobei
- _{R}2: gegebenenfalls substituiertes Tetrahydrooxazol-2-yl, Tetrahydrothiazol-2-yl, 1,3-Dioxolan-2-yl, 1,3-Oxathiolan-2-yl, 1,3-Dithiolan-2-yl, 4,5-Dihydroisoxazol-3-yl, 4,5-Dihydro-1H-imidazol-2-yl, 4,5-Dihydrooxazol-2-yl, 4,5-Dihydrothiazol-2-yl;
bedeutet.

Außerordentlich bevorzugt sind die Verbindungen der Formel I, wobei
- R²: gegebenenfalls substituiertes 1,3-Dioxolan-2-yl, 1,3-Dithiolan-2-yl, 4,5-Dihydrothiazol-2-yl, 4,5-Dihydroisoxazol-3-yl oder 4,5-Dihydrooxazol-2-yl; insbesondere bevorzugt gegebenenfalls substituiertes 1,3-Dithiolan-2-yl, 4,5-Dihydrothiazol-2-yl oder 4,5-Dihydroisoxazol-3-yl; außerordentlich bevorzugt gegebenenfalls substituiertes 4,5-Dihydroisoxazol-3-yl;
bedeutet.

Ebenso insbesondere bevorzugt sind die Verbindungen der Formel I, wobei
- R²: gegebenenfalls substituiertes Pyrazol-3-yl, Pyrazol-4-yl, Isoxazol-3-yl, Isoxazol-4-yl, Isothiazol-3-yl, Oxazol-2-yl, Oxazol-5-yl, Thiazol-2-yl, Thiazol-4-yl, Thiazol-5-yl;
- R⁷: C₁-C₆-Alkyl, C₃-C₆-Alkenyl, C₃-C₆-Alkinyl, C₁-C₆-Alkyl-carbonyl, C₂-C₆-Alkenylcarbonyl, C₁-C₆-Alkoxycarbonyl, Di-(C₁-C₆-alkyl)-aminocarbonyl, N-(C₁-C₆-Alkoxy)-N-(C₁-C₆-alkyl)-aminocarbonyl, Di-(C₁-C₆-alkyl)-aminothiocarbonyl, wobei die genannten Alkyl- oder Alkoxyreste partiell oder vollständig halogeniert sein können und/oder eine bis drei der folgenden Gruppen tragen können:
Cyano, C₁-C₄-Alkoxy,
C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, C₁-C₄-Alkylaminocarbonyl, Di-(C₁-C₄-alkyl)-aminocarbonyl, C₁-C₄-Alkylcarbonyloxy ;
bedeutet.

Außerordentlich bevorzugt sind hierbei die Verbindungen der Formel I, wobei
- R²: gegebenenfalls substituiertes Isoxazol-3-yl, Oxazol-2-yl oder Thiazol-2-yl; insbesondere bevorzugt gegebenenfalls substituiertes Isoxazol-3-yl oder Thiazol-2-yl;
bedeutet.

Insbesondere außerordentlich bevorzugt sind die Verbindungen Ia1 ( 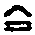 I mit R¹ = Cl, R³ = SO₂CH₃, R⁶, R⁷ = CH₃ und R⁸ = H), insbesondere die Verbindungen der Tabelle 1.

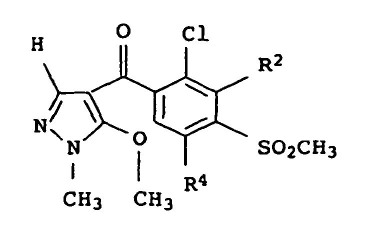

**Tabelle 1**

| Nr. | R² | R⁴ |
|---|---|---|
| | | |
| | | |
| | | |
| | | |
| Ia.5 | 3-Methyl-isoxazol-5-yl | H |
| Ia.6 | 5-Thiazolyl | H |
| Ia.7 | 4-Thiazolyl | H |
| Ia.8 | 2-Thiazolyl | H |
| Ia.9 | 3-Methyl-isothiazol-5-yl | H |
| Ia.10 | 3-Isoxazolyl | H |
| | | |
| | | |
| | | |
| | | |
| | | |
| | | |
| | | |
| | | |
| | | |
| Ia.20 | 2-Oxazolyl | H |
| | | |
| Ia.22 | 1-Methyl-pyrazol-3-yl | H |
| Ia.23 | 1-Methyl-pyrazol-5-yl | H |
| Ia.24 | 1,5-Dimethyl- pyrazol-3-yl | H |
| | | |
| Ia.26 | 1,4-Dimethyl- pyrazol-5-yl | H |
| Ia.27 | 1,3-Dimethyl- pyrazol-4-yl | H |
| Ia.28 | 1,5-Dimethyl- pyrazol-4-yl | H |
| Ia.29 | 1-Methyl-pyrazol-4-yl | H |
| Ia.30 | 1,3-Dimethyl- pyrazol-5-yl | H |
| Ia.31 | 4-Methyl-oxazol-2-yl | H |
| | | |
| Ia.33 | 4-Methoxy-1-methyl- pyrazol-5-yl | H |
| Ia.34 | 3-Cyclopropyl- isoxazol-5-yl | H |
| Ia.35 | 5-Methyl- isoxazol-3-yl | H |
| | | |
| Ia.37 | 5-Methyl-thiazol-2-yl | H |
| | | |
| | | |
| | | |
| Ia.41 | 4-Methyl-thiazol-2-yl | H |
| | | |
| Ia.43 | 4,5-Dimethyl- thiazol-2-yl | H |
| | | |
| Ia.45 | 2-Methoxy-thiazol-5-yl | H |
| | | |
| | | |
| | | |
| | | |
| | | |
| | | |
| | | |
| | | |
| | | |
| | | |
| | | |
| Ia.57 | 3-Ethoxycarbonyl-1- methyl-pyrazol-5-yl | H |
| | | |
| | | |
| | | |
| | | |
| | | |
| Ia.63 | 4,5-Dihydro-thiazol-2-yl | H |
| Ia.64 | 5-Methyl-oxazol-2-yl | H |
| | | |
| Ia.66 | 2-Methyl-oxazol-5-yl | H |
| | | H |
| | | H |
| | | H |
| | | H |
| | | H |
| | | H |
| | | H |
| | | H |
| | | H |
| | | H |
| Ia.77 | 1,3-Dithiolan-2-yl | H |
| Ia.78 | 1,3-Dioxolan-2-yl | H |
| | | |
| | | |
| | | |
| | | |
| Ia.83 | 1,3-Oxathiolan-2-yl | H |
| | | |
| | | |
| Ia.91 | 5,5 -Dimethyl-4,5-dihydro-isoxazol-3-yl | H |
| Ia.92 | 4,5-Dihydroisoxazol-3-yl | H |
| Ia.93 | 5-Ethyl-4,5-dibydroisoxazol-3-yl | H |
| Ia.94 | 5,5-Diethyl-4,5-dihydro-isoxazol-3-yl | H |
| Ia.95 | (4,5-Dibydro-isoxazol-5-spiro-cyclopentan)-3-yl | H |
| Ia.96 | 4,5-Dihydro-oxazol-2-yl | H |
| Ia.97 | 5-Methyl-4,5-dihydro-oxazol-2-yl | H |
| Ia.98 | 4,4-Dimethyl-4,5-dihydro-oxazol-2-yl | H |
| Ia.99 | 5-Ethyl-4,5-dihydro-oxazol-2-yl | H |
| Ia.100 | 5,5-Dimethyl-4,5-dihydro-oxazol-2-yl | H |
| Ia.101 | 4,5-Dirnethyl-4,5-dihydro-oxazol-2-yl | H |
| Ia.102 | 4,5-Diethyl-4,5-dihydro-oxazol-2-yl | H |
| | | |
| | | |
| Ia.108 | 5-Methyl-4,5-dihydro-thiazol-2-yl | H |
| Ia.109 | 5-Ethyl-4,5-dihydro-thiazol-2-yl | H |
| Ia.110 | 4,4-Dimethyl-4,5-dihydro-thiazol-2-yl | H |
| Ia.111 | 5,5-Dimethyl-4,5-dihydro-thiazol-2-yl | H |
| Ia.112 | 4,5-Dimethyl-4,5-dihydro-thiazol-2-yl | H |
| Ia.113 | 5-Methyl-4,5-dihydro-imidazol-2-yl | H |
| Ia.114 | 5-Ethyl-4,5-dihydro-imidazol-2-yl | H |
| Ia.115 | 4,5-Dibydro-inüdazol-2-yl | H |
| Ia.116 | 5,5-Dimethyl-4,5-dihydro-imidazol-2-yl | H |
| Ia.117 | 4,4-Dimethyl-4,5-dihydro-imidazol-2-yl | H |
| Ia.118 | 4,5-Dimethyl-4,5-dihydro-imidazol-2-yl | H |
| Ia.119 | 1,5-Dimethyl-4,5-dihydro-imidazol-2-yl | H |
| Ia.120 | 1-Methyl-5-ethyl-4,5-dihydro-imidazol-2-yl | H |
| Ia.121 | 1-Methyl-4,5-dihydro-imidazol-2-yl | H |
| | | |
| | | |
| | | |
| Ia.138 | 5-Methoxycarbonyl-4,5-dihydro-isoxazol-3-yl | H |
| Ia.139 | 5-Ethoxycarbooyl-4,5-dihydro-isoxazol-3-yl | H |
| | | |
| Ia.143 | 5-Methyl-4,5-dihydro-isoxazol-3-yl | H |
| Ia.144 | 5-Isopropyl-4,5-dihydro-isoxazol-3-yl | H |
| Ia.145 | 5,5-Dimethyl-4,5-dihydro-isoxazol-3-yl | H |
| Ia.146 | (4,5-Dihydro-isoxazol-5-spiro-4-cyclopentan)-3-yl | H |
| Ia.147 | 4,5-Dimethyl-4,5-dibydroisoxazol-3-yl | H |

- Ebenso insbesondere außerordentlich bevorzugt sind die Verbindungen Ia2; insbesondere die Verbindungen Ia2.1-Ia2.164, die sich von den Verbindungen Ia1.1-Ia1.164 dadurch unterscheiden, daß R⁶ für Ethyl steht: 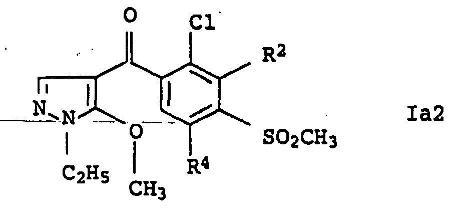
- Ebenso insbesondere außerordentlich bevorzugt sind die Verbindungen Ia3; insbesondere die Verbindungen Ia3.1-Ia3.164, die sich von den Verbindungen Ia1.1-Ia1.164 dadurch unterscheiden, daß R⁶ für n-Propyl steht: 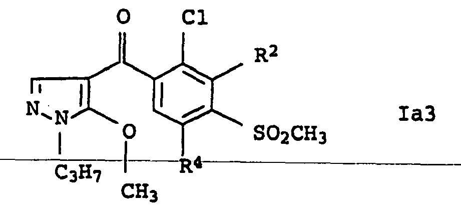
- Ebenso insbesondere außerordentlich bevorzugt sind die Verbindungen Ia4; insbesondere die Verbindungen Ia4.1-Ia4.164, die sich von den Verbindungen Ia1.1-Ia1.164 dadurch unterscheiden, daß R⁶ für iso-Butyl steht: 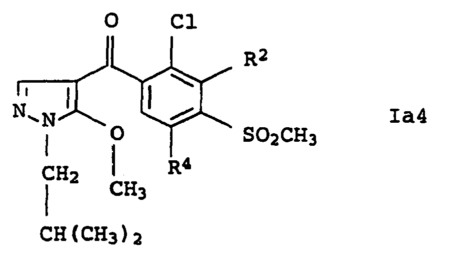
- Ebenso insbesondere außerordentlich bevorzugt sind die Verbindungen Ia5; insbesondere die Verbindungen Ia5.1-Ia5.164, die sich von den Verbindungen Ia1.1-Ia1.164 dadurch unterscheiden, daß R⁸ für Methyl steht: 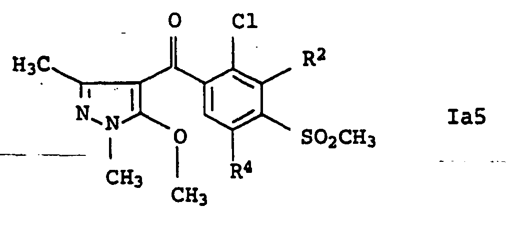
- Ebenso insbesondere außerordentlich bevorzugt sind die Verbindungen Ia.6; insbesondere die Verbindungen Ia6.1-Ia6.164, die sich von den Verbindungen Ia1.1-Ia1.164 dadurch unterscheiden, daß R⁷ für Ethyl steht: 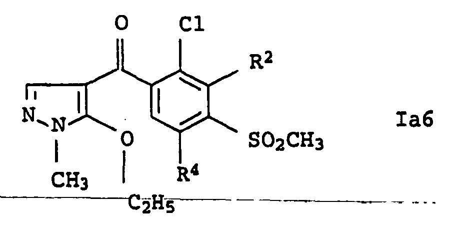
- Ebenso insbesondere außerordentlich bevorzugt sind die Verbindungen Ia7; insbesondere die Verbindungen Ia7.1-Ia7.164, die sich von den Verbindungen Ia1.1-Ia1.164 dadurch unterscheiden, daß R⁶ und R⁷ für Ethyl stehen: 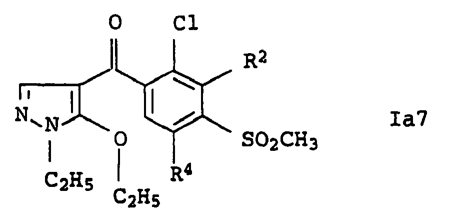
- Ebenso insbesondere außerordentlich bevorzugt sind die Verbindungen Ia8; insbesondere die Verbindungen Ia8.1-Ia8.164, die sich von den Verbindungen Ia1.1-Ia1.164 dadurch unterscheiden, daß R⁶ für n-Propyl und R⁷ für Ethyl stehen: 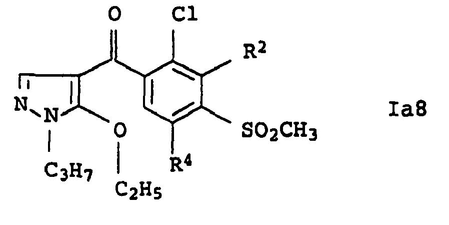
- Ebenso insbesondere außerordentlich bevorzugt sind die Verbindungen Ia9; insbesondere die Verbindungen Ia9.1-Ia9.164, die sich von den Verbindungen Ia1.1-Ia1.164 dadurch unterscheiden, daß R⁶ für iso-Butyl und R⁷ für Ethyl stehen: 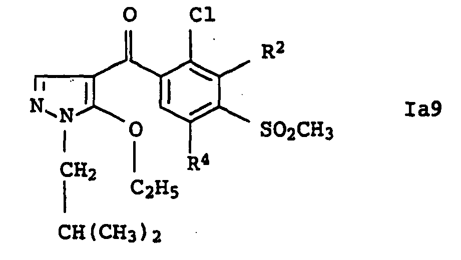
- Ebenso insbesondere außerordentlich bevorzugt sind die Verbindungen Ia10; insbesondere die Verbindungen Ia10.1-Ia10.164, die sich von den Verbindungen Ia1.1-Ia1.164 dadurch unterscheiden, daß R⁷ für Ethyl und R⁸ für Methyl stehen: 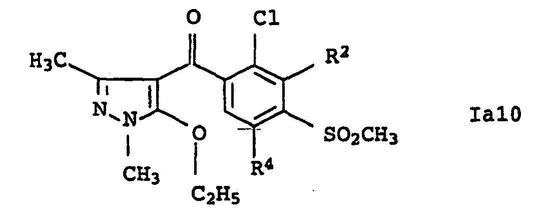
- Ebenso insbesondere außerordentlich bevorzugt sind die Verbindungen Ia11; insbesondere die Verbindungen Ia11.1-Ia11.164, die sich von den Verbindungen Ia1.1-Ia1.164 dadurch unterscheiden, daß R⁷ für Cyanomethyl steht: 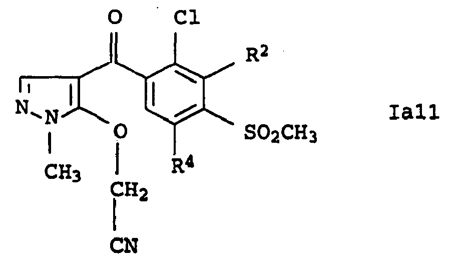
- Ebenso insbesondere außerordentlich bevorzugt sind die Verbindungen Ia12; insbesondere die Verbindungen Ia12.1-Ia12.164, die sich von den Verbindungen Ia1.1-Ia1.164 dadurch unterscheiden, daß R⁶ für Ethyl und R⁷ für Cyanomethyl stehen: 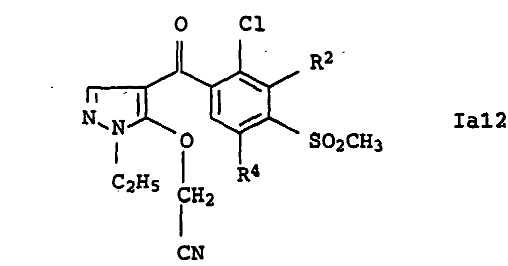
- Ebenso insbesondere außerordentlich bevorzugt sind die Verbindungen Ia13; insbesondere die Verbindungen Ia13.1-Ia13.164, die sich von den Verbindungen Ia1.1-Ia1.164 dadurch unterscheiden, daß R⁷ für Cyanomethyl und R⁸ für Methyl stehen: 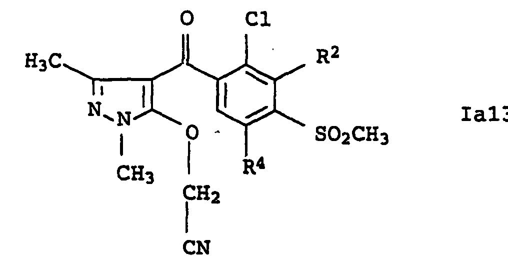
- Ebenso insbesondere außerordentlich bevorzugt sind die Verbindungen Ia14; insbesondere die Verbindungen Ia14.1-Ia14.164, die sich von den Verbindungen Ia1.1-Ia1.164 dadurch unterscheiden, daß R⁷ für Methoxymethyl steht: 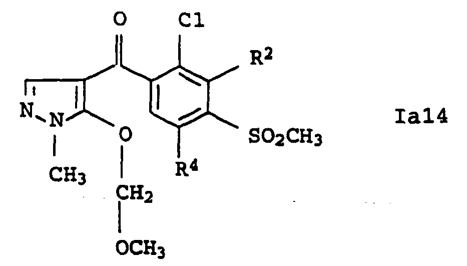
- Ebenso insbesondere außerordentlich bevorzugt sind die Verbindungen Ia15; insbesondere die Verbindungen Ia15.1-Ia15.164, die sich von den Verbindungen Ia1.1-Ia1.164 dadurch unterscheiden, daß R⁶ für Ethyl und R⁷ für Methoxymethyl stehen: 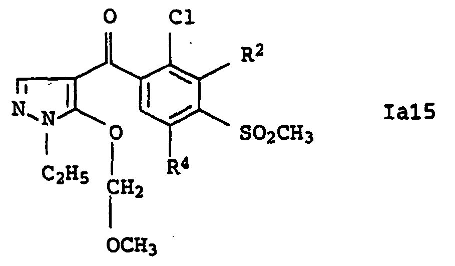
- Ebenso insbesondere außerordentlich bevorzugt sind die Verbindungen Ia16; insbesondere die Verbindungen Ia16.1-Ia16.164, die sich von den Verbindungen Ia1.1-Ia1.164 dadurch unterscheiden, daß R⁷ für Methoxymethyl und R⁸ für Methyl stehen: 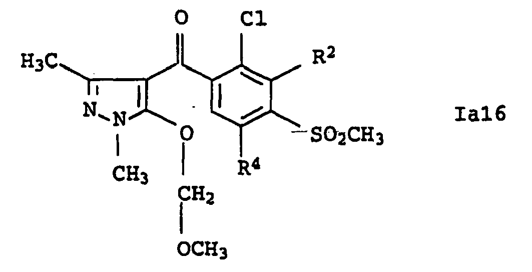
- Ebenso insbesondere außerordentlich bevorzugt sind die Verbindungen Ia17; insbesondere die Verbindungen Ia17.1-Ia17.164, die sich von den Verbindungen Ia1.1-Ia1.164 dadurch unterscheiden, daß R⁷ für Methylcarbonyloxymethyl steht: 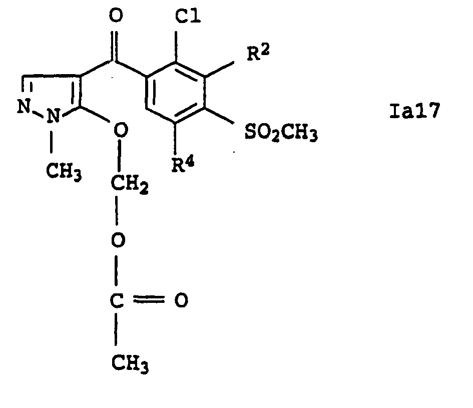
- Ebenso insbesondere außerordentlich bevorzugt sind die Verbindungen Ia18; insbesondere die Verbindungen Ia18.1-Ia18.164, die sich von den Verbindungen Ia1.1-Ia1.164 dadurch unterscheiden, daß R⁶ für Ethyl und R⁷ für Methylcarbonyloxymethyl stehen: 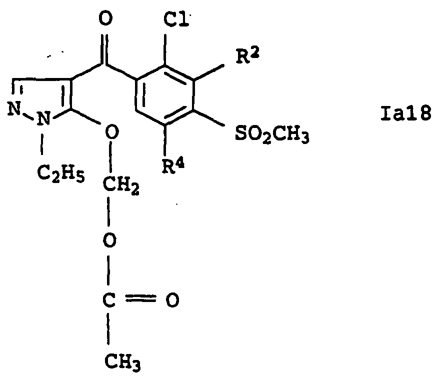
- Ebenso insbesondere außerordentlich bevorzugt sind die Verbindungen Ia19; insbesondere die Verbindungen Ia19.1-Ia19.164, die sich von den Verbindungen Ia1.1-Ia1.164 dadurch unterscheiden, daß R⁷ für Methylcarbonyloxymethyl und R⁸ für Methyl stehen: 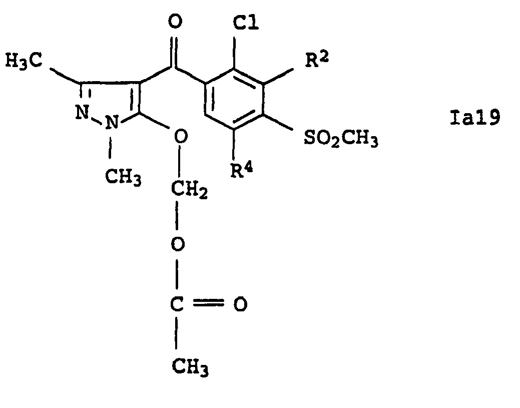
- Ebenso insbesondere außerordentlich bevorzugt sind die Verbindungen Ia20; insbesondere die Verbindungen Ia20.1-Ia20.164, die sich von den Verbindungen Ia1.1-Ia1.164 dadurch unterscheiden, daß R⁷ für tert.-Butylcarbonyloxymethyl steht: 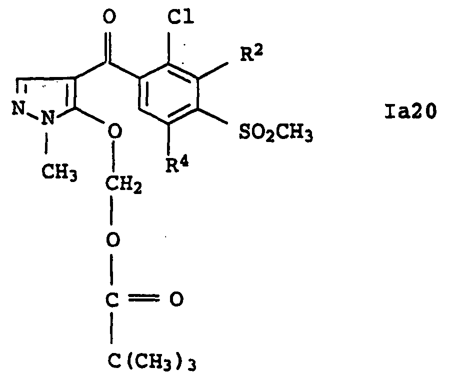
- Ebenso insbesondere außerordentlich bevorzugt sind die Verbindungen Ia21; insbesondere die Verbindungen Ia21.1-Ia21.164, die sich von den Verbindungen Ia1.1-Ia1.164 dadurch unterscheiden, daß R⁶ für Ethyl und R⁷ für tert.-Butylcarbonyloxymethyl stehen: 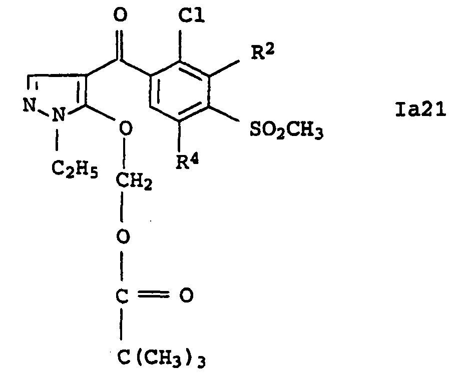
- Ebenso insbesondere außerordentlich bevorzugt sind die Verbindungen Ia22; insbesondere die Verbindungen Ia22.1-Ia22.164, die sich von den Verbindungen Ia1.1-Ia1.164 dadurch unterscheiden, daß R⁷ für tert.-Butylcarbonyloxymethyl und R⁸ für Methyl stehen: 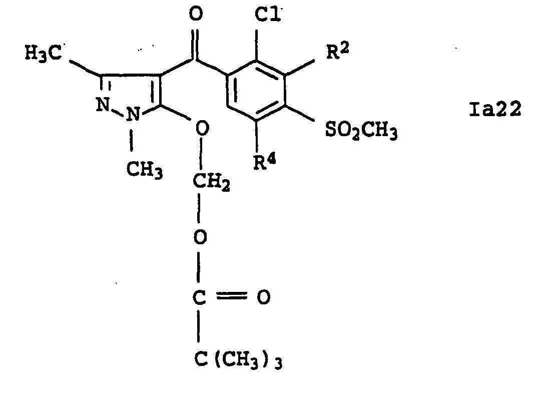
- Ebenso insbesondere außerordentlich bevorzugt sind die Verbindungen Ia23; insbesondere die Verbindungen Ia23.1-Ia23.164, die sich von den Verbindungen Ia1.1-Ia1.164 dadurch unterscheiden, daß R⁷ für Methoxycarbonylmethyl steht : 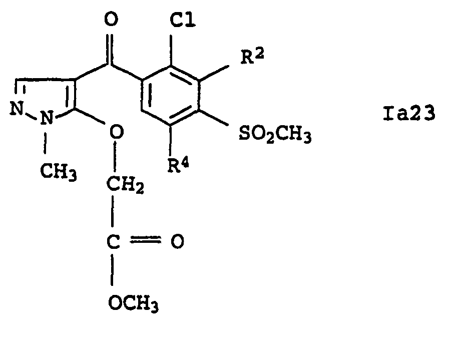
- Ebenso insbesondere außerordentlich bevorzugt sind die verbindungen Ia24; insbesondere die Verbindungen Ia24.1-Ia24.164, die sich von den Verbindungen Ia1.1-Ia1.164 dadurch unterscheiden, daß R⁶ für Ethyl und R⁷ für Methoxycarbonylmethyl stehen: 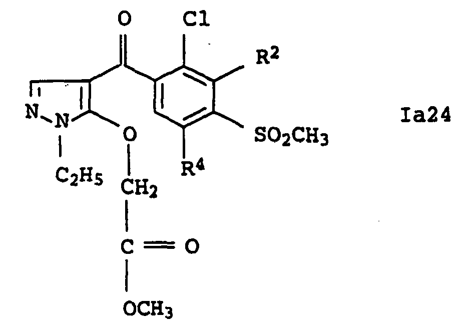
- Ebenso insbesondere außerordentlich bevorzugt sind die Verbindungen Ia25; insbesondere die Verbindungen Ia25.1-Ia25.164, die sich von den Verbindungen Ia1.1-Ia1.164 dadurch unterscheiden, daß R⁷ für Methoxycarbonylmethyl und R⁸ für Methyl stehen: 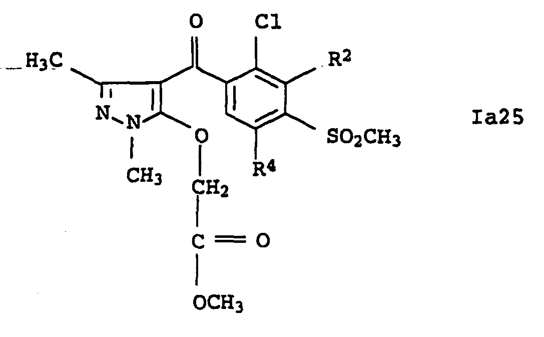
- Ebenso insbesondere außerordentlich bevorzugt sind die Verbindungen Ia26; insbesondere die Verbindungen Ia26.1-Ia26.164, die sich von den Verbindungen Ia1.1-Ia1.164 dadurch unterscheiden, daß R⁷ für Ethoxycarbonylmethyl steht: 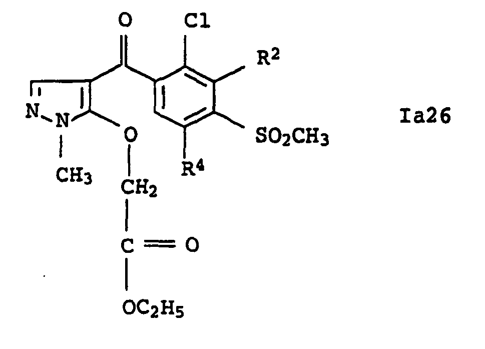
- Ebenso insbesondere außerordentlich bevorzugt sind die Verbindungen Ia27; insbesondere die Verbindungen Ia27.1-Ia27.164, die sich von den Verbindungen Ia1.1-Ia1.164 dadurch unterscheiden, daß R⁶ für Ethyl und R⁷ für Ethoxycarbonylmethyl stehen: 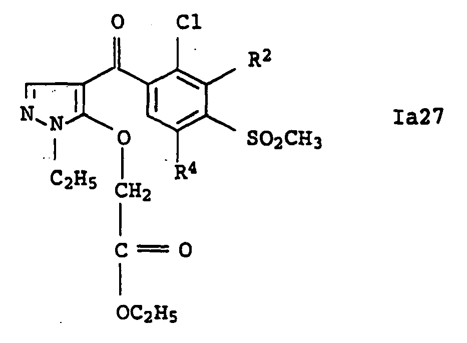
- Ebenso insbesondere außerordentlich bevorzugt sind die Verbindungen Ia28; insbesondere die Verbindungen Ia28.1-Ia28.164, die sich von den Verbindungen Ia1.1-Ia1.164 dadurch unterscheiden, daß R⁷ für Ethoxycarbonylmethyl und R⁸ für Methyl stehen: 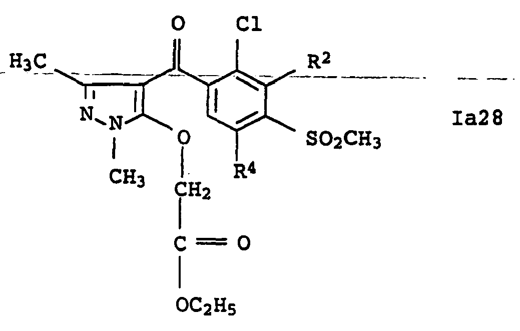
- Ebenso insbesondere außerordentlich bevorzugt sind die Verbindungen Ia29; insbesondere die Verbindungen Ia29.1-Ia29.164, die sich von den Verbindungen Ia1.1-Ia1.164 dadurch unterscheiden, daß R⁷ für 1-Methoxycarbonyl-eth-1-yl steht: 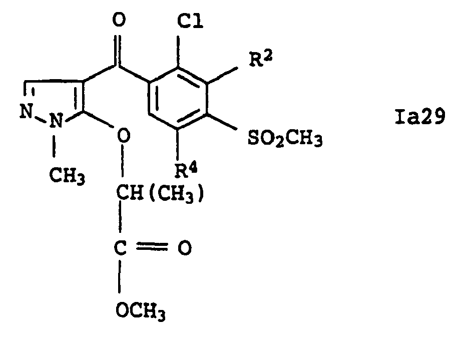
- Ebenso insbesondere außerordentlich bevorzugt sind die Verbindungen Ia30; insbesondere die Verbindungen Ia30.1-Ia30.164, die sich von den Verbindungen Ia1.1-Ia1.164 dadurch unterscheiden, daß R⁶ für Ethyl und R⁷ für 1-Methoxycarbonyl-eth-1-yl stehen: 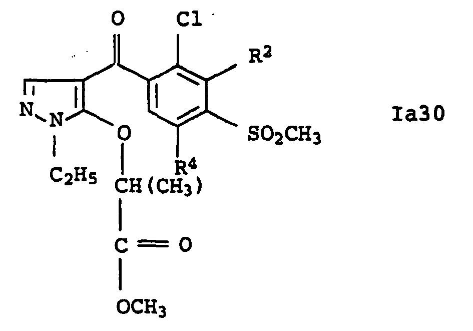
- Ebenso insbesondere außerordentlich bevorzugt sind die Verbindungen Ia31; insbesondere die Verbindungen Ia31.1-Ia31.164, die sich von den Verbindungen Ia1.1-Ia1.164 dadurch unterscheiden, daß R⁷ für 1-Methoxycarbonyl-eth-1-yl und R⁸ für Methyl stehen: 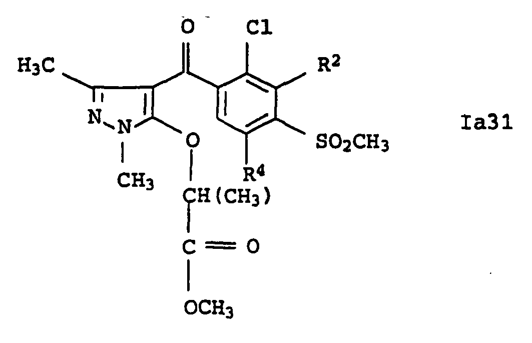
- Ebenso insbesondere außerordentlich bevorzugt sind die Verbindungen Ia32; insbesondere die Verbindungen Ia32.1-Ia32.164, die sich von den Verbindungen Ia1.1-Ia1.164 dadurch unterscheiden, daß R⁷ für 1-Ethoxycarbonyl-eth-1-yl steht: j 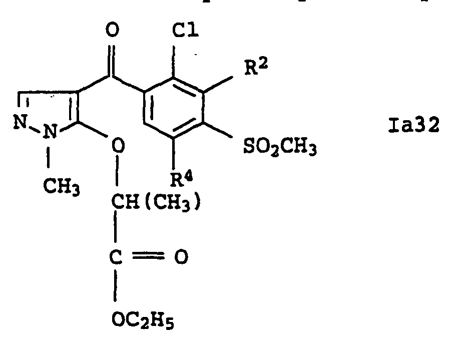
- Ebenso insbesondere außerordentlich bevorzugt sind die Verbindungen Ia33; insbesondere die Verbindungen Ia33.1-Ia33.164, die sich von den Verbindungen Ia1.1-Ia1.164 dadurch unterscheiden, daß R⁶ für Ethyl und R⁷ für 1-Ethoxycarbonyl-eth-1-yl stehen: 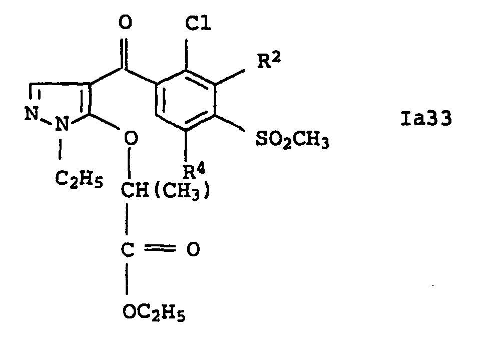
- Ebenso insbesondere außerordentlich bevorzugt sind die Verbindungen Ia34; insbesondere die Verbindungen Ia34.1-Ia34.164, die sich von den Verbindungen Ia1.1-Ia1.164 dadurch unterscheiden, daß R⁷ für 1-Ethoxycarbonyl-eth-1-yl und R⁸ für Methyl stehen: 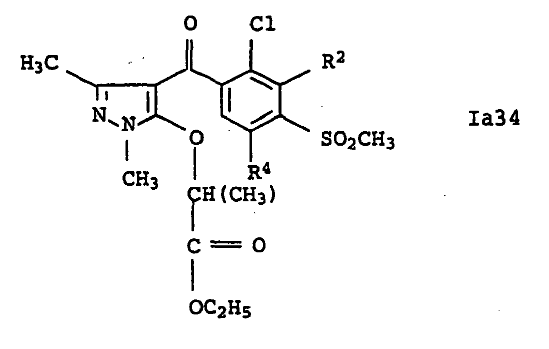
- Ebenso insbesondere außerordentlich bevorzugt sind die Verbindungen Ia35; insbesondere die Verbindungen Ia35.1-Ia35.164, die sich von den Verbindungen Ia1.1-Ia1.164 dadurch unterscheiden, daß R⁷ für 2-Methoxy-eth-1-oxycarbonylmethyl steht: 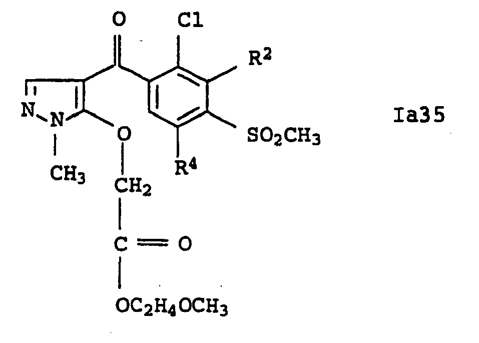
- Ebenso insbesondere außerordentlich bevorzugt sind die Verbindungen Ia36; insbesondere die Verbindungen Ia36.1-Ia36.164, die sich von den Verbindungen Ia1.1-Ia1.164 dadurch unterscheiden, daß R⁶ für Ethyl und R⁷ für 2-Methoxy-eth-1-oxycarbonylmethyl stehen: 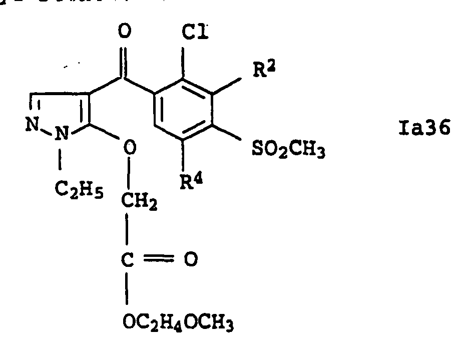
- Ebenso insbesondere außerordentlich bevorzugt sind die Verbindungen Ia37; insbesondere die Verbindungen Ia37.1-Ia37.164, die sich von den Verbindungen Ia1.1-Ia1.164 dadurch unterscheiden, daß R⁷ für 2-Methoxy-eth-1-oxycarbonylmethyl und R⁸ für Methyl stehen: 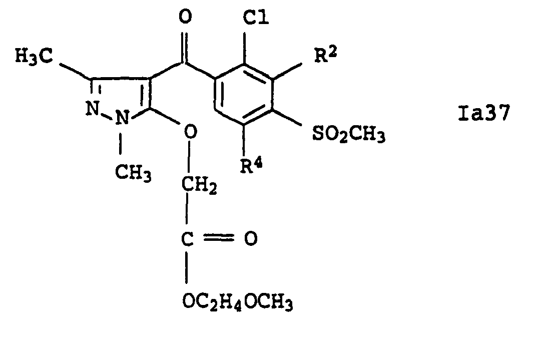
- Ebenso insbesondere außerordentlich bevorzugt sind die Verbindungen Ia38; insbesondere die Verbindungen Ia38.1-Ia38.164, die sich von den Verbindungen Ia1.1-Ia1.164 dadurch unterscheiden, daß R⁷ für 2-Dimethylamino-eth-1-oxycarbonylmethyl steht: 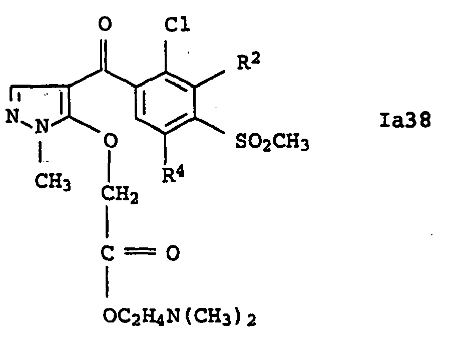
- Ebenso insbesondere außerordentlich bevorzugt sind die Verbindungen Ia39; insbesondere die Verbindungen Ia39.1-Ia39.164, die sich von den Verbindungen Ia1.1-Ia1.164 dadurch unterscheiden, daß R⁶ für Ethyl und R⁷ für 2-Dimethylamino-eth-1-oxycarbonylmethyl stehen: 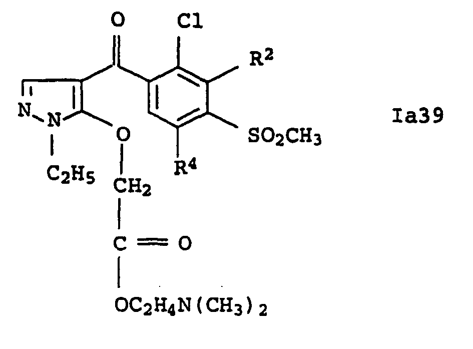
- Ebenso insbesondere außerordentlich bevorzugt sind die Verbindungen Ia40; insbesondere die Verbindungen Ia40.1-Ia40.164, die sich von den Verbindungen Ia1.1-Ia1.164 dadurch unterscheiden, daß R⁷ für 2-Dimethylamino-eth-1-oxycarbonylmethyl und R⁸ für Methyl stehen: 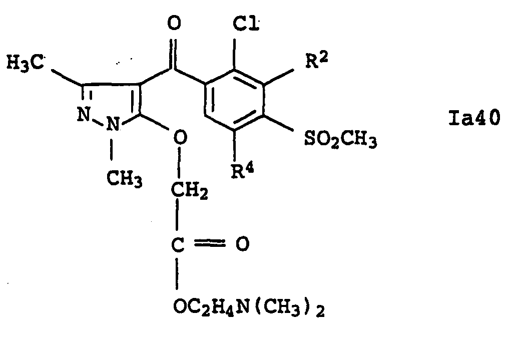
- Ebenso insbesondere außerordentlich bevorzugt sind die Verbindungen Ia41; insbesondere die Verbindungen Ia41.1-Ia41.164, die sich von den Verbindungen Ia1.1-Ia1.164 dadurch unterscheiden, daß R⁷ für Methylaminocarbonylmethyl steht: 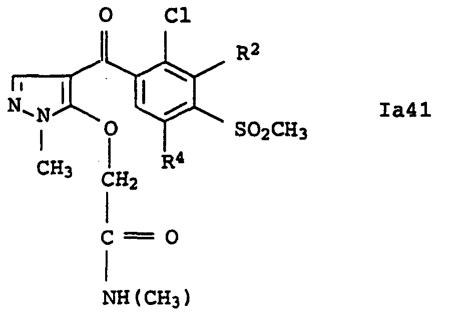
- Ebenso insbesondere außerordentlich bevorzugt sind die Verbindungen Ia42; insbesondere die Verbindungen Ia42.1-Ia42.164, die sich von den Verbindungen Ia1.1-Ia1.164 dadurch unterscheiden, daß R⁶ für Ethyl und R⁷ für Methylaminocarbonylmethyl stehen: 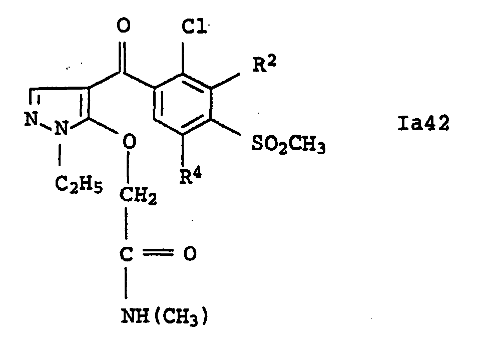
- Ebenso insbesondere außerordentlich bevorzugt sind die Verbindungen Ia43; insbesondere die Verbindungen Ia43.1-Ia43.164, die sich von den Verbindungen Ial.l-Ial.164 dadurch unterscheiden, daß R⁷ für Methylaminocarbonylmethyl und R⁸ für Methyl stehen: 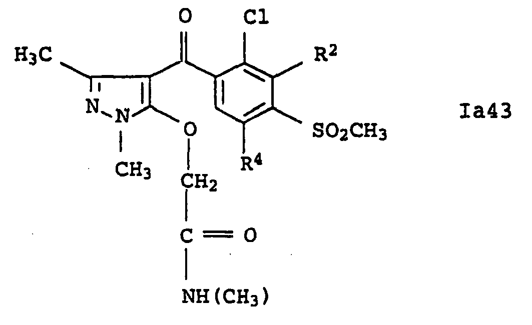
- Ebenso insbesondere außerordentlich bevorzugt sind die Verbindungen Ia44; insbesondere die Verbindungen Ia44.1-Ia44.164, die sich von den Verbindungen Ia1.1-Ia1.164 dadurch unterscheiden, daß R⁷ für Ethylaminocarbonylmethyl steht: 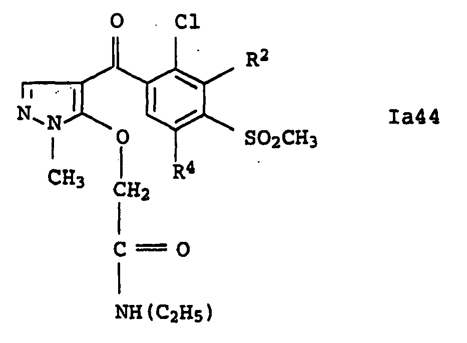
- Ebenso insbesondere außerordentlich bevorzugt sind die Verbindungen Ia45; insbesondere die Verbindungen Ia45.1-Ia45.164, die sich von den Verbindungen Ia1.1-Ia1.164 dadurch unterscheiden, daß R⁶ für Ethyl und R⁷ für Ethylaminocarbonylmethyl stehen: 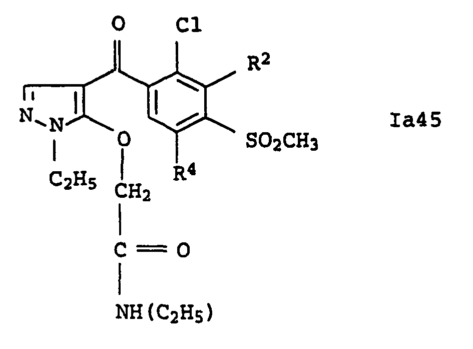
- Ebenso insbesondere außerordentlich bevorzugt sind die Verbindungen Ia46; insbesondere die Verbindungen Ia46.1-Ia46.164, die sich von den Verbindungen Ia1.1-Ia1.164 dadurch unterscheiden, daß R⁷ für Ethylaminocarbonylmethyl und R^{B} für Methyl stehen: 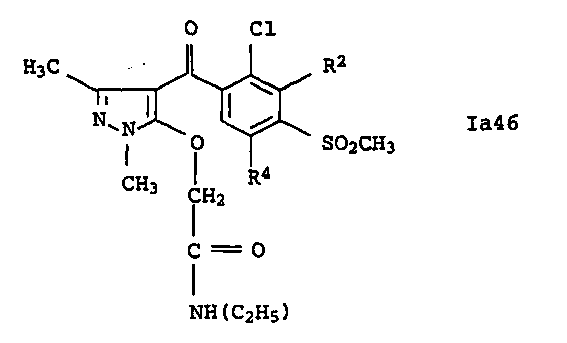
- Ebenso insbesondere außerordentlich bevorzugt sind die Verbindungen Ia47; insbesondere die Verbindungen Ia47.1-Ia47.164, die sich von den Verbindungen Ia1.1-Ia1.164 dadurch unterscheiden, daß R⁷ für Dimethylaminocarbonylmethyl steht: 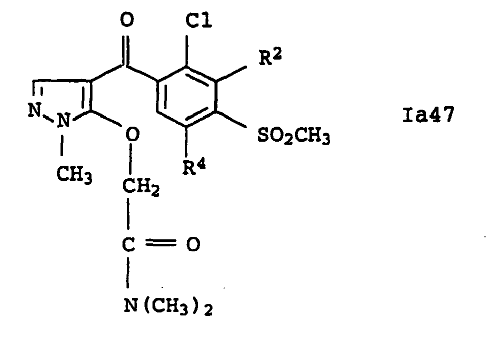
- Ebenso insbesondere außerordentlich bevorzugt sind die Verbindungen Ia48; insbesondere die Verbindungen Ia48.1-Ia48.164, die sich von den Verbindungen Ia1.1-Ia1.164 dadurch unterscheiden, daß R⁶ für Ethyl und R⁷ für Dimethylaminocarbonylmethyl stehen: 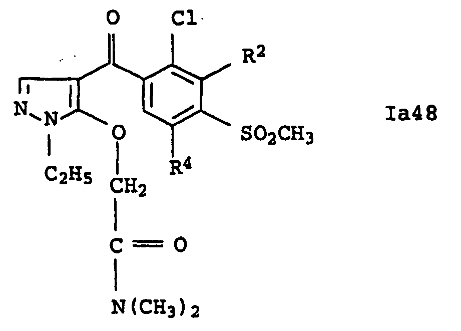
- Ebenso insbesondere außerordentlich bevorzugt sind die Verbindungen Ia49; insbesondere die Verbindungen Ia49.1-Ia49.164, die sich von den Verbindungen Ia1.1-Ia1.164 dadurch unterscheiden, daß R⁷ für Dimethylaminocarbonylmethyl und R⁸ für Methyl stehen: 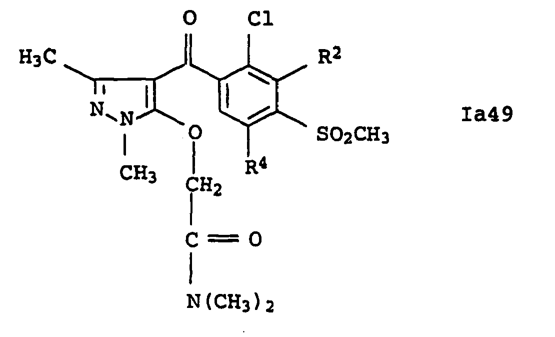
- Ebenso insbesondere außerordentlich bevorzugt sind die Verbindungen Ia50; insbesondere die Verbindungen Ia50.1-Ia50.164, die sich von den Verbindungen Ia1.1-Ia1.164 dadurch unterscheiden, daß R⁷ für Diethylaminocarbonylmethyl steht: 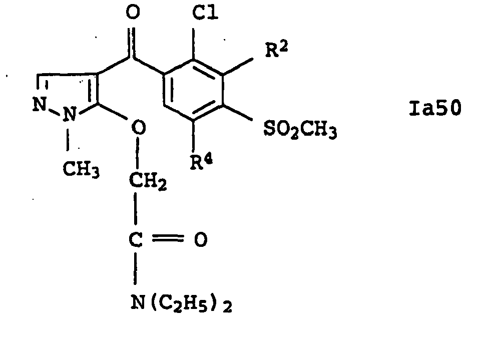
- Ebenso insbesondere außerordentlich bevorzugt sind die Verbindungen Ia51; insbesondere die Verbindungen Ia51.1-Ia51.164, die sich von den Verbindungen Ia1.1-Ia1.164 dadurch unterscheiden, daß R⁶ für Ethyl und R⁷ für Diethylaminocarbonylmethyl stehen: 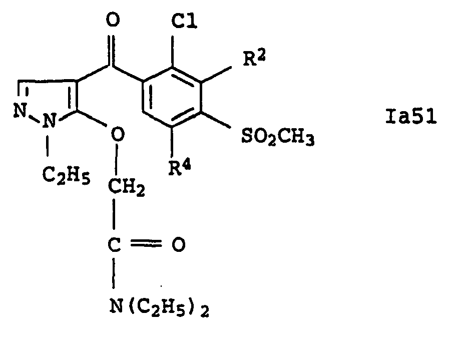
- Ebenso insbesondere außerordentlich bevorzugt sind die Verbindungen Ia52; insbesondere die Verbindungen Ia52.1-Ia52.164, die sich von den Verbindungen Ia1.1-Ia1.164 dadurch unterscheiden, daß R⁷ für Diethylaminocarbonylmethyl und R⁸ für Methyl stehen: 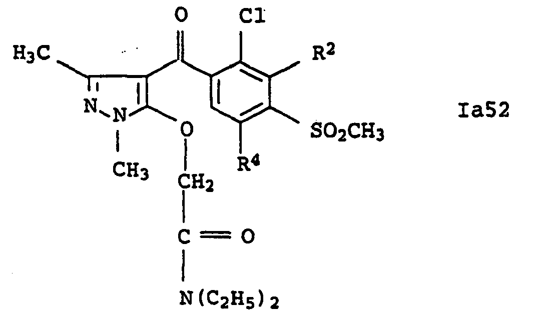
- Ebenso insbesondere außerordentlich bevorzugt sind die Verbindungen Ia53; insbesondere die Verbindungen Ia53.1-Ia53.164, die sich von den Verbindungen Ia1.1-Ia1.164 dadurch unterscheiden, daß R⁷ für Allyl steht: 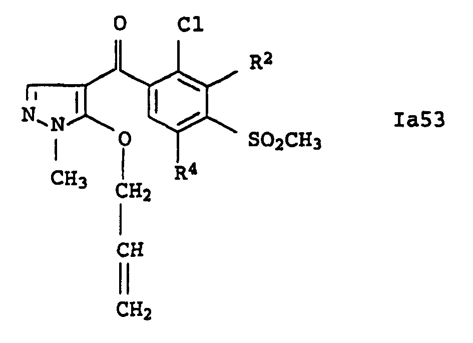
- Ebenso insbesondere außerordentlich bevorzugt sind die Verbindungen Ia54; insbesondere die Verbindungen Ia54.1-Ia54.164, die sich von den Verbindungen Ia1.1-Ia1.164 dadurch unterscheiden, daß R⁶ für Ethyl und R⁷ für Allyl stehen: 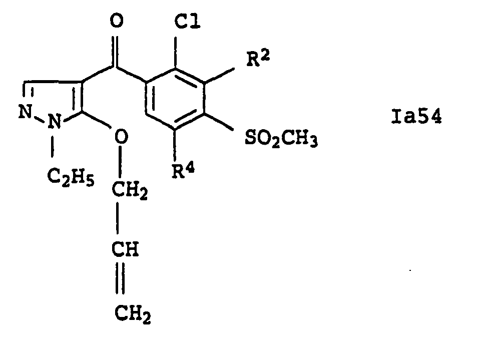
- Ebenso insbesondere außerordentlich bevorzugt sind die Verbindungen Ia55; insbesondere die Verbindungen Ia55.1-Ia55.164, die sich von den Verbindungen Ia1.1-Ia1.164 dadurch unterscheiden, daß R⁷ für Allyl und R⁸ für Methyl stehen: 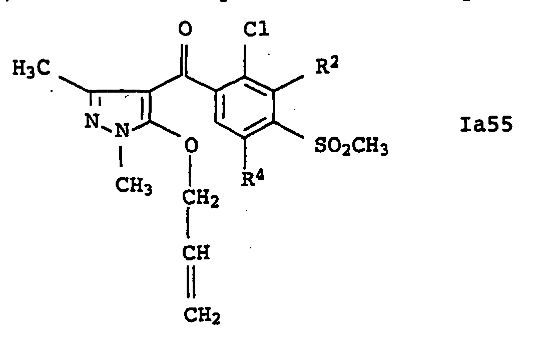
- Ebenso insbesondere außerordentlich bevorzugt sind die Verbindungen Ia56; insbesondere die Verbindungen Ia56.1-Ia56.164, die sich von den Verbindungen Ia1.1-Ia1.164 dadurch unterscheiden, daß R⁷ für Propargyl steht: 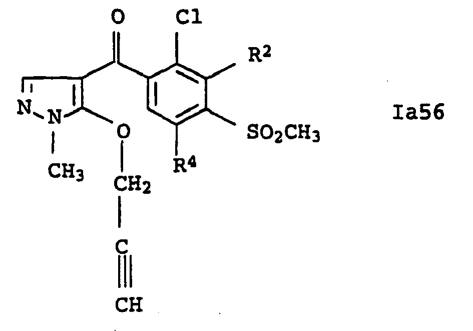
- Ebenso insbesondere außerordentlich bevorzugt sind die Verbindungen Ia57; insbesondere die Verbindungen Ia57.1-Ia57.164, die sich von den Verbindungen Ia1.1-Ia1.164 dadurch unterscheiden, daß R⁶ für Ethyl und R⁷ für Propargyl stehen: 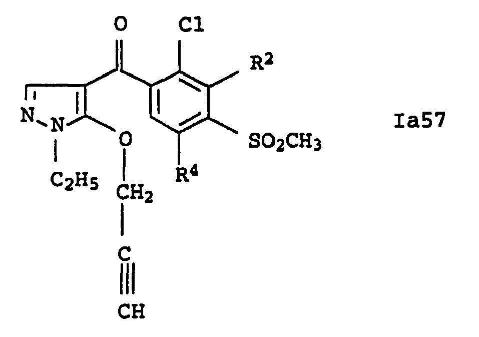
- Ebenso insbesondere außerordentlich bevorzugt sind die Verbindungen Ia58; insbesondere die Verbindungen Ia58.1-Ia58.164, die sich von den Verbindungen Ia1.1-Ia1.164 dadurch unterscheiden, daß R⁷ für Propargyl und R⁸ für Methyl stehen: 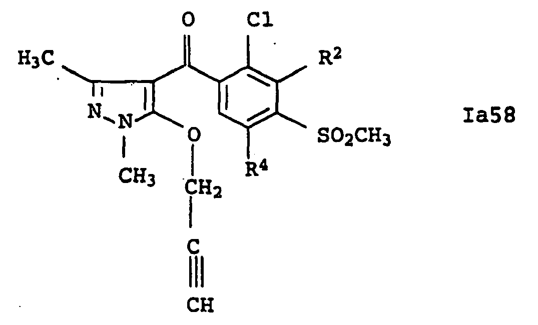
- Ebenso insbesondere außerordentlich bevorzugt sind die Verbindungen Ia59; insbesondere die Verbindungen Ia59.1-Ia59.164, die sich von den Verbindungen Ia1.1-Ia1.164 dadurch unterscheiden, daß R⁷ für Methylcarbonyl steht: 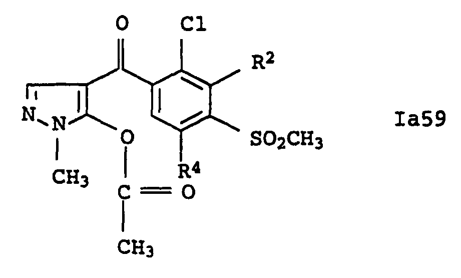
- Ebenso insbesondere außerordentlich bevorzugt sind die Verbindungen Ia60; insbesondere die Verbindungen Ia60.1-Ia60.164, die sich von den Verbindungen Ia1.1-Ia1.164 dadurch unterscheiden, daß R⁶ für Ethyl und R⁷ für Methylcarbonyl stehen: 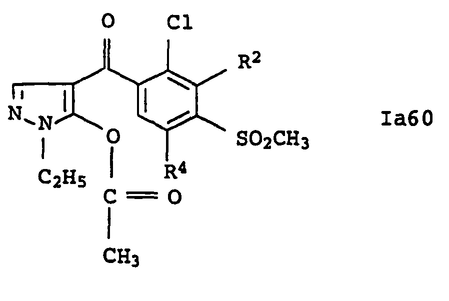
- Ebenso insbesondere außerordentlich bevorzugt sind die Verbindungen Ia61; insbesondere die Verbindungen Ia61.1-Ia61.164, die sich von den Verbindungen Ia1.1-Ia1.164 dadurch unterscheiden, daß R⁶ für n-Propyl und R⁷ für Methylcarbonyl stehen: 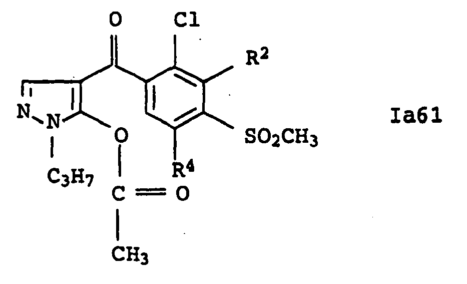
- Ebenso insbesondere außerordentlich bevorzugt sind die Verbindungen Ia62; insbesondere die Verbindungen Ia62.1-Ia62.164, die sich von den Verbindungen Ia1.1-Ia1.164 dadurch unterscheiden, daß R⁶ für iso-Butyl und R⁷ für Methylcarbonyl stehen: 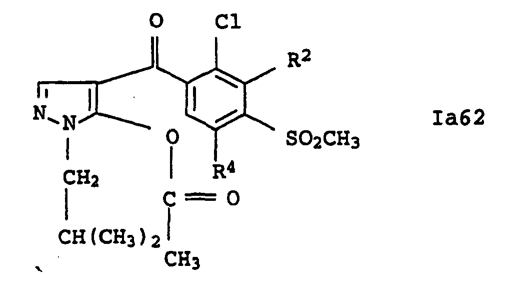
- Ebenso insbesondere außerordentlich bevorzugt sind die Verbindungen Ia63; insbesondere die Verbindungen Ia63.1-Ia63.164, die sich von den Verbindungen Ia1.1-Ia1.164 dadurch unterscheiden, daß R⁷ für Methylcarbonyl und R⁸ für Methyl stehen: 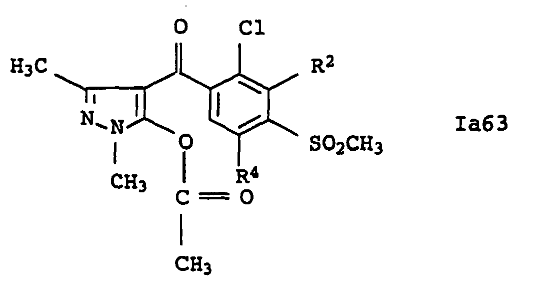
- Ebenso insbesondere außerordentlich bevorzugt sind die Verbindungen Ia64; insbesondere die Verbindungen Ia64.1-Ia64.164, die sich von den Verbindungen Ia1.1-Ia1.164 dadurch unterscheiden, daß R⁷ für Ethylcarbonyl steht: 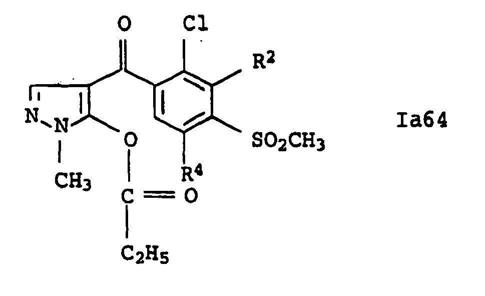
- Ebenso insbesondere außerordentlich bevorzugt sind die Verbindungen Ia65; insbesondere die Verbindungen Ia65.1-Ia65.164, die sich von den Verbindungen Ia1.1-Ia1.164 dadurch unterscheiden, daß R⁶ für Ethyl und R⁷ für Ethylcarbonyl stehen: 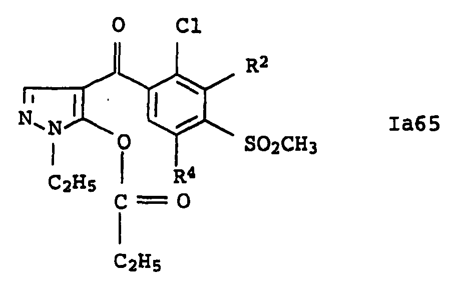
- Ebenso insbesondere außerordentlich bevorzugt sind die Verbindungen Ia66; insbesondere die Verbindungen Ia66.1-Ia66.164, die sich von den Verbindungen Ia1.1-Ia1.164 dadurch unterscheiden, daß R⁶ für n-Propyl und R⁷ für Ethylcarbonyl stehen: 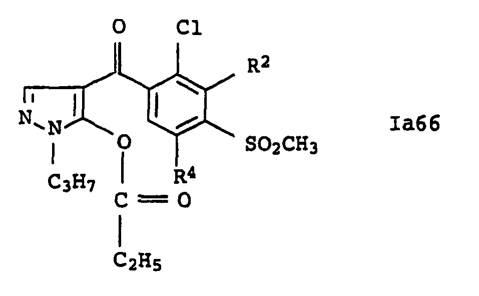
- Ebenso insbesondere außerordentlich bevorzugt sind die Verbindungen Ia67; insbesondere die Verbindungen Ia67.1-Ia67.164, die sich von den Verbindungen Ia1.1-Ia1.164 dadurch unterscheiden, daß R⁶ für iso-Butyl und R⁷ für Ethylcarbonyl stehen: 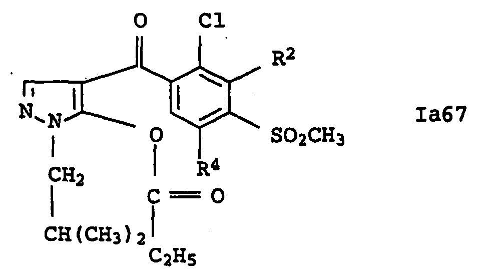
- Ebenso insbesondere außerordentlich bevorzugt sind die Verbindungen Ia68.; insbesondere die Verbindungen Ia68.1-Ia68.164, die sich von den Verbindungen Ia1.1-Ia1.164 dadurch unterscheiden, daß R⁷ für Ethylcarbonyl und R⁸ für Methyl stehen: 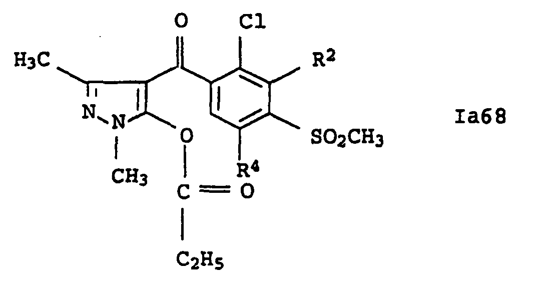
- Ebenso insbesondere außerordentlich bevorzugt sind die Verbindungen Ia69; insbesondere die Verbindungen Ia69.1-Ia69.164, die sich von den Verbindungen Ia1.1-Ia1.164 dadurch unterscheiden, daß R⁷ für Methoxycarbonyl steht: 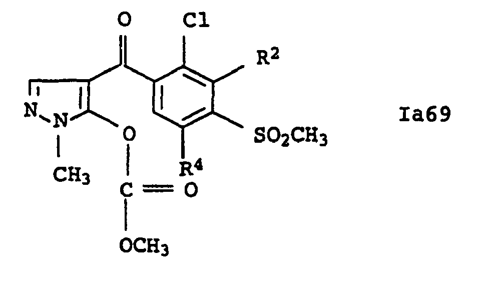
- Ebenso insbesondere außerordentlich bevorzugt sind die Verbindungen Ia70; insbesondere die Verbindungen Ia70.1-Ia70.164, die sich von den Verbindungen Ia1.1-Ia1.164 dadurch unterscheiden, daß R⁶ für Ethyl und R⁷ für Methoxycarbonyl stehen: 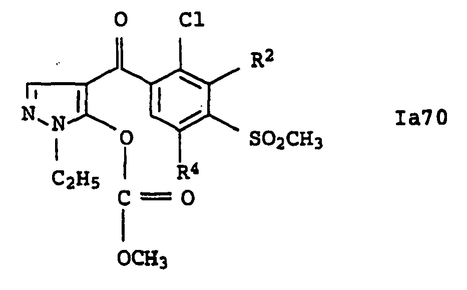
- Ebenso insbesondere außerordentlich bevorzugt sind die Verbindungen Ia71; insbesondere die Verbindungen Ia71.1-Ia71.164, die sich von den Verbindungen Ia1.1-Ia1.164 dadurch unterscheiden, daß R⁷ für Methoxycarbonyl und R⁸ für Methyl stehen: 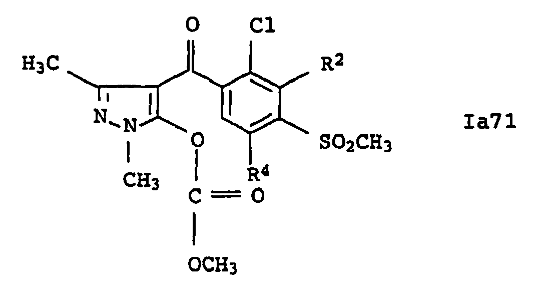
- Ebenso insbesondere außerordentlich bevorzugt sind die Verbindungen Ia72; insbesondere die Verbindungen Ia72.1-Ia72.164, die sich von den Verbindungen Ia1.1-Ia1.164 dadurch unterscheiden, daß R⁷ für Ethoxycarbonyl steht: 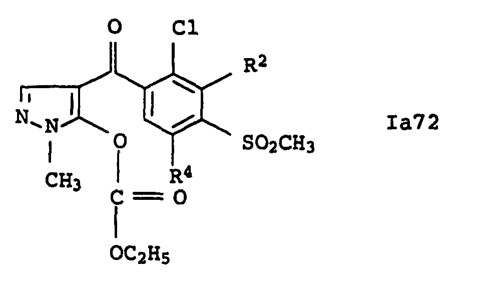
- Ebenso insbesondere außerordentlich bevorzugt sind die Verbindungen Ia73; insbesondere die Verbindungen Ia73.1-Ia73.164, die sich von den Verbindungen Ia1.1-Ia1.164 dadurch unterscheiden, daß R⁶ für Ethyl und R⁷ für Ethoxycarbonyl stehen: 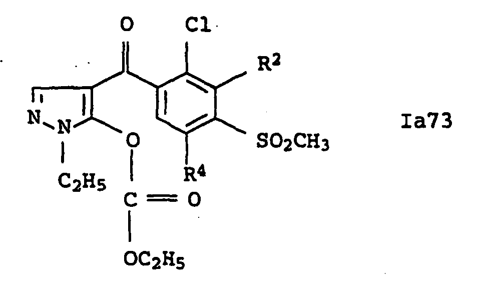
- Ebenso insbesondere außerordentlich bevorzugt sind die Verbindungen Ia74; insbesondere die Verbindungen Ia74.1-Ia74.164, die sich von den Verbindungen Ia1.1-Ia1.164 dadurch unterscheiden, daß R⁷ für Ethoxycarbonyl und R⁸ für Methyl stehen: 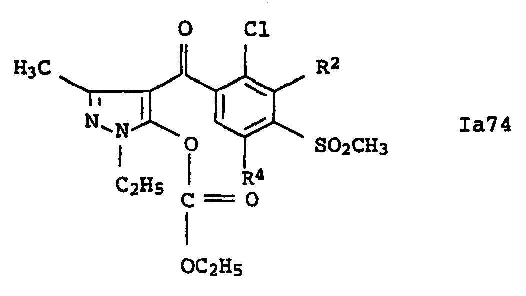
- Ebenso insbesondere außerordentlich bevorzugt sind die Verbindungen Ia75; insbesondere die Verbindungen Ia75.1-Ia75.164, die sich von den Verbindungen Ia1.1-Ia1.164 dadurch unterscheiden, daß R⁷ für Dimethylaminocarbonyl steht: 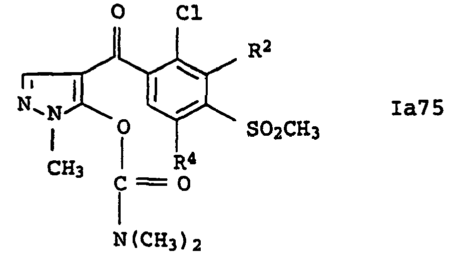
- Ebenso insbesondere außerordentlich bevorzugt sind die Verbindungen Ia76; insbesondere die Verbindungen Ia76.1-Ia76.164, die sich von den Verbindungen Ia1.1-Ia1.164 dadurch unterscheiden, daß R⁶ für Ethyl und R⁷ für Dimethylaminocarbonyl stehen: 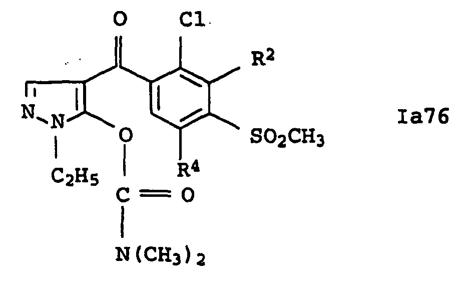
- Ebenso insbesondere außerordentlich bevorzugt sind die Verbindungen Ia77; insbesondere die Verbindungen Ia77.1-Ia77.164, die sich von den Verbindungen Ia1.1-Ia1.164 dadurch unterscheiden, daß R⁷ für Dimethylaminocarbonyl und R^{B} für Methyl stehen: 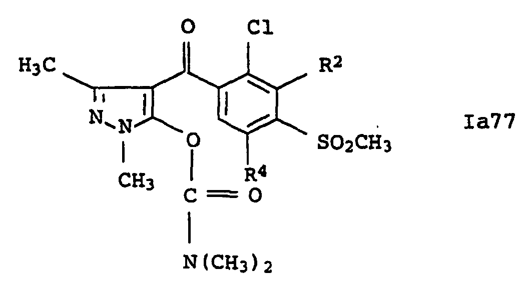
- Ebenso insbesondere außerordentlich bevorzugt sind die Verbindungen Ia78; insbesondere die Verbindungen Ia78.1-Ia78.164, die sich von den Verbindungen Ia1.1-Ia1.164 dadurch unterscheiden, daß R⁷ für Diethylaminocarbonyl steht: 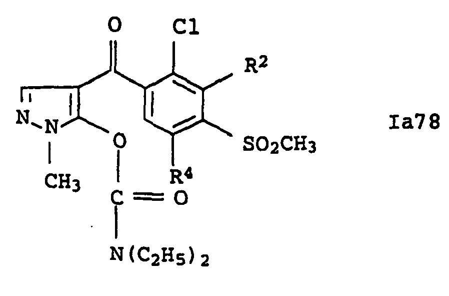
- Ebenso insbesondere außerordentlich bevorzugt sind die Verbindungen Ia79; insbesondere die Verbindungen Ia79.1-Ia79.164, die sich von den Verbindungen Ia1.1-Ia1.164 dadurch unterscheiden, daß R⁶ für Ethyl und R⁷ für Diethylaminocarbonyl stehen: 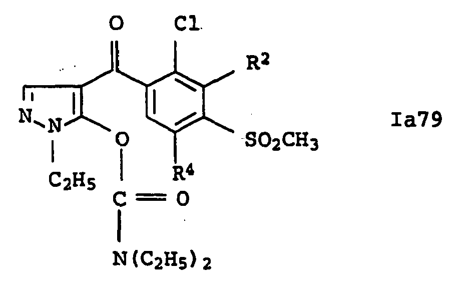
- Ebenso insbesondere außerordentlich bevorzugt sind die Verbindungen Ia80; insbesondere die Verbindungen Ia80.1-Ia80.164, die sich von den Verbindungen Ia1.1-Ia1.164 dadurch unterscheiden, daß R⁷ für Diethylaminocarbonyl und R⁸ für Methyl stehen: 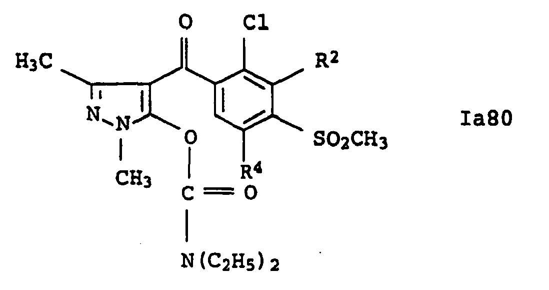
- Ebenso insbesondere außerordentlich bevorzugt sind die Verbindungen Ia81; insbesondere die Verbindungen Ia81.1-Ia81.164, die sich von den Verbindungen Ia1.1-Ia1.164 dadurch unterscheiden, daß R⁷ für N-Methoxy-N-methylaminocarbonyl steht: 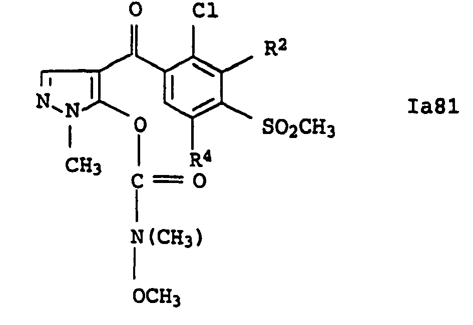
- Ebenso insbesondere außerordentlich bevorzugt sind die Verbindungen Ia82; insbesondere die Verbindungen Ia82.1-Ia82.164, die sich von den Verbindungen Ia1.1-Ia1.164 dadurch unterscheiden, daß R⁶ für Ethyl und R⁷ für N-Methoxy-N-methylaminocarbonyl stehen: 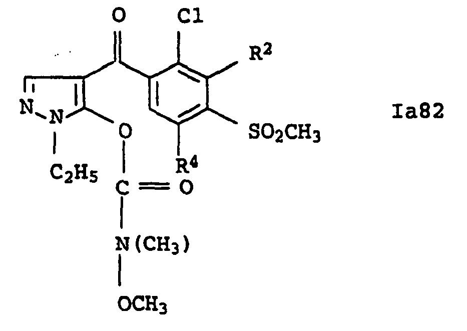
- Ebenso insbesondere außerordentlich bevorzugt sind die Verbindungen Ia83; insbesondere die Verbindungen Ia83.1-Ia83.164, die sich von den Verbindungen Ia1.1-Ia1.164 dadurch unterscheiden, daß R⁷ für N-Methoxy-N-methylaminocarbonyl und R^{B} für Methyl stehen: 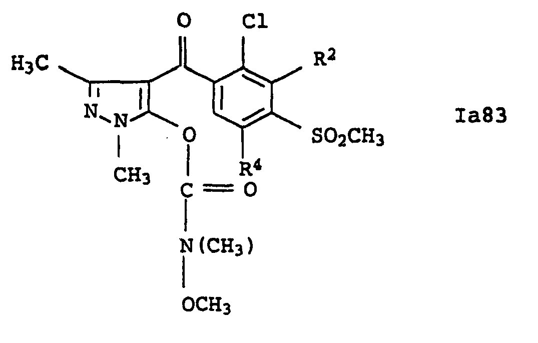
- Ebenso insbesondere außerordentlich bevorzugt sind die Verbindungen Ia84; insbesondere die Verbindungen Ia84.1-Ia84.164, die sich von den Verbindungen Ia1.1-Ia1.164 dadurch unterscheiden, daß R⁷ für Benzyl steht: 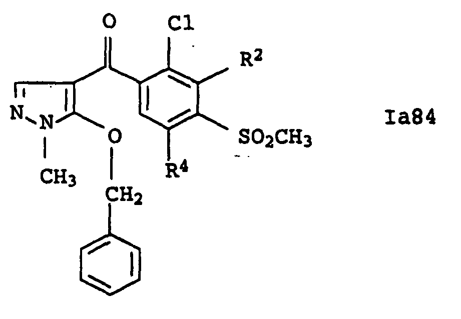
- Ebenso insbesondere außerordentlich bevorzugt sind die Verbindungen Ia85; insbesondere die Verbindungen Ia85.1-Ia85.164, die sich von den Verbindungen Ial.1-Ial.164 dadurch unterscheiden, daß R⁶ für Ethyl und R⁷ für Benzyl stehen: 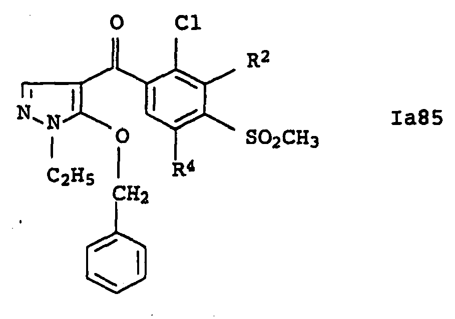
- Ebenso insbesondere außerordentlich bevorzugt sind die Verbindungen Ia86; insbesondere die Verbindungen Ia86.1-Ia86.164, die sich von den Verbindungen Ia1.1-Ia1.164 dadurch unterscheiden, daß R⁶ für n-Propyl und R⁷ für Benzyl stehen: 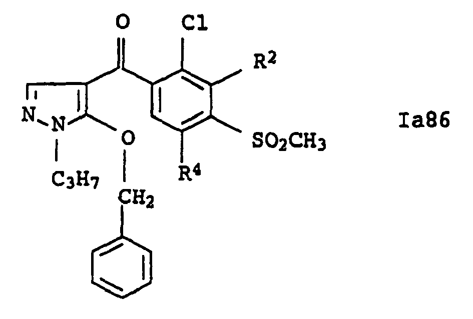
- Ebenso insbesondere außerordentlich bevorzugt sind die Verbindungen Ia87; insbesondere die Verbindungen Ia87.1-Ia87.164, die sich von den Verbindungen Ia1.1-Ia1.164 dadurch unterscheiden, daß R⁶ für iso-Butyl und R⁷ für Benzyl stehen: 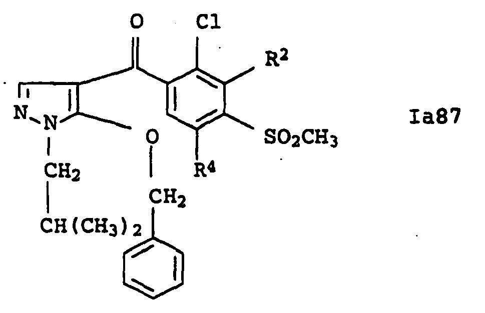
- Ebenso insbesondere außerordentlich bevorzugt sind die Verbindungen Ia88; insbesondere die Verbindungen Ia88.1-Ia88.164, die sich von den Verbindungen Ia1.1-Ia1.164 dadurch unterscheiden, daß R⁷ für Benzyl und R⁸ für Methyl stehen: 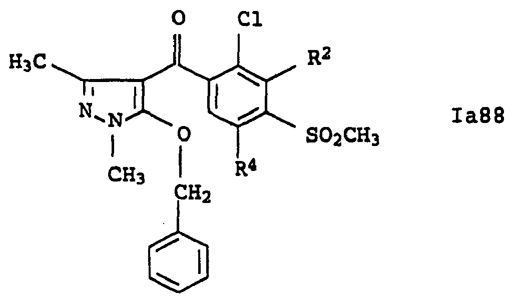
- Ebenso insbesondere außerordentlich bevorzugt sind die Verbindungen Ia89; insbesondere die Verbindungen Ia89.1-Ia89.164, die sich von den Verbindungen Ia1.1-Ia1.164 dadurch unterscheiden, daß R⁷ für 4-Methylphenylmethyl steht: 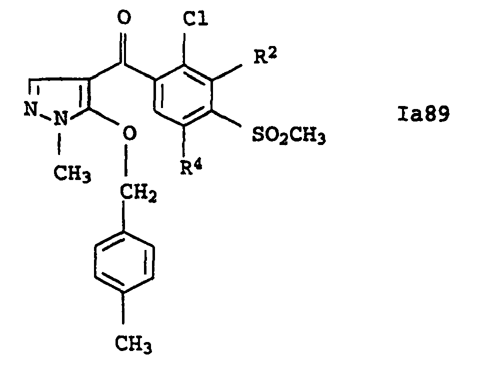
- Ebenso insbesondere außerordentlich bevorzugt sind die Verbindungen Ia90; insbesondere die Verbindungen Ia90.1-Ia90.164, die sich von den Verbindungen Ia1.1-Ia1.164 dadurch unterscheiden, daß R⁶ für Ethyl und R⁷ für 4-Methylphenylmethyl stehen: 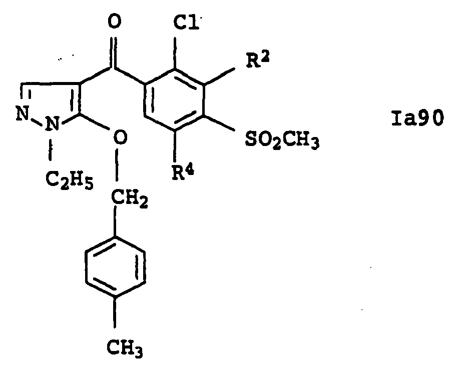
- Ebenso insbesondere außerordentlich bevorzugt sind die Verbindungen Ia91; insbesondere die Verbindungen Ia91.1-Ia91.164, die sich von den Verbindungen Ia1.1-Ia1.164 dadurch unterscheiden, daß R⁶ für n-Propyl und R⁷ für 4-Methylphenylmethyl stehen: 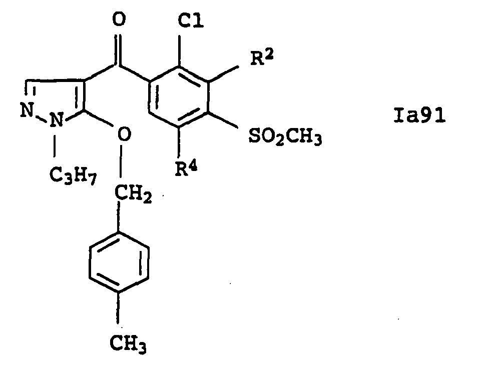
- Ebenso insbesondere außerordentlich bevorzugt sind die Verbindungen Ia92; insbesondere die Verbindungen 1a92.1-Ia92.164, die sich von den Verbindungen Ia1.1-Ia1.164 dadurch unterscheiden, daß R⁶ für iso-Butyl und R⁷ für 4-Methylphenylmethyl stehen: 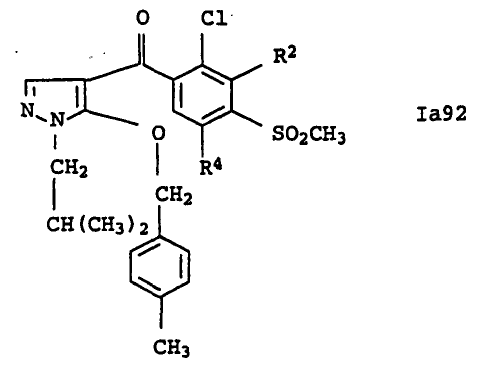
- Ebenso insbesondere außerordentlich bevorzugt sind die Verbindungen Ia93; insbesondere die Verbindungen Ia93.1-Ia93.164, die sich von den Verbindungen Ia1.1-Ia1.164 dadurch unterscheiden, daß R⁷ für 4-Methylphenylmethyl und R⁸ für Methyl stehen: 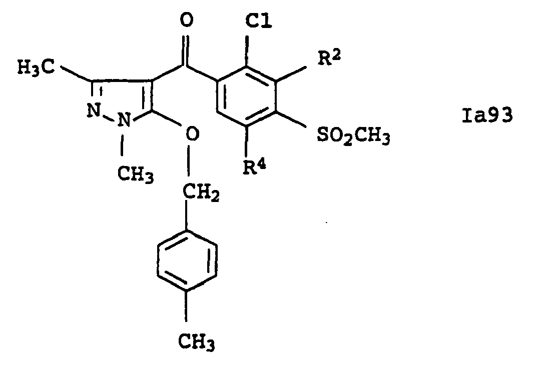
- Ebenso insbesondere außerordentlich bevorzugt sind die Verbindungen Ia94; insbesondere die Verbindungen Ia94.1-Ia94.164, die sich von den Verbindungen Ia1.1-Ia1.164 dadurch unterscheiden, daß R⁷ für 4-Chlorphenylmethyl steht: 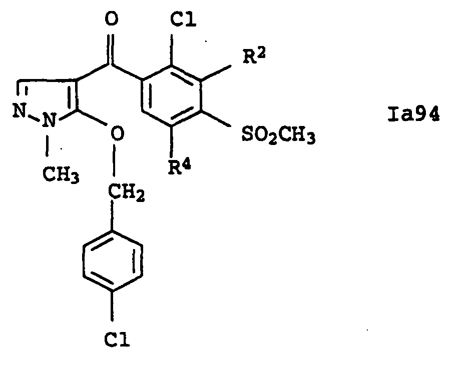
- Ebenso insbesondere außerordentlich bevorzugt sind die Verbindungen Ia95; insbesondere die Verbindungen Ia95.1-Ia95.164, die sich von den Verbindungen Ia1.1-Ia1.164 dadurch unterscheiden, daß R⁶ für Ethyl und R⁷ für 4-Chlorphenylmethyl stehen: 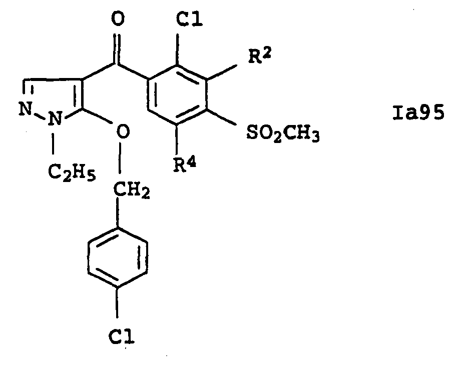
- Ebenso insbesondere außerordentlich bevorzugt sind die Verbindungen Ia96; insbesondere die Verbindungen Ia96.1-Ia96.164, die sich von den Verbindungen Ia1.1-Ia1.164 dadurch unterscheiden, daß R⁶ für n-Propyl und R⁷ für 4-Chlorphenylmethyl stehen: 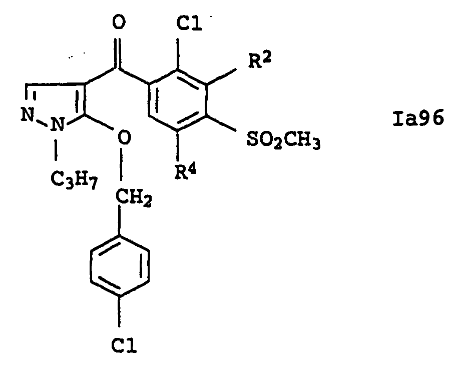
- Ebenso insbesondere außerordentlich bevorzugt sind die Verbindungen Ia97; insbesondere die Verbindungen Ia97.1-Ia97.164, die sich von den Verbindungen Ia1.1-Ia1.164 dadurch unterscheiden, daß R⁶ für iso-Butyl und R⁷ für 4-Chlorphenylmethyl stehen: 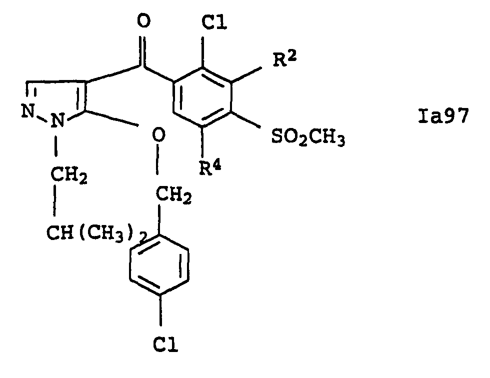
- Ebenso insbesondere außerordentlich bevorzugt sind die Verbindungen Ia98; insbesondere die Verbindungen Ia98.1-Ia98.164, die sich von den Verbindungen Ia1.1-Ia1.164 dadurch unterscheiden, daß R⁷ für 4-Chlorphenylmethyl und R⁸ für Methyl stehen: 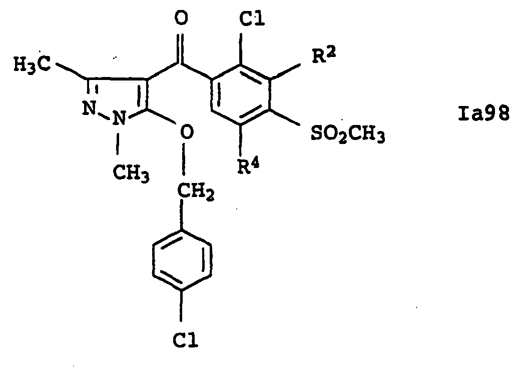
- Ebenso insbesondere außerordentlich bevorzugt sind die Verbindungen Ia99; insbesondere die Verbindungen Ia99.1-Ia99.164, die sich von den Verbindungen Ia1.1-Ia1.164 dadurch unterscheiden, daß R⁷ für 4-Methoxyphenylmethyl steht: 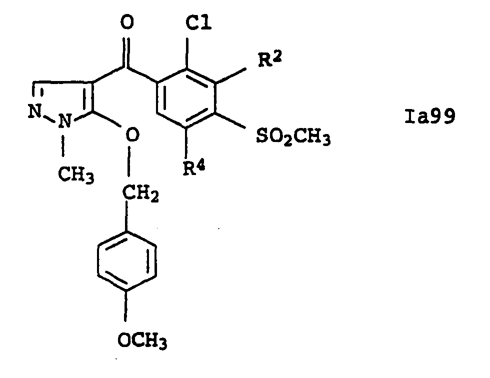
- Ebenso insbesondere außerordentlich bevorzugt sind die Verbindungen Ia100; insbesondere die Verbindungen Ia100.1-Ia100.164, die sich von den Verbindungen Ia1.1-Ia1.164 dadurch unterscheiden, daß R⁶ für Ethyl und R⁷ für 4-Methoxyphenylmethyl stehen: 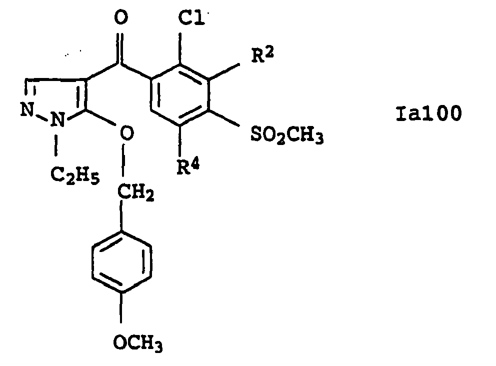
- Ebenso insbesondere außerordentlich bevorzugt sind die Verbindungen Ia101; insbesondere die Verbindungen Ia101.1-Ia101.164, die sich von den Verbindungen Ia1.1-Ia1.164 dadurch unterscheiden, daß R⁷ für 4-Methoxyphenylmethyl und R⁸ für Methyl stehen: 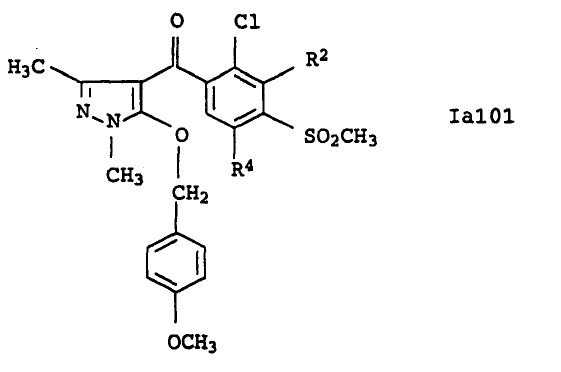
- Ebenso insbesondere außerordentlich bevorzugt sind die Verbindungen Ia102; insbesondere die Verbindungen Ia102.1-Ia102.164, die sich von den Verbindungen Ia1.1-Ia1.164 dadurch unterscheiden, daß R⁷ für 3-Trifluormethylphenylmethyl steht: 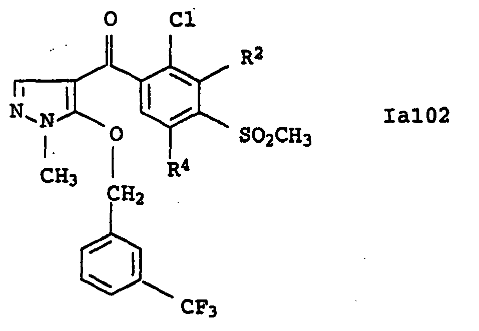
- Ebenso insbesondere außerordentlich bevorzugt sind die Verbindungen Ia103; insbesondere die Verbindungen Ia103.1-Ia103.164, die sich von den Verbindungen Ia1.1-Ia1.164 dadurch unterscheiden, daß R⁶ für Ethyl und R⁷ für 3-Trifluormethylphenylmethyl stehen: 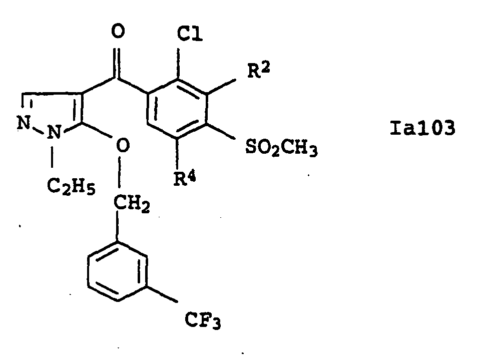
- Ebenso insbesondere außerordentlich bevorzugt sind die Verbindungen Ia104; insbesondere die Verbindungen Ia104.1-Ia104.164, die sich von den Verbindungen Ia1.1-Ia1.164 dadurch unterscheiden, daß R⁷ für 3-Trifluormethylphenylmethyl und R⁸ für Methyl stehen: 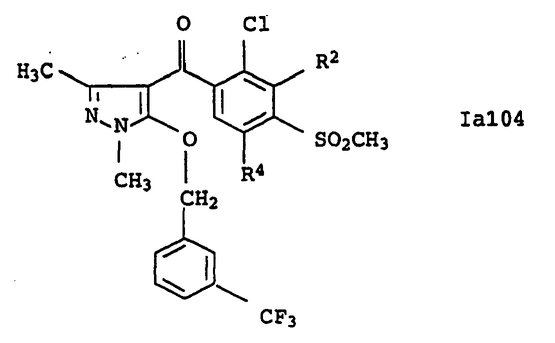
- Ebenso insbesondere außerordentlich bevorzugt sind die Verbindungen Ia105; insbesondere die Verbindungen Ia105.1-Ia105.164, die sich von den Verbindungen Ia1.1-Ia1.164 dadurch unterscheiden, daß R⁷ für 2,4-Dichlorphenylmethyl steht: 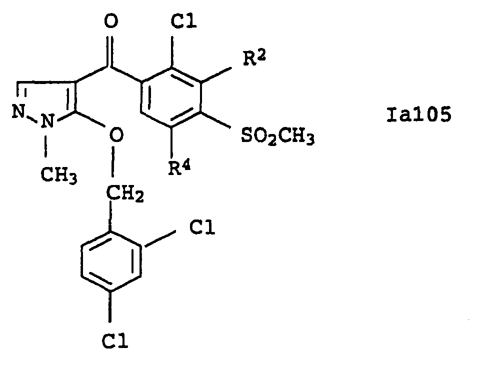
- Ebenso insbesondere außerordentlich bevorzugt sind die Verbindungen Ia106; insbesondere die Verbindungen Ia106.1-Ia106.164, die sich von den Verbindungen Ia1.1-Ia1.164 dadurch unterscheiden, daß R⁶ für Ethyl und R⁷ für 2,4-Dichlorphenylmethyl stehen: 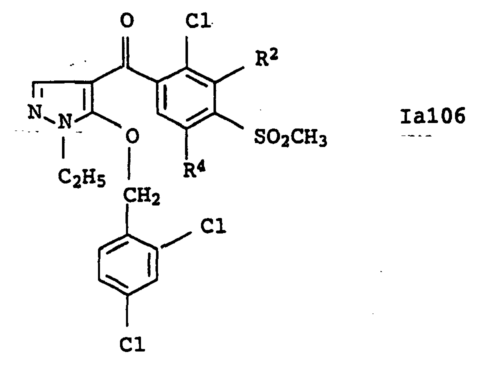
- Ebenso insbesondere außerordentlich bevorzugt sind die Verbindungen Ia107; insbesondere die Verbindungen Ia107.1-Ia107.164, die sich von den Verbindungen Ia1.1-Ia1.164 dadurch unterscheiden, daß R⁷ für 2,4-Dichlorphenylmethyl und R⁸ für Methyl-stehen: 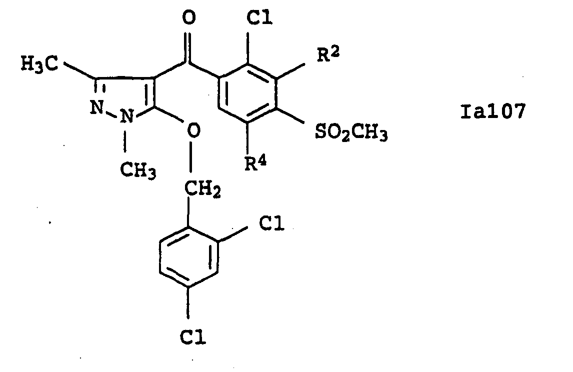
- Ebenso insbesondere außerordentlich bevorzugt sind die Verbindungen Ia108; insbesondere die Verbindungen Ia108.1-Ia108.164, die sich von den Verbindungen Ia1.1-Ia1.164 dadurch unterscheiden, daß R⁷ für Phenylcarbonylmethyl steht: 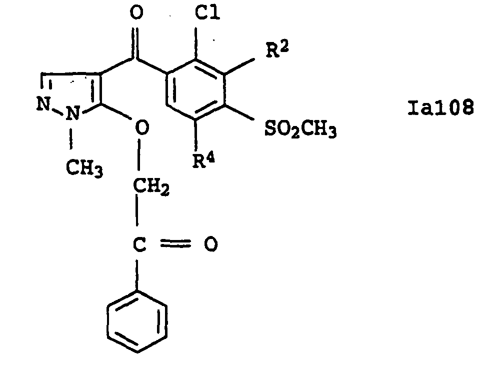
- Ebenso insbesondere außerordentlich bevorzugt sind die Verbindungen Ia109; insbesondere die Verbindungen Ia109.1-Ia109.164, die sich von den Verbindungen Ia1.1-Ia1.164 dadurch unterscheiden, daß R⁶ für Ethyl und R⁷ für Phenylcarbonylmethyl stehen: 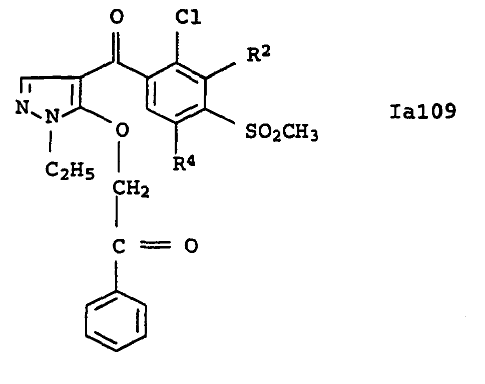
- Ebenso insbesondere außerordentlich bevorzugt sind die Verbindungen Ia110; insbesondere die Verbindungen Ia110.1-Ia110.164, die sich von den Verbindungen Ia1.1-Ia1.164 dadurch unterscheiden, daß R⁶ für n-Propyl und R⁷ für Phenylcarbonylmethyl stehen: 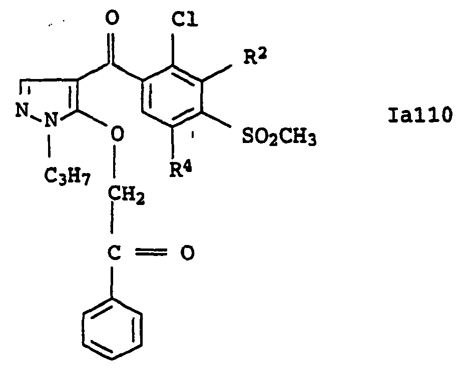
- Ebenso insbesondere außerordentlich bevorzugt sind die Verbindungen Ia111; insbesondere die Verbindungen Ia111.1-Ia111.164, die sich von den verbindungen Ia1.1-Ia1.164 dadurch unterscheiden, daß R⁶ für iso-Butyl und R⁷ für Phenylcarbonylmethyl stehen: 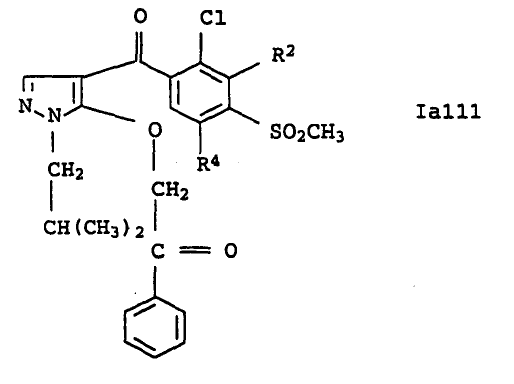
- Ebenso insbesondere außerordentlich bevorzugt sind die Verbindungen Ia112; insbesondere die Verbindungen Ia112.1-1a112.164, die sich von den Verbindungen Ia1.1-Ia1.164 dadurch unterscheiden, daß R⁷ für Phenylcarbonylmethyl und R⁸ für Methyl stehen: 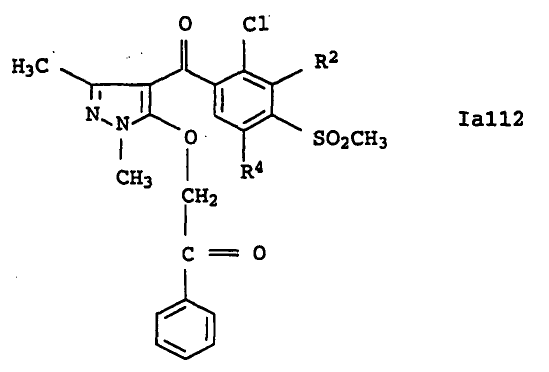
- Ebenso insbesondere außerordentlich bevorzugt sind die Verbindungen Ia113; insbesondere die Verbindungen Ia113.1-Ia113.164, die sich von den Verbindungen Ia1.1-Ia1.164 dadurch unterscheiden, daß R⁷ für 4-Methylphenylcarbonylmethyl steht: 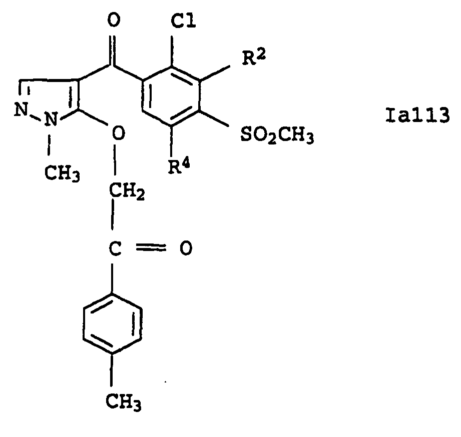
- Ebenso insbesondere außerordentlich bevorzugt sind die Verbindungen Ia114; insbesondere die Verbindungen Ia114.1-Ia114.164, die sich von den Verbindungen Ia1.1-Ia1.164 dadurch unterscheiden, daß R⁶ für Ethyl und R⁷ für 4-Methylphenylcarbonylmethyl stehen: 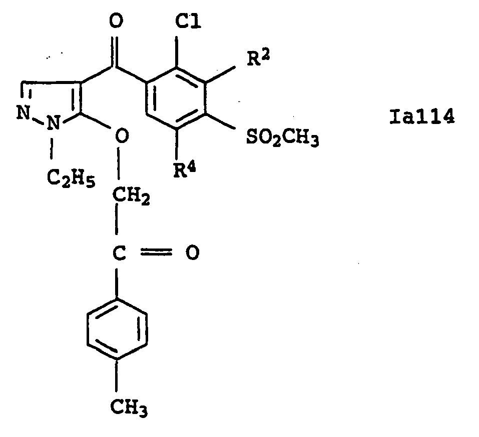
- Ebenso insbesondere außerordentlich bevorzugt sind die Verbindungen Ia115; insbesondere die Verbindungen Ia115.1-Ia115.164, die sich von den Verbindungen Ia1.1-Ia1.164 dadurch unterscheiden, daß R⁷ für 4-Methylphenylcarbonylmethyl und R⁸ für Methyl stehen: 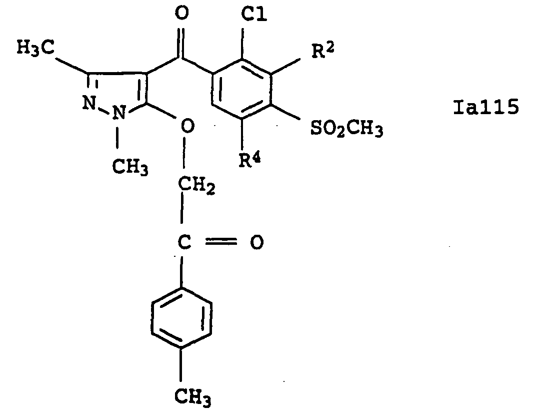
- Ebenso insbesondere außerordentlich bevorzugt sind die Verbindungen Ia116; insbesondere die Verbindungen Ia116.1-Ia116.164, die sich von den Verbindungen Ia1.1-Ia1.164 dadurch unterscheiden, daß R⁷ für 1-(Phenylcarbonyl)-eth-1-yl steht: 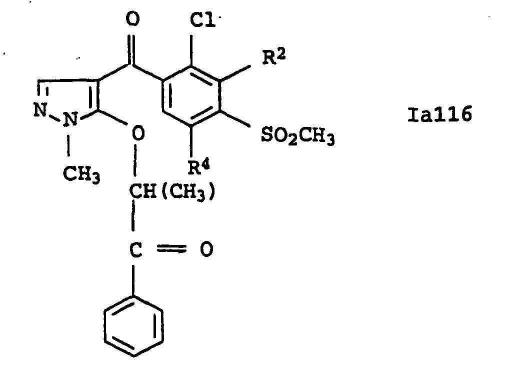
- Ebenso insbesondere außerordentlich bevorzugt sind die Verbindungen Ia117; insbesondere die Verbindungen Ia117.1-Ia117.164, die sich von den Verbindungen Ia1.1-Ia1.164 dadurch unterscheiden, daß R⁶ für Ethyl und R⁷ für 1-(Phenylcarbonyl)-eth-1-yl stehen: 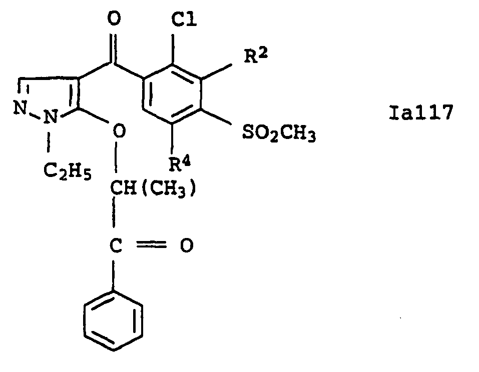
- Ebenso insbesondere außerordentlich bevorzugt sind die Verbindungen Ia118; insbesondere die Verbindungen Ia118.1-Ia118.164, die sich von den Verbindungen Ia1.1-Ia1.164 dadurch unterscheiden, daß R⁷ für 1-(Phenylcarbonyl)-eth-1-yl und R⁸ für Methyl stehen: 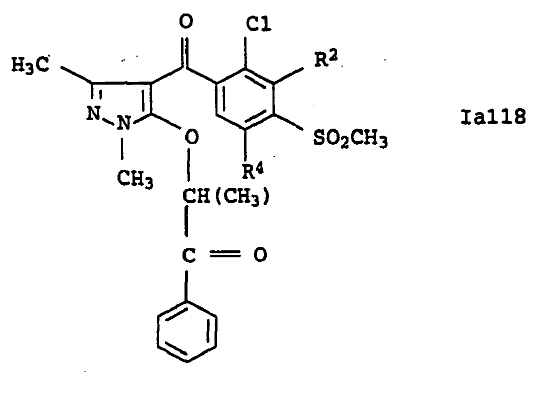
- Ebenso insbesondere außerordentlich bevorzugt sind die Verbindungen Ia119; insbesondere die Verbindungen Ia119.1-Ia119.164, die sich von den Verbindungen Ia1.1-Ia1.164 dadurch unterscheiden, daß R⁷ für Phenylcarbonyl steht : 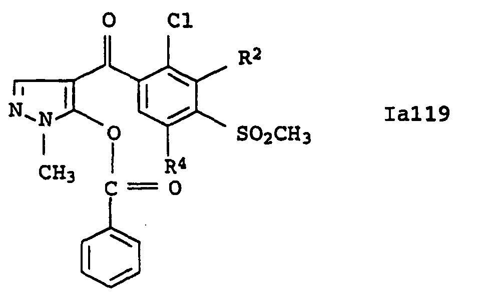
- Ebenso insbesondere außerordentlich bevorzugt sind die Verbindungen Ia120; insbesondere die Verbindungen Ia120.1-Ia120.164, die sich von den Verbindungen Ia1.1-Ia1.164 dadurch unterscheiden, daß R⁶ für Ethyl und R⁷ für Phenylcarbonyl stehen: 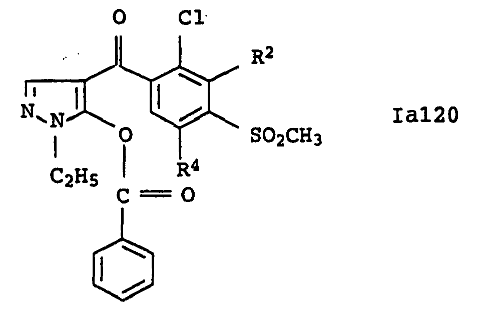
- Ebenso insbesondere außerordentlich bevorzugt sind die Verbindungen Ia121; insbesondere die Verbindungen Ia121.1-Ia121.164, die sich von den Verbindungen Ia1.1-Ia1.164 dadurch unterscheiden, daß R⁷ für Phenylcarbonyl und R⁸ für Methyl stehen: 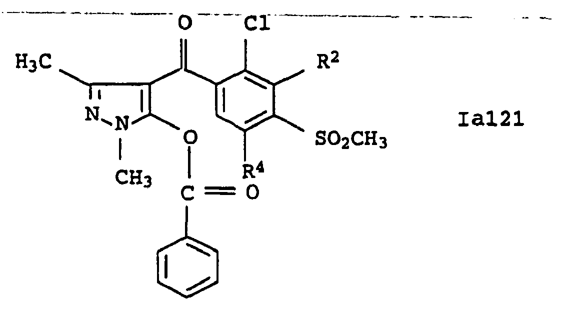
- Ebenso insbesondere außerordentlich bevorzugt sind die Verbindungen Ia122; insbesondere die Verbindungen Ia122.1-Ia122.164, die sich von den Verbindungen Ia1.1-Ia1.164 dadurch unterscheiden, daß R⁷ für 4-Methylphenylcarbonyl steht: 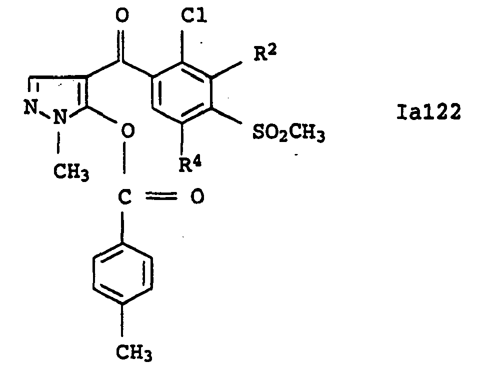
- Ebenso insbesondere außerordentlich bevorzugt sind die Verbindungen Ia123; insbesondere die Verbindungen Ia123.1-Ia123.164, die sich von den Verbindungen Ia1.1-Ia1.164 dadurch unterscheiden, daß R⁶ für Ethyl und R⁷ für 4-Methylphenylcarbonyl stehen: 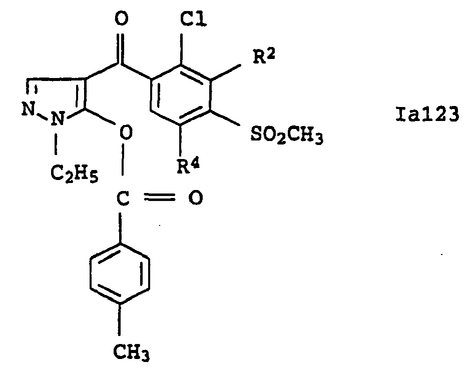
- Ebenso insbesondere außerordentlich bevorzugt sind die Verbindungen Ia124; insbesondere die Verbindungen Ia124.1-Ia124.164, die sich von den Verbindungen Ia1.1-Ia1.164 dadurch unterscheiden, daß R⁷ für 4-Methylphenylcarbonyl und R⁸ für Methyl stehen: 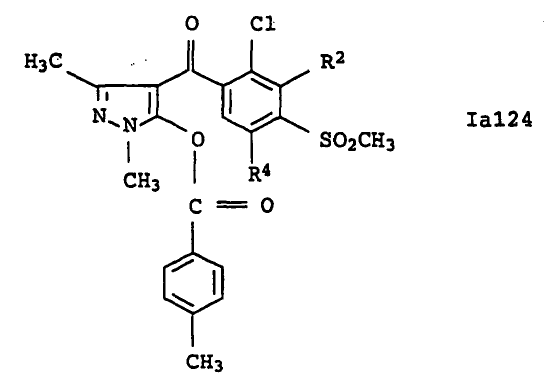
- Ebenso insbesondere außerordentlich bevorzugt sind die Verbindungen Ia125; insbesondere die Verbindungen Ia125.1-Ia125.164, die sich von den Verbindungen Ia1.1-Ia1.164 dadurch unterscheiden, daß R⁷ für 4-Chlorphenylcarbonyl steht: 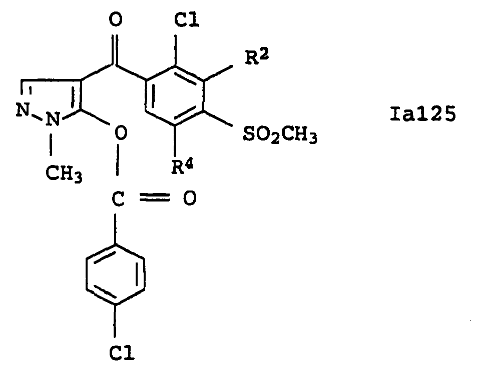
- Ebenso insbesondere außerordentlich bevorzugt sind die Verbindungen Ia126; insbesondere die Verbindungen Ia126.1-Ia126.164, die sich von den Verbindungen Ia1.1-Ia1.164 dadurch unterscheiden, daß R⁶ für Ethyl und R⁷ für 4-Chlorphenylcarbonyl stehen: 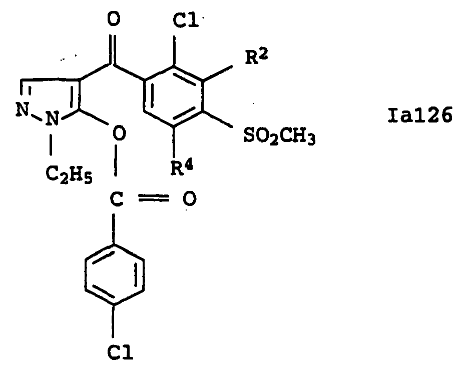
- Ebenso insbesondere außerordentlich bevorzugt sind die Verbindungen Ia127; insbesondere die Verbindungen Ia127.1-Ia127.164, die sich von den Verbindungen Ia1.1-Ia1.164 dadurch unterscheiden, daß R⁷ für 4-Chlorphenylcarbonyl und R⁸ für Methyl stehen: 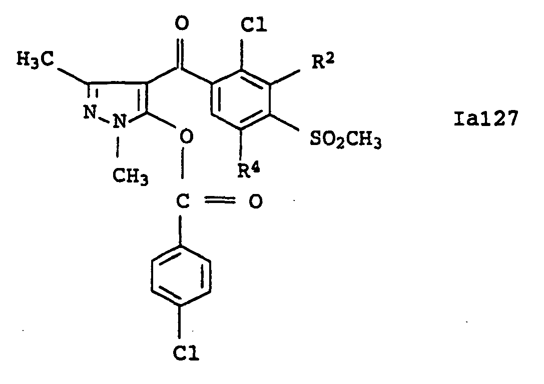
- Ebenso insbesondere außerordentlich bevorzugt sind die Verbindungen Ia128; insbesondere die Verbindungen Ia128.1-Ia128.164, die sich von den Verbindungen Ia1.1-Ia1.164 dadurch unterscheiden, daß R⁷ für 4-Methoxyphenylcarbonyl steht: 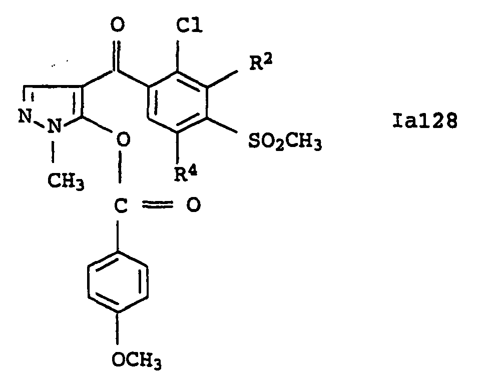
- Ebenso insbesondere außerordentlich bevorzugt sind die Verbindungen Ia129; insbesondere die Verbindungen Ia129.1-Ia129.164, die sich von den Verbindungen Ia1.1-Ia1.164 dadurch unterscheiden, daß R⁶ für Ethyl und R⁷ für 4-Methoxyphenylcarbonyl stehen: 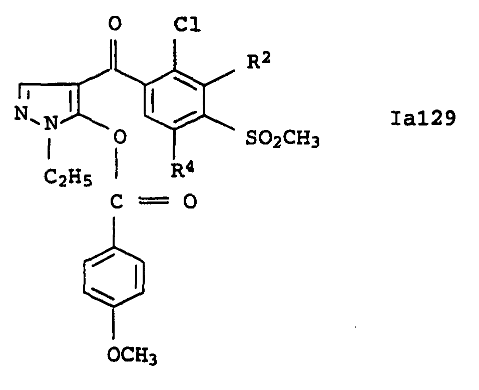
- Ebenso insbesondere außerordentlich bevorzugt sind die Verbindungen Ia130; insbesondere die Verbindungen Ia130.1-Ia130.164, die sich von den Verbindungen Ia1.1-Ia1.164 dadurch unterscheiden, daß R⁷ für 4-Methoxyphenylcarbonyl und R⁸ für Methyl stehen: 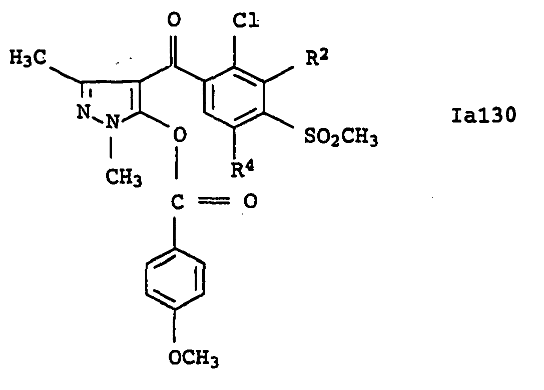
- Ebenso insbesondere außerordentlich bevorzugt sind die Verbindungen Ia131; insbesondere die Verbindungen Ia131.1-Ia131.164, die sich von den Verbindungen Ia1.1-Ia1.164 dadurch unterscheiden, daß R⁷ für 4-Trifluormethylphenylcarbonyl steht: 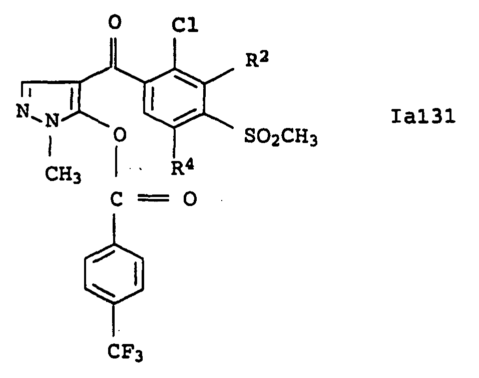
- Ebenso insbesondere außerordentlich bevorzugt sind die Verbindungen Ia132; insbesondere die Verbindungen Ia132.1-Ia132.164, die-sich von den Verbindungen Ia1.1-Ia1.164 dadurch unterscheiden, daß R⁶ für Ethyl und R⁷ für 4-Trifluormethylphenylcarbonyl stehen: 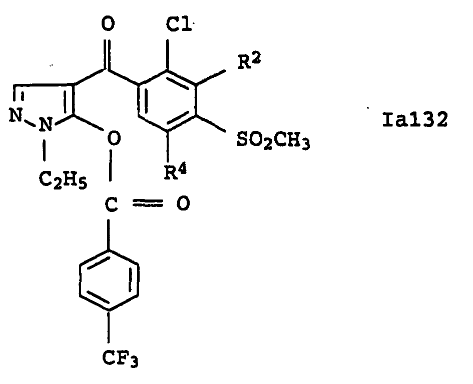
- Ebenso insbesondere außerordentlich bevorzugt sind die Verbindungen Ia133; insbesondere die Verbindungen Ia133.1-Ia133.164, die sich von den Verbindungen Ia1.1-Ia1.164 dadurch unterscheiden, daß R⁷ für 4-Trifluormethylphenylcarbonyl und R⁸ für Methyl stehen: 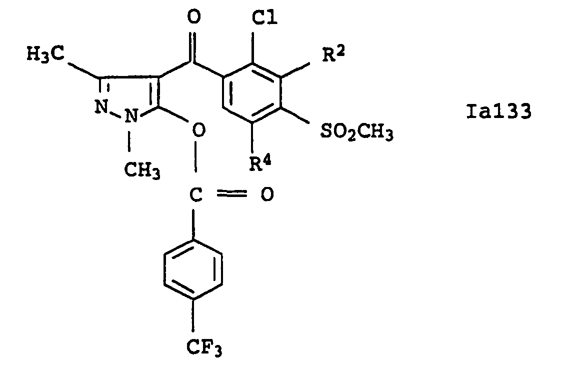
- Ebenso insbesondere außerordentlich bevorzugt sind die Verbindungen Ia134; insbesondere die Verbindungen Ia134.1-Ia134.164, die sich von den Verbindungen Ia1.1-Ia1.164 dadurch unterscheiden, daß R⁷ für 2,4-Dichlorphenylcarbonyl steht: 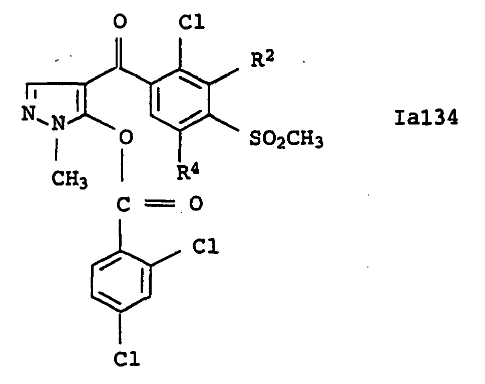
- Ebenso insbesondere außerordentlich bevorzugt sind die Verbindungen Ia135; insbesondere die Verbindungen Ia135.1-Ia135.164, die sich von den Verbindungen Ia1.1-Ia1.164 dadurch unterscheiden, daß R⁶ für Ethyl und R⁷ für 2,4 - Dichlorphenylcarbonyl stehen: 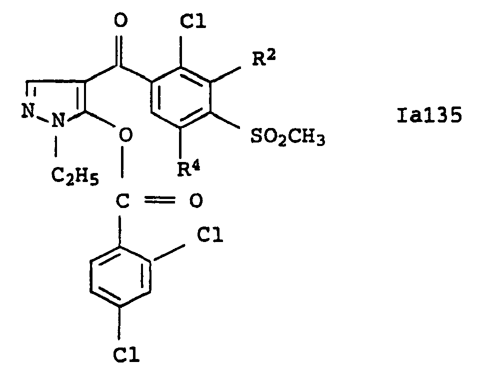
- Ebenso insbesondere außerordentlich bevorzugt sind die Verbindungen Ia136; insbesondere die Verbindungen Ia136.1-Ia136.164, die sich von den Verbindungen Ia1.1-Ia1.164 dadurch unterscheiden, daß R⁷ für 2,4-Dichlorphenylcarbonyl und R⁸ für Methyl stehen: 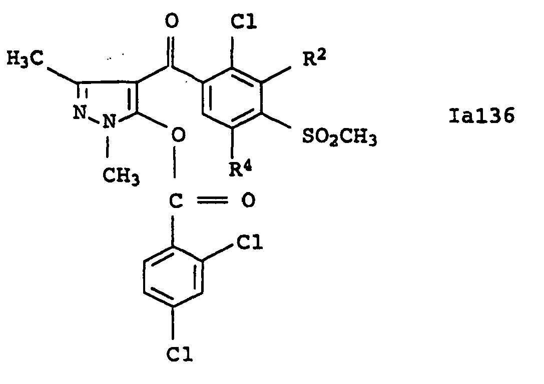
- Ebenso insbesondere außerordentlich bevorzugt sind die Verbindungen Ia137; insbesondere die Verbindungen Ia137.1-Ia137.164, die sich von den Verbindungen Ia1.1-Ia1.164 dadurch unterscheiden, daß R³ für Chlor steht: 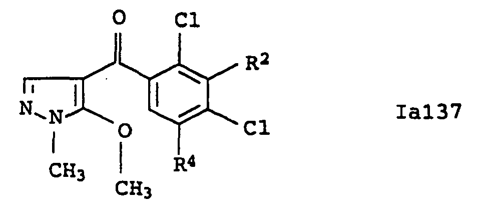
- Ebenso insbesondere außerordentlich bevorzugt sind die Verbindungen Ia138; insbesondere die Verbindungen Ia138.1-Ia138.164, die sich von den Verbindungen Ia1.1-Ia1.164 dadurch unterscheiden, daß R³ für Chlor und R⁶ für Ethyl stehen: 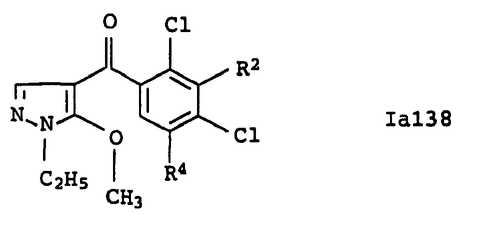
- Ebenso insbesondere außerordentlich bevorzugt sind die Verbindungen Ia139; insbesondere die Verbindungen Ia139.1-Ia139.164, die sich von den Verbindungen Ia1.1-Ial1164 dadurch unterscheiden, daß R³ für Chlor und R⁶ für n-Propyl stehen: 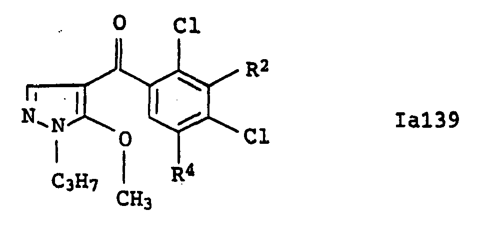
- Ebenso insbesondere außerordentlich bevorzugt sind die Verbindungen Ia140; insbesondere die Verbindungen Ia140.1-Ia140.164, die sich von den Verbindungen Ia1.1-Ia1.164 dadurch unterscheiden, daß R³ für Chlor und R⁶ für iso-Butyl stehen: 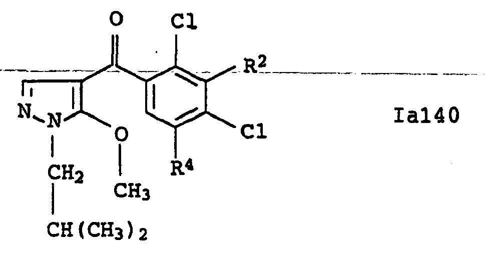
- Ebenso insbesondere außerordentlich bevorzugt sind die Verbindungen Ia141; insbesondere die Verbindungen Ia141.1-Ia141.164, die sich von den Verbindungen Ia1.1-Ia1.164 dadurch unterscheiden, daß R³ für Chlor und R⁸ für Methyl stehen: 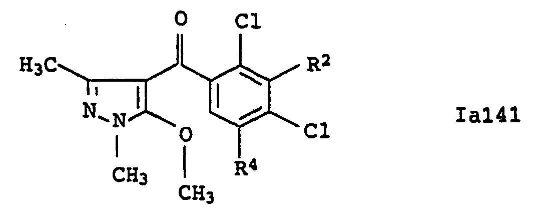
- Ebenso insbesondere außerordentlich bevorzugt sind die Verbindungen Ia142; insbesondere die Verbindungen Ia142.1-Ia142.164, die sich von den Verbindungen Ia1.1-Ia1.164 dadurch unterscheiden, daß R³ für Chlor und R⁷ für Ethyl stehen: 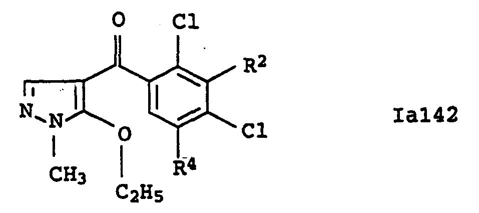
- Ebenso insbesondere außerordentlich bevorzugt sind die Verbindungen Ia143; insbesondere die Verbindungen Ia143.1-Ia143.164, die sich von den Verbindungen Ia1.1-Ia1.164 dadurch unterscheiden, daß R³ für Chlor, R⁶ und R⁷ für Ethyl stehen: 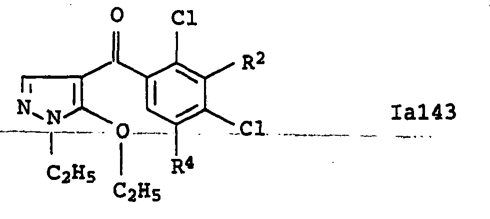
- Ebenso insbesondere außerordentlich bevorzugt sind die Verbindungen Ia144; insbesondere die Verbindungen Ia144.1-Ia144.164, die sich von den Verbindungen Ia1.1-Ia1.164 dadurch unterscheiden, daß R³ für Chlor, R⁶ für n-Propyl und R⁷ für Ethyl stehen: 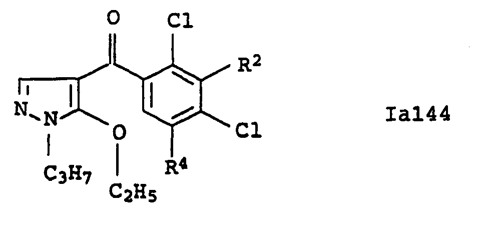
- Ebenso insbesondere außerordentlich bevorzugt sind die Verbindungen Ia145; insbesondere die Verbindungen Ia145.1-Ia145.164, die sich von den Verbindungen Ia1.1-Ia1.164 dadurch unterscheiden, daß R³ für Chlor, R⁶ für iso-Butyl und R⁷ für Ethyl stehen: 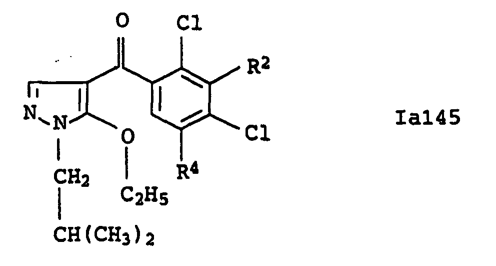
- Ebenso insbesondere außerordentlich bevorzugt sind die Verbindungen Ia146; insbesondere die Verbindungen Ia146.1-Ia146.164, die sich von den Verbindungen Ia1.1-Ia1.164 dadurch unterscheiden, daß R³ für Chlor, R⁷ für Ethyl und R⁸ für Methyl stehen: 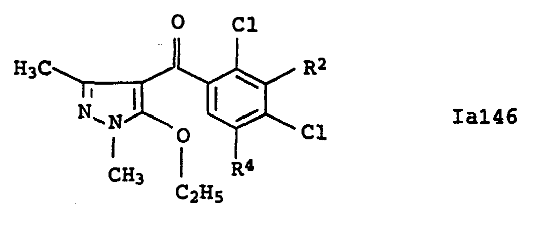
- Ebenso insbesondere außerordentlich bevorzugt sind die Verbindungen Ia147; insbesondere die Verbindungen Ia147.1-Ia147.164, die sich von den Verbindungen Ia1.1-Ia1.164 dadurch unterscheiden, daß R³ für Chlor und R⁷ für Cyanomethyl stehen: 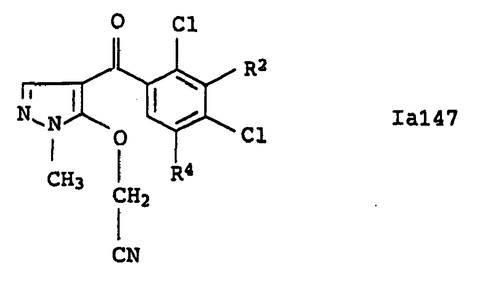
- Ebenso insbesondere außerordentlich bevorzugt sind die Verbindungen Ia148; insbesondere die Verbindungen Ia148.1-Ia148.164, die sich von den Verbindungen Ia1.1-Ia1.164 dadurch unterscheiden, daß R³ für Chlor, R⁶ für Ethyl und R⁷ für Cyanomethyl stehen: 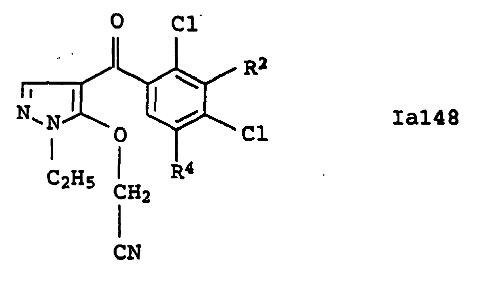
- Ebenso insbesondere außerordentlich bevorzugt sind die Verbindungen Ia149; insbesondere die Verbindungen Ia149.1-Ia149.164, die sich von den Verbindungen Ia1.1-Ia1.164 dadurch unterscheiden, daß R³ für Chlor, R⁷ für Cyanomethyl und R⁸ für Methyl stehen: 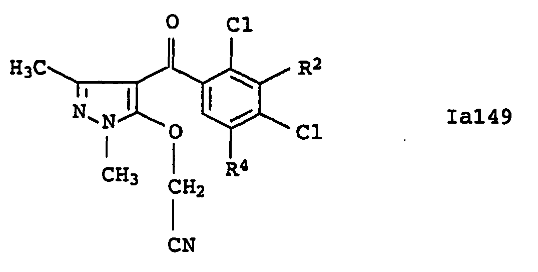
- Ebenso insbesondere außerordentlich bevorzugt sind die Verbindungen Ia150; insbesondere die Verbindungen Ia150.1-Ia150.164, die sich von den Verbindungen Ia1.1-Ia1.164 dadurch unterscheiden, daß R³ für Chlor und R⁷ für Methoxymethyl stehen: 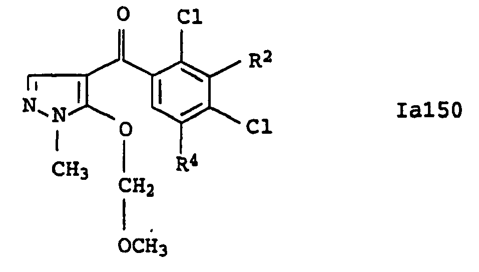
- Ebenso insbesondere außerordentlich bevorzugt sind die Verbindungen Ia151; insbesondere die Verbindungen Ia151.1-Ia151.164, die sich von den Verbindungen Ia1.1-Ia1.164 dadurch unterscheiden, daß R³ für Chlor, R⁶ für Ethyl und R⁷ für Methoxymethyl stehen: 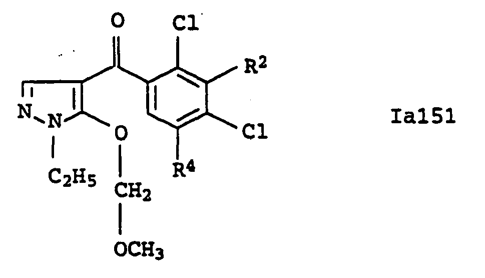
- Ebenso insbesondere außerordentlich bevorzugt sind die Verbindungen Ia152; insbesondere die Verbindungen Ia152.1-Ia152.164, die sich von den Verbindungen Ia1.1-Ia1.164 dadurch unterscheiden, daß R³ für Chlor, R⁷ für Methoxymethyl und R⁸ für Methyl stehen: 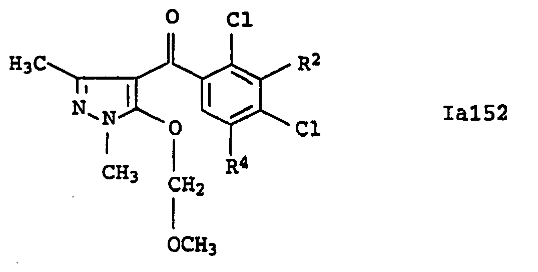
- Ebenso insbesondere außerordentlich bevorzugt sind die Verbindungen Ia153; insbesondere die Verbindungen Ia153.1-Ia153.164, die sich von den Verbindungen Ia1.1-Ia1.164 dadurch unterscheiden, daß R³ für Chlor und R⁷ für Methylcarbonyloxymethyl stehen: 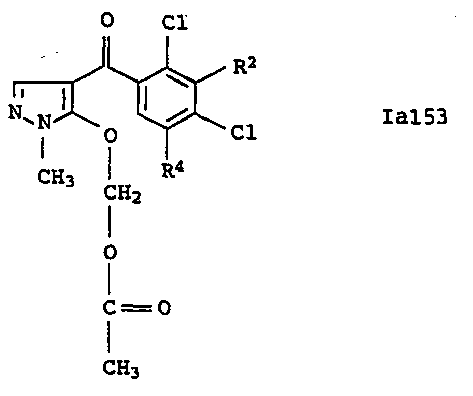
- Ebenso insbesondere außerordentlich bevorzugt sind die Verbindungen Ia154; insbesondere die Verbindungen Ia154.1-Ia54.164, die sich von den Verbindungen Ia1.1-Ia1.164 dadurch unterscheiden, daß R³ für Chlor, R⁶ für Ethyl und R⁷ für Methylcarbonyloxymethyl stehen: 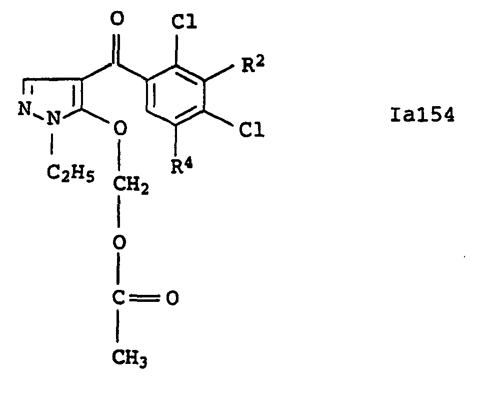
- Ebenso insbesondere außerordentlich bevorzugt sind die Verbindungen Ia155; insbesondere die Verbindungen Ia155.1-Ia155.164, die'sich von den Verbindungen Ia1.1-Ia1.164 dadurch unterscheiden, daß R³ für Chlor, R⁷ für Methylcarbonyloxymethyl und R⁸ für Methyl stehen: 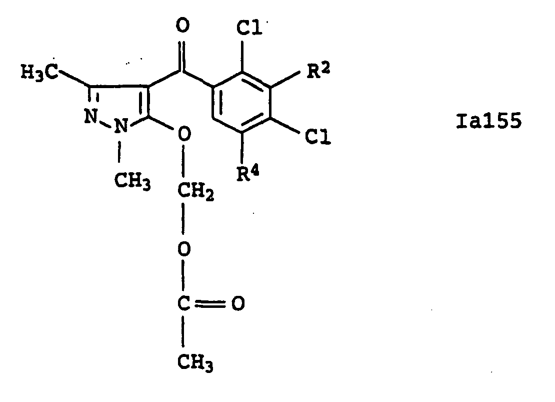
- Ebenso insbesondere außerordentlich bevorzugt sind die Verbindungen Ia156; insbesondere die Verbindungen Ia156.1-Ia156.164, die sich von den Verbindungen Ia1.1-Ia1.164 dadurch unterscheiden, daß R³ für Chlor und R⁷ für tert. -Butylcarbonyloxymethyl stehen: 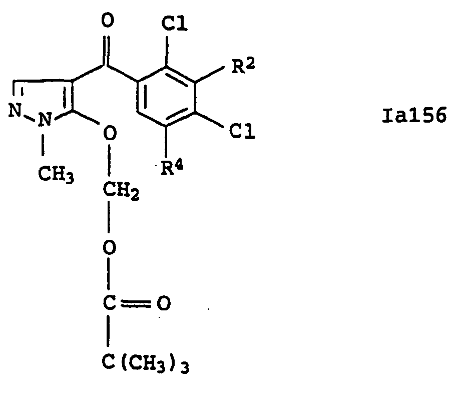
- Ebenso insbesondere außerordentlich bevorzugt sind die Verbindungen Ia157; insbesondere die Verbindungen Ia157.1-Ia157.164, die sich von den Verbindungen Ia1.1-Ia1.164 dadurch unterscheiden, daß R³ für Chlor, R⁶ für Ethyl und R⁷ für tert.-Butylcarbonyloxymethyl stehen: 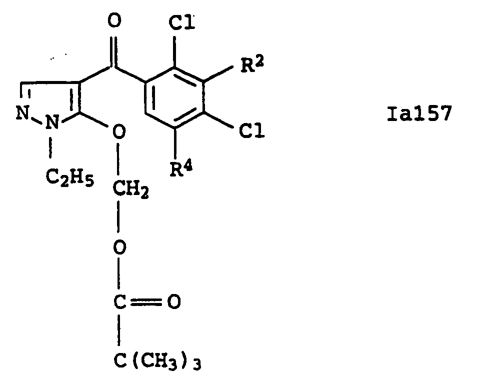
- Ebenso insbesondere außerordentlich bevorzugt sind die Verbindungen Ia158; insbesondere die Verbindungen Ia158.1-Ia158.164, die sich von den Verbindungen Ia1.1-Ia1.164 dadurch unterscheiden, daß R³ für Chlor, R⁷ für tert.-Butylcarbonyloxymethyl und R⁸ für Methyl stehen: 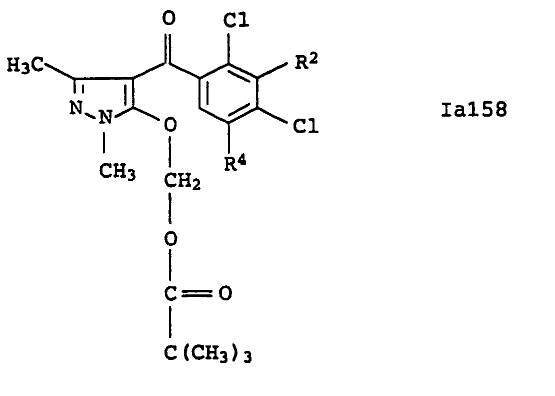
- Ebenso insbesondere außerordentlich bevorzugt sind die Verbindungen Ia159: insbesondere die Verbindungen Ia159.1-Ia159.164, die sich von den Verbindungen Ia1.1-Ia1.164 dadurch unterscheiden, daß R³ für Chlor und R⁷ für Methoxycarbonylmethyl stehen: 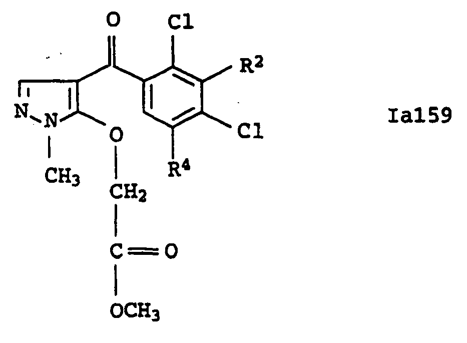
- Ebenso insbesondere außerordentlich bevorzugt sind die Verbindungen Ia160; insbesondere die Verbindungen Ia160.1-Ia160.164, die sich von den Verbindungen Ia1.1-Ia1.164 dadurch unterscheiden, daß R³ für Chlor, R⁶ für Ethyl und R⁷ für Methoxycarbonylmethyl stehen: 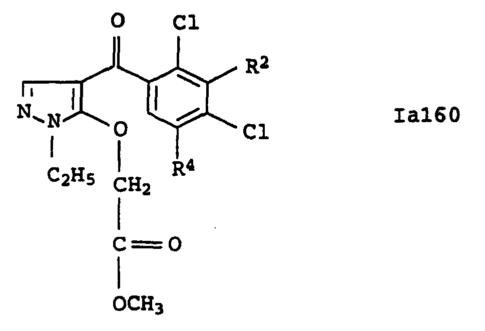
- Ebenso insbesondere außerordentlich bevorzugt sind die Verbindungen Ia161; insbesondere die Verbindungen Ia161.1-Ia161.164, die sich von den Verbindungen Ia1.1-Ia1.164 dadurch unterscheiden, daß R³ für Chlor, R⁷ für Methoxycarbonylmethyl und R⁸ für Methyl stehen: 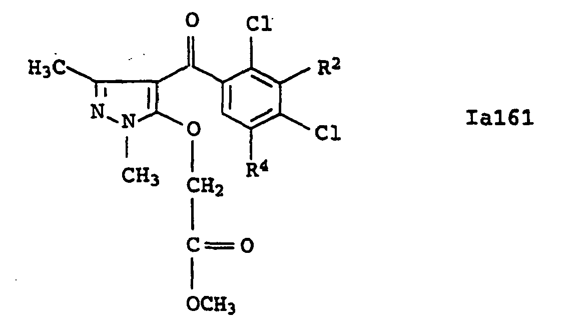
- Ebenso insbesondere außerordentlich bevorzugt sind die Verbindungen Ia162; insbesondere die Verbindungen Ia162.1-Ia162.164, die sich von den Verbindungen Ia1.1-Ia1.164 dadurch unterscheiden, daß R³ für Chlor und R⁷ für Ethoxycarbonylmethyl stehen: 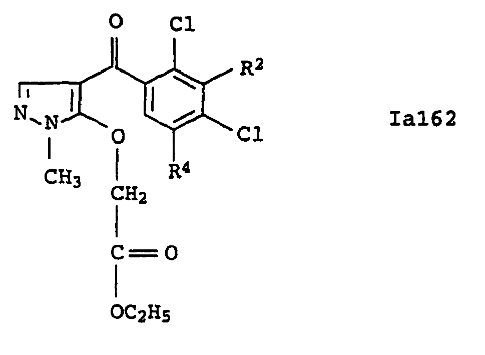
- Ebenso insbesondere außerordentlich bevorzugt sind die Verbindungen Ia163; insbesondere die Verbindungen Ia163.1-Ia163.164, die-sich von den Verbindungen Ia1.1-Ia1.164 dadurch unterscheiden, daß R³ für Chlor, R⁶ für Ethyl und R⁷ für Ethoxycarbonylmethyl stehen: 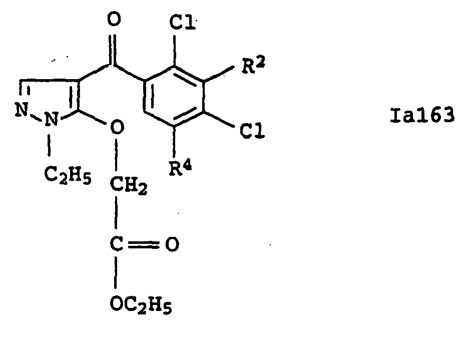
- Ebenso insbesondere außerordentlich bevorzugt sind die Verbindungen Ia164; insbesondere die Verbindungen Ia164.1-Ia164.164, die sich von den Verbindungen Ia1.1-Ia1.164 dadurch unterscheiden, daß R³ für Chlor, R⁷ für Ethoxycarbonylmethyl und R⁸ für Methyl stehen 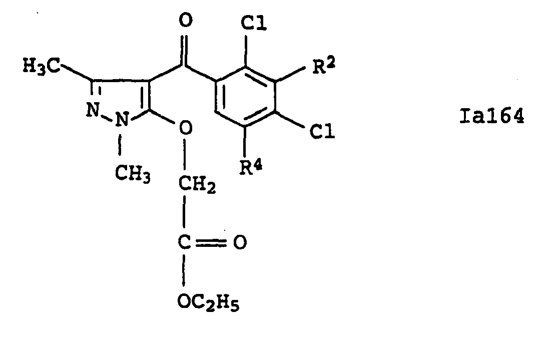
- Ebenso insbesondere außerordentlich bevorzugt sind die Verbindungen Ia165; insbesondere die Verbindungen Ia165.1-Ia165.164, die sich von den Verbindungen Ia1.1-Ia1.164 dadurch unterscheiden, daß R³ für Chlor und R⁷ für 1-Methoxycarbonyl-eth-1-yl stehen: 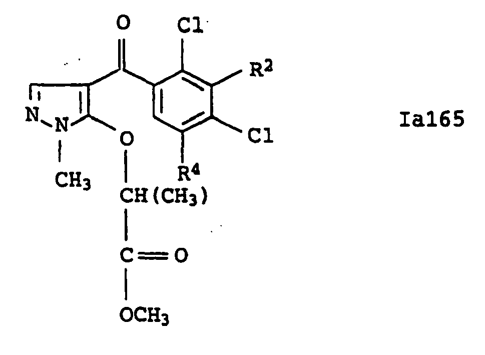
- Ebenso insbesondere außerordentlich bevorzugt sind die Verbindungen Ia166; insbesondere die Verbindungen Ia166.1-Ia166.164, die sich von den Verbindungen Ia1.1-Ia1.164 dadurch unterscheiden, daß R³ für Chlor, R⁶ für Ethyl und R⁷ für 1-Methoxyearbonyl-eth-1-yl stehen: 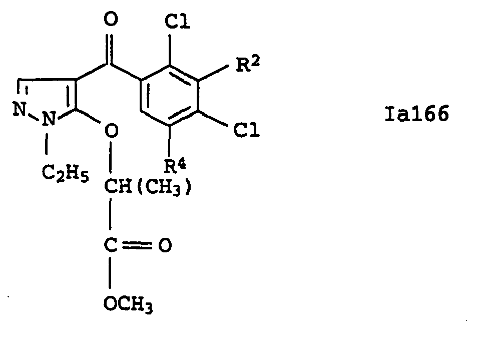
- Ebenso insbesondere außerordentlich bevorzugt sind die Verbindungen Ia167; insbesondere die Verbindungen Ia167.1-Ia167.164, die sich von den Verbindungen Ia1.1-Ia1.164 dadurch unterscheiden, daß R³ für Chlor, R⁷ für 1-Methoxycarbonyl-eth-1-yl und R⁸ für Methyl stehen: 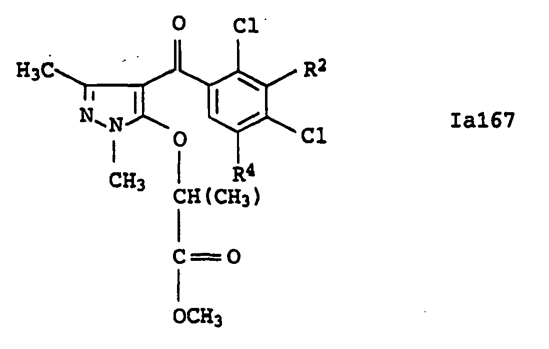
- Ebenso insbesondere außerordentlich bevorzugt sind die Verbindungen Ia168; insbesondere die Verbindungen Ia168.1-Ia168.164, die sich von den Verbindungen Ia1.1-Ia1.164 dadurch unterscheiden, daß R³ für Chlor und R⁷ für 1-Ethoxycarbonyl-eth-1-yl stehen: 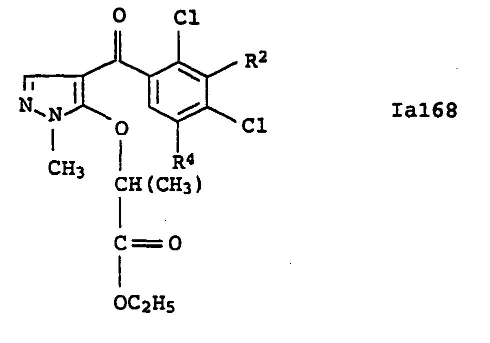
- Ebenso insbesondere außerordentlich bevorzugt sind die Verbindungen Ia169; insbesondere die Verbindungen Ia169.1-Ia169.164, die sich von den Verbindungen Ia1.1-Ia1.164 dadurch unterscheiden, daß R³ für Chlor, R⁶ für Ethyl und R⁷ für 1-Ethoxycarbonyl-eth-1-yl stehen: 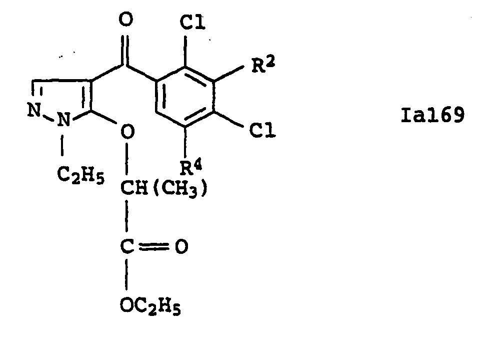
- Ebenso insbesondere außerordentlich bevorzugt sind die Verbindungen Ia170; insbesondere die Verbindungen Ia170.1-Ia170.164, die sich von den Verbindungen Ia1.1-Ia1.164 dadurch unterscheiden, daß R³ für Chlor, R⁷ für 1-Ethoxycarbonyl-eth-1-yl und R⁸ für Methyl stehen: 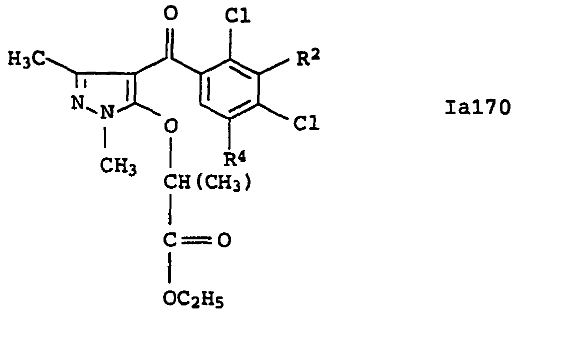
- Ebenso insbesondere außerordentlich bevorzugt sind die Verbindungen Ia171; insbesondere die Verbindungen Ia171.1-Ia171.164, die sich von den Verbindungen Ia1.1-Ia1.164 dadurch unterscheiden, daß R³ für Chlor und R⁷ für 2-Methoxy-eth-1-oxycarbonylmethyl stehen: 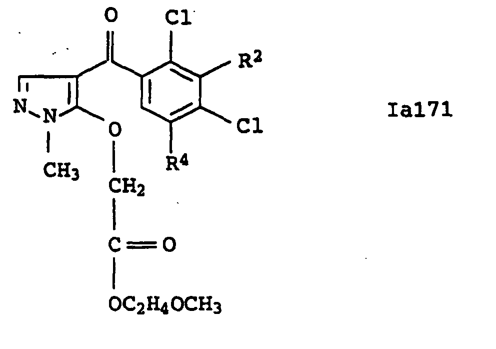
- Ebenso insbesondere außerordentlich bevorzugt sind die Verbindungen Ia172; insbesondere die Verbindungen Ia172.1-Ia172.164, die sich von den Verbindungen Ia1.1-Ia1.164 dadurch unterscheiden, daß R³ für Chlor, R⁶ für Ethyl und R⁷ für 2-Methoxy-eth-1-oxycarbonylmethyl stehen: 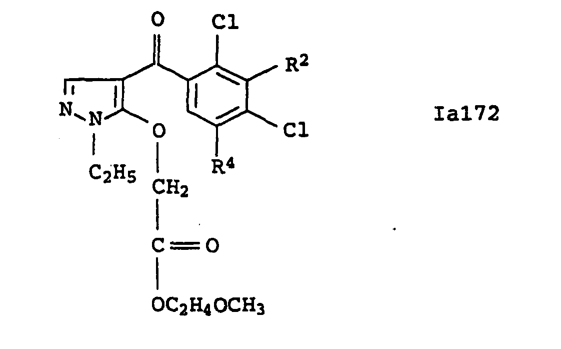
- Ebenso insbesondere außerordentlich bevorzugt sind die Verbindungen Ia173: insbesondere die Verbindungen Ia173.1-Ia173.164, die sich von den Verbindungen Ia1.1-Ia1.164 dadurch unterscheiden, daß R³ für Chlor, R⁷ für 2-Methoxy-eth-1-oxycarbonylmethyl und R⁸ für Methyl stehen: 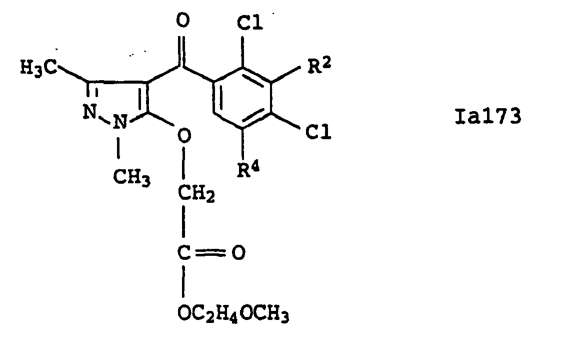
- Ebenso insbesondere außerordentlich bevorzugt sind die Verbindungen Ia174; insbesondere die Verbindungen Ia174.1-Ia174.164, die sich von den Verbindungen Ia1.1-Ia1.164 dadurch unterscheiden, daß R³ für Chlor und R⁷ für 2-Dimethylamino-eth-1-oxycarbonylmethyl stehen: 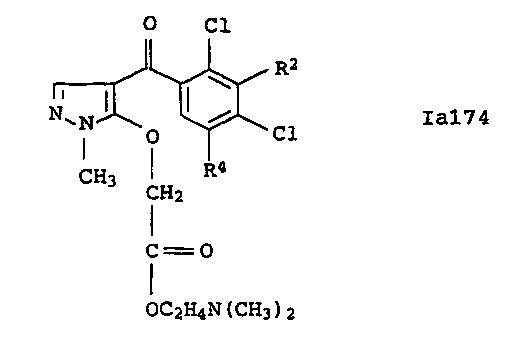
- Ebenso insbesondere außerordentlich bevorzugt sind die Verbindungen Ia175; insbesondere die Verbindungen Ia175.1-Ia175.164, die sich von den Verbindungen Ia1.1-Ia1.164 dadurch unterscheiden, daß R³ für Chlor, R⁶ für Ethyl und R⁷ für 2-Dimethylamino-eth-1-oxycarbonylmethy stehen: 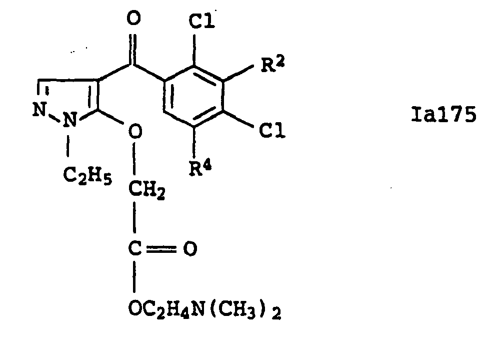
- Ebenso insbesondere außerordentlich bevorzugt sind die Verbindungen Ia176; insbesondere die Verbindungen Ia176.1-Ia176.164, die sich von den Verbindungen Ia1.1-Ia1.164 dadurch unterscheiden, daß R³ für Chlor, R⁷ für 2-Dimethylamino-eth-1-oxycarbonylmethyl und R⁸ für Methyl stehen: 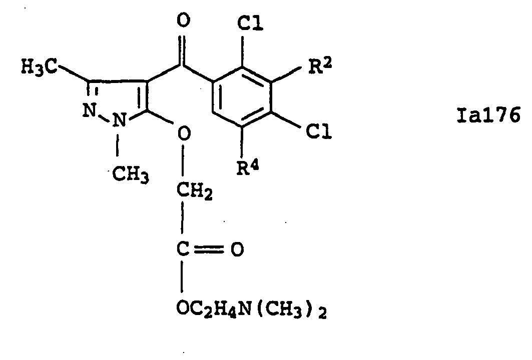
- Ebenso insbesondere außerordentlich bevorzugt sind die Verbindungen Ia177; insbesondere die Verbindungen Ia177.1-Ia177.164, die sich von den Verbindungen Ia1.1-Ia1.164 dadurch unterscheiden, daß R³ für Chlor und R⁷ für Methylaminocarbonylmethyl stehen: 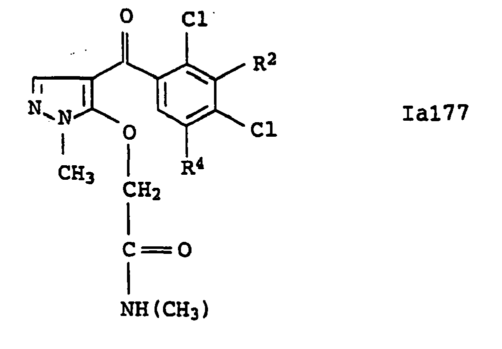
- Ebenso insbesondere außerordentlich bevorzugt sind die Verbindungen Ia178; insbesondere die Verbindungen Ia178.1-Ia178.164, die sich von den Verbindungen Ia1.1-Ia1.164 dadurch unterscheiden, daß R³ für Chlor, R⁶ für Ethyl und R⁷ für Methylamiaocarbonylmethyl stehen: 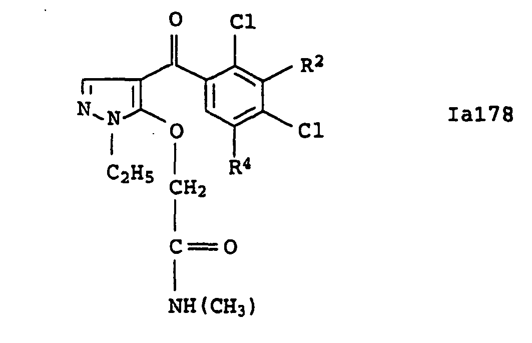
- Ebenso insbesondere außerordentlich bevorzugt sind die Verbindungen Ia179; insbesondere die Verbindungen Ia179.1-Ia179.164, die sich von den Verbindungen Ia1.1-Ia1.164 dadurch unterscheiden, daß R³ für Chlor, R⁷ für Methylaminocarbonylmethyl und R⁸ für Methyl stehen: 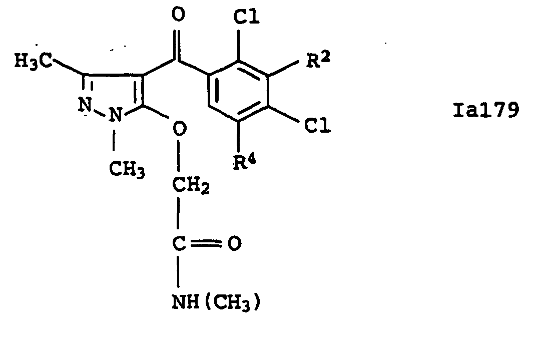
- Ebenso insbesondere außerordentlich bevorzugt sind die Verbindungen Ia180; insbesondere die Verbindungen Ia180.1-Ia180.164, die sich von den Verbindungen Ia1.1-Ia1.164 dadurch unterscheiden, daß R³ für Chlor und R⁷ für Ethylaminocarbonylmethyl stehen: 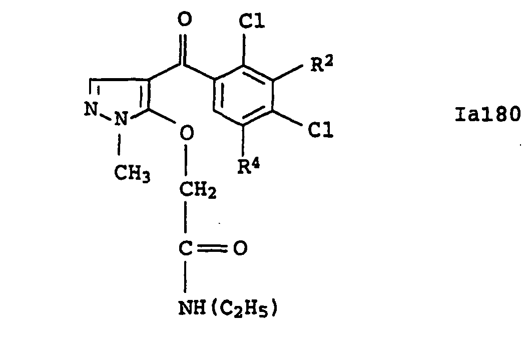
- Ebenso insbesondere außerordentlich bevorzugt sind die Verbindungen Ia181; insbesondere die Verbindungen Ia181.1-Ia181.164, die sich von den Verbindungen Ia1.1-Ia1.164 dadurch unterscheiden, daß R³ für Chlor, R⁶ für Ethyl und R⁷ für Ethylaminocarbonylmethyl stehen: 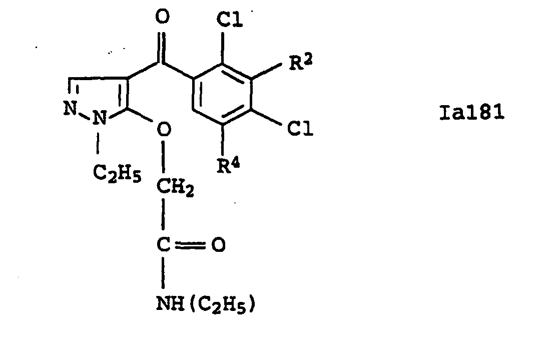
- Ebenso insbesondere außerordentlich bevorzugt sind die Verbindungen Ia182; insbesondere die Verbindungen Ia182.1-Ia182.164, die sich von den Verbindungen Ia1.1-Ia1.164 dadurch unterscheiden, daß R³ für Chlor, R⁷ für Ethylaminocarbonylmethyl und R⁸ für Methyl stehen: 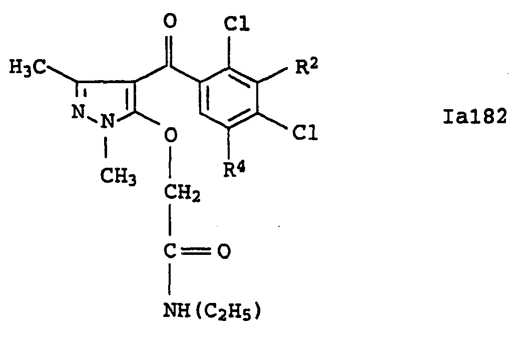
- Ebenso insbesondere außerordentlich bevorzugt sind die Verbindungen Ia183; insbesondere die Verbindungen Ia183.1-Ia183.164, die sich von den Verbindungen Ia1.1-Ia1.164 dadurch unterscheiden, daß R³ für Chlor und R⁷ für Dimethylaminocarbonylmethyl stehen: 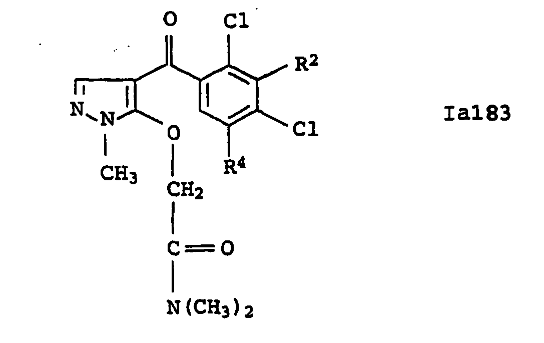
- Ebenso insbesondere außerordentlich bevorzugt sind die Verbindungen Ia184; insbesondere die Verbindungen Ia184.1-1a184.164, die sich von den Verbindungen Ia1.1-Ia1.164 dadurch unterscheiden, daß R³ für Chlor, R⁶ für Ethyl und R⁷ für Dimethylaminocarbonylmethyl stehen: 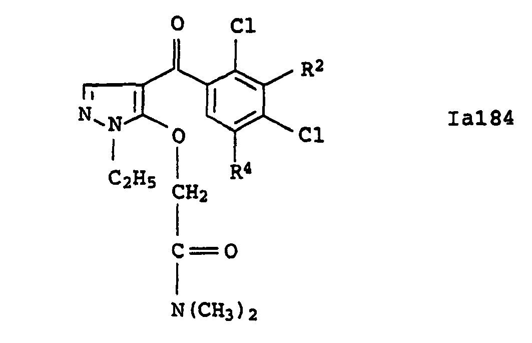
- Ebenso insbesondere außerordentlich bevorzugt sind die Verbindungen Ia185; insbesondere die Verbindungen Ia185.1-Ia185.164, die sich von den Verbindungen Ia1.1-Ia1.164 dadurch unterscheiden, daß R³ für Chlor, R⁷ für Dimethylaminocarbonylmethyl und R⁸ für Methyl stehen: 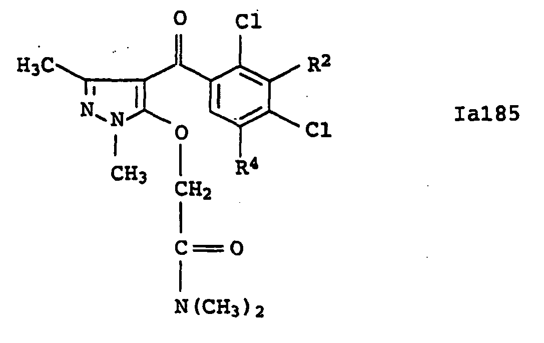
- Ebenso insbesondere außerordentlich bevorzugt sind die Verbindungen Ia186; insbesondere die Verbindungen Ia186.1-Ia186.164, die sich von den Verbindungen Ia1.1-Ia1.164 dadurch unterscheiden, daß R³ für Chlor und R⁷ für Diethylaminocarbonylmethyl stehen: 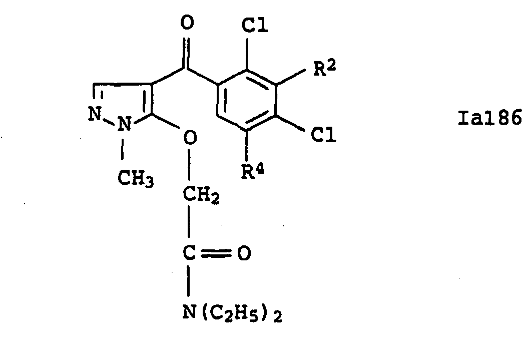
- Ebenso insbesondere außerordentlich bevorzugt sind die Verbindungen Ia187; insbesondere die Verbindungen Ia187.1-Ia187.164, die sich von den Verbindungen Ia1.1-Ia1.164 dadurch unterscheiden, daß R³ für Chlor, R⁶ für Ethyl und R⁷ für Diethylaminocarbonylmethyl stehen: 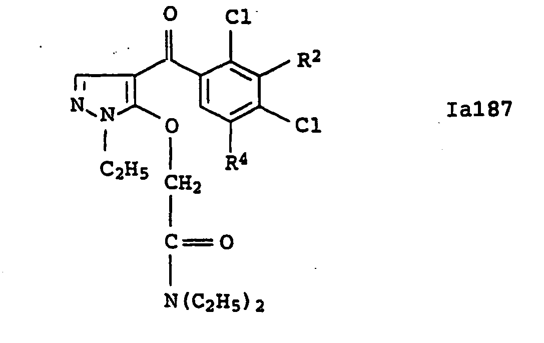
- Ebenso insbesondere außerordentlich bevorzugt sind die Verbindungen Ia188; insbesondere die Verbindungen Ia188.1-Ia188.164, die sich von den Verbindungen Ia1.1-Ia1.164 dadurch unterscheiden, daß R³ für Chlor, R⁷ für Diethylaminocarbonylmethyl und R⁸ für Methyl stehen: 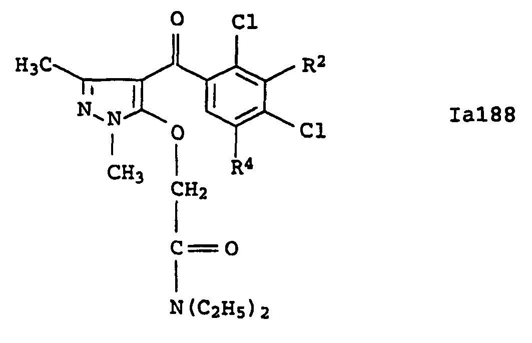
- Ebenso insbesondere außerordentlich bevorzugt sind die Verbindungen Ia189; insbesondere die Verbindungen Ia189.1-Ia189.164, die sich von den Verbindungen Ia1.1-Ia1.164 dadurch unterscheiden, daß R³ für Chlor und R⁷ für Allyl stehen: 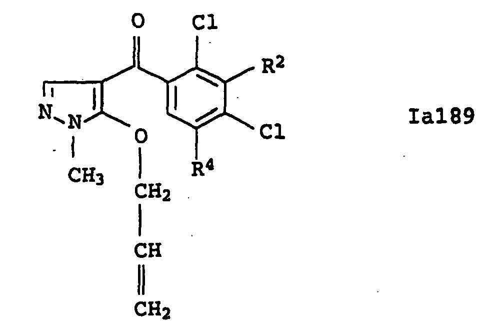
- Ebenso insbesondere außerordentlich bevorzugt sind die Verbindungen Ia190; insbesondere die Verbindungen Ia190.1-Ia190.164, die sich von den Verbindungen Ia1.1-Ia1.164 dadurch unterscheiden, daß R³ für Chlor, R⁶ für Ethyl und R⁷ für Allyl stehen: 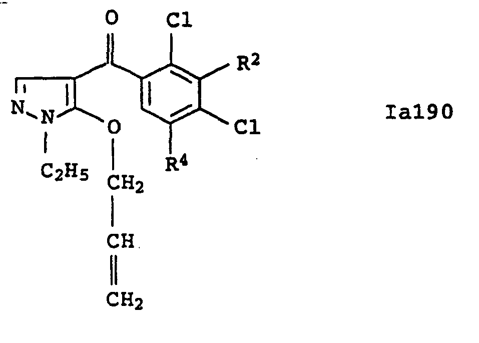
- Ebenso insbesondere außerordentlich bevorzugt sind die Verbindungen Ia191; insbesondere die Verbindungen Ia191.1-Ia191.164, die sich von den Verbindungen Ia1.1-Ia1.164 dadurch unterscheiden, daß R³ für Chlor, R⁷ für Allyl und R⁸ für Methyl stehen: 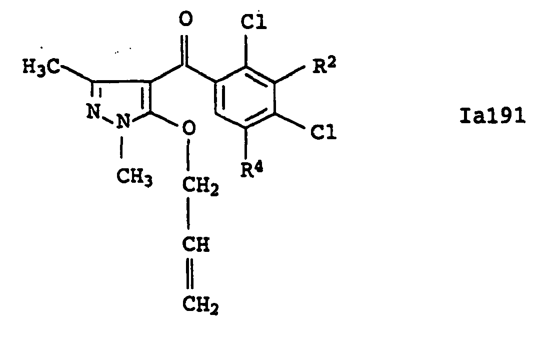
- Ebenso insbesondere außerordentlich bevorzugt sind die Verbindungen Ia192; insbesondere die Verbindungen Ia192.1-Ia192.164, die sich von den Verbindungen Ia1.1-Ia1.164 dadurch unterscheiden, daß R³ für Chlor und R⁷ für Propargyl stehen: 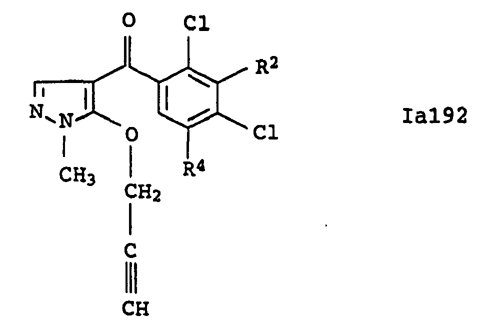
- Ebenso insbesondere außerordentlich bevorzugt sind die Verbindungen Ia193; insbesondere die Verbindungen Ia193.1-Ia193.164, die sich von den Verbindungen Ia1.1-Ia1.164 dadurch unterscheiden, daß R³ für Chlor, R⁶ für Ethyl und R⁷ für Propargyl stehen: 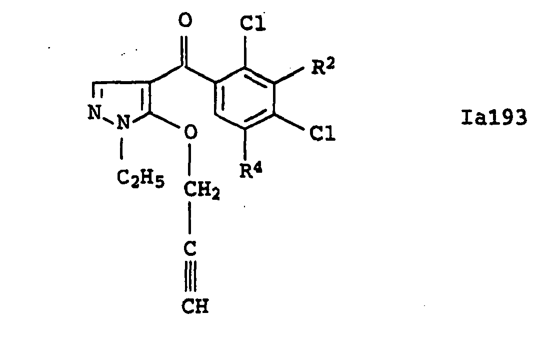
- Ebenso insbesondere außerordentlich bevorzugt sind die Verbindungen Ia194; insbesondere die Verbindungen Ia194.1-Ia194.164, die sich von den Verbindungen Ia1.1-Ia1.164 dadurch unterscheiden, daß R³ für Chlor, R⁷ für Propargyl und R⁸ für Methyl stehen: 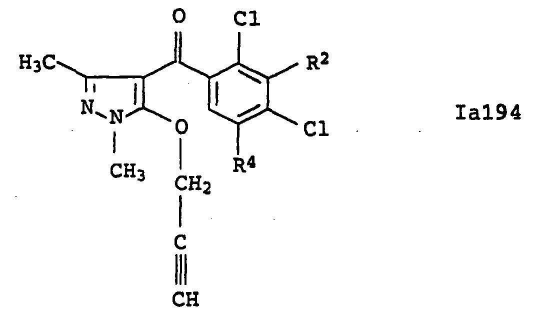
- Ebenso insbesondere außerordentlich bevorzugt sind die Verbindungen Ia195; insbesondere die Verbindungen Ia195.1-Ia195.164, die sich von den Verbindungen Ia1.1-Ia1.164 dadurch unterscheiden, daß R³ für Chlor und R⁷ für Methylcarbonyl stehen: 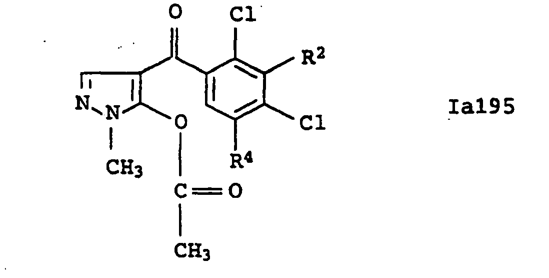
- Ebenso insbesondere außerordentlich bevorzugt sind die Verbindungen Ia196; insbesondere die Verbindungen Ia196.1-Ia196.164, die sich von den Verbindungen Ia1.1-Ia1.164 dadurch unterscheiden, daß R³ für Chlor, R⁶ für Ethyl und R⁷ für Methylcarbonyl stehen: 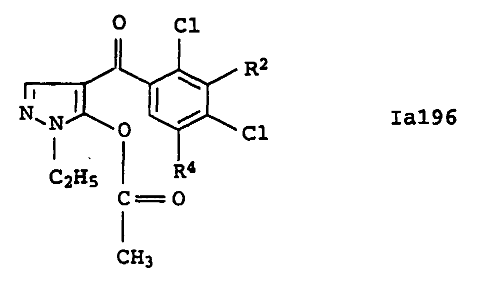
- Ebenso insbesondere außerordentlich bevorzugt sind die Verbindungen Ia197; insbesondere die Verbindungen Ia197.1-Ia197.164, die sich von den Verbindungen Ia1.1-Ia1.164 dadurch unterscheiden, daß R³ für Chlor, R⁶ für n-Propyl und R⁷ für Methylcarbonyl stehen: 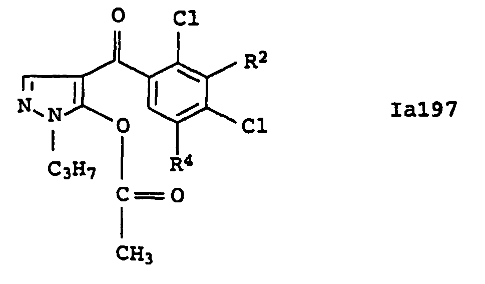
- Ebenso insbesondere außerordentlich bevorzugt sind die Verbindungen Ia198; insbesondere die Verbindungen Ia198.1-Ia198.164, die sich von den Verbindungen Ia1.1-Ia1.164 dadurch unterscheiden, daß R³ für Chlor, R⁶ für iso-Butyl und R⁷ für Methylcarbonyl stehen: 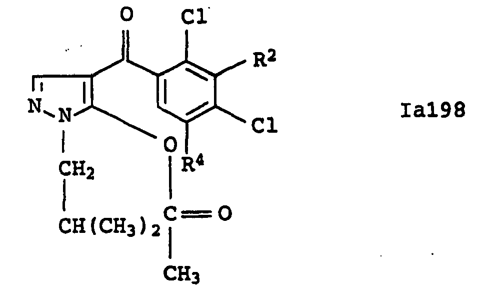
- Ebenso insbesondere außerordentlich bevorzugt sind die Verbindungen Ia199; insbesondere die Verbindungen Ia199.1-Ia199.164, die sich von den Verbindungen Ia1.1-Ia1.164 dadurch unterscheiden, daß R³ für Chlor, R⁷ für Methylcarbonyl und R⁸ für Methyl stehen: 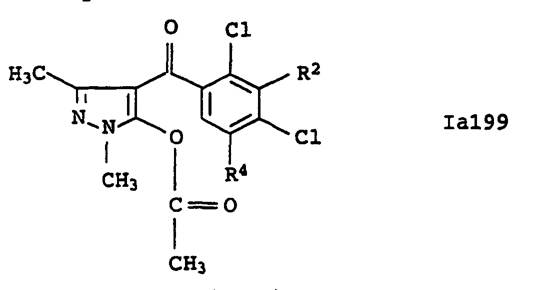
- Ebenso insbesondere außerordentlich bevorzugt sind die Verbindungen Ia200; insbesondere die Verbindungen Ia200.1-Ia200.164, die sich von den Verbindungen Ia1.1-Ia1.164 dadurch unterscheiden, daß R³ für Chlor und R⁷ für Ethylcarbonyl stehen: 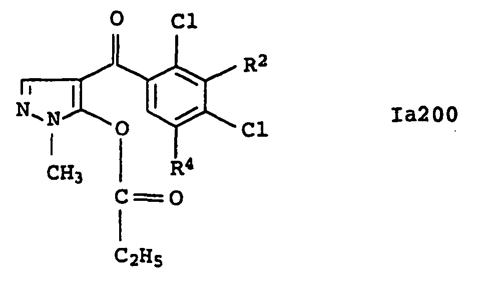
- Ebenso insbesondere außerordentlich bevorzugt sind die Verbindungen Ia201; insbesondere die Verbindungen Ia201.1-Ia201.164, die sich von den Verbindungen Ia1.1-Ia1.164 dadurch unterscheiden, daß R³ für Chlor, R⁶ für Ethyl und R⁷ für Ethylcarbonyl stehen: 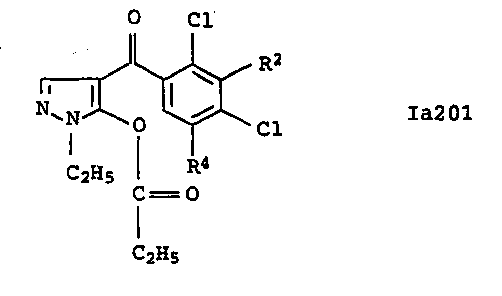
- Ebenso insbesondere außerordentlich bevorzugt sind die Verbindungen Ia202; insbesondere die Verbindungen Ia202.1-Ia202.164, die sich von den Verbindungen Ia1.1-Ia1.164 dadurch unterscheiden, daß R³ für Chlor, R⁶ für n-Propyl und R⁷ für Ethylcarbonyl stehen: 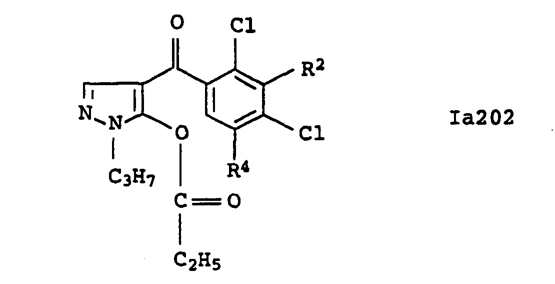
- Ebenso insbesondere außerordentlich bevorzugt sind die Verbindungen Ia203; insbesondere die Verbindungen Ia203.1-1a203.164, die sich von den Verbindungen Ia1.1-Ia1.164 dadurch unterscheiden, daß R³ für Chlor, R⁶ für iso-Butyl und R⁷ für Ethylcarbonyl stehen: 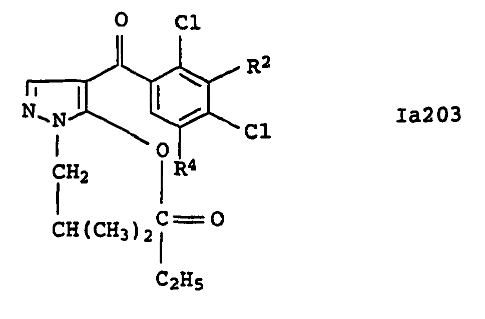
- Ebenso insbesondere außerordentlich bevorzugt sind die Verbindungen Ia204; insbesondere die Verbindungen Ia204.1-Ia204.164, die sich von den Verbindungen Ia1.1-Ia1.164 dadurch unterscheiden, daß R³ für Chlor, R⁷ für Ethylcarbonyl und R⁸ für Methyl stehen: 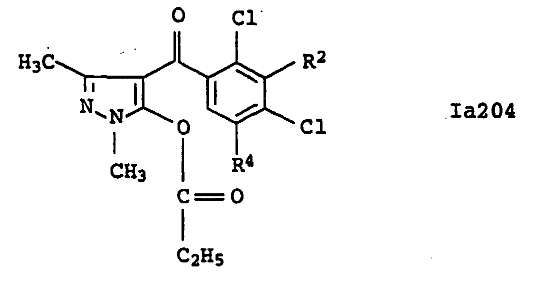
- Ebenso insbesondere außerordentlich bevorzugt sind die Verbindungen Ia205; insbesondere die Verbindungen Ia205.1-Ia205.164, die sich von den Verbindungen Ia1.1-Ia1.164 dadurch unterscheiden, daß R³ für Chlor und R⁷ für Methoxycarbonyl stehen: 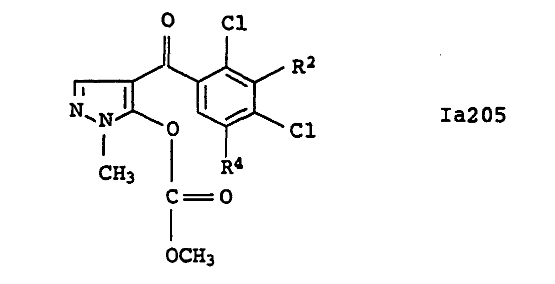
- Ebenso insbesondere außerordentlich bevorzugt sind die Verbindungen Ia206; insbesondere die Verbindungen Ia206.1-Ia206.164, die sich von den Verbindungen Ia1.1-Ia1.164 dadurch unterscheiden, daß R³ für Chlor, R⁶ für Ethyl und R⁷ für Methoxycarbonyl stehen: 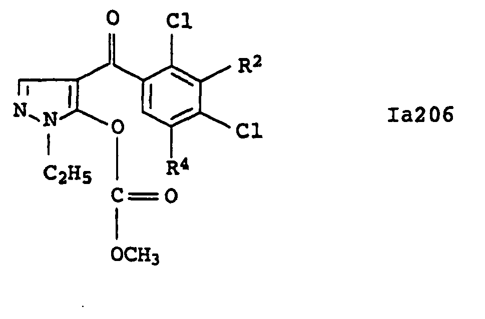
- Ebenso insbesondere außerordentlich bevorzugt sind die Verbindungen Ia207; insbesondere die Verbindungen Ia207.1-Ia207.164, die sich von den Verbindungen Ia1.1-Ia1.164 dadurch unterscheiden, daß R³ für Chlor, R⁷ für Methoxycarbonyl und R⁸ für Methyl stehen: 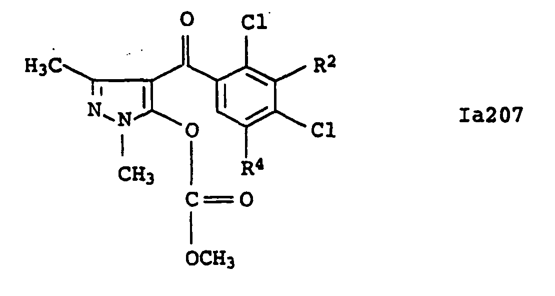
- Ebenso insbesondere außerordentlich bevorzugt sind die Verbindungen Ia208; insbesondere die Verbindungen Ia208.1-Ia208.164, die sich von den Verbindungen Ia1.1-Ia1.164 dadurch unterscheiden, daß R³ für Chlor und R⁷ für Ethoxycarbonyl stehen: 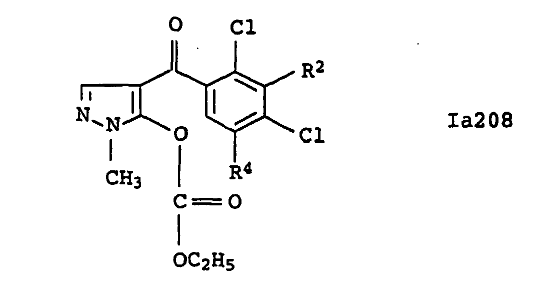
- Ebenso insbesondere außerordentlich bevorzugt sind die Verbindungen Ia209; insbesondere die Verbindungen 1a209.1-Ia209.164, die sich von den Verbindungen Ia1.1-Ia1.164 dadurch unterscheiden, daß R³ für Chlor, R⁶ für Ethyl und R⁷ für Ethoxycarbonyl stehen: 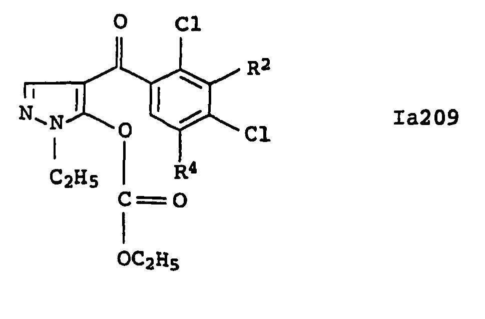
- Ebenso insbesondere außerordentlich bevorzugt sind die Verbindungen Ia210; insbesondere die Verbindungen Ia210.1-Ia210.164, die sich von den Verbindungen Ia1.1-Ia1.164 dadurch unterscheiden, daß R³ für Chlor, R⁷ für Ethoxycarbonyl und R⁸ für Methyl stehen: 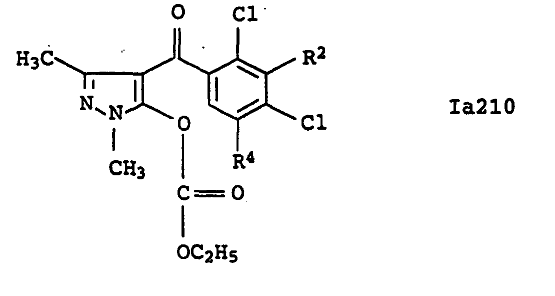
- Ebenso insbesondere außerordentlich bevorzugt sind die Verbindungen Ia211; insbesondere die Verbindungen Ia211.1-Ia211.164, die sich von den Verbindungen Ia1.1-Ia1.164 dadurch unterscheiden, daß R³ für Chlor und R⁷ für Dimethylaminocarbonyl stehen: 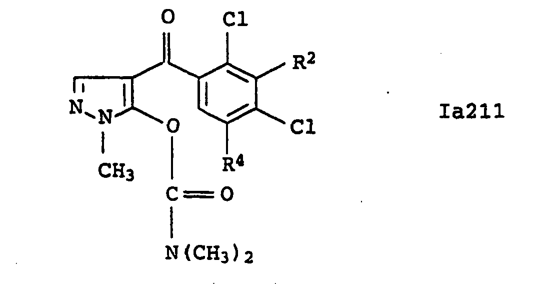
- Ebenso insbesondere außerordentlich bevorzugt sind die Verbindungen Ia212; insbesondere die Verbindungen Ia212.1-Ia212.164, die sich von den Verbindungen Ia1.1-Ia1.164 dadurch unterscheiden, daß R³ für Chlor, R⁶ für Ethyl und R⁷ für Dimethylaminocarbonyl stehen: 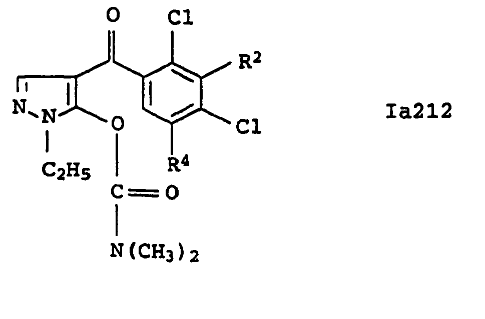
- Ebenso insbesondere außerordentlich bevorzugt sind die Verbindungen Ia213; insbesondere die Verbindungen Ia213.1-Ia213.164, die sich von den Verbindungen Ia1.1-Ia1.164 dadurch unterscheiden, daß R³ für Chlor, R⁷ für Dimethylaminocarbonyl und R⁸ für Methyl stehen: 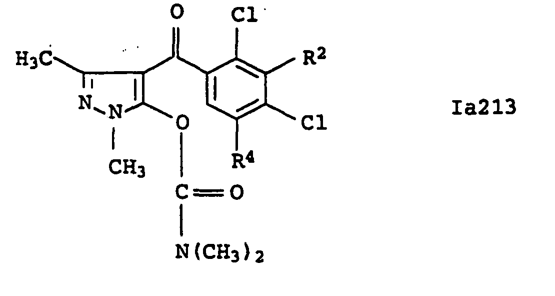
- Ebenso insbesondere außerordentlich bevorzugt sind die Verbindungen Ia214; insbesondere die Verbindungen Ia214.1-Ia214.164, die sich von den Verbindungen Ia1.1-Ia1.164 dadurch unterscheiden, daß R³ für Chlor und R⁷ für Diethylaminocarbonyl stehen: 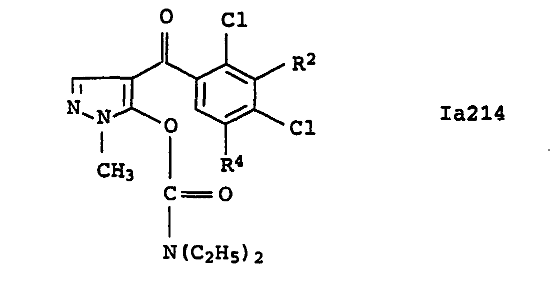
- Ebenso insbesondere außerordentlich bevorzugt sind die Verbindungen Ia215; insbesondere die Verbindungen Ia215.1-Ia215.164, die sich von den Verbindungen Ia1.1-Ia1.164 dadurch unterscheiden, daß R³ für Chlor, R⁶ für Ethyl und R⁷ für Diethylaminocarbonyl stehen: 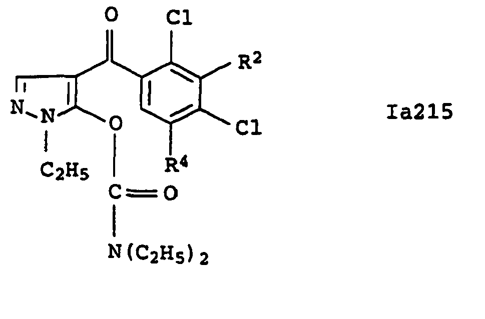
- Ebenso insbesondere außerordentlich bevorzugt sind die Verbindungen Ia216; insbesondere die Verbindungen Ia216.1-Ia216.164, die sich von den Verbindungen Ia1.1-Ia1.164 dadurch unterscheiden, daß R³ für Chlor, R⁷ für Diethylaminocarbonyl und R⁸ für Methyl stehen: 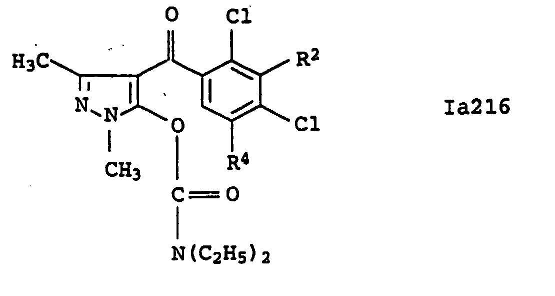
- Ebenso insbesondere außerordentlich bevorzugt sind die Verbindungen Ia217; insbesondere die Verbindungen Ia217.1-Ia217.164, die sich von den Verbindungen Ia1.1-Ia1.164 dadurch unterscheiden, daß R³ für Chlor und R⁷ für N-Methoxy-N-methylaminocarbonyl stehen: 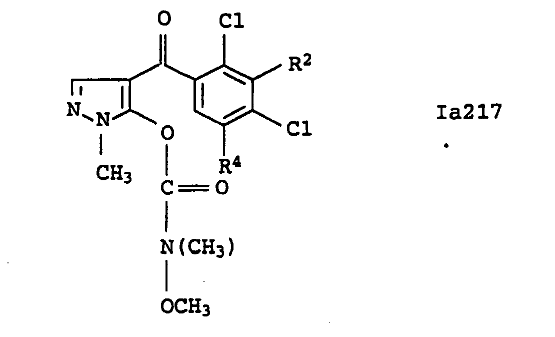
- Ebenso insbesondere außerordentlich bevorzugt sind die Verbindungen Ia218; insbesondere die Verbindungen Ia218.1-Ia218.164, die sich von den Verbindungen Ia1.1-Ia1.164 dadurch unterscheiden, daß R³ für Chlor, R⁶ für Ethyl und R⁷ für N-Methoxy-N-methylaminocarbonyl stehen: 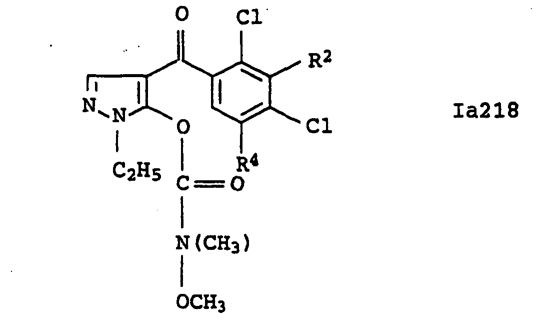
- Ebenso insbesondere außerordentlich bevorzugt sind die Verbindungen Ia219; insbesondere die Verbindungen Ia219.1-1a219.164, die sich von den Verbindungen Ia1.1-Ia1.164 dadurch unterscheiden, daß R³ für Chlor, R⁷ für N-Methoxy-N-methylaminocarbonyl und R⁸ für Methyl stehen: 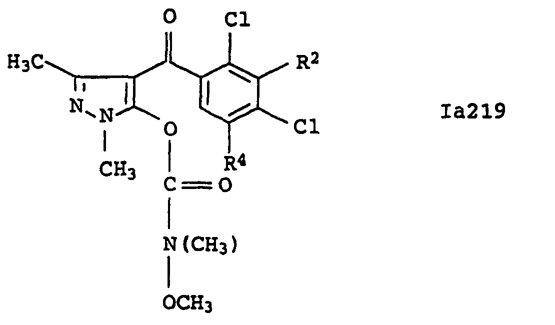
- Ebenso insbesondere außerordentlich bevorzugt sind die Verbindungen Ia220; insbesondere die Verbindungen 1a220.1-Ia220.164, die sich von den Verbindungen Ia1.1-Ia1.164 dadurch unterscheiden, daß R³ für Chlor und R⁷ für Benzyl stehen: 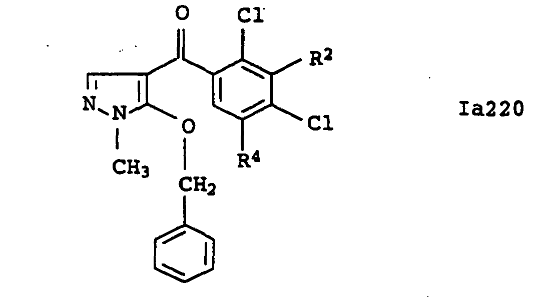
- Ebenso insbesondere außerordentlich bevorzugt sind die Verbindungen Ia221; insbesondere die Verbindungen Ia221.1-Ia221.164, die sich von den Verbindungen Ia1.1-Ia1.164 dadurch unterscheiden, daß R³ für Chlor, R⁶ für Ethyl und R⁷ für Benzyl stehen: 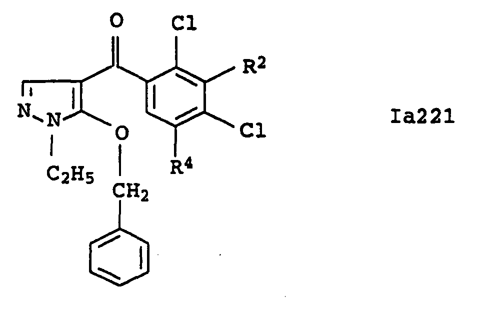
- Ebenso insbesondere außerordentlich bevorzugt sind die Verbindungen Ia222; insbesondere die Verbindungen Ia222.1-Ia222.164, die sich von den Verbindungen Ia1.1-Ia1.164 dadurch unterscheiden, daß R³ für Chlor, R⁶ für n-Propyl und R⁷ für Benzyl stehen: 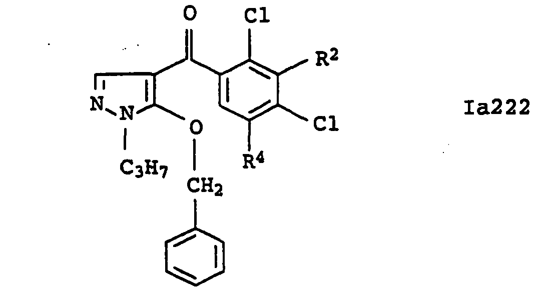
- Ebenso insbesondere außerordentlich bevorzugt sind die Verbindungen Ia223; insbesondere die Verbindungen Ia223.1-Ia223.164, die sich von den Verbindungen Ia1.1-Ia1.164 dadurch unterscheiden, daß R³ für Chlor, R⁶ für iso-Butyl und R⁷ für Benzyl stehen: 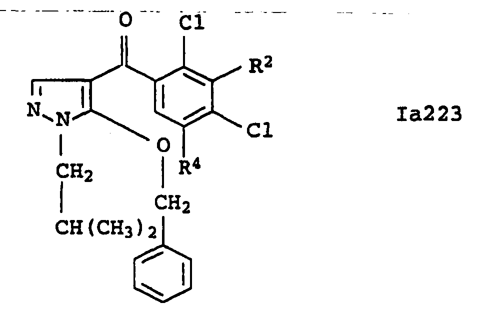
- Ebenso insbesondere außerordentlich bevorzugt sind die Verbindungen Ia224; insbesondere die Verbindungen Ia224.1-Ia224.164, die sich von den Verbindungen Ia1.1-Ia1.164 dadurch unterscheiden, daß R³ für Chlor, R⁷ für Benzyl und R⁸ für Methyl stehen: 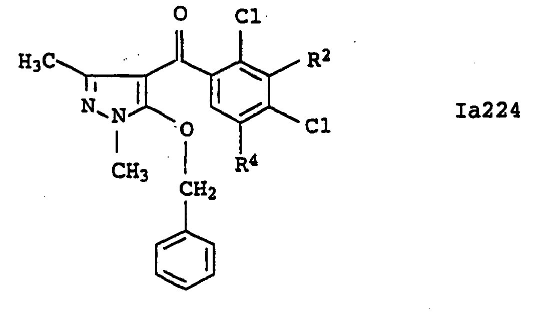
- Ebenso insbesondere außerordentlich bevorzugt sind die Verbindungen Ia225; insbesondere die Verbindungen Ia225.1-Ia225.164, die sich von den Verbindungen Ia1.1-Ia1.164 dadurch unterscheiden, daß R³ für Chlor und R⁷ für 4-Methylphenylmethyl stehen: 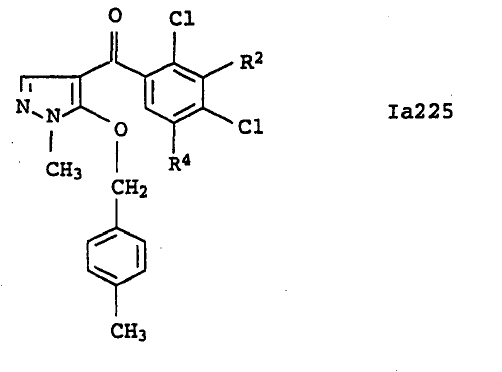
- Ebenso insbesondere außerordentlich bevorzugt sind die Verbindungen Ia226; insbesondere die Verbindungen Ia226.1-Ia226.164, die sich von den Verbindungen Ia1.1-Ia1.164 dadurch unterscheiden, daß R³ für Chlor, R⁶ für Ethyl und i R⁷ für 4-Methylphenylmethyl stehen: 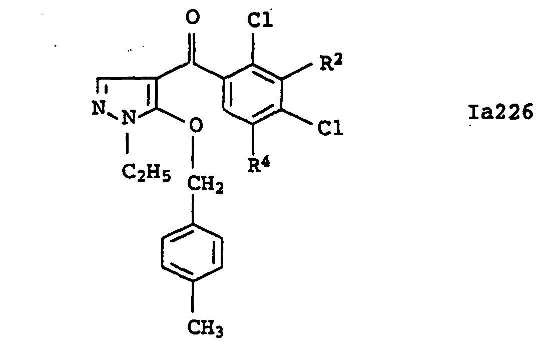
- Ebenso insbesondere außerordentlich bevorzugt sind die Verbindungen Ia227; insbesondere die Verbindungen Ia227.1-Ia227.164, die sich von den Verbindungen Ia1.1-Ia1.164 dadurch unterscheiden, daß R³ für Chlor, R⁶ für n-Propyl und R⁷ für 4-Methylphenylmethyl stehen: 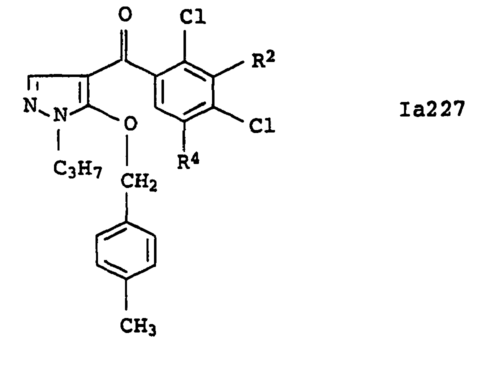
- Ebenso insbesondere außerordentlich bevorzugt sind die Verbindungen Ia228; insbesondere die Verbindungen Ia228.1-Ia228.164, die sich von den Verbindungen Ia1.1-Ia1.164 dadurch unterscheiden, daß R³ für Chlor, R⁶ für iso-Butyl und R⁷ für 4-Methylphenylmethyl stehen: 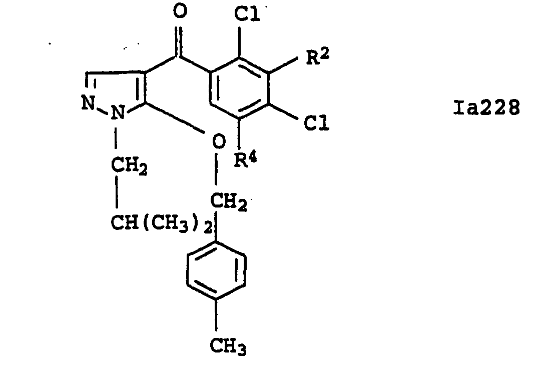
- Ebenso insbesondere außerordentlich bevorzugt sind die Verbindungen Ia229; insbesondere die Verbindungen Ia229.1-Ia229.164, die sich von den Verbindungen Ia1.1-Ia1.164 dadurch unterscheiden, daß R³ für Chlor, R⁷ für 4-Methylphenylmethyl und R⁸ für Methyl stehen: 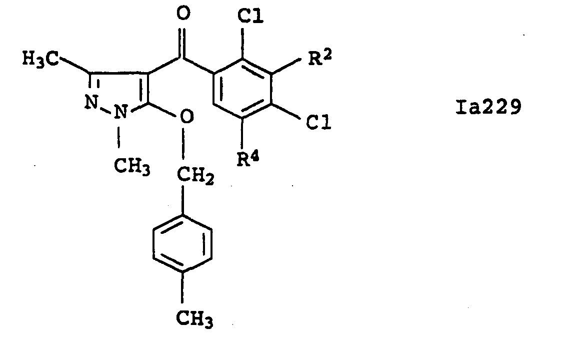
- Ebenso insbesondere außerordentlich bevorzugt sind die Verbindungen Ia230; insbesondere die Verbindungen Ia230.1-Ia230.164, die sich von den Verbindungen Ia1.1-Ia1.164 dadurch unterscheiden, daß R³ für Chlor und R⁷ für 4-Chlorphenylmethyl stehen: 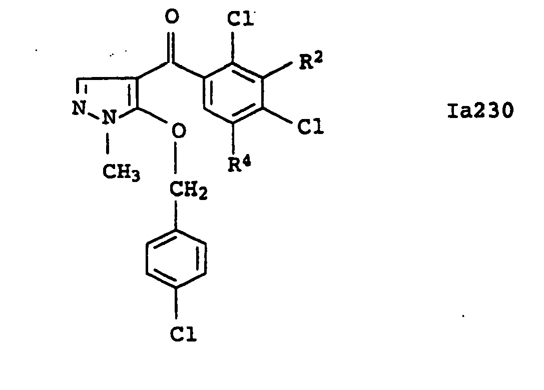
- Ebenso insbesondere außerordentlich bevorzugt sind die Verbindungen Ia231; insbesondere die Verbindungen Ia231.1-Ia231.164, die sich von den Verbindungen Ia1.1-Ia1.164 dadurch unterscheiden, daß R³ für Chlor, R⁶ für Ethyl und R⁷ für 4-Chlorphenylmethyl stehen: 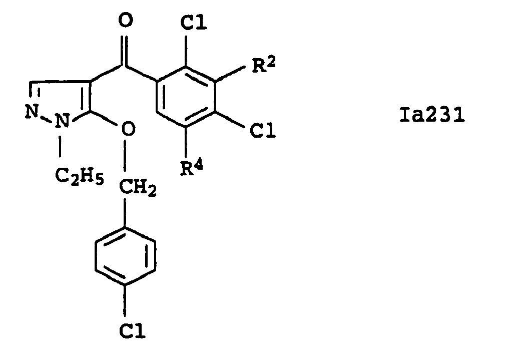
- Ebenso insbesondere außerordentlich bevorzugt sind die Verbindungen Ia232; insbesondere die Verbindungen 1a232.1-Ia232.164, die sich von den Verbindungen Ia1.1-Ia1.164 dadurch unterscheiden, daß R³ für Chlor, R⁶ für n-Propyl und R⁷ für 4-Chlorphenylmethyl stehen: 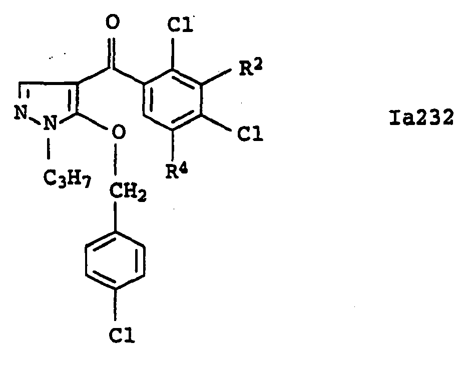
- Ebenso insbesondere außerordentlich bevorzugt sind die Verbindungen Ia233; insbesondere die Verbindungen Ia2233.1-Ia233.164, die sich von den Verbindungen Ia1.1-Ia1.164 dadurch unterscheiden, daß R³ für Chlor, R⁶ für iso-Butyl und R⁷ für 4-Chlorphenylmethyl stehen: 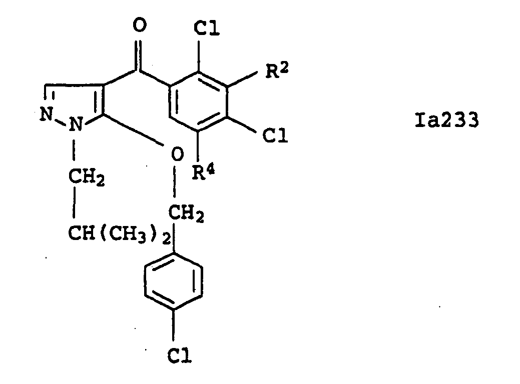
- Ebenso insbesondere außerordentlich bevorzugt sind die Verbindungen Ia234; insbesondere die Verbindungen Ia234.1-Ia234.164, die sich von den Verbindungen Ia1.1-Ia1.164 dadurch unterscheiden, daß R³ für Chlor, R⁷ für 4-Chlorphenylmethyl und R⁸ für Methyl stehen: 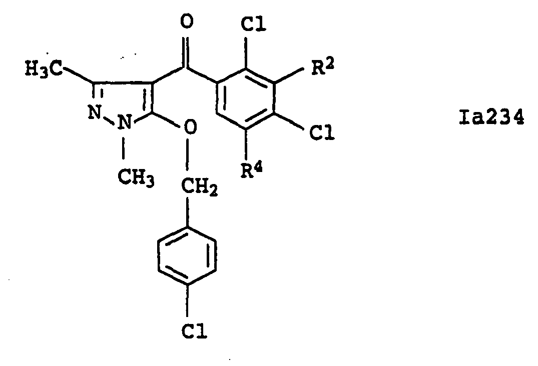
- Ebenso insbesondere außerordentlich bevorzugt sind die Verbindungen Ia235; insbesondere die Verbindungen Ia235.1-Ia235.164, die sich von den Verbindungen Ia1.1-Ia1.164 dadurch unterscheiden, daß R³ für Chlor und R⁷ für 4-Methoxyphenylmethyl stehen: 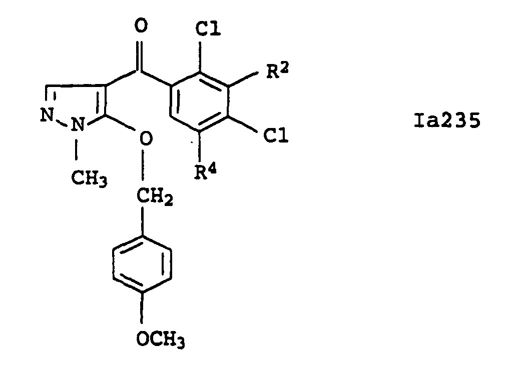
- Ebenso insbesondere außerordentlich bevorzugt sind die Verbindungen Ia236; insbesondere die Verbindungen Ia236.1-Ia236.164, die sich von den Verbindungen Ia1.1-Ia1.164 dadurch unterscheiden, daß R³ für Chlor, R⁶ für Ethyl und R⁷ für 4-Methoxyphenylmethyl stehen: 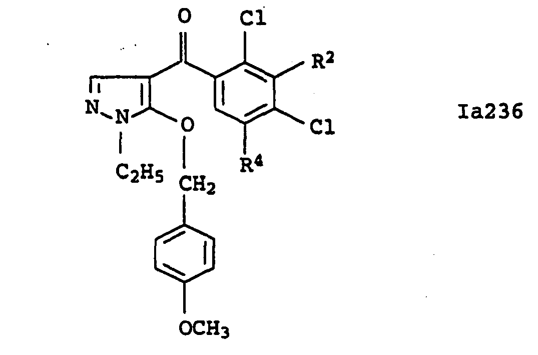
- Ebenso insbesondere außerordentlich bevorzugt sind die Verbindungen Ia237; insbesondere die Verbindungen Ia237.1-Ia237.164, die sich von den Verbindungen Ia1.1-Ia1.164 dadurch unterscheiden, daß R³ für Chlor, R⁷ für 4-Methoxyphenylmethyl und R⁸ für Methyl stehen: 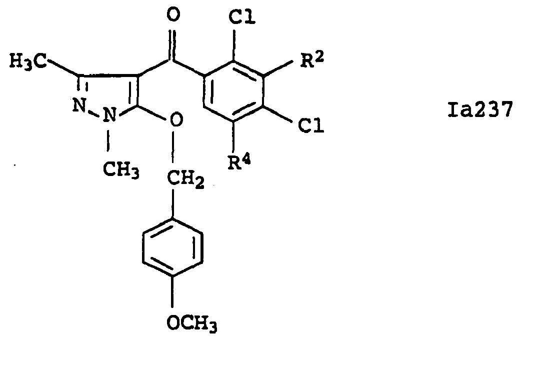
- Ebenso insbesondere außerordentlich bevorzugt sind die Verbindungen Ia238; insbesondere die Verbindungen Ia238.1-Ia238.164, die sich von den Verbindungen Ia1.1-Ia1.164 dadurch unterscheiden, daß R³ für Chlor und R⁷ für 3-Trifluqrmethylphenylmethyl stehen: 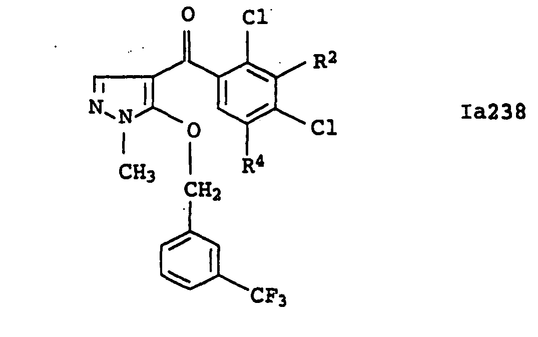
- Ebenso insbesondere außerordentlich bevorzugt sind die Verbindungen Ia239; insbesondere die Verbindungen Ia239.1-Ia239.164, die sich von den Verbindungen Ia1.1-Ia1.164 dadurch unterscheiden, daß R³ für Chlor, R⁶ für Ethyl und R⁷ für 3-Trifluormethylphenylmethyl stehen: 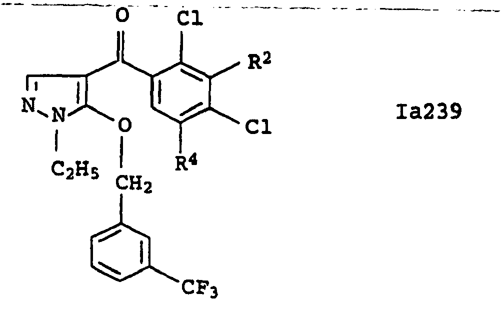
- Ebenso insbesondere außerordentlich bevorzugt sind die Verbindungen Ia237; insbesondere die Verbindungen Ia237.1-Ia237.164, die sich von den Verbindungen Ia1.1-Ia1.164 dadurch unterscheiden, daß R³ für Chlor, R⁷ für 3-Trifluormethylphenylmethyl und R⁸ für Methyl stehen: 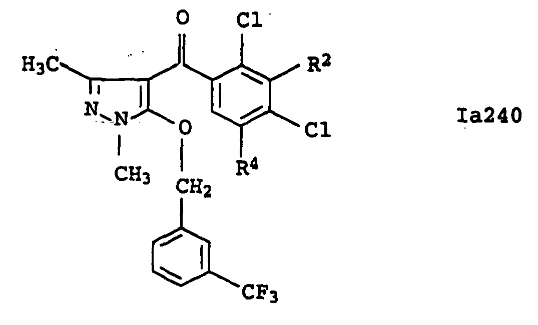
- Ebenso insbesondere außerordentlich bevorzugt sind die Verbindungen Ia241; insbesondere die Verbindungen Ia241.1-Ia241.164, die sich von den Verbindungen Ia1.1-Ia1.164 dadurch unterscheiden, daß R³ für Chlor und R⁷ für 2,4-Dichlorphenylmethyl stehen: 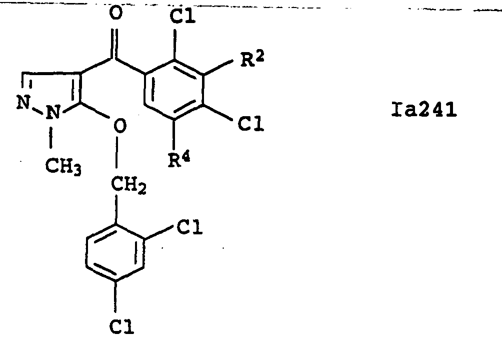
- Ebenso insbesondere außerordentlich bevorzugt sind die Verbindungen Ia242; insbesondere die Verbindungen Ia242.1-Ia242.164, die sich von den Verbindungen Ia1.1-Ia1.164 dadurch unterscheiden, daß R³ für Chlor, R⁶ für Ethyl und R⁷ für 2,4-Dichlorphenylmethyl stehen: 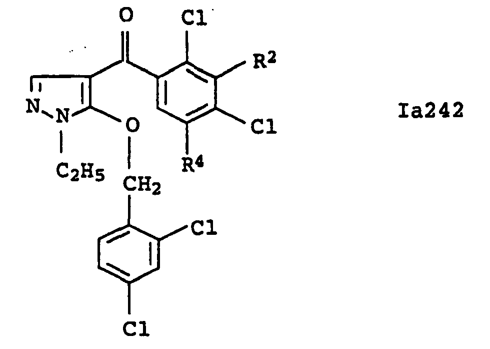
- Ebenso insbesondere außerordentlich bevorzugt sind die Verbindungen Ia243; insbesondere die Verbindungen Ia243.1-Ia243.164, die sich von den Verbindungen Ia1.1-Ia1.164 dadurch unterscheiden, daß R³ für Chlor, R⁷ für 2,4-Dichlorphenylmethyl und R⁸ für Methyl stehen: 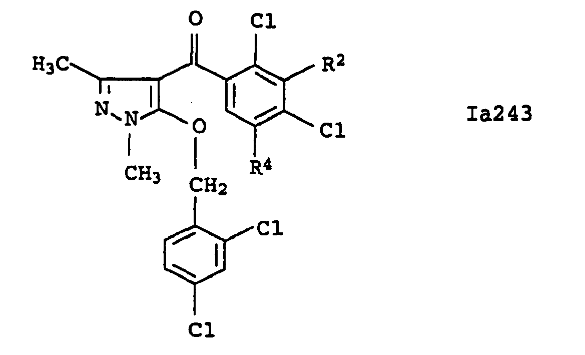
- Ebenso insbesondere außerordentlich bevorzugt sind die Verbindungen Ia244; insbesondere die Verbindungen Ia244.1-Ia244.164, die sich von den Verbindungen Ia1.1-Ia1.164 dadurch unterscheiden, daß R³ für Chlor und R⁷ für Phenylcarbonylmethyl stehen: 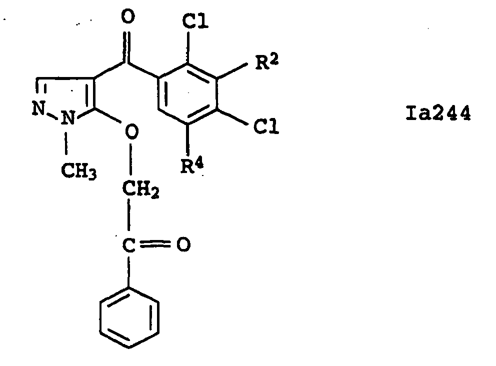
- Ebenso insbesondere außerordentlich bevorzugt sind die Verbindungen Ia245; insbesondere die Verbindungen Ia245.1-Ia245.164, die sich von den Verbindungen Ia1.1-Ia1.164 dadurch unterscheiden, daß R³ für Chlor, R⁶ für Ethyl und R⁷ für Phenylcarbonylmethyl stehen: 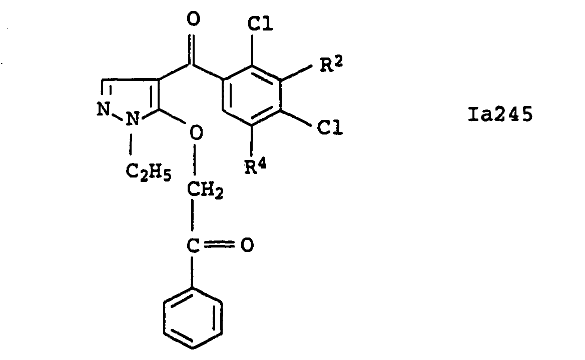
- Ebenso insbesondere außerordentlich bevorzugt sind die Verbindungen Ia246; insbesondere die Verbindungen Ia246.1-Ia246.164, die sich von den Verbindungen Ia1.1-Ia1.164 dadurch unterscheiden, daß R³ für Chlor, R⁶ für n-Propyl und R⁷ für Phenylcarbonylmethyl stehen: 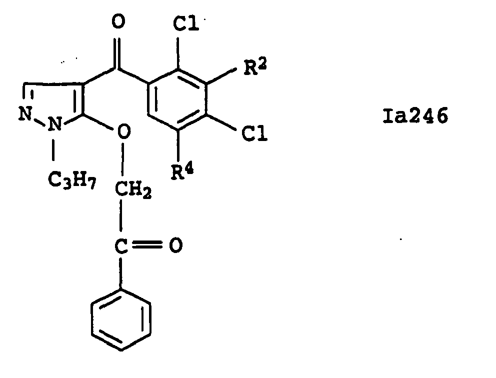
- Ebenso insbesondere außerordentlich bevorzugt sind die Verbindungen Ia247; insbesondere die Verbindungen Ia247.1-Ia247.164, die sich von den Verbindungen Ia1.1-Ia1.164 dadurch unterscheiden, daß R³ für Chlor, R⁶ für iso-Butyl und R⁷ für Phenylcarbonylmethyl stehen: 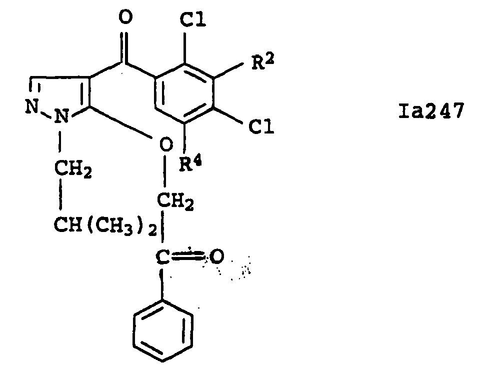
- Ebenso insbesondere außerordentlich bevorzugt sind die Verbindungen Ia248; insbesondere die Verbindungen Ia248.1-Ia248.164, die sich von den Verbindungen Ia1.1-Ia1.164 dadurch unterscheiden, daß R³ für Chlor, R⁷ für Phenylcarbonylmethyl und R⁸ für Methyl stehen: 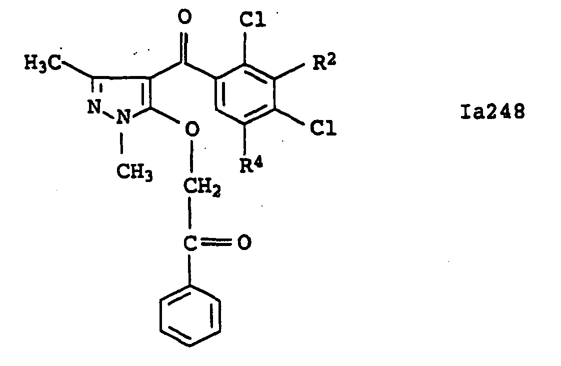
- Ebenso insbesondere außerordentlich bevorzugt sind die Verbindungen Ia249; insbesondere die Verbindungen Ia249.1-Ia249.164, die sich von den Verbindungen Ia1.1-Ia1.164 dadurch unterscheiden, daß R³ für Chlor und R⁷ für 4-Methylphenylcarbonylmethyl stehen: 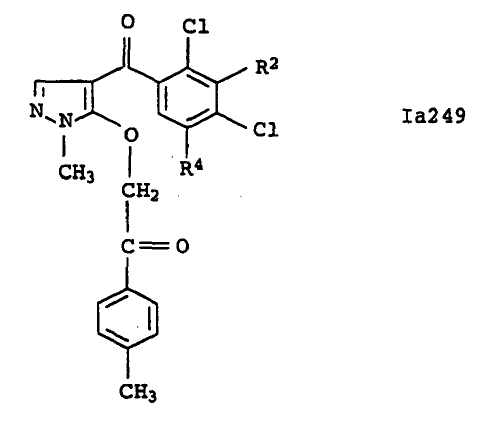
- Ebenso insbesondere außerordentlich bevorzugt sind die Verbindungen Ia250; insbesondere die Verbindungen Ia250.1-Ia250.164, die sich von den Verbindungen Ia1.1-Ia1.164 dadurch unterscheiden, daß R³ für Chlor, R⁶ für Ethyl und R⁷ für 4-Methylphenylcarbonylmethyl stehen: 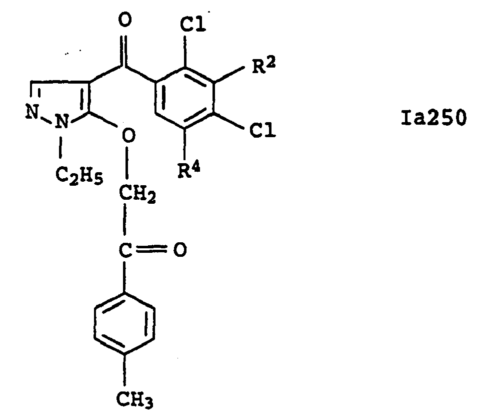
- Ebenso insbesondere außerordentlich bevorzugt sind die Verbindungen Ia251; insbesondere die Verbindungen Ia251.1-Ia251.164, die sich von den Verbindungen Ia1.1-Ia1.164 dadurch unterscheiden, daß R³ für Chlor, R⁷ für 4-Methylphenylcarbonylmethyl und R⁸ für Methyl stehen: 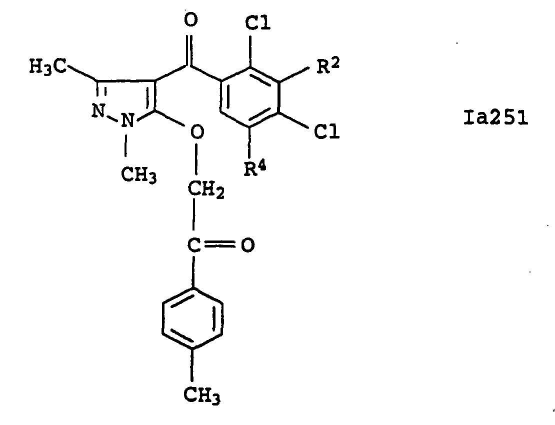
- Ebenso insbesondere außerordentlich bevorzugt sind die Verbindungen Ia252; insbesondere die Verbindungen Ia252.1-Ia252.164, die sich von den Verbindungen Ia1.1-Ia1.164 dadurch unterscheiden, daß R³ für Chlor und R⁷ für 1-(Phenylcarbonyl)-eth-1-yl stehen: 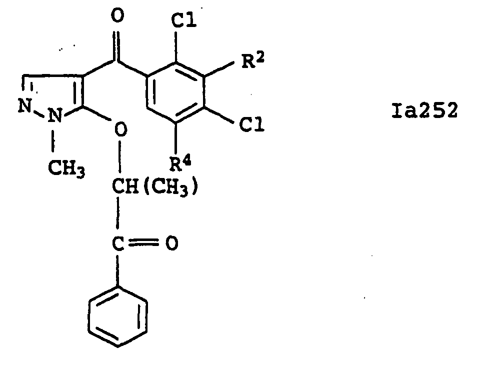
- Ebenso insbesondere außerordentlich bevorzugt sind die Verbindungen Ia253; insbesondere die Verbindungen 1a253.1-Ia253.164, die sich von den Verbindungen Ia1.1-Ia1.164 dadurch unterscheiden, daß R³ für Chlor, R⁶ für Ethyl und R⁷ für 1-(Phenylcarbonyl)-eth-1-yl stehen: 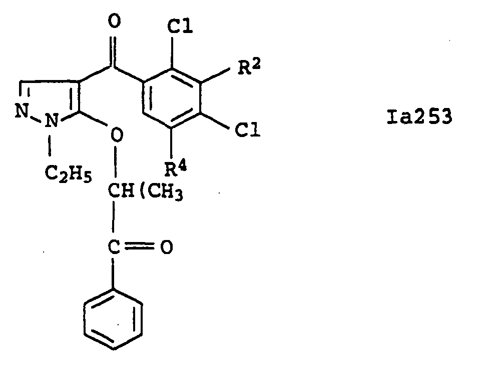
- Ebenso insbesondere außerordentlich bevorzugt sind die Verbindungen Ia254; insbesondere die Verbindungen Ia254.1-Ia254.164, die sich von den Verbindungen Ia1.1-Ia1.164 dadurch unterscheiden, daß R³ für Chlor, R⁷ für 1-(phenylcarbonyl)-eth-1-yl und R⁸ für Methyl stehen: 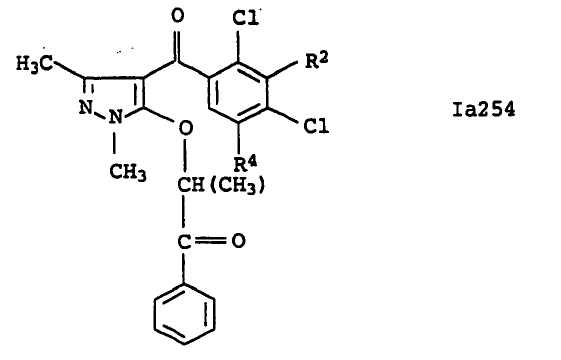
- Ebenso insbesondere außerordentlich bevorzugt sind die Verbindungen Ia255; insbesondere die Verbindungen Ia255.1-Ia255.164, die sich von den Verbindungen Ia1.1-Ia1.164 dadurch unterscheiden, daß R³ für Chlor und R⁷ für Phenyl - carbonyl stehen: 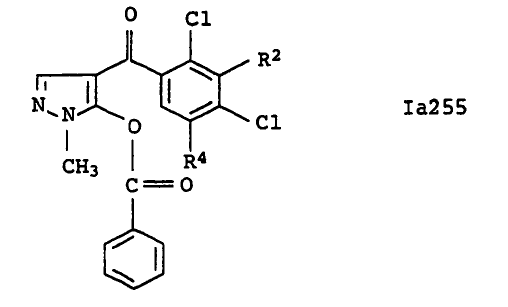
- Ebenso insbesondere außerordentlich bevorzugt sind die Verbindungen Ia256; insbesondere die Verbindungen Ia256.1-Ia256.164, die sich von den Verbindungen Ia1.1-Ia1.164 dadurch unterscheiden, daß R³ für Chlor, R⁶ für Ethyl und R⁷ für Phenylcarbonyl stehen: 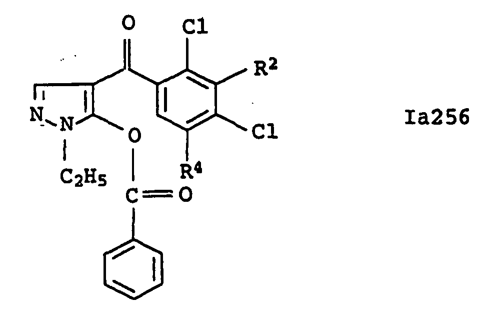
- Ebenso insbesondere außerordentlich bevorzugt sind die Verbindungen Ia257; insbesondere die Verbindungen Ia257.1-Ia257.164, die sich von den Verbindungen Ia1.1-Ia1.164 dadurch unterscheiden, daß R³ für Chlor, R⁷ für Phenylcarbonyl und R⁸ für Methyl stehen: 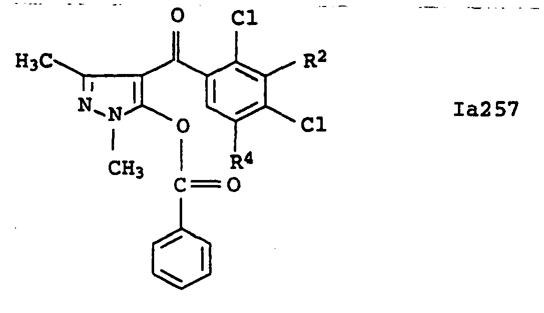
- Ebenso insbesondere außerordentlich bevorzugt sind die Verbindungen Ia258; insbesondere die Verbindungen Ia258.1-Ia258.164, die sich von den Verbindungen Ia1.1-Ia1.164 dadurch unterscheiden, daß R³ für Chlor und R⁷ für 4-Methylphenylcarbonyl stehen: 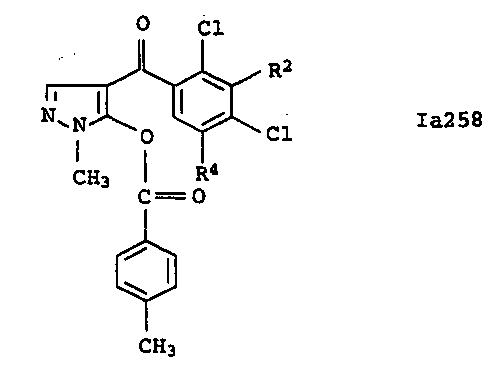
- Ebenso insbesondere außerordentlich bevorzugt sind die Verbindungen Ia259; insbesondere die Verbindungen Ia259.1-Ia259.164, die sich von den Verbindungen Ia1.1-Ia1.164 dadurch unterscheiden, daß R³ für Chlor, R⁶ für Ethyl und R⁷ für 4-Methylphenylcarbonyl stehen: 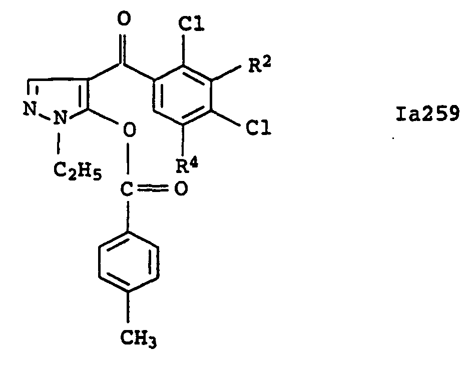
- Ebenso insbesondere außerordentlich bevorzugt sind die Verbindungen Ia260; insbesondere die Verbindungen Ia260.1-Ia260.164, die sich von den Verbindungen Ia1.1-Ia1.164 dadurch unterscheiden, daß R³ für Chlor, R⁷ für 4-Methylphenylcarbonyl und R⁸ für Methyl stehen: 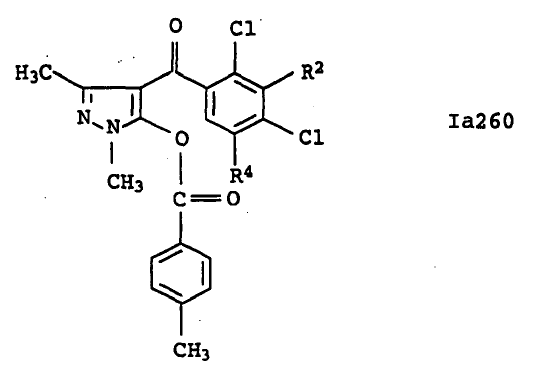
- Ebenso insbesondere außerordentlich bevorzugt sind die Verbindungen Ia261; insbesondere die Verbindungen Ia261.1-Ia261.164, die sich von den Verbindungen Ia1.1-Ia1.164 dadurch unterscheiden, daß R³ für Chlor und R⁷ für 4-Chlorphenylcarbonyl stehen: 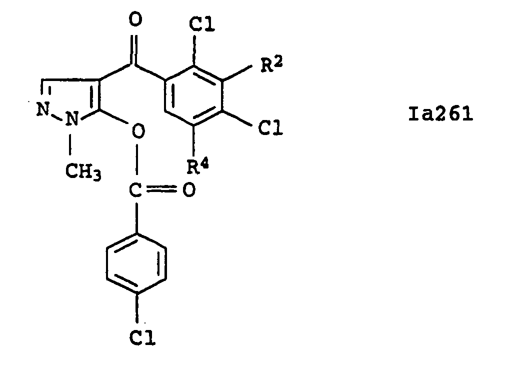
- Ebenso insbesondere außerordentlich bevorzugt sind die Verbindungen Ia262; insbesondere die Verbindungen Ia262.1-Ia262.164, die sich von den Verbindungen Ia1.1-Ia1.164 dadurch unterscheiden, daß R³ für Chlor, R⁶ für Ethyl und R⁷ für 4-Chlorphenylcarbonyl stehen: 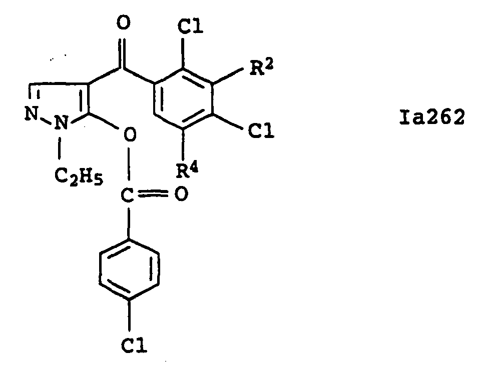
- Ebenso insbesondere außerordentlich bevorzugt sind die Verbindungen Ia263; insbesondere die Verbindungen Ia263.1-Ia263.164, die sich von den Verbindungen Ia1.1-Ia1.164 dadurch unterscheiden, daß R³ für Chlor, R⁷ für 4-Chlorphenylcarbonyl und R⁸ für Methyl stehen: 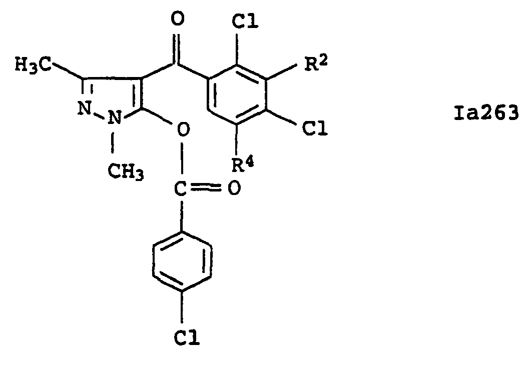
- Ebenso insbesondere außerordentlich bevorzugt sind die Verbindungen Ia264; insbesondere die Verbindungen Ia264.1-Ia264.164, die sich von den Verbindungen Ia1.1-Ia1.164 dadurch unterscheiden, daß R³ für Chlor und R⁷ für 4-Methoxyphenylcarbonyl stehen: 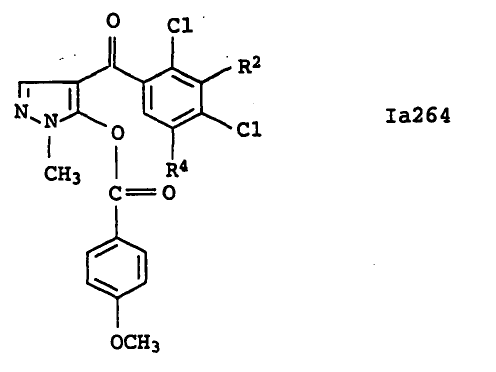
- Ebenso insbesondere außerordentlich bevorzugt sind die Verbindungen Ia265; insbesondere die Verbindungen Ia265.1-Ia265.164, die sich von den Verbindungen Ia1.1-Ia1.164 dadurch unterscheiden, daß R³ für Chlor, R⁶ für Ethyl und R⁷ für 4-Methoxyphenylcarbonyl stehen: 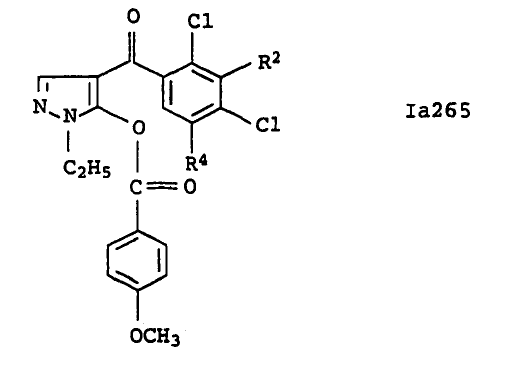
- Ebenso insbesondere außerordentlich bevorzugt sind die Verbindungen Ia266; insbesondere die Verbindungen Ia266.1-Ia266.164, die sich von den Verbindungen Ia1.1-Ia1.164 dadurch unterscheiden, daß R³ für Chlor, R⁷ für 4-Methoxyphenylcarbonyl und R⁸ für Methyl stehen: 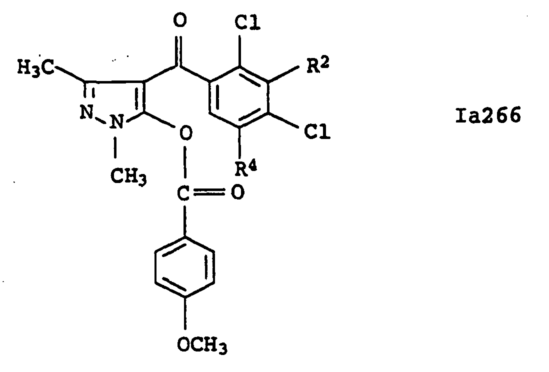
- Ebenso insbesondere außerordentlich bevorzugt sind die Verbindungen Ia267; insbesondere die Verbindungen Ia267.1-Ia267.164, die sich von den Verbindungen Ia1.1-Ia1.164 dadurch unterscheiden, daß R³ für Chlor und R⁷ für 4-Trifluormethylphenylcarbonyl stehen: 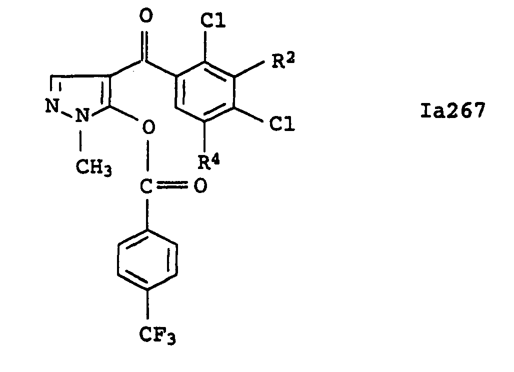
- Ebenso insbesondere außerordentlich bevorzugt sind die Verbindungen Ia268; insbesondere die Verbindungen Ia268.1-Ia268.164, die sich von den Verbindungen Ia1.1-Ia1.164 dadurch unterscheiden, daß R³ für Chlor, R⁶ für Ethyl und R⁷ für 4-Trifluormethylphenylcarbonyl stehen: 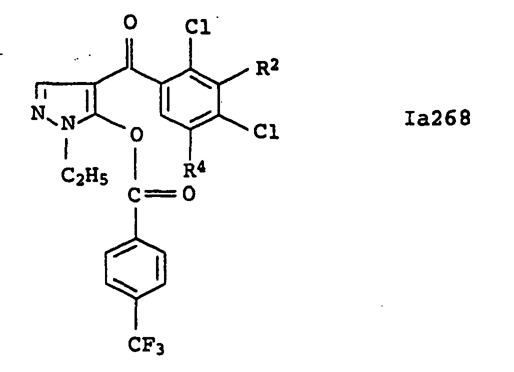
- Ebenso insbesondere außerordentlich bevorzugt sind die Verbindungen Ia269; insbesondere die Verbindungen Ia269.1-Ia269.164, die sich von den Verbindungen Ia1.1-Ia1.164 dadurch unterscheiden, daß R³ für Chlor, R⁷ für 4-Trifluormethylphenylcarbonyl und R⁸ für Methyl stehen: 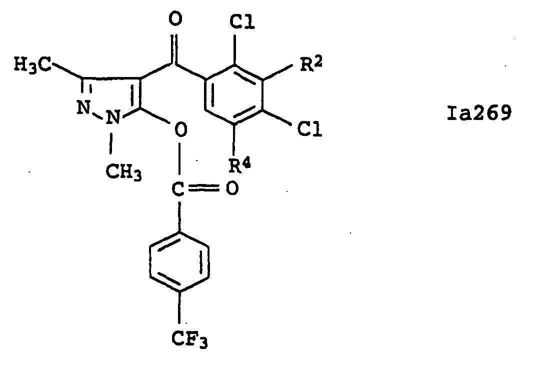
- Ebenso insbesondere außerordentlich bevorzugt sind die Verbindungen Ia270; insbesondere die Verbindungen Ia270.1-Ia270.164, die sich von den Verbindungen Ia1.1-Ia1.164 dadurch unterscheiden, daß R³ für Chlor und R⁷ für 2,4-Dichlorphenylcarbonyl stehen: 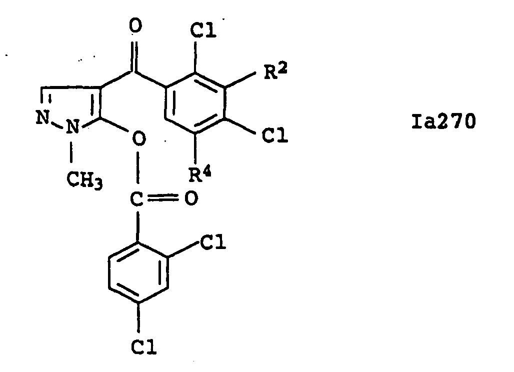
- Ebenso insbesondere außerordentlich bevorzugt sind die Verbindungen Ia271; insbesondere die Verbindungen Ia271.1-Ia271.164, die sich von den Verbindungen Ia1.1-Ia1.164 dadurch unterscheiden, daß R³ für Chlor, R⁶ für Ethyl und R⁷ für 2,4-Dichlorphenylcarbonyl stehen: 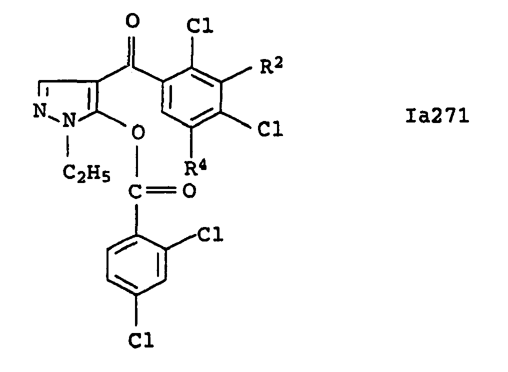
- Ebenso insbesondere außerordentlich bevorzugt sind die Verbindungen Ia272; insbesondere die Verbindungen Ia272.1-Ia272.164, die sich von den Verbindungen Ia1.1-Ia1.164 dadurch unterscheiden, daß R³ für Chlor, R⁷ für 2,4-Dichlorphenylcarbonyl und R⁸ für Methyl stehen: 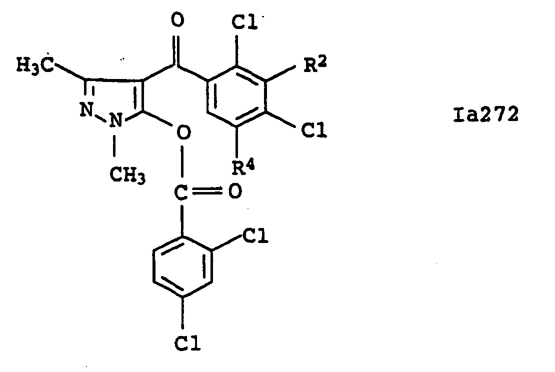
- Ebenso insbesondere außerordentlich bevorzugt sind die Verbindungen Ia273; insbesondere die Verbindungen Ia273.1-Ia273.164, die sich von den Verbindungen Ia1.1-Ia1.164 dadurch unterscheiden, daß R¹ für Methyl steht: 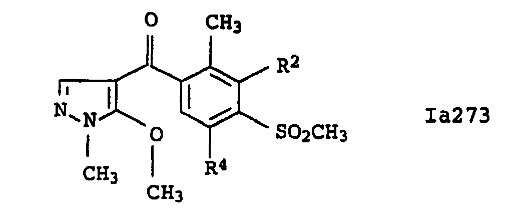
- Ebenso insbesondere außerordentlich bevorzugt sind die Verbindungen Ia274; insbesondere die Verbindungen Ia274.1-Ia274.164, die sich von den Verbindungen Ia1.1-Ia1.164 dadurch unterscheiden, daß R¹ für Methyl und R⁶ für Ethyl stehen: 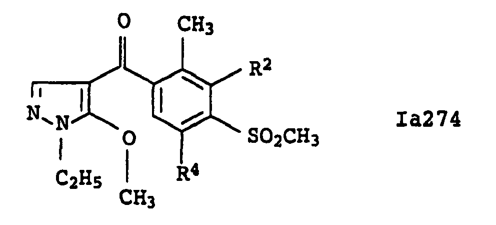
- Ebenso insbesondere außerordentlich bevorzugt sind die Verbindungen Ia275; insbesondere die Verbindungen Ia275.1-Ia275.164, die sich von den Verbindungen Ia1.1-Ia1.164 dadurch unterscheiden, daß R¹ für Methyl und R⁶ für n-Propyl stehen: 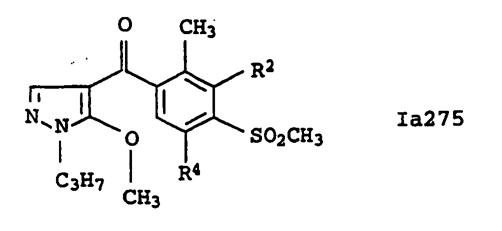
- Ebenso insbesondere außerordentlich bevorzugt sind die Verbindungen Ia276; insbesondere die Verbindungen Ia276.1-Ia276.164, die sich von den Verbindungen Ia1.1-Ia1.164 dadurch unterscheiden, daß R¹ für Methyl und R⁶ für iso-Butyl stehen: 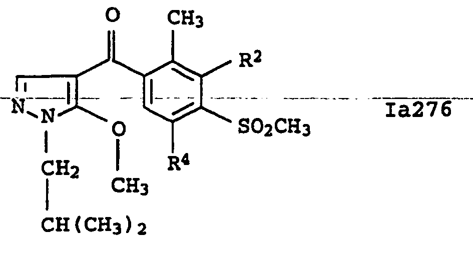
- Ebenso insbesondere außerordentlich bevorzugt sind die Verbindungen Ia277; insbesondere die Verbindungen Ia277.1-Ia277.164, die sich von den Verbindungen Ia1.1-Ia1.164 dadurch unterscheiden, daß R¹ und R⁸ für Methyl stehen: 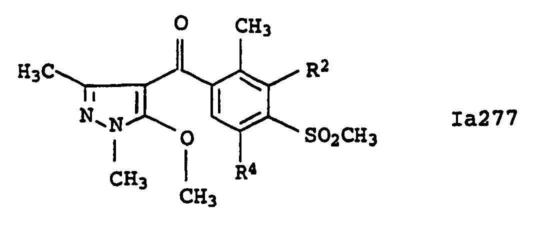
- Ebenso insbesondere außerordentlich bevorzugt sind die Verbindungen Ia278; insbesondere die Verbindungen Ia278.1-Ia278.164, die sich von den Verbindungen Ia1.1-Ia1.164 dadurch unterscheiden, daß R¹ für Methyl und R⁷ für Ethyl stehen: 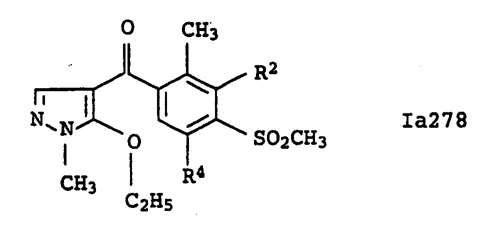
- Ebenso insbesondere außerordentlich bevorzugt sind die Verbindungen Ia279; insbesondere die Verbindungen Ia279.1-Ia279.164, die sich von den Verbindungen Ia1.1-Ia1.164 dadurch unterscheiden, daß R¹ für Methyl, R⁶ und R⁷ für Ethyl stehen: 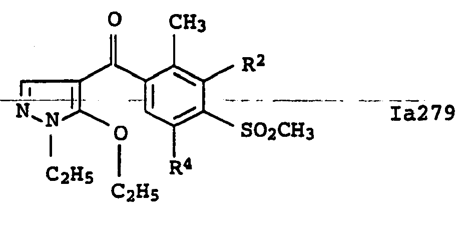
- Ebenso insbesondere außerordentlich bevorzugt sind die Verbindungen Ia280; insbesondere die Verbindungen Ia280.1-Ia280.164, die sich von den Verbindungen Ia1.1-Ia1.164 dadurch unterscheiden, daß R¹ für Methyl, R⁶ für n-Propyl und R⁷ für Ethyl stehen: 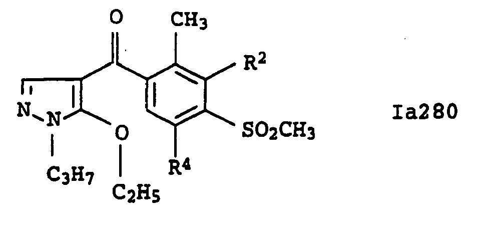
- Ebenso insbesondere außerordentlich bevorzugt sind die Verbindungen Ia281; insbesondere die Verbindungen Ia281.1-Ia281.164, die sich von den Verbindungen Ia1.1-Ia1.164 dadurch unterscheiden, daß R¹ für Methyl, R⁶ für iso-Butyl und R⁷ für Ethyl stehen: 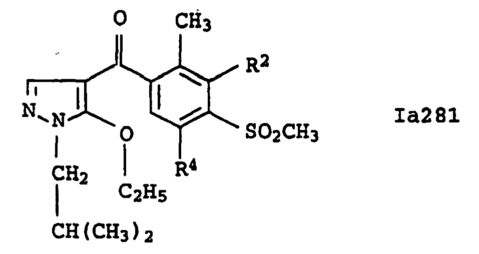
- Ebenso insbesondere außerordentlich bevorzugt sind die Verbindungen Ia282; insbesondere die Verbindungen Ia282.1-Ia282.164, die sich von den Verbindungen Ia1.1-Ia1.164 dadurch unterscheiden, daß R¹ für Methyl, R⁷ für Ethyl und R⁸ für Methyl stehen: 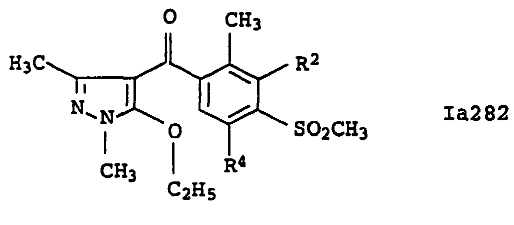
- Ebenso insbesondere außerordentlich bevorzugt sind die Verbindungen Ia283; insbesondere die Verbindungen Ia283.1-Ia283.164, die sich von den Verbindungen Ia1.1-Ia1.164 dadurch unterscheiden, daß R¹ für Methyl und R⁷ für Cyanomethyl stehen: 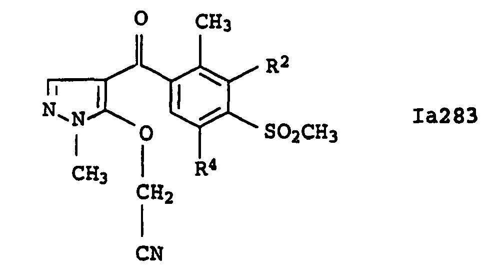
- Ebenso insbesondere außerordentlich bevorzugt sind die Verbindungen Ia284; insbesondere die Verbindungen Ia284.1-Ia284.164, die sich von den Verbindungen Ia1.1-Ia1.164 dadurch unterscheiden, daß R¹ für Methyl, R⁶ für Ethyl und R⁷ für Cyanomethyl stehen: 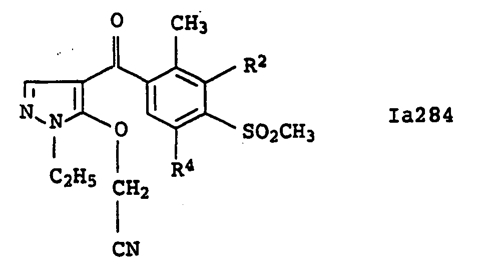
- Ebenso insbesondere außerordentlich bevorzugt sind die Verbindungen Ia285; insbesondere die Verbindungen Ia285.1-Ia285.164, die sich von den Verbindungen Ia1.1-Ia1.164 dadurch unterscheiden, daß R¹ für Methyl, R⁷ für Cyanomethyl und R⁸ für Methyl stehen: 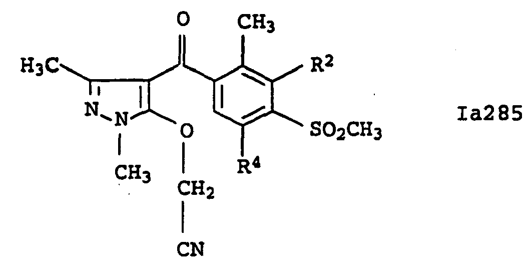
- Ebenso insbesondere außerordentlich bevorzugt sind die Verbindungen Ia286; insbesondere die Verbindungen Ia286.1-Ia286.164, die sich von den Verbindungen Ia1.1-Ia1.164 dadurch unterscheiden, daß R¹ für Methyl und R⁷ für Methoxymethyl stehen: 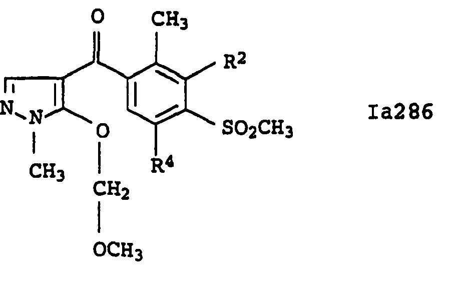
- Ebenso insbesondere außerordentlich bevorzugt sind die Verbindungen Ia287; insbesondere die Verbindungen Ia287.1-Ia287.164, die sich von den Verbindungen Ia1.1-Ia1.164 dadurch unterscheiden, daß R¹ für Methyl, R⁶ für Ethyl und R⁷ für Methoxymethyl stehen: 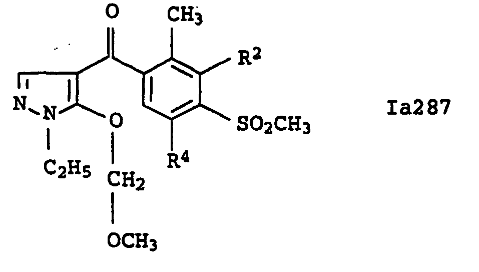
- Ebenso insbesondere außerordentlich bevorzugt sind die Verbindungen Ia288; insbesondere die Verbindungen Ia288.1-Ia288.164, die sich von den Verbindungen Ia1.1-Ia1.164 dadurch unterscheiden, daß R¹ für Methyl, R⁷ für Methoxymethyl und R⁸ für Methyl stehen: 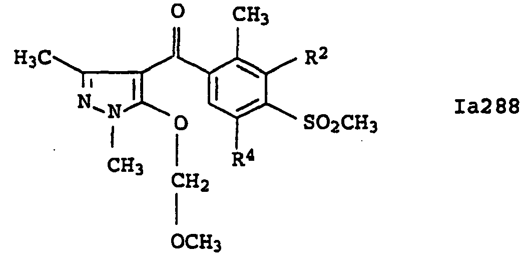
- Ebenso insbesondere außerordentlich bevorzugt sind die Verbindungen Ia289; insbesondere die Verbindungen Ia289.1-Ia289.164, die sich von den Verbindungen Ia1.1-Ia1.164 dadurch unterscheiden, daß R¹ für Methyl und R⁷ für Methylcarbonyloxymethyl stehen: 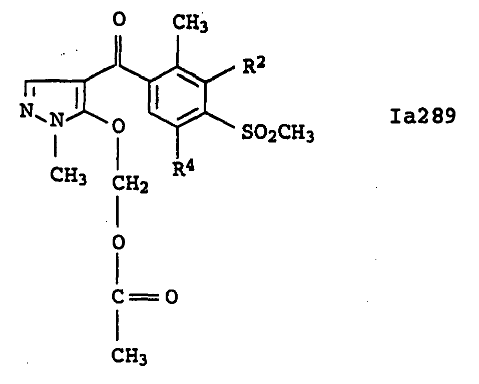
- Ebenso insbesondere außerordentlich bevorzugt sind die Verbindungen Ia290; insbesondere die Verbindungen Ia290.1-Ia290.164, die sich von den Verbindungen Ia1.1-Ia1.164 dadurch unterscheiden, daß R¹ für Methyl, R⁶ für Ethyl und R⁷ für Methylcarbonyloxymethyl stehen: 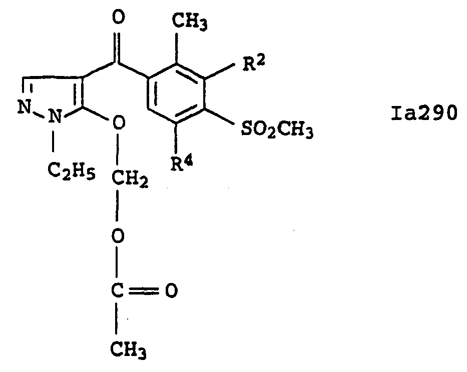
- Ebenso insbesondere außerordentlich bevorzugt sind die Verbindungen Ia291; insbesondere die Verbindungen Ia291.1-Ia291.164, die sich von den Verbindungen Ia1.1-Ia1.164 dadurch unterscheiden, daß R¹ für Methyl, R⁷ für Methylcarbonyloxymethyl und R⁸ für Methyl stehen: 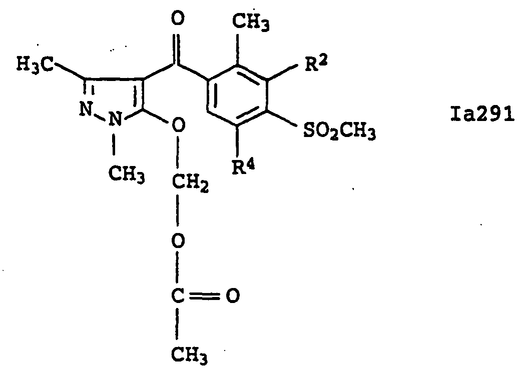
- Ebenso insbesondere außerordentlich bevorzugt sind die Verbindungen Ia292; insbesondere die Verbindungen Ia292.1-Ia292.164, die sich von den Verbindungen Ia1.1-Ia1.164 dadurch unterscheiden, daß R¹ für Methyl und R⁷ für tert.-Butylcarbonyloxymethyl stehen: 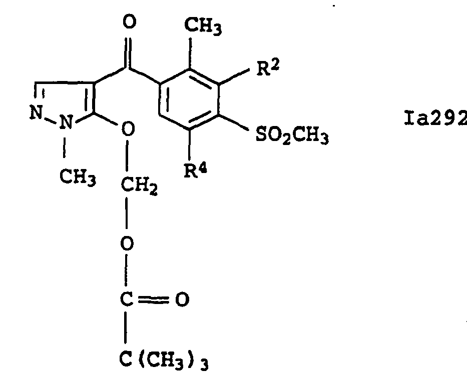
- Ebenso insbesondere außerordentlich bevorzugt sind die Verbindungen Ia293; insbesondere die Verbindungen Ia293.1-Ia293.164, die sich von den Verbindungen Ia1.1-Ia1.164 dadurch unterscheiden, daß R¹ für Methyl, R⁶ für Ethyl und R⁷ für tert.-Butylcarbonyloxymethyl stehen: 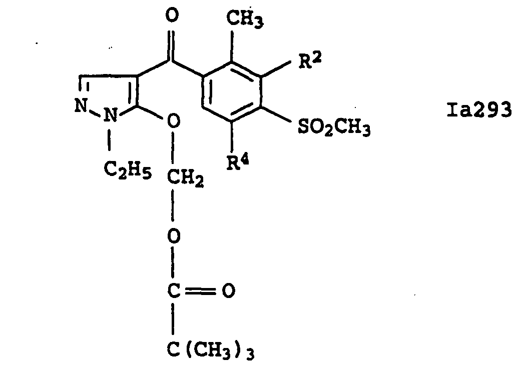
- Ebenso insbesondere außerordentlich bevorzugt sind die Verbindungen Ia294; insbesondere die Verbindungen Ia294.1-Ia294.164, die sich von den Verbindungen Ia1.1-Ia1.164 dadurch unterscheiden, daß R¹ für Methyl, R⁷ für tert.-Butylcarbonyloxymethyl und R⁸ für Methyl stehen: 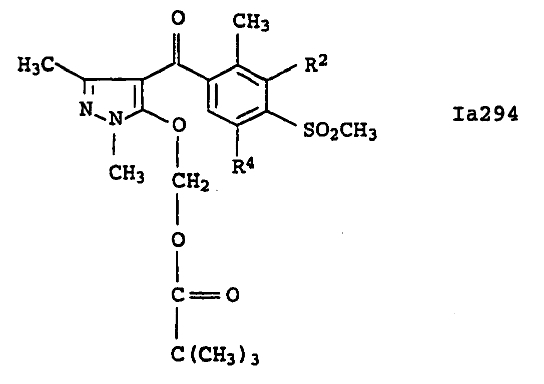
- Ebenso insbesondere außerordentlich bevorzugt sind die Verbindungen Ia295; insbesondere die Verbindungen Ia295.1-Ia295.164, die sich von den Verbindungen Ia1.1-Ia1.164 dadurch unterscheiden, daß R¹ für Methyl und R⁷ für Methoxycarbonylmethyl stehen: 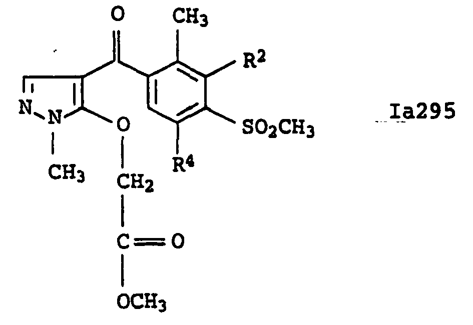
- Ebenso insbesondere außerordentlich bevorzugt sind die Verbindungen Ia296; insbesondere die Verbindungen Ia296.1-Ia296.164, die sich von den Verbindungen Ia1.1-Ia1.164 dadurch unterscheiden, daß R¹ für Methyl, R⁶ für Ethyl und R⁷ für Methoxycarbonylmethyl stehen: 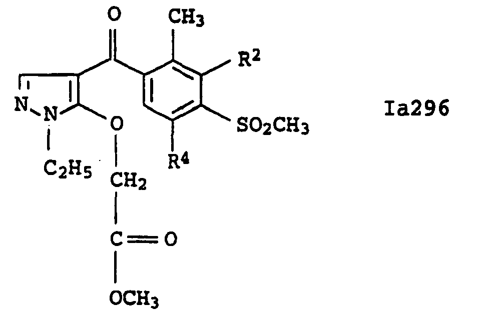
- Ebenso insbesondere außerordentlich bevorzugt sind die Verbindungen Ia297; insbesondere die Verbindungen Ia297.1-Ia297.164, die sich von den Verbindungen Ia1.1-Ia1.164 dadurch unterscheiden, daß R¹ für Methyl, R⁷ für Methoxycarbonylmethyl und R⁸ für Methyl stehen: 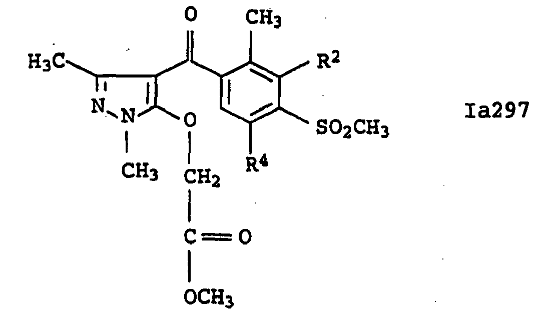
- Ebenso insbesondere außerordentlich bevorzugt sind die Verbindungen Ia298; insbesondere die Verbindungen Ia298.1-Ia298.164, die sich von den Verbindungen Ia1.1-Ia1.164 dadurch unterscheiden, daß R¹ für Methyl und R⁷ für Ethoxycarbonylmethyl stehen: 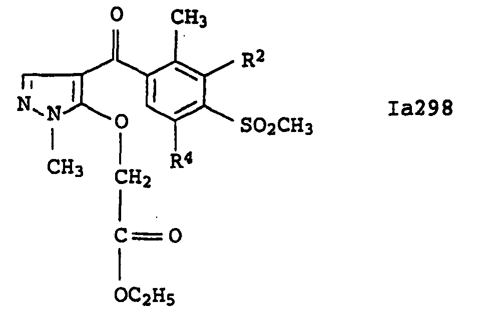
- Ebenso insbesondere außerordentlich bevorzugt sind die Verbindungen Ia299; insbesondere die Verbindungen Ia299.1-Ia299.164, die sich von den Verbindungen Ia1.1-Ia1.164 dadurch unterscheiden, daß R¹ für Methyl, R⁶ für Ethyl und R⁷ für Ethoxycarbonylmethyl stehen: 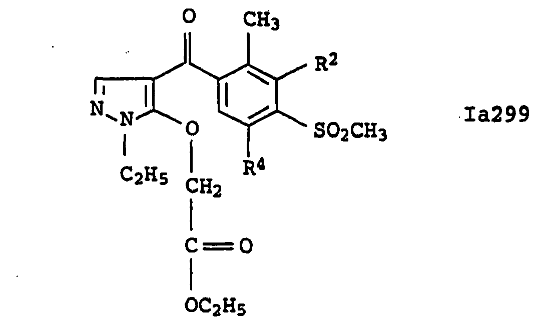
- Ebenso insbesondere außerordentlich bevorzugt sind die Verbindungen Ia300; insbesondere die Verbindungen Ia300.1-Ia300.164, die sich von den Verbindungen Ia1.1-Ia1.164 dadurch unterscheiden, daß R¹ für Methyl, R⁷ für Ethoxycarbonylmethyl und R⁸ für Methyl stehen: 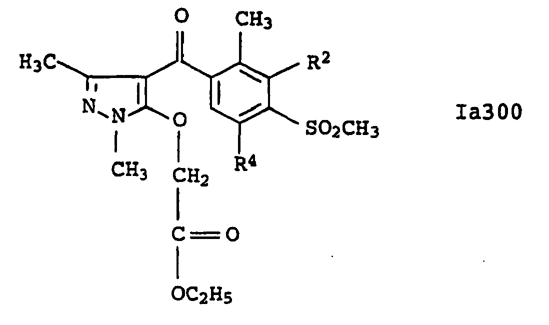
- Ebenso insbesondere außerordentlich bevorzugt sind die Verbindungen Ia301; insbesondere die Verbindungen Ia301.1-Ia301.164, die sich von den Verbindungen Ia1.1-Ia1.164 dadurch unterscheiden, daß R¹ für Methyl und R⁷ für 1-Methoxycarbonyl-eth-1-yl stehen: 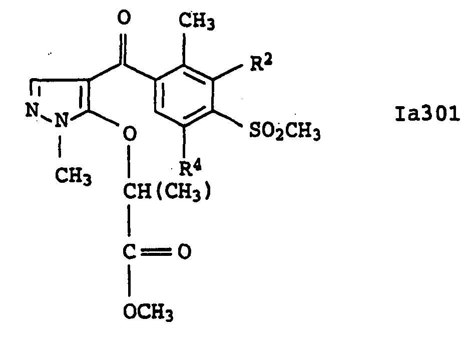
- Ebenso insbesondere außerordentlich bevorzugt sind die Verbindungen Ia302; insbesondere die Verbindungen Ia302.1-Ia302.164, die sich von den Verbindungen Ia1.1-Ia1.164 dadurch unterscheiden, daß R¹ für Methyl, R⁶ für Ethyl und R⁷ für 1-Methoxycarbonyl-eth-1-yl stehen: 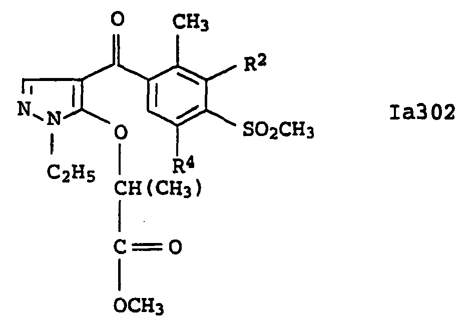
- Ebenso insbesondere außerordentlich bevorzugt sind die Verbindungen Ia303; insbesondere die Verbindungen Ia303.1-Ia303.164, die sich von den Verbindungen Ia1.1-Ia1.164 dadurch unterscheiden, daß R¹ für Methyl, R⁷ für 1-Methoxycarbonyl-eth-1-yl und R⁸ für Methyl stehen: 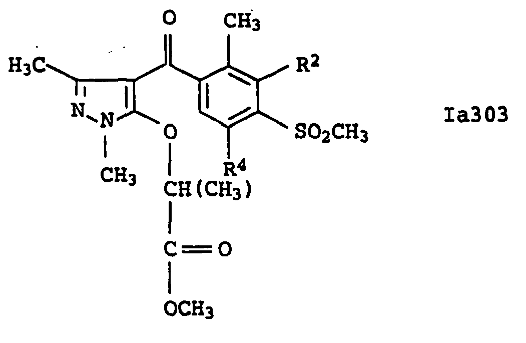
- Ebenso insbesondere außerordentlich bevorzugt sind die Verbindungen Ia304; insbesondere die Verbindungen Ia304.1-Ia304.164, die sich von den Verbindungen Ia1.1-Ia1.164 dadurch unterscheiden, daß R¹ für Methyl und R⁷ für 1-Ethoxycarbonyl-eth-1-yl stehen: 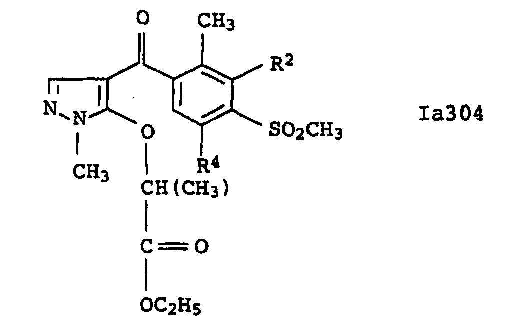
- Ebenso insbesondere außerordentlich bevorzugt sind die Verbindungen Ia305; insbesondere die Verbindungen Ia305.1-Ia305.164, die sich von den Verbindungen Ia1.1-Ia1.164 dadurch unterscheiden, daß R¹ für Methyl, R⁶ für Ethyl und R⁷ für 1-Ethoxycarbonyl-eth-1-yl stehen: 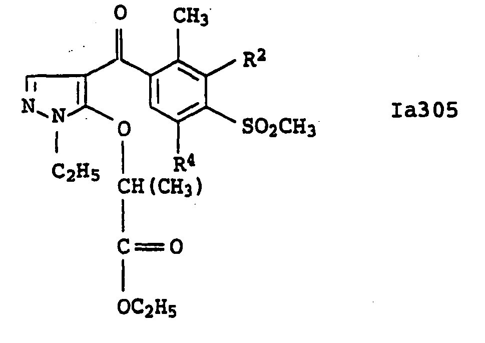
- Ebenso insbesondere außerordentlich bevorzugt sind die Verbindungen Ia306; insbesondere die Verbindungen Ia306.1-Ia306.164, die sich von den Verbindungen Ia1.1-Ia1.164 dadurch unterscheiden, daß R¹ für Methyl, R⁷ für 1-Ethoxycarbonyl-eth-1-yl und R⁸ für Methyl stehen: 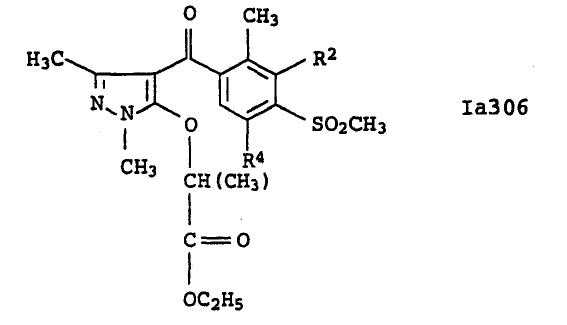
- Ebenso insbesondere außerordentlich bevorzugt sind die Verbindungen Ia307; insbesondere die Verbindungen Ia307.1-Ia307.164, die sich von den Verbindungen Ia1.1-Ia1.164 dadurch unterscheiden, daß R¹ für Methyl und R⁷ für 2-Methoxy-eth-1-oxycarbonylmethyl stehen: 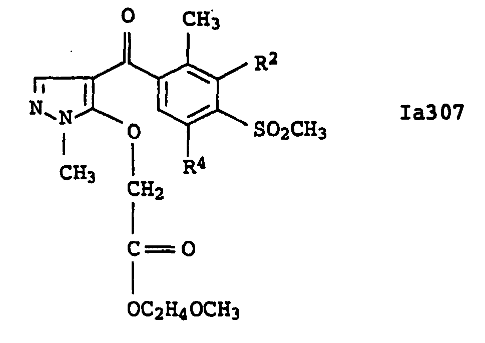
- Ebenso insbesondere außerordentlich bevorzugt sind die Verbindungen Ia308; insbesondere die Verbindungen Ia308.1-Ia308.164, die sich von den Verbindungen Ia1.1-Ia1.164 dadurch unterscheiden, daß R¹ für Methyl, R⁶ für Ethyl und R⁷ für 2-Methoxy-eth-1-oxycarbonylmethyl stehen: 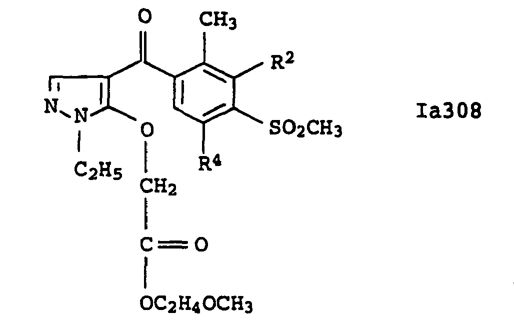
- Ebenso insbesondere außerordentlich bevorzugt sind die Verbindungen Ia309; insbesondere die Verbindungen Ia309.1-Ia309.164, die sich von den Verbindungen Ia1.1-Ia1.164 dadurch unterscheiden, daß R¹ für Methyl, R⁷ für 2-Methoxy-eth-1-oxycarbonylmetyl und R⁸ für Methyl stehen: 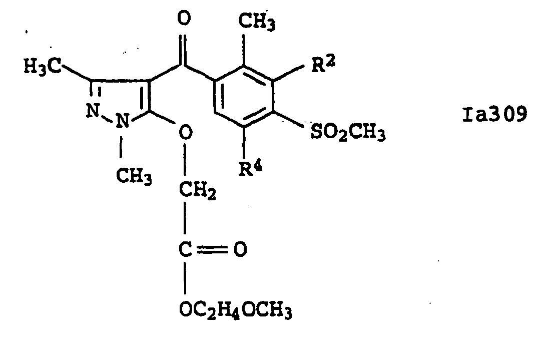
- Ebenso insbesondere außerordentlich bevorzugt sind die Verbindungen Ia310; insbesondere die Verbindungen Ia310.1-Ia310.164, die sich von den Verbindungen Ia1.1-Ia1.164 dadurch unterscheiden, daß R¹ für Methyl und R⁷ für 2-Dimethylamino-eth-1-oxycarbonylmethyl stehen: 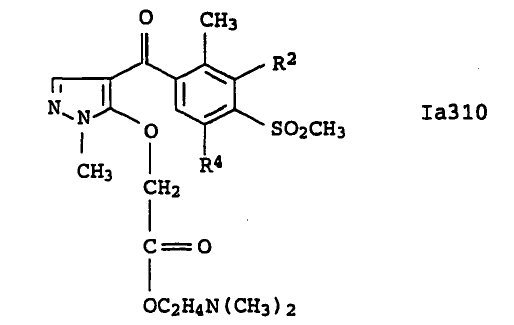
- Ebenso insbesondere außerordentlich bevorzugt sind die Verbindungen Ia311; insbesondere die Verbindungen Ia311.1-Ia311.164, die sich von den Verbindungen Ia1.1-Ia1.164 dadurch unterscheiden, daß R¹ für Methyl, R⁶ für Ethyl und R⁷ für 2-Dimethylamino-eth-1-oxycarbonylmethyl stehen: 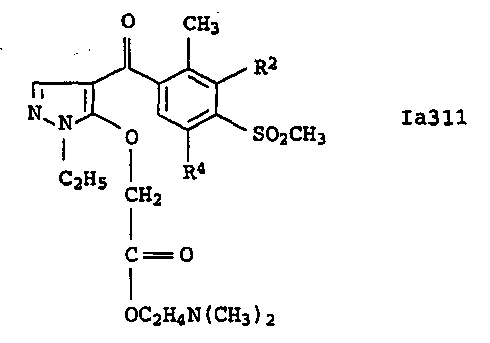
- Ebenso insbesondere außerordentlich bevorzugt sind die Verbindungen Ia312; insbesondere die Verbindungen Ia312.1-Ia313.164, die sich von den Verbindungen Ia1.1-Ia1.164 dadurch unterscheiden, daß R¹ für Methyl, R⁷ für 2-Dimethylamino-eth-1-oxycarbonylmethyl und R⁸ für Methyl stehen: 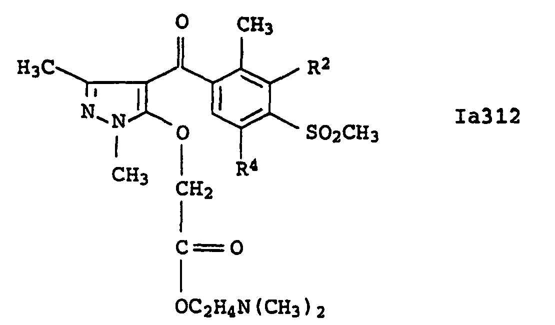
- Ebenso insbesondere außerordentlich bevorzugt sind die Verbindungen Ia313; insbesondere die Verbindungen Ia313.1-Ia313.164, die sich von den Verbindungen Ia1.1-Ia1.164 dadurch unterscheiden, daß R¹ für Methyl und R⁷ für Methylaminocarbonylmethyl stehen: 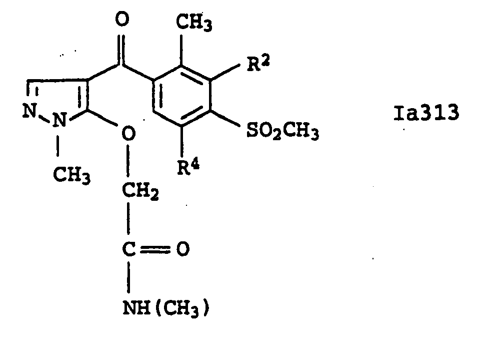
- Ebenso insbesondere außerordentlich bevorzugt sind die Verbindungen Ia314; insbesondere die Verbindungen Ia314.1-Ia314.164, die sich von den Verbindungen Ia1.1-Ia1.164 dadurch unterscheiden, daß R¹ für Methyl, R⁶ für Ethyl und R⁷ für Methylaminocarbonylmethyl stehen: 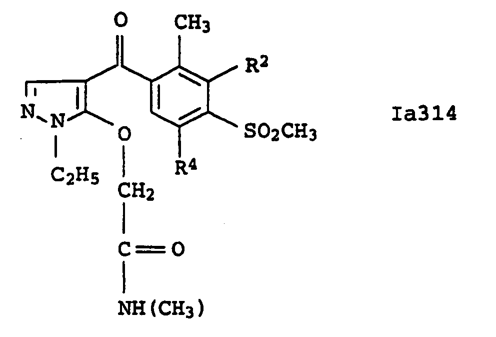
- Ebenso insbesondere außerordentlich bevorzugt sind die Verbindungen Ia315; insbesondere die Verbindungen Ia315.1-Ia315.164, die sich von den Verbindungen Ia1.1-Ia1.164 dadurch unterscheiden, daß R¹ für Methyl, R⁷ für Methylaminocarbonylmethyl und R⁸ für Methyl stehen: 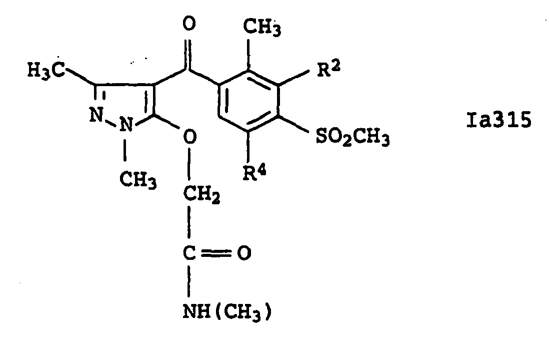
- Ebenso insbesondere außerordentlich bevorzugt sind die Verbindungen Ia316; insbesondere die Verbindungen Ia316.1-Ia316.164, die sich von den Verbindungen Ial.1-Ial.164 dadurch unterscheiden, daß R¹ für Methyl und R⁷ für Ethylaminocarbonylmethyl stehen : 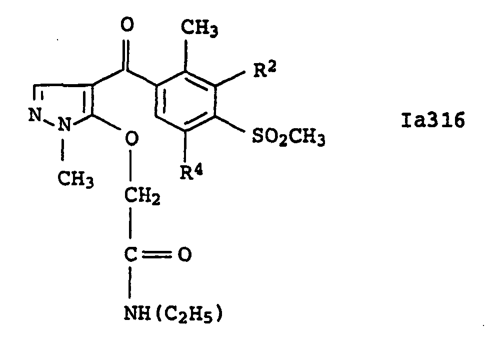
- Ebenso insbesondere außerordentlich bevorzugt sind die Verbindungen Ia317; insbesondere die Verbindungen Ia317.1-Ia317.164, die sich von den Verbindungen Ia1.1-Ia1.164 dadurch unterscheiden, daß R¹ für Methyl, R⁶ für Ethyl und R⁷ für Ethylaminocarbonylmethyl stehen: 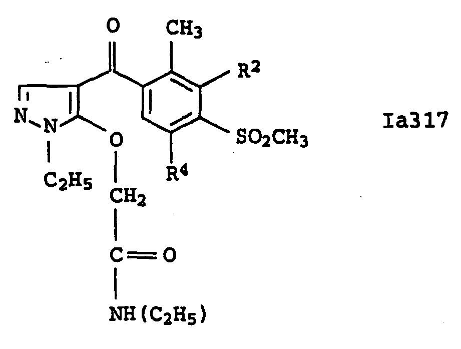
- Ebenso insbesondere außerordentlich bevorzugt sind die Verbindungen Ia318; insbesondere die Verbindungen Ia318.1-Ia318.164, die sich von den Verbindungen Ia1.1-Ia1.164 dadurch unterscheiden, daß R¹ für Methyl, R⁷ für Ethylaminocarbonylmethyl und R⁸ für Methyl stehen: 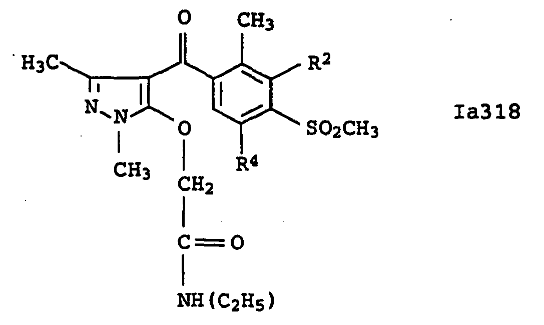
- Ebenso insbesondere außerordentlich bevorzugt sind die Verbindungen Ia319; insbesondere die Verbindungen Ia319.1-Ia319.164, die sich von den Verbindungen Ia1.1-Ia1.164 dadurch unterscheiden, daß R¹ für Methyl und R⁷ für Dimethylaminocarbonylmethyl stehen: 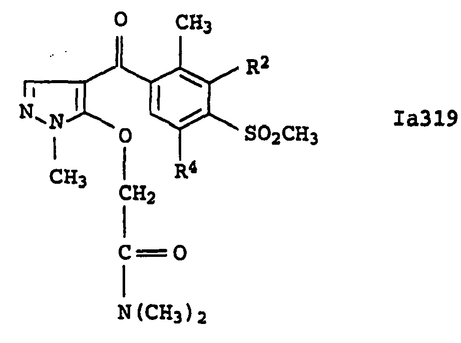
- Ebenso insbesondere außerordentlich bevorzugt sind die Verbindungen Ia320; insbesondere die Verbindungen Ia320.1-Ia320.164, die sich von den Verbindungen Ia1.1-Ia1.164 dadurch unterscheiden, daß R¹ für Methyl, R⁶ für Ethyl und R⁷ für Dimethylaminocarbonylmethyl stehen: 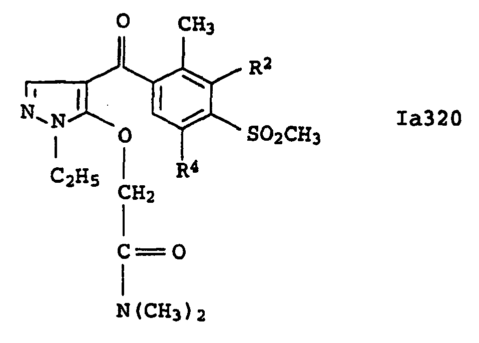
- Ebenso insbesondere außerordentlich bevorzugt sind die Verbindungen Ia321; insbesondere die Verbindungen Ia321.1-Ia321.164, die sich von den Verbindungen Ia1.1-Ia1.164 dadurch unterscheiden, daß R¹ für Methyl, R⁷ für Dimethylaminocarbonylmethyl und R⁸ für Methyl stehen: 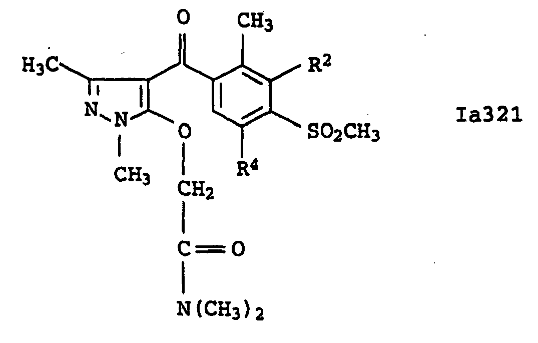
- Ebenso insbesondere außerordentlich bevorzugt sind die Verbindungen Ia322; insbesondere die Verbindungen Ia322.1-Ia322.164, die sich von den Verbindungen Ia1.1-Ia1.164 dadurch unterscheiden, daß R¹ für Methyl und R⁷ für Diethylaminocarbonylmethyl stehen: 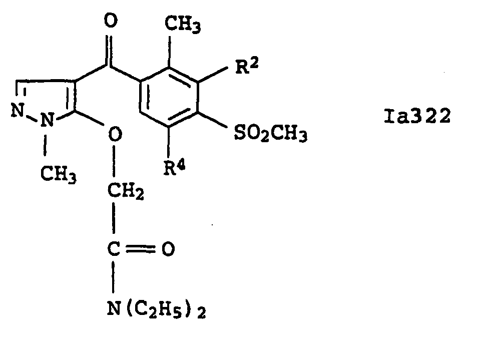
- Ebenso insbesondere außerordentlich bevorzugt sind die Verbindungen Ia323; insbesondere die Verbindungen Ia323.1-Ia323.164, die sich von den Verbindungen Ia1.1-Ia1.164 dadurch unterscheiden, daß R¹ für Methyl, R⁶ für Ethyl und R⁷ für Diethylaminocarbonylmethyl stehen: 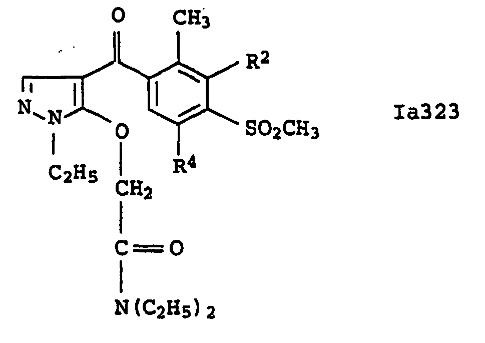
- Ebenso insbesondere außerordentlich bevorzugt sind die Verbindungen Ia324; insbesondere die Verbindungen Ia324.1-Ia324.164, die sich von den Verbindungen Ia1.1-Ia1.164 dadurch unterscheiden, daß R¹ für Methyl, R⁷ für Diethylaminocarbonylmethyl und R⁸ für Methyl Stehen: 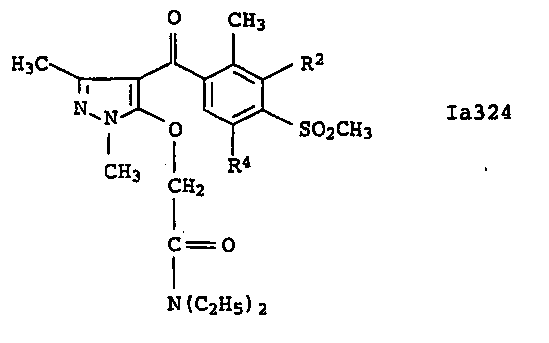
- Ebenso insbesondere außerordentlich bevorzugt sind die Verbindungen Ia325; insbesondere die Verbindungen Ia325.1-Ia325.164, die sich von den Verbindungen Ia1.1-Ia1.164 dadurch unterscheiden, daß R¹ für Methyl und R⁷ für Allyl stehen: 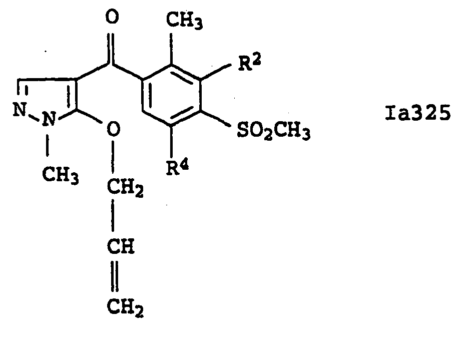
- Ebenso insbesondere außerordentlich bevorzugt sind die Verbindungen Ia326; insbesondere die Verbindungen Ia326.1-Ia326.164, die sich von den Verbindungen Ia1.1-Ia1.164 dadurch unterscheiden, daß R¹ für Methyl, R⁶ für Ethyl und R⁷ für Allyl stehen: 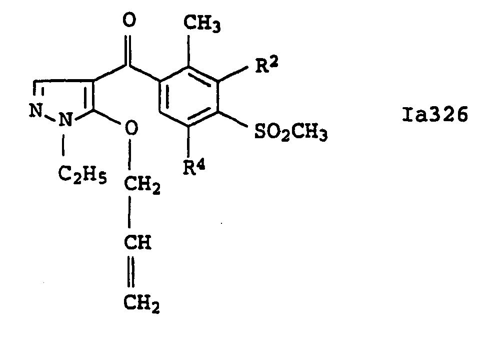
- Ebenso insbesondere außerordentlich bevorzugt sind die Verbindungen Ia327; insbesondere die Verbindungen Ia327.1-Ia327.164, die sich von den Verbindungen Ia1.1-Ia1.164 dadurch unterscheiden, daß R¹ für Methyl, R⁷ für Allyl und R⁸ für Methyl stehen: 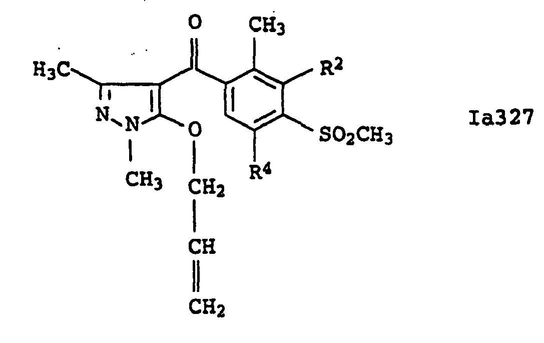
- Ebenso insbesondere außerordentlich bevorzugt sind die Verbindungen Ia328; insbesondere die Verbindungen Ia328.1-Ia328.164, die sich von den Verbindungen Ia1.1-Ia1.164 dadurch unterscheiden, daß R¹ für Methyl und R⁷ für Propargyl stehen: 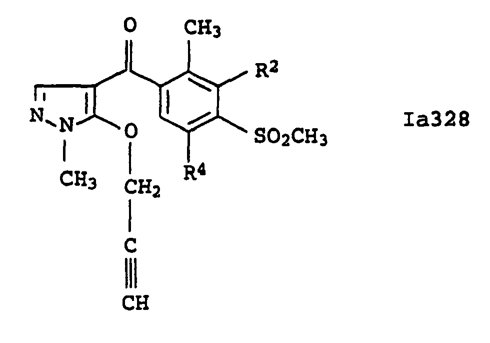
- Ebenso insbesondere außerordentlich bevorzugt sind die Verbindungen Ia329; insbesondere die Verbindungen Ia329.1-1a329.164, die sich von den Verbindungen Ia1.1-Ia1.164 dadurch unterscheiden, daß R¹ für Methyl, R⁶ für Ethyl und R⁷ für Propargyl stehen: 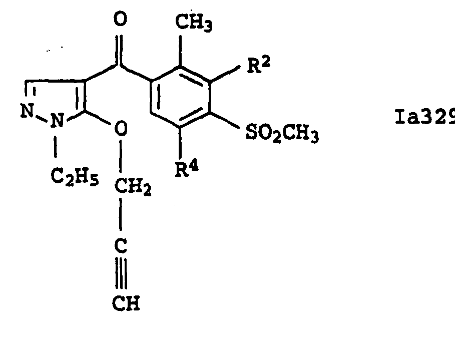
- Ebenso insbesondere außerordentlich bevorzugt sind die Verbindungen Ia330; insbesondere die Verbindungen Ia330.1-Ia330.164, die sich von den Verbindungen Ia1.1-Ia1.164 dadurch unterscheiden, daß R¹ für Methyl, R⁷ für Propargyl und R⁸ für Methyl stehen: 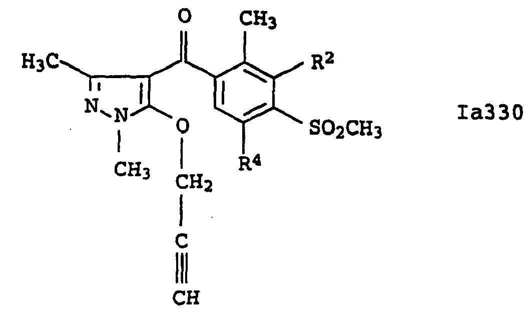
- Ebenso insbesondere außerordentlich bevorzugt sind die Verbindungen Ia331; insbesondere die Verbindungen Ia331.1-Ia331.164, die sich von den Verbindungen Ia1.1-Ia1.164 dadurch unterscheiden, daß R¹ für Methyl und R⁷ für Methylcarbonyl stehen: 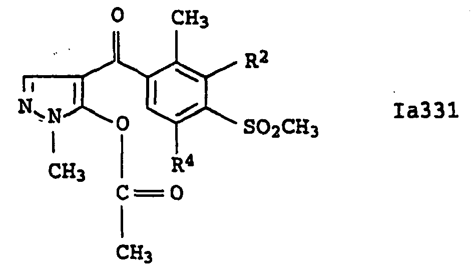
- Ebenso insbesondere außerordentlich bevorzugt sind die Verbindungen Ia332; insbesondere die Verbindungen Ia332.1-Ia332.164, die sich von den Verbindungen Ia1.1-Ia1.164 dadurch unterscheiden, daß R¹ für Methyl, R⁶ für Ethyl und R⁷ für Methylcarbonyl stehen: 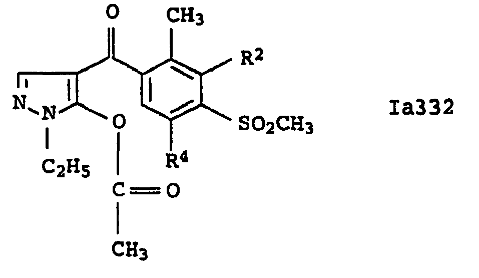
- Ebenso insbesondere außerordentlich bevorzugt sind die Verbindungen Ia333; insbesondere die Verbindungen Ia333.1-Ia333.164, die sich von den Verbindungen Ia1.1-Ia1.164 dadurch unterscheiden, daß R¹ für Methyl, R⁶ für n-Propyl und R⁷ für Methylcarbonyl stehen: 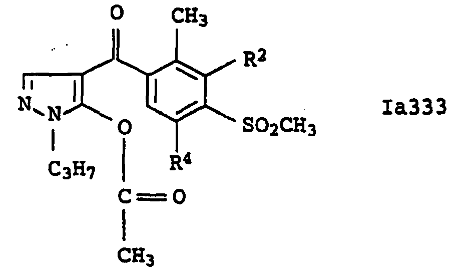
- Ebenso insbesondere außerordentlich bevorzugt sind die Verbindungen Ia334; insbesondere die Verbindungen Ia334.1-Ia334.164, die sich von den Verbindungen Ia1.1-Ia1.164 dadurch unterscheiden, daß R¹ für Methyl, R⁶ für iso-Butyl und R⁸ für Methylcarbonyl stehen: 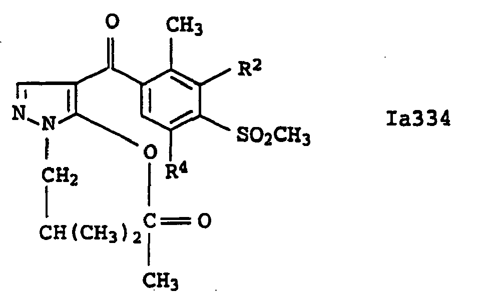
- Ebenso insbesondere außerordentlich bevorzugt sind die Verbindungen Ia335; insbesondere die Verbindungen Ia335.1-Ia335.164, die sich von den Verbindungen Ia1.1-Ia1.164 dadurch unterscheiden, daß R¹ für Methyl, R⁷ für Methylcarbonyl und R⁸ für Methyl stehen: 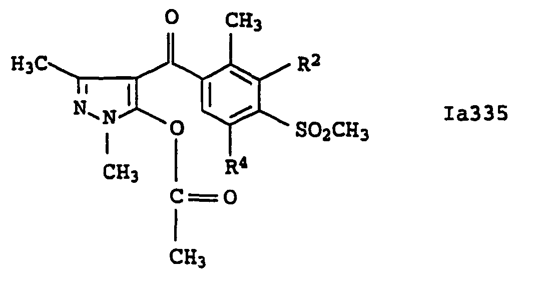
- Ebenso insbesondere außerordentlich bevorzugt sind die Verbindungen Ia336; insbesondere die Verbindungen Ia336.1-Ia336.164, die sich von den Verbindungen Ia1.1-Ia1.164 dadurch unterscheiden, daß R¹ für Methyl und R⁷ für Ethylcarbonyl stehen: 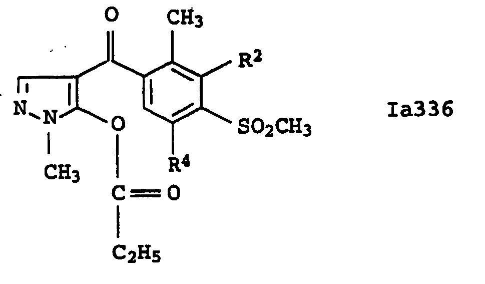
- Ebenso insbesondere außerordentlich bevorzugt sind die Verbindungen Ia337; insbesondere die Verbindungen Ia337.1-Ia337.164, die sich von den Verbindungen Ia1.1-Ia1.164 dadurch unterscheiden, daß R¹ für Methyl, R⁶ für Ethyl und R⁷ für Ethylcarbonyl stehen: 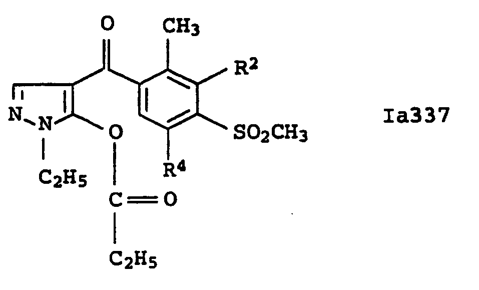
- Ebenso insbesondere außerordentlich bevorzugt sind die Verbindungen Ia338; insbesondere die Verbindungen Ia338.1-Ia338.164, die sich von den Verbindungen Ia1.1-Ia1.164 dadurch unterscheiden, daß R¹ für Methyl, R⁶ für n-Propyl und R⁷ für Ethylcarbonyl stehen: 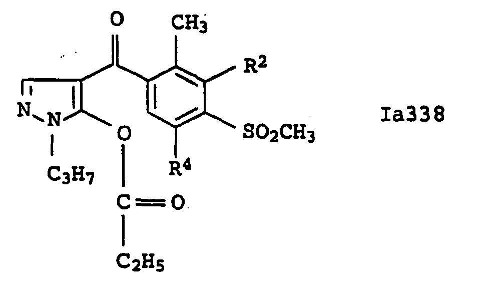
- Ebenso insbesondere außerordentlich bevorzugt sind die Verbindungen Ia339; insbesondere die Verbindungen Ia339.1-Ia339.164, die sich von den Verbindungen Ia1.1-Ia1.164 dadurch unterscheiden, daß R¹ für Methyl, R⁶ für iso-Butyl und R⁷ für Ethylcarbonyl stehen: 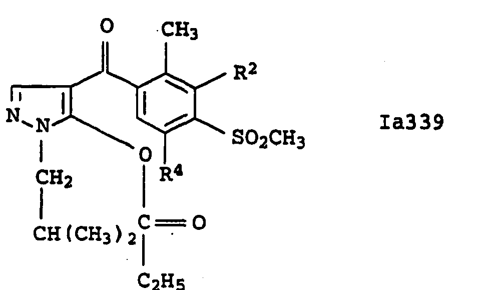
- Ebenso insbesondere außerordentlich bevorzugt sind die Verbindungen Ia340; insbesondere die Verbindungen Ia340.1-Ia340.164, die sich von den Verbindungen Ia1.1-Ia1.164 dadurch unterscheiden, daß R¹ für Methyl, R⁷ für Ethylcarbonyl und R⁸ für Methyl stehen: 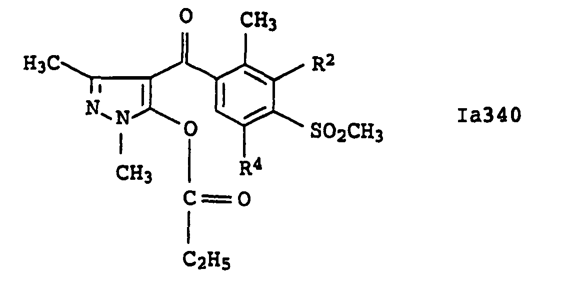
- Ebenso insbesondere außerordentlich bevorzugt sind die Verbindungen Ia341; insbesondere die Verbindungen Ia341.1-Ia341.164, die sich von den Verbindungen Ia1.1-Ia1.164 dadurch unterscheiden, daß R¹ für Methyl und R⁷ für Methoxycarbonyl stehen: 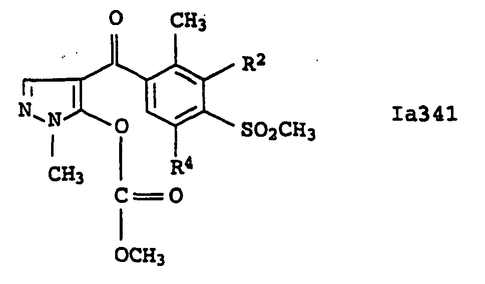
- Ebenso insbesondere außerordentlich bevorzugt sind die Verbindungen Ia342; insbesondere die Verbindungen Ia342.1-Ia342.164, die sich von den Verbindungen Ia1.1-Ia1.164 dadurch unterscheiden, daß R¹ für Methyl, R⁶ für Ethyl und R⁷ für Methoxycarbonyl stehen: 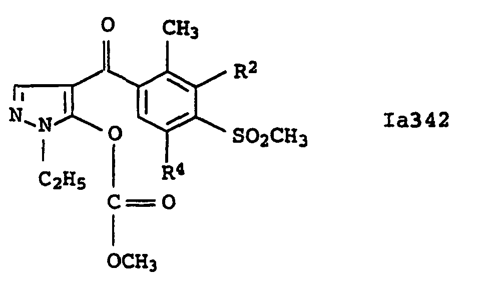
- Ebenso insbesondere außerordentlich bevorzugt sind die Verbindungen Ia343; insbesondere die Verbindungen Ia343.1-Ia343.164, die sich von den Verbindungen Ia1.1-Ia1.164 dadurch unterscheiden, daß R¹ für Methyl, R⁷ für Methoxycarbonyl und R⁸ für Methyl stehen: 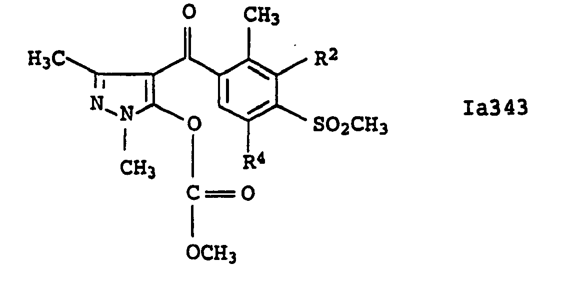
- Ebenso insbesondere außerordentlich bevorzugt sind die Verbindungen Ia344; insbesondere die Verbindungen Ia344.1-Ia344.164, die sich von den Verbindungen Ia1.1-Ia1.164 dadurch unterscheiden, daß R¹ für Methyl und R⁷ für Ethoxycarbonyl stehen: 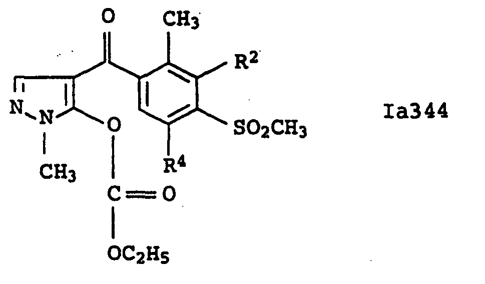
- Ebenso insbesondere außerordentlich bevorzugt sind die Verbindungen Ia345; insbesondere die Verbindungen Ia345.1-Ia345.164, die sich von den Verbindungen Ia1.1-Ia1.164 dadurch unterscheiden, daß R¹ für Methyl, R⁶ für Ethyl und R⁷ für Ethoxycarbonyl stehen: 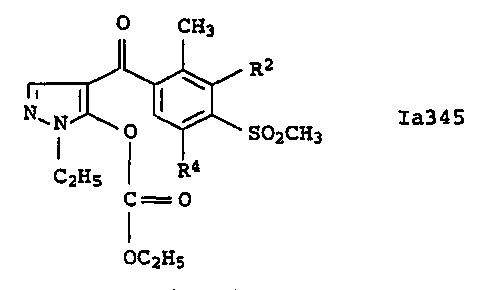
- Ebenso insbesondere außerordentlich bevorzugt sind die Verbindungen Ia346; insbesondere die Verbindungen Ia346.1-Ia346.164, die sich von den Verbindungen Ia1.1-Ia1.164 dadurch unterscheiden, daß R¹ für Methyl, R⁷ für Ethoxycarbonyl und R⁸ für Methyl stehen: 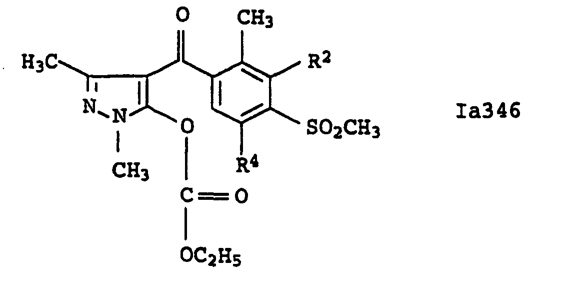
- Ebenso insbesondere außerordentlich bevorzugt sind die Verbindungen Ia347; insbesondere die Verbindungen Ia347.1-Ia347.164, die sich von den Verbindungen Ia1.1-Ia1.164 dadurch unterscheiden, daß R¹ für Methyl und R⁷ für Dimethylaminocarbonyl stehen: 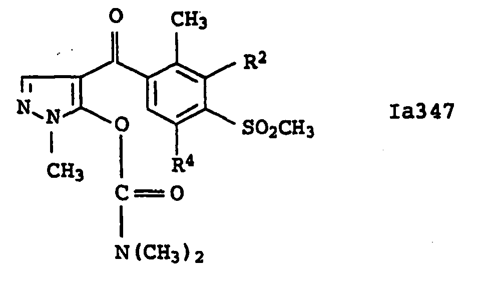
- Ebenso insbesondere außerordentlich bevorzugt sind die Verbindungen Ia348; insbesondere die Verbindungen Ia348.1-Ia348.164, die sich von den Verbindungen Ia1.1-Ia1.164 dadurch unterscheiden, daß R¹ für Methyl, R⁶ für Ethyl und R⁷ für Dimethylaminocarbonyl stehen: 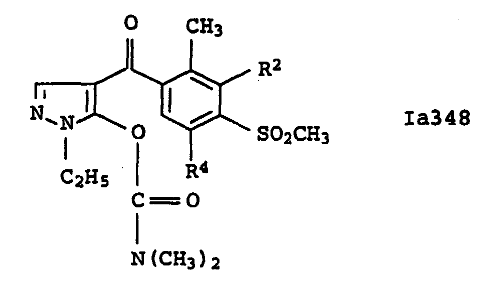
- Ebenso insbesondere außerordentlich bevorzugt sind die Verbindungen Ia349; insbesondere die Verbindungen Ia349.1-Ia349.164, die sich von den Verbindungen Ia1.1-Ia1.164 dadurch unterscheiden, daß R¹ für Methyl, R⁷ für Dimethylaminocarbonyl und R⁸ für Methyl stehen: 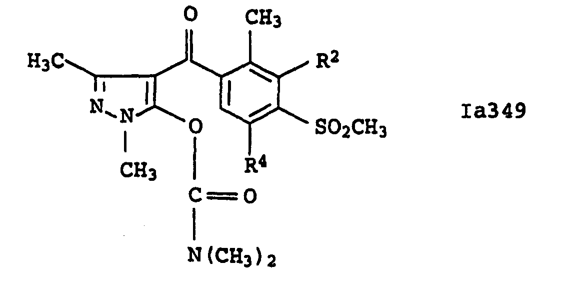
- Ebenso insbesondere außerordentlich bevorzugt sind die Verbindungen Ia350; insbesondere die Verbindungen Ia350.1-Ia350.164, die sich von den Verbindungen Ia1.1-Ia1.164 dadurch unterscheiden, daß R¹ für Methyl und R⁷ für Diethylaminocarbonyl stehen: 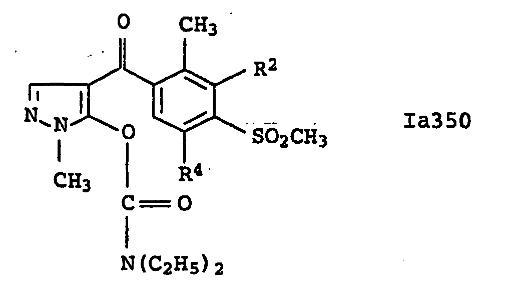
- Ebenso insbesondere außerordentlich bevorzugt sind die Verbindungen Ia351; insbesondere die Verbindungen Ia351.1-Ia351.164, die sich von den Verbindungen Ia1.1-Ia1.164 dadurch unterscheiden, daß R¹ für Methyl, R⁶ für Ethyl und R⁷ für Diethylaminocarbonyl stehen: 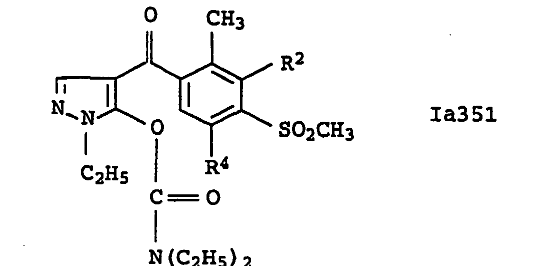
- Ebenso insbesondere außerordentlich bevorzugt sind die Verbindungen Ia352; insbesondere die Verbindungen Ia352.1-Ia352.164, die sich von den Verbindungen Ia1.1-Ia1.164 dadurch unterscheiden, daß R¹ für Methyl, R⁷ für Diethylaminocarbonyl und R⁸ für Methyl stehen: 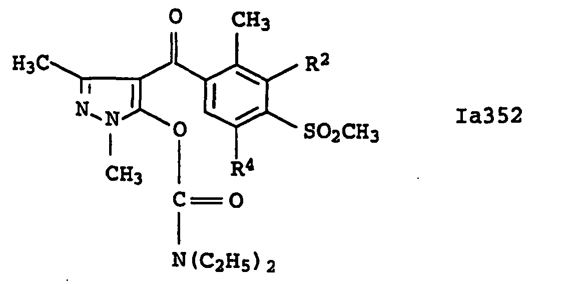
- Ebenso insbesondere außerordentlich bevorzugt sind die Verbindungen Ia353; insbesondere die Verbindungen Ia353.1-Ia353.164, die sich von den Verbindungen Ia1.1-Ia1.164 dadurch unterscheiden, daß R¹ für Methyl und R⁷ für N-Methoxy-N-methylaminocarbonyl stehen: 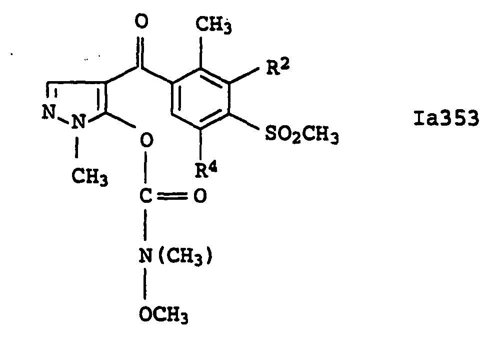
- Ebenso insbesondere außerordentlich bevorzugt sind die Verbindungen Ia354; insbesondere die Verbindungen Ia354.1-Ia354.164, die sich von den Verbindungen Ia1.1-Ia1.164 dadurch unterscheiden, daß R¹ für Methyl, R⁶ für Ethyl und R⁷ für N-Methoxy-N-methylaminocarbonyl stehen: 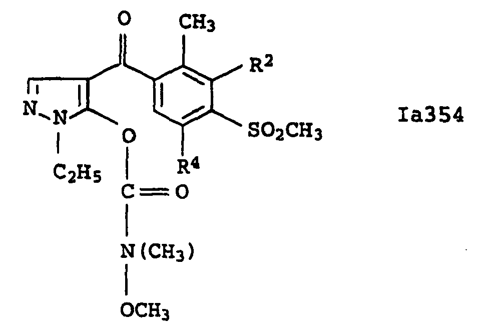
- Ebenso insbesondere außerordentlich bevorzugt sind die Verbindungen Ia355; insbesondere die Verbindungen Ia355.1-Ia355.164, die sich von den Verbindungen Ia1.1-Ia1.164 dadurch unterscheiden, daß R¹ für Methyl, R⁷ für N-Methoxy-N-methylaminocarbonyl und R⁸ für Methyl stehen: 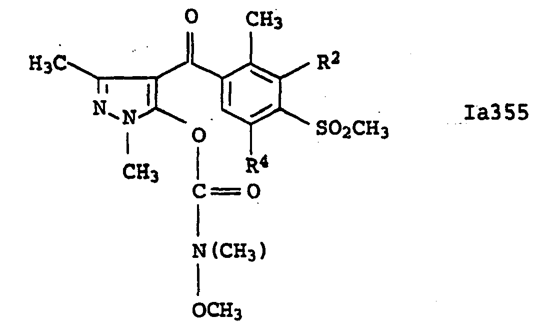
- Ebenso insbesondere außerordentlich bevorzugt sind die Verbindungen Ia356; insbesondere die Verbindungen Ia356.1-Ia356.164, die sich von den Verbindungen Ia1.1-Ia1.164 dadurch unterscheiden, daß R¹ für Methyl und R⁷ für Benzyl stehen: 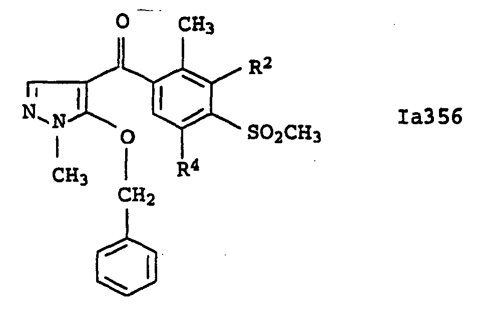
- Ebenso insbesondere außerordentlich bevorzugt sind die Verbindungen Ia357; insbesondere die Verbindungen Ia357.1-Ia357.164, die sich von den Verbindungen Ial.l-Ial.164 dadurch unterscheiden, daß R¹ für Methyl, R⁶ für Ethyl und R⁷ für Benzyl stehen: 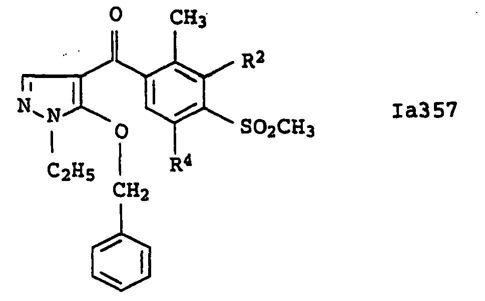
- Ebenso insbesondere außerordentlich bevorzugt sind die Verbindungen Ia358; insbesondere die Verbindungen Ia358.1-Ia358.164, die sich von den Verbindungen Ia1.1-Ia1.164 dadurch unterscheiden, daß R¹ für Methyl, R⁶ für n-Propyl und R⁷ für Benzyl stehen: 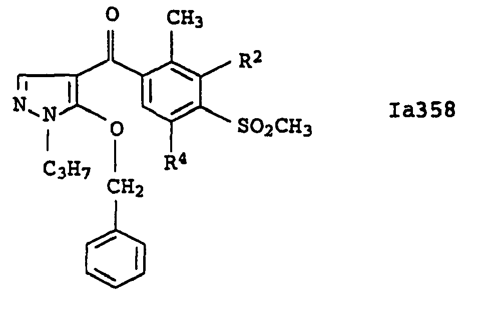
- Ebenso insbesondere außerordentlich bevorzugt sind die Verbindungen Ia359; insbesondere die Verbindungen Ia359.1-Ia359.164, die sich von den Verbindungen Ia1.1-Ia1.164 dadurch unterscheiden, daß R¹ für Methyl, R⁶ für iso-Butyl und R⁷ für Benzyl stehen: 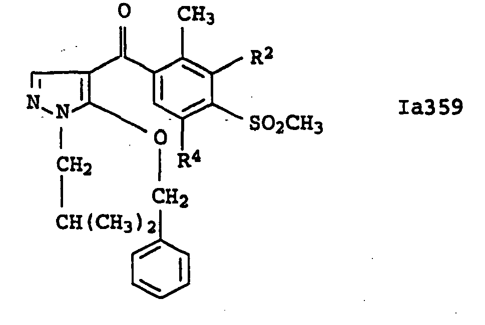
- Ebenso insbesondere außerordentlich bevorzugt sind die Verbindungen Ia360; insbesondere die Verbindungen Ia360.1-Ia360.164, die sich von den Verbindungen Ia1.1-Ia1.164 dadurch unterscheiden, daß R¹ für Methyl, R⁷ für Benzyl und R⁸ für Methyl stehen: 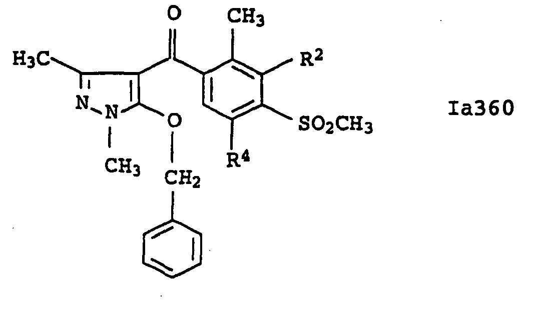
- Ebenso insbesondere außerordentlich bevorzugt sind die Verbindungen Ia361; insbesondere die Verbindungen Ia361.1-Ia361.164, die sich von den Verbindungen Ia1.1-Ia1.164 dadurch unterscheiden, daß R¹ für Methyl und R⁷ für 4-Methylphenylmethyl stehen: 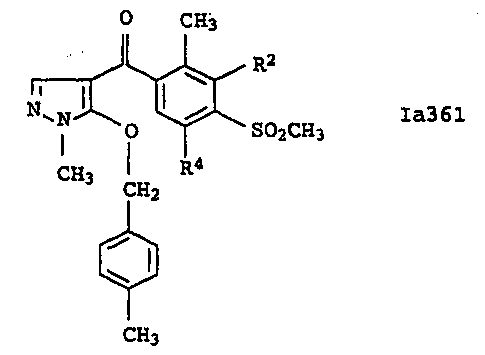
- Ebenso insbesondere außerordentlich bevorzugt sind die Verbindungen Ia362; insbesondere die Verbindungen Ia362.1-Ia362.164, die sich von den Verbindungen Ia1.1-Ia1.164 dadurch unterscheiden, daß R¹ für Methyl, R⁶ für Ethyl und R⁷ für 4-Methylphenylmethyl stehen: 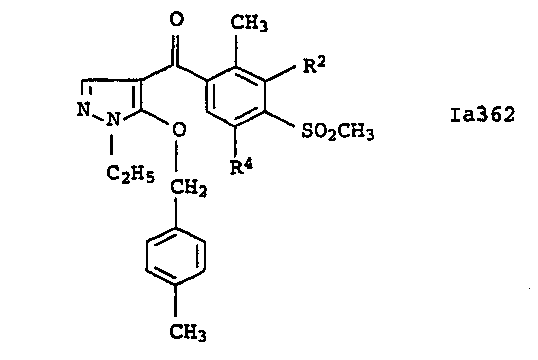
- Ebenso insbesondere außerordentlich bevorzugt sind die Verbindungen Ia363; insbesondere die Verbindungen Ia363.1-Ia363.164, die sich von den Verbindungen Ia1.1-Ia1.164 dadurch unterscheiden, daß R¹ für Methyl, R⁶ für n-Propyl und R⁷ für 4-Methylphenylmethyl stehen: 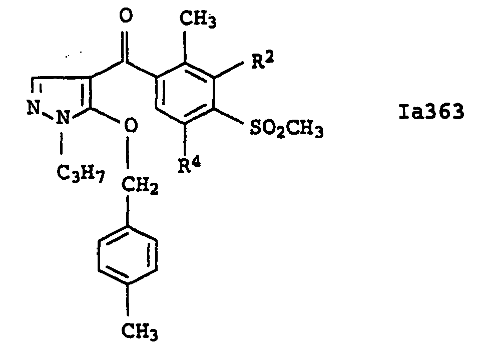
- Ebenso insbesondere außerordentlich bevorzugt sind die Verbindungen Ia364; insbesondere die Verbindungen Ia364.1-Ia364.164, die sich von den Verbindungen Ia1.1-Ia1.164 dadurch unterscheiden, daß R¹ für Methyl, R⁶ für iso-Butyl und R⁷ für 4-Methylphenylmethyl stehen: 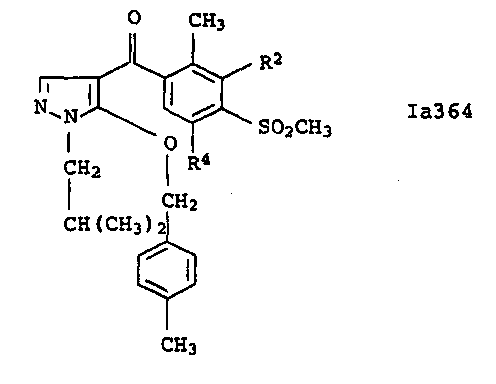
- Ebenso insbesondere außerordentlich bevorzugt sind die Verbindungen Ia365; insbesondere die Verbindungen Ia365.1-Ia365.164, die sich von den Verbindungen Ia1.1-Ia1.164 dadurch unterscheiden, daß R¹ für Methyl, R⁷ für 4-Methylphenylmethyl und R⁸ für Methyl stehen: 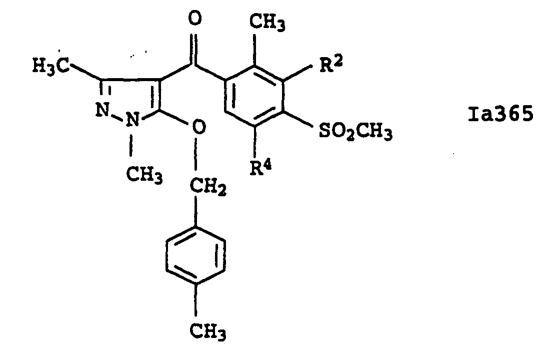
- Ebenso insbesondere außerordentlich bevorzugt sind die Verbindungen Ia366; insbesondere die Verbindungen Ia366.1-Ia366.164, die sich von den Verbindungen Ia1.1-Ia1.164 dadurch unterscheiden, daß R¹ für Methyl und R⁷ für 4-Chlorphenylmethyl stehen: 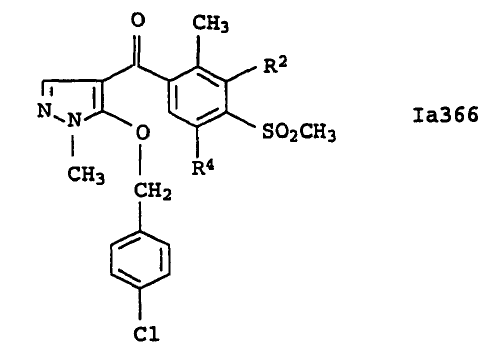
- Ebenso insbesondere außerordentlich bevorzugt sind die Verbindungen Ia367; insbesondere die Verbindungen Ia367.1-Ia367.164, die sich von den Verbindungen Ia1.1-Ia1.164 dadurch unterscheiden, daß R¹ für Methyl, R⁶ für Ethyl und R⁷ für 4-Chlorphenylmethyl stehen: 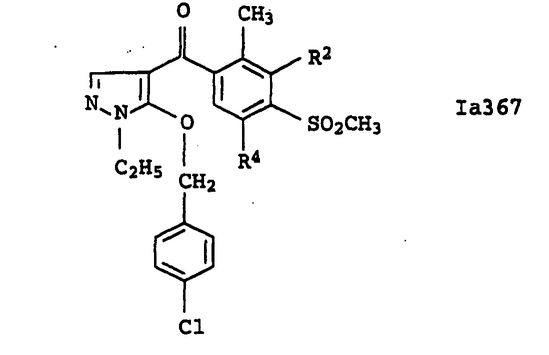
- Ebenso insbesondere außerordentlich bevorzugt sind die Verbindungen Ia368; insbesondere die Verbindungen Ia368.1-Ia368.164, die sich von den Verbindungen Ia1.1-Ia1.164 dadurch unterscheiden, daß R¹ für Methyl, R⁶ für n-Propyl und R⁷ für 4-Chlorphenylmethyl stehen: 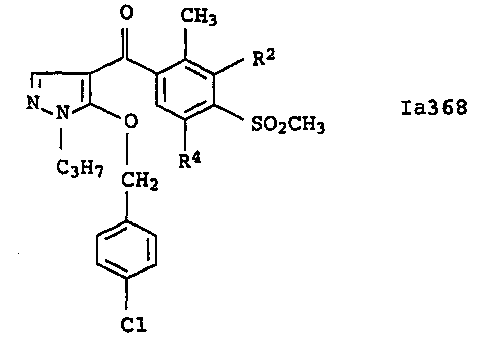
- Ebenso insbesondere außerordentlich bevorzugt sind die Verbindungen Ia369; insbesondere die Verbindungen Ia369.1-Ia369.164, die sich von den Verbindungen Ia1.1-Ia1.164 dadurch unterscheiden, daß R¹ für Methyl, R⁶ für iso-Butyl und R⁷ für 4-Chlorphenylmethyl stehen: 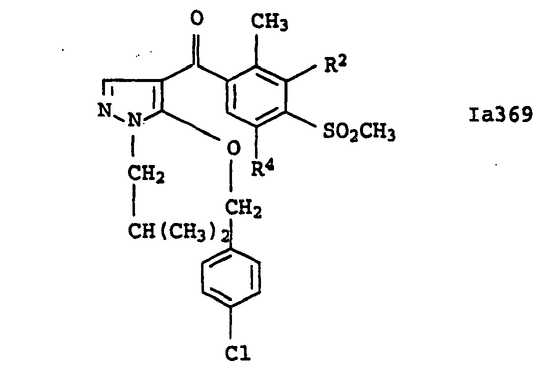
- Ebenso insbesondere außerordentlich bevorzugt sind die Verbindungen Ia370; insbesondere die Verbindungen Ia370.1-1a370.164, die sich von den Verbindungen Ial.1-Ial.164 dadurch unterscheiden, daß R¹ für Methyl, R⁷ für 4-Chlorphenylmethyl und R⁸ für Methyl stehen: 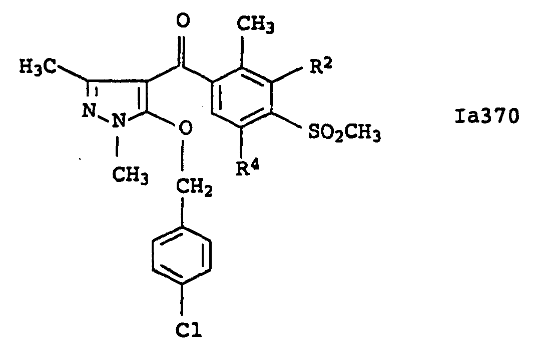
- Ebenso insbesondere außerordentlich bevorzugt sind die Verbindungen Ia371; insbesondere die Verbindungen Ia371.1-Ia371.164, die sich von den Verbindungen Ia1.1-Ia1.164 dadurch unterscheiden, daß R¹ für Methyl und R⁷ für 4-Methoxyphenylmethyl stehen: 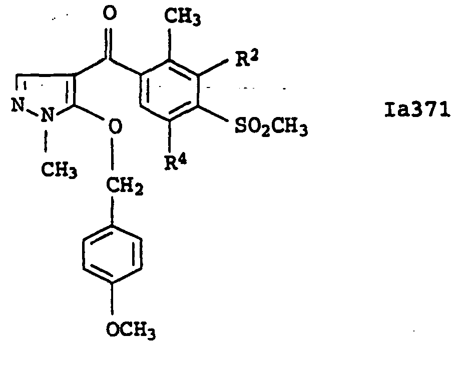
- Ebenso insbesondere außerordentlich bevorzugt sind die Verbindungen Ia372; insbesondere die Verbindungen Ia372.1-Ia372.164, die sich von den Verbindungen Ia1.1-Ia1.164 dadurch unterscheiden, daß R¹ für Methyl, R⁶ für Ethyl und R⁷ für 4-Methoxyphenylmethyl stehen: 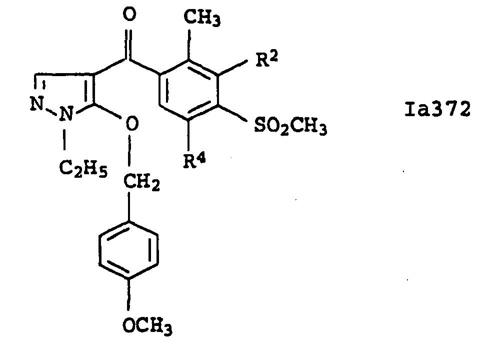
- Ebenso insbesondere außerordentlich bevorzugt sind die Verbindungen Ia373; insbesondere die Verbindungen Ia373.1-Ia373.164, die-sich von den Verbindungen Ia1.1-Ia1.164 dadurch unterscheiden, daß R¹ für Methyl, R⁷ für 4-Methoxyphenylmethyl und R⁸ für Methyl stehen: 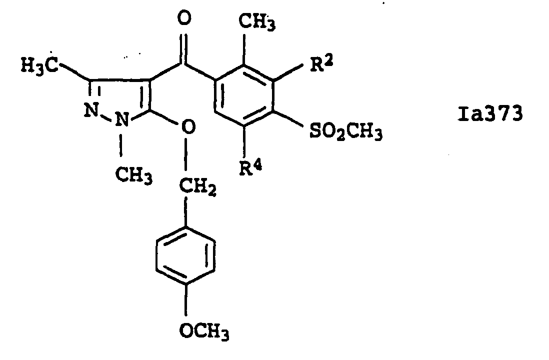
- Ebenso insbesondere außerordentlich bevorzugt sind die Verbindungen Ia374; insbesondere die Verbindungen Ia374.1-Ia374.164, die sich von den Verbindungen Ia1,1-Ia1.164 dadurch unterscheiden, daß R¹ für Methyl und R⁷ für 3-Trifluormethylphenylmethyl stehen: 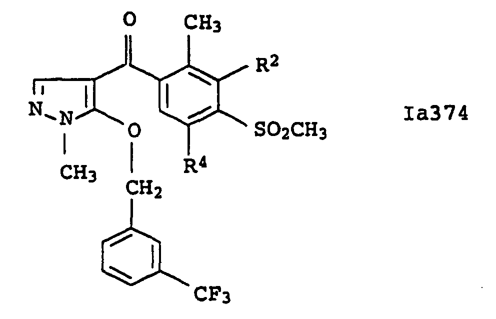
- Ebenso insbesondere außerordentlich bevorzugt sind die Verbindungen Ia375; insbesondere die Verbindungen Ia375.1-Ia375.164, die sich von den Verbindungen Ia1.1-Ia1.164 dadurch unterscheiden, daß R¹ für Methyl, R⁶ für Ethyl und R⁷ für 3-Trifluormethylphenylmethyl stehen: 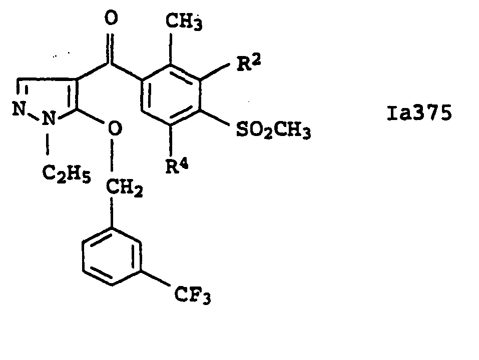
- Ebenso insbesondere außerordentlich bevorzugt sind die Verbindungen Ia376; insbesondere die Verbindungen Ia376.1-Ia376.164, die sich von den Verbindungen Ia1.1-Ia1.164 dadurch unterscheiden, daß R¹ für Methyl, R⁷ für 3-Trifluormethylphenylmethyl und R⁸ für Methyl stehen: 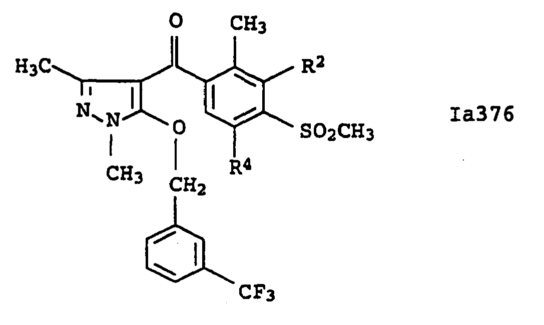
- Ebenso insbesondere außerordentlich bevorzugt sind die Verbindungen Ia377; insbesondere die Verbindungen Ia377.1-Ia377.164, die-sich von den Verbindungen Ia1.1-Ia1.164 dadurch unterscheiden, daß R¹ für Methyl und R⁷ für 2,4-Dichlorphenylmethyl stehen: 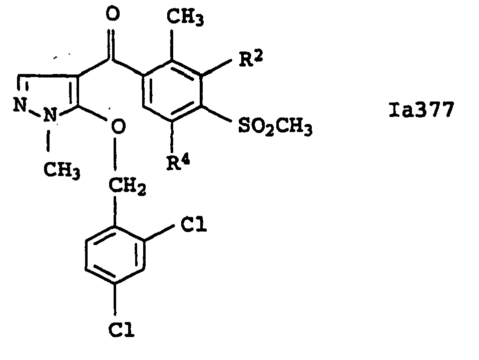
- Ebenso insbesondere außerordentlich bevorzugt sind die Verbindungen Ia378; insbesondere die Verbindungen Ia378.1-Ia378.164, die sich von den Verbindungen Ia1.1-Ia1.164 dadurch unterscheiden, daß R¹ für Methyl, R⁶ für Ethyl und R⁷ für 2,4-Dichlorphenylmethyl stehen: 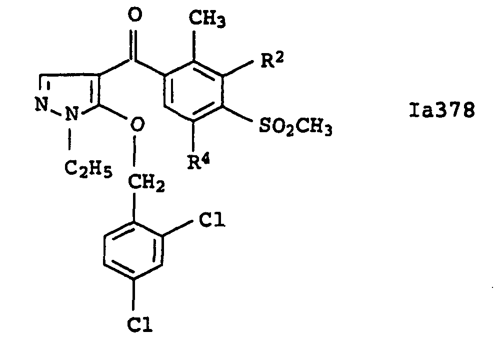
- Ebenso insbesondere außerordentlich bevorzugt sind die Verbindungen Ia379; insbesondere die Verbindungen Ia379.1-Ia379.164, die sich von den Verbindungen Ia1.1-Ia1.164 dadurch unterscheiden, daß R¹ für Methyl, R⁷ für 2,4-Dichlorphenylmethyl und R⁸ für Methyl stehen: 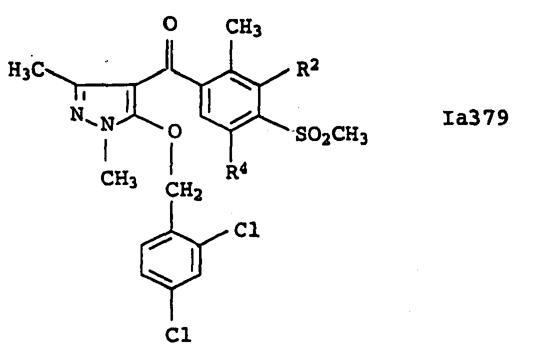
- Ebenso insbesondere außerordentlich bevorzugt sind die Verbindungen Ia380; insbesondere die Verbindungen Ia380.1-Ia380.164, die sich von den Verbindungen Ia1.1-Ia1.164 dadurch unterscheiden, daß R¹ für Methyl und R⁷ für Phenylcarbonylmethyl stehen: 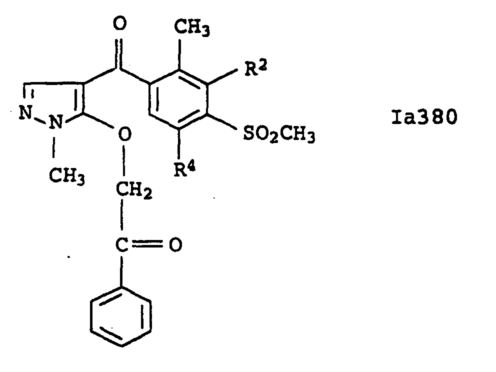
- Ebenso insbesondere außerordentlich bevorzugt sind die Verbindungen Ia381; insbesondere die Verbindungen Ia381.1-Ia381.164, die-sich von den Verbindungen Ia1.1-Ia1.164 dadurch unterscheiden, daß R¹ für Methyl, R⁶ für Ethyl und R⁷ für Phenylcarbonylmethyl stehen: 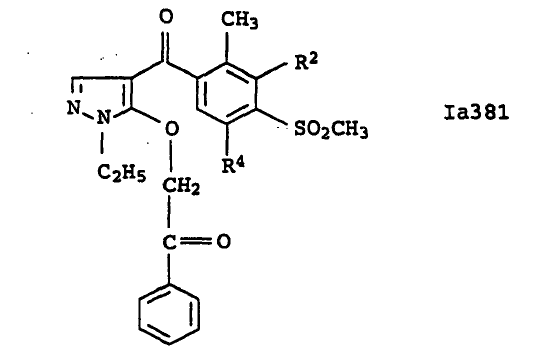
- Ebenso insbesondere außerordentlich bevorzugt sind die Verbindungen Ia382; insbesondere die Verbindungen Ia382.1-Ia382.164, die sich von den Verbindungen Ia1.1-Ia1.164 dadurch unterscheiden, daß R¹ für Methyl, R⁶ für n-Propyl und R⁷ für Phenylcarbonylmethyl stehen: 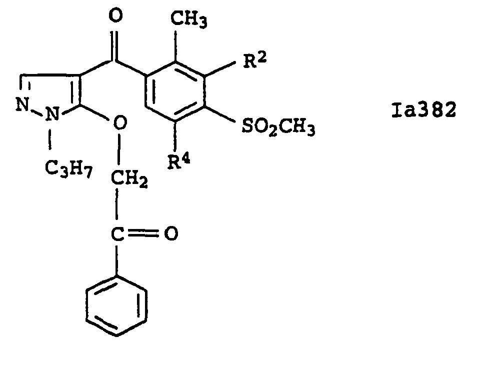
- Ebenso insbesondere außerordentlich bevorzugt sind die Verbindungen Ia383; insbesondere die Verbindungen Ia383.1-Ia383.164, die sich von den Verbindungen Ia1.1-Ia1.164 dadurch unterscheiden, daß R¹ für Methyl, R⁶ für iso-Butyl und R⁷ für Phenylcarbonylmethyl stehen: 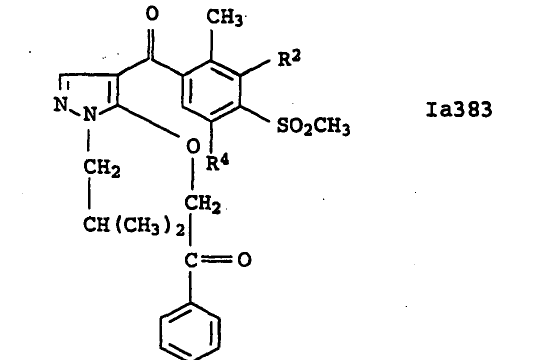
- Ebenso insbesondere außerordentlich bevorzugt sind die Verbindungen Ia384; insbesondere die Verbindungen Ia384.1-Ia384.164, die sich von den Verbindungen Ia1.1-Ia1.164 dadurch unterscheiden, daß R¹ für Methyl, R⁷ für Phenylcarbonylmethyl und R⁸ für Methyl stehen: 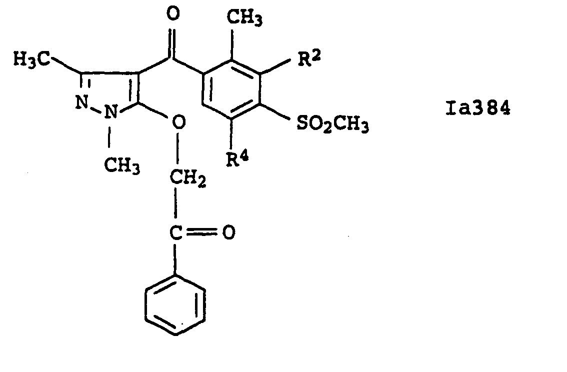
- Ebenso insbesondere außerordentlich bevorzugt sind die Verbindungen Ia385; insbesondere die Verbindungen Ia385.1-Ia385.164, die sich von den Verbindungen Ia1.1-Ia1.164 dadurch unterscheiden, daß R¹ für Methyl und R⁷ für 4-Methylphenylcarbonylmethyl stehen: 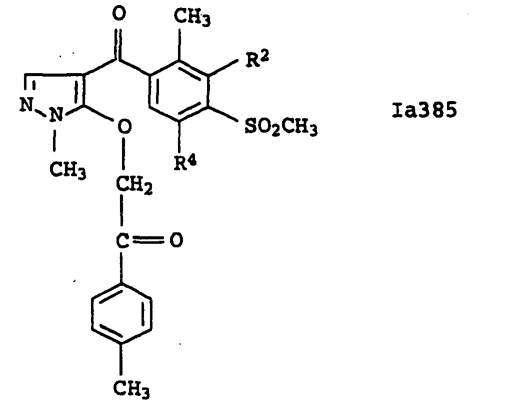
- Ebenso insbesondere außerordentlich bevorzugt sind die Verbindungen Ia386; insbesondere die Verbindungen Ia386.1-Ia386.164, die sich von den Verbindungen Ia1.1-Ia1.164 dadurch unterscheiden, daß R¹ für Methyl, R⁶ für Ethyl und R⁷ für 4-Methylphenylcarbonylmethyl stehen: 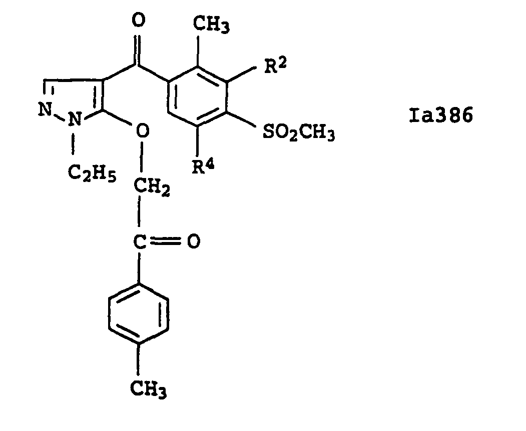
- Ebenso insbesondere außerordentlich bevorzugt sind die Verbindungen Ia387; insbesondere die Verbindungen Ia387.1-Ia387.164, die-sich von den Verbindungen Ia1.1-Ia1.164 dadurch unterscheiden, daß R¹ für Methyl, R⁷ für 4-Methylphenylcarbonylmethyl und R⁸ für Methyl stehen: 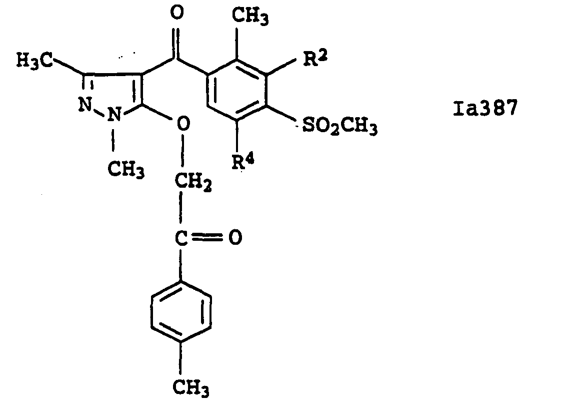
- Ebenso insbesondere außerordentlich bevorzugt sind die Verbindungen Ia388; insbesondere die Verbindungen Ia388.1-Ia388.164, die sich von den Verbindungen Ia1.1-Ia1.164 dadurch unterscheiden, daß R¹ für Methyl und R⁷ für 1-(Phenylcarbonyl)-eth-1-yl stehen: 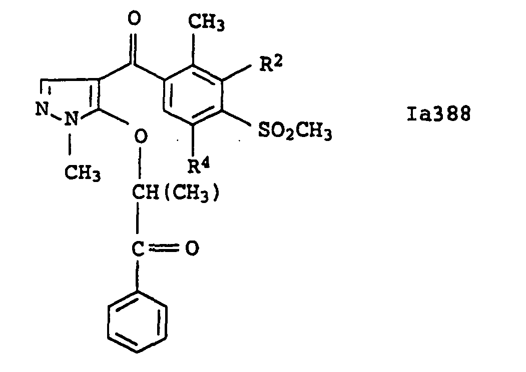
- Ebenso insbesondere außerordentlich bevorzugt sind die Verbindungen Ia389; insbesondere die Verbindungen Ia389.1-Ia389.164, die sich von den Verbindungen Ia1.1-Ia1.164 dadurch unterscheiden, daß R¹ für Methyl, R⁶ für Ethyl und R⁷ für 1-(Phenylcarbonyl)-eth-1-yl stehen: 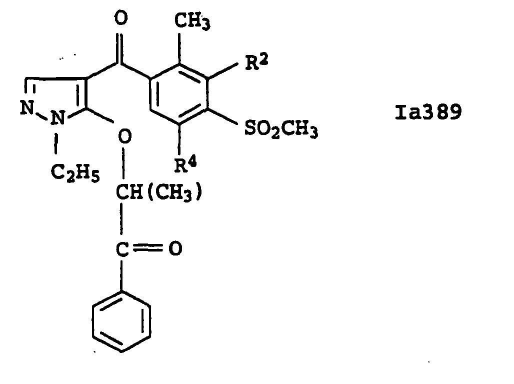
- Ebenso insbesondere außerordentlich bevorzugt sind die Verbindungen Ia390; insbesondere die Verbindungen Ia390.1-Ia390.164, die sich von den Verbindungen Ia1.1-Ia1.164 dadurch unterscheiden, daß R¹ für Methyl, R⁷ für 1 (Phenylcarbonyl)-eth-1-yl und R⁸ für Methyl stehen: 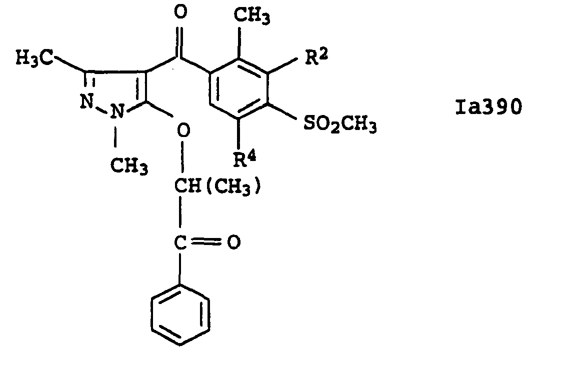
- Ebenso insbesondere außerordentlich bevorzugt sind die Verbindungen Ia391; insbesondere die Verbindungen Ia391.1-Ia391.164, die sich von den Verbindungen Ia1.1-Ia1.164 dadurch unterscheiden, daß R¹ für Methyl und R⁷ für Phenylcarbonyl stehen: 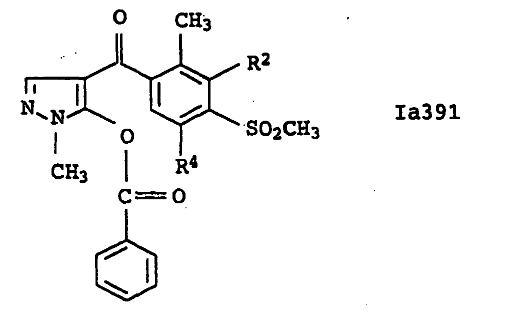
- Ebenso insbesondere außerordentlich bevorzugt sind die Verbindungen Ia392; insbesondere die Verbindungen Ia392.1-Ia392.164, die sich von den Verbindungen Ia1.1-Ia1.164 dadurch unterscheiden, daß R¹ für Methyl, R⁶ für Ethyl und R⁷ für Phenylcarbonyl stehen: 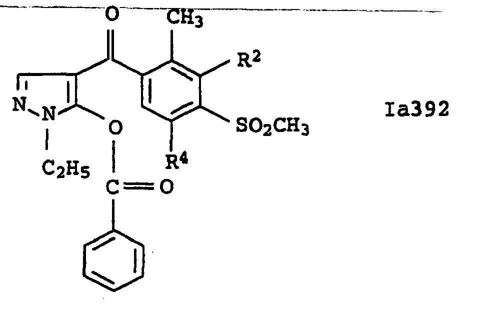
- Ebenso insbesondere außerordentlich bevorzugt sind die Verbindungen Ia393; insbesondere die Verbindungen Ia393.1-Ia393.164, die sich von den Verbindungen Ia1.1-Ia1.164 dadurch unterscheiden, daß R¹ für Methyl, R⁷ für Phenylcarbonyl und R⁸ für Methyl stehen: 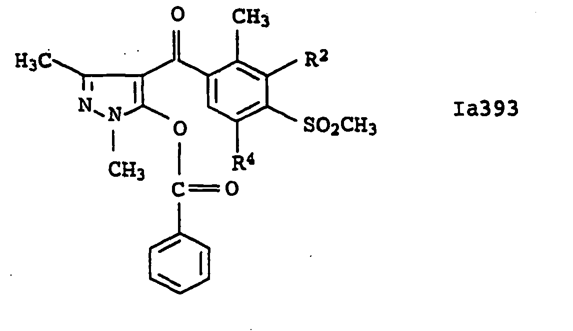
- Ebenso insbesondere außerordentlich bevorzugt sind die Verbindungen Ia394; insbesondere die Verbindungen Ia394.1-Ia394.164, die sich von den Verbindungen Ia1.1-Ia1.164 dadurch unterscheiden, daß R¹ für Methyl und R⁷ für 4-Methylphenylcarbonyl stehen: 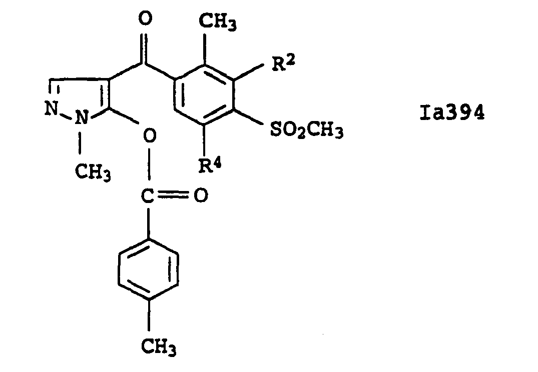
- Ebenso insbesondere außerordentlich bevorzugt sind die Verbindungen Ia395; insbesondere die Verbindungen Ia395.1-Ia395.164, die sich von den Verbindungen Ia1.1-Ia1.164 dadurch unterscheiden, daß R¹ für Methyl, R⁶ für Ethyl und R⁷ für 4-Methylphenylcarbonyl stehen: 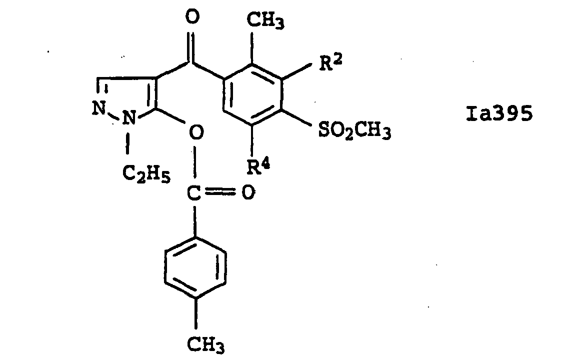
- Ebenso insbesondere außerordentlich bevorzugt sind die Verbindungen Ia396; insbesondere die Verbindungen Ia396.1-Ia396.164, die sich von den Verbindungen Ia1.1-Ia1.164 dadurch unterscheiden, daß R¹ für Methyl, R⁷ für 4-Methylphenylcarbonyl und R⁸ für Methyl stehen: 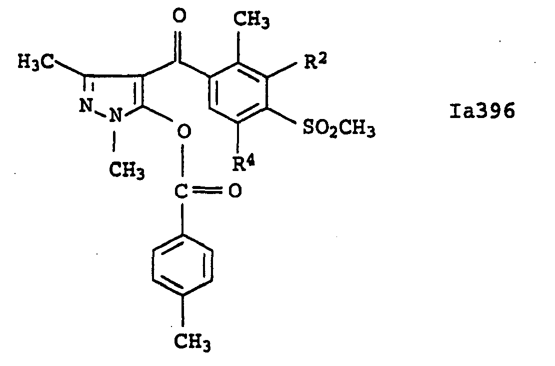
- Ebenso insbesondere außerordentlich bevorzugt sind die Verbindungen Ia397; insbesondere die Verbindungen 1a397.1-Ia397.164, die sich von den Verbindungen Ia1.1-Ia1.164 dadurch unterscheiden, daß R¹ für Methyl und R⁷ für 4-Chlorphenylcarbonyl stehen: 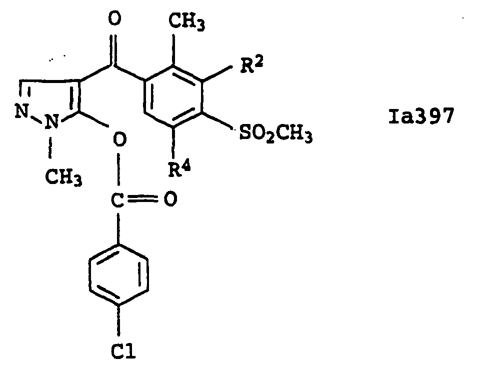
- Ebenso insbesondere außerordentlich bevorzugt sind die Verbindungen Ia398; insbesondere die Verbindungen Ia398.1-Ia398.164, die sich von den Verbindungen Ia1.1-Ia1.164 dadurch unterscheiden, daß R¹ für Methyl, R⁶ für Ethyl und R⁷ für 4-Chlorphenylcarbonyl stehen: 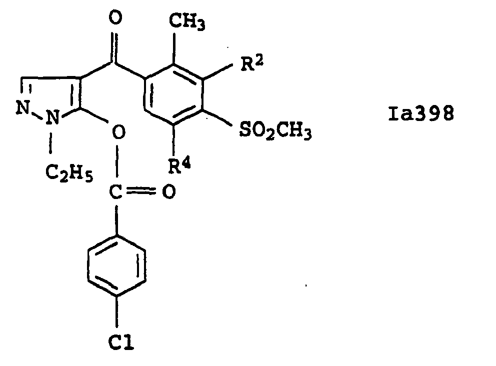
- Ebenso insbesondere außerordentlich bevorzugt sind die Verbindungen Ia399; insbesondere die Verbindungen Ia399.1-Ia399.164, die sich von den Verbindungen Ia1.1-Ia1.164 dadurch unterscheiden, daß R¹ für Methyl, R⁷ für 4-Chlorphenylcarbonyl und R⁸ für Methyl stehen: 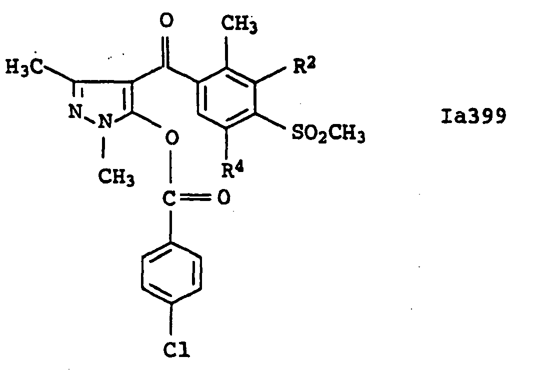
- Ebenso insbesondere außerordentlich bevorzugt sind die Verbindungen Ia400; insbesondere die Verbindungen Ia400.1-Ia400.164, die sich von den Verbindungen Ia1.1-Ia1.164 dadurch unterscheiden, daß R¹ für Methyl und R⁷ für 4-Methoxyphenylcarbonyl stehen: 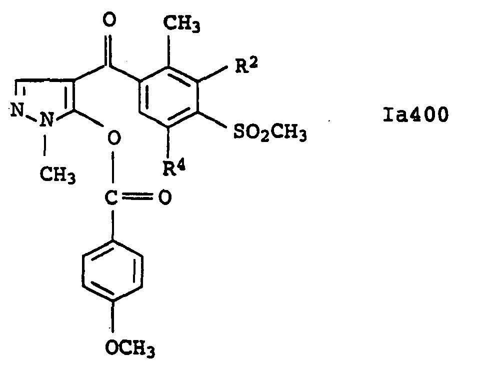
- Ebenso insbesondere außerordentlich bevorzugt sind die Verbindungen Ia401; insbesondere die Verbindungen Ia401.1-Ia401.164, die sich von den Verbindungen Ia1.1-Ia1.164 dadurch unterscheiden, daß R¹ für Methyl, R⁶ für Ethyl und R⁷ für 4-Methoxyphenylcarbonyl stehen: 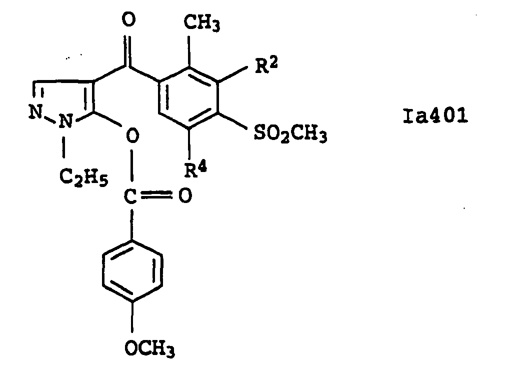
- Ebenso insbesondere außerordentlich bevorzugt sind die Verbindungen Ia402; insbesondere die Verbindungen Ia402.1-Ia402.164, die sich von den Verbindungen Ia1.1-Ia1.164 dadurch unterscheiden, daß R¹ für Methyl, R⁷ für 4 Methoxy phenylcarbonyl und R⁸ für Methyl stehen: 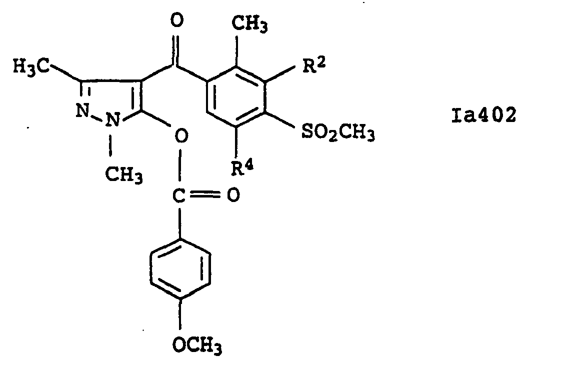
- Ebenso insbesondere außerordentlich bevorzugt sind die Verbindungen Ia403; insbesondere die Verbindungen Ia403.1-Ia403.164, die sich von den Verbindungen Ia1.1-Ia1.164 dadurch unterscheiden, daß R¹ für Methyl und R⁷ für 4-Trifluormethylphenylcarbonyl stehen: 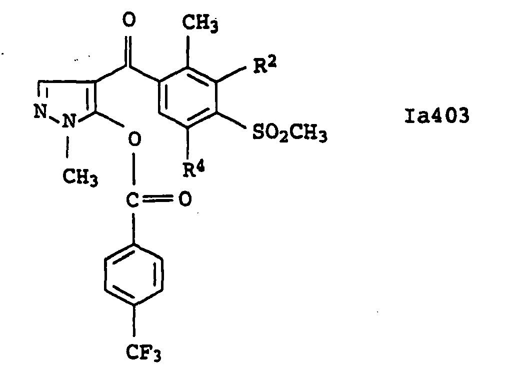
- Ebenso insbesondere außerordentlich bevorzugt sind die Verbindungen Ia404; insbesondere die Verbindungen Ia404.1-Ia404.164, die sich von den Verbindungen Ia1.1-Ia1.164 dadurch unterscheiden, daß R¹ für Methyl, R⁶ für Ethyl und R⁷ für 4-Trifluormethylphenylcarbonyl stehen: 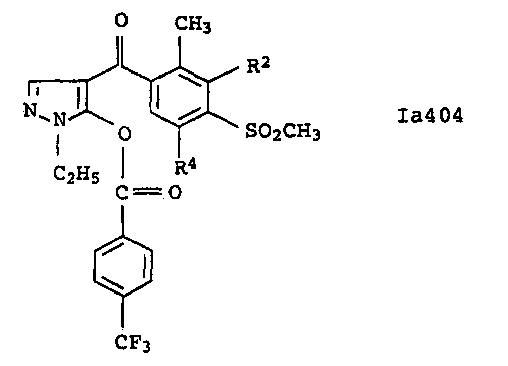
- Ebenso insbesondere außerordentlich bevorzugt sind die Verbindungen Ia405; insbesondere die Verbindungen Ia405.1-Ia405.164, die sich von den Verbindungen Ia1.1-Ia1.164 dadurch unterscheiden, daß R¹ für Methyl, R⁷ für 4-Trifluormethylphenylcarbonyl und R⁸ für Methyl stehen: 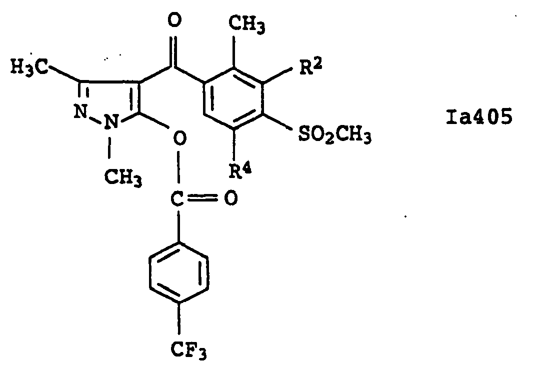
- Ebenso insbesondere außerordentlich bevorzugt sind die Verbindungen Ia406; insbesondere die Verbindungen Ia406.1-Ia406.164, die sich von den Verbindungen Ia1.1-Ia1.164 dadurch unterscheiden, daß R¹ für Methyl und R⁷ für 2,4-Dichlorphenylcarbonyl stehen: 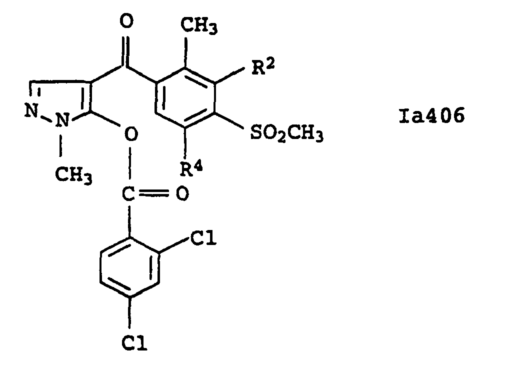
- Ebenso insbesondere außerordentlich bevorzugt sind die Verbindungen Ia407; insbesondere die Verbindungen Ia407.1-Ia407.164, die sich von den Verbindungen Ia1.1-Ia1.164 dadurch unterscheiden, daß R¹ für Methyl, R⁶ für Ethyl und R⁷ für 2,4-Dichlorphenylcarbonyl stehen: 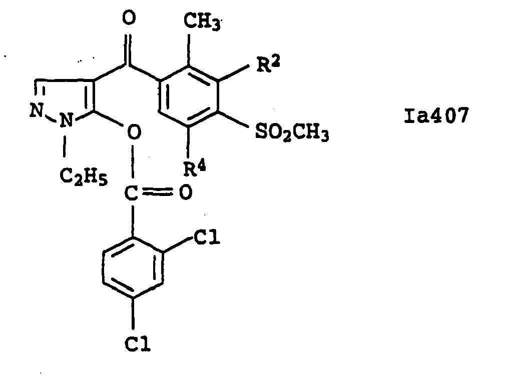
- Ebenso insbesondere außerordentlich bevorzugt sind die Verbindungen Ia408; insbesondere die Verbindungen Ia408.1-Ia408.164, die sich von den Verbindungen Ia1.1-Ia1.164 dadurch unterscheiden, daß R¹ für Methyl, R⁷ für 2,4-Dichlorphenylcarbonyl und R⁸ für Methyl stehen: 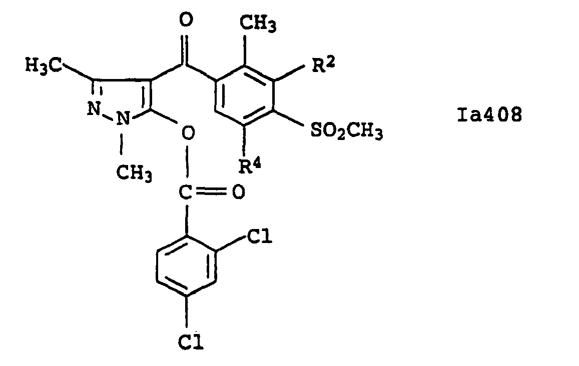
- Ebenso insbesondere außerordentlich bevorzugt sind die Verbindungen Ia409; insbesondere die Verbindungen Ia409.1-Ia409.164, die sich von den Verbindungen Ia1.1-Ia1.164 dadurch unterscheiden, daß R¹ für Methoxy steht: 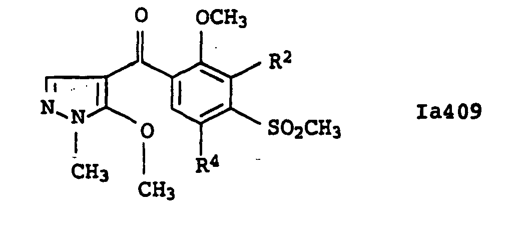
- Ebenso insbesondere außerordentlich bevorzugt sind die Verbindungen Ia410; insbesondere die Verbindungen Ia410.1-Ia410.164, die sich von den Verbindungen Ia1.1-Ia1.164 dadurch unterscheiden, daß R¹ für Methoxy und R⁶ für Ethyl stehen: 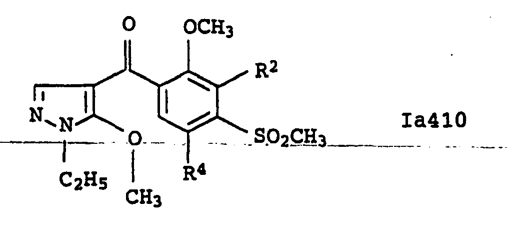
- Ebenso insbesondere außerordentlich bevorzugt sind die Verbindungen Ia411; insbesondere die Verbindungen Ia411.1-Ia411.164, die sich von den Verbindungen Ia1.1-Ia1.164 dadurch unterscheiden, daß R¹ für Methoxy und R⁸ für Methyl stehen: 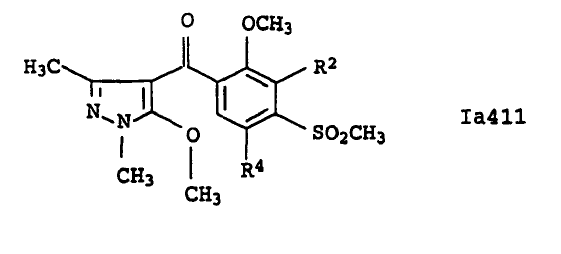
- Ebenso insbesondere außerordentlich bevorzugt sind die Verbindungen Ia412; insbesondere die Verbindungen Ia412.1-Ia412.164, die sich von den Verbindungen Ia1.1-Ia1.164 dadurch unterscheiden, daß R¹ für Methoxy und R⁷ für Ethyl stehen: 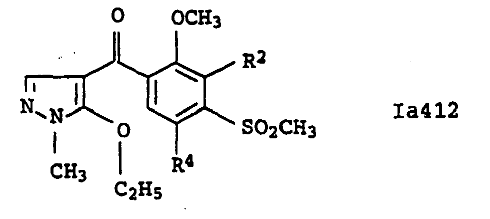
- Ebenso insbesondere außerordentlich bevorzugt sind die Verbindungen Ia413; insbesondere die Verbindungen Ia413.1-Ia413.164, die sich von den Verbindungen Ia1.1-Ia1.164 dadurch unterscheiden, daß R¹ für Methoxy und R⁶ und R⁷ für Ethyl stehen: 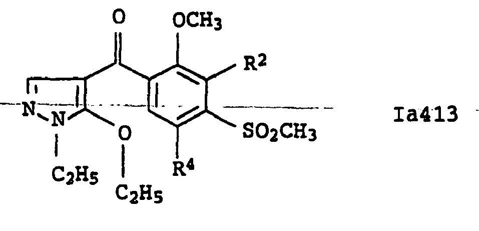
- Ebenso insbesondere außerordentlich bevorzugt sind die Verbindungen Ia414; insbesondere die Verbindungen Ia414.1-Ia414.164, die sich von den Verbindungen Ia1.1-Ia1.164 dadurch unterscheiden, daß R¹ für Methoxy, R⁷ für Ethyl und R⁸ für Methyl stehen: 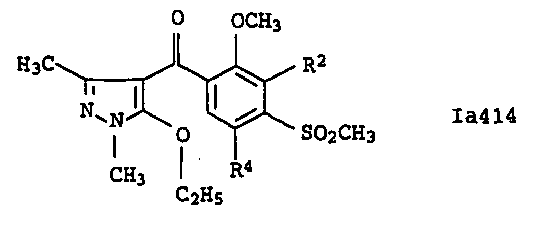
- Ebenso insbesondere außerordentlich bevorzugt sind die Verbindungen Ia415; insbesondere die Verbindungen Ia415.1-Ia415.164, die-sich von den Verbindungen Ia1.1-Ia1.164 dadurch unterscheiden, daß R¹ für Methoxy und R⁷ für Allyl stehen: 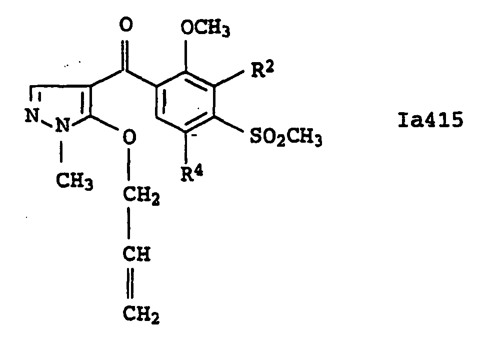
- Ebenso insbesondere außerordentlich bevorzugt sind die Verbindungen Ia416; insbesondere die Verbindungen Ia416.1-Ia416.164, die sich von den Verbindungen Ia1.1-Ia1.164 dadurch unterscheiden, daß R¹ für Methoxy, R⁶ für Ethyl und R⁷ für Allyl stehen: 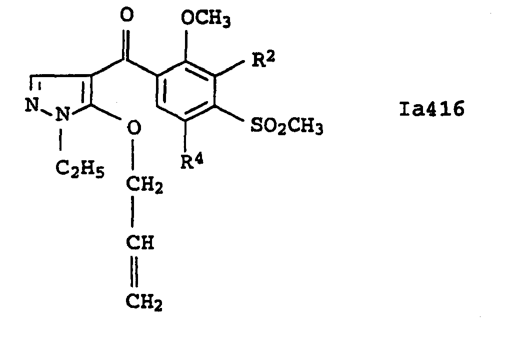
- Ebenso insbesondere außerordentlich bevorzugt sind die Verbindungen Ia417; insbesondere die Verbindungen Ia417.1-Ia417.164, die sich von den Verbindungen Ia1.1-Ia1.164 dadurch unterscheiden, daß R¹ für Methoxy, R⁷ für Allyl und R⁸ für Methyl stehen: 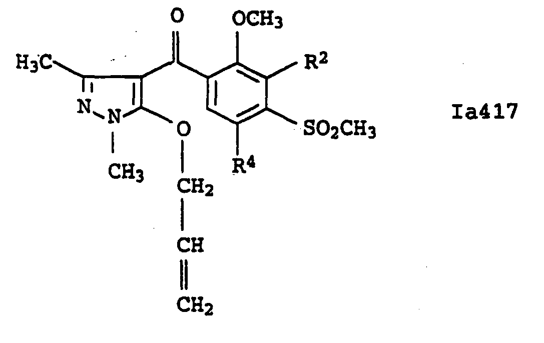
- Ebenso insbesondere außerordentlich bevorzugt sind die Verbindungen Ia418; insbesondere die Verbindungen Ia418.1-Ia418.164, die sich von den Verbindungen Ia1.1-Ia1.164 dadurch unterscheiden, daß R¹ für Methoxy und R⁷ für Methylcarbonyl stehen: 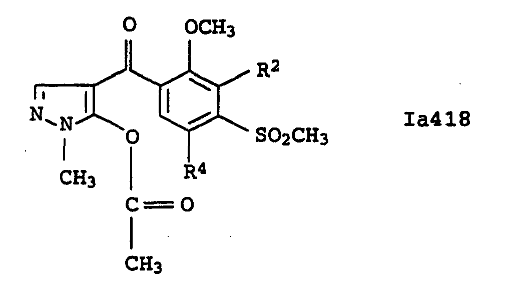
- Ebenso insbesondere außerordentlich bevorzugt sind die Verbindungen Ia419; insbesondere die Verbindungen Ia419.1-Ia419.164, die-sich von den Verbindungen Ia1.1-Ia1.164 dadurch unterscheiden, daß R¹ für Methoxy, R⁶ für Ethyl und R⁷ für Methylcarbonyl stehen: 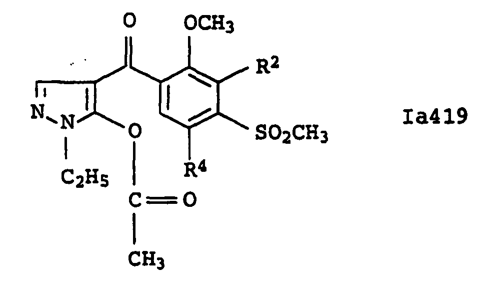
- Ebenso insbesondere außerordentlich bevorzugt sind die Verbindungen Ia420; insbesondere die Verbindungen Ia420.1-Ia420.164, die sich von den Verbindungen Ia1.1-Ia1.164 dadurch unterscheiden, daß R¹ für Methoxy, R⁷ für Methylcarbonyl und R⁸ für Methyl stehen: 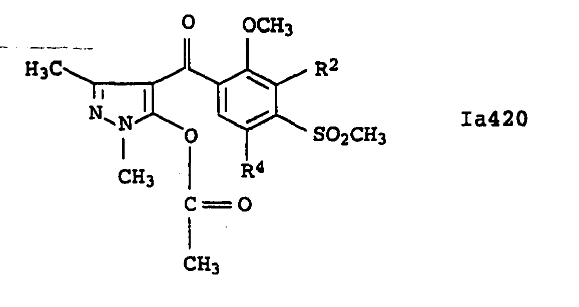
- Ebenso insbesondere außerordentlich bevorzugt sind die Verbindungen Ia421; insbesondere die Verbindungen Ia421.1-Ia421.164, die sich von den Verbindungen Ia1.1-Ia1.164 dadurch unterscheiden, daß R¹ für Methoxy und R⁷ für Methoxycarbonyl stehen: 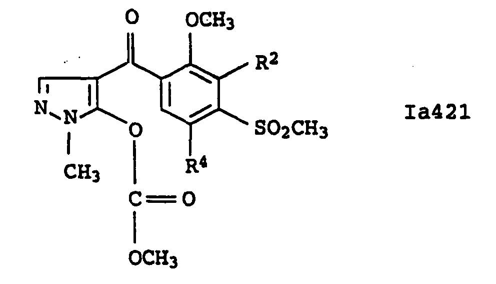
- Ebenso insbesondere außerordentlich bevorzugt sind die Verbindungen Ia422; insbesondere die Verbindungen Ia422.1-Ia422.164, die sich von den Verbindungen Ia1.1-Ia1.164 dadurch unterscheiden, daß R¹ für Methoxy, R⁶ für Ethyl und R⁷ für Methoxycarbonyl stehen: 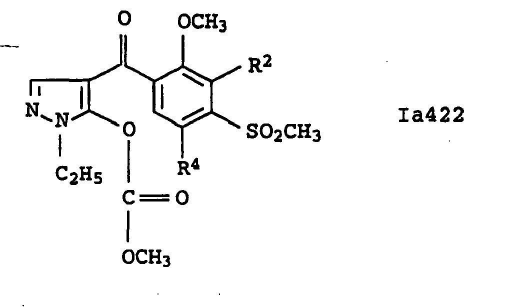
- Ebenso insbesondere außerordentlich bevorzugt sind die Verbindungen Ia423; insbesondere die Verbindungen Ia423.1-Ia423.164, die sich von den Verbindungen Ia1.1-Ia1.164 dadurch unterscheiden, daß R¹ für Methoxy, R⁷ für Methoxycarbonyl und R⁸ für Methyl stehen: 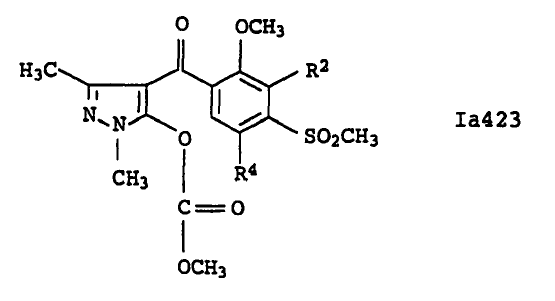
- Ebenso insbesondere außerordentlich bevorzugt sind die Verbindungen Ia424; insbesondere die Verbindungen Ia424.1-Ia424.164, die sich von den Verbindungen Ia1.1-Ia1.164 dadurch unterscheiden, daß R¹ für Methoxy und R⁷ für Dimethylaminocarbonyl stehen: 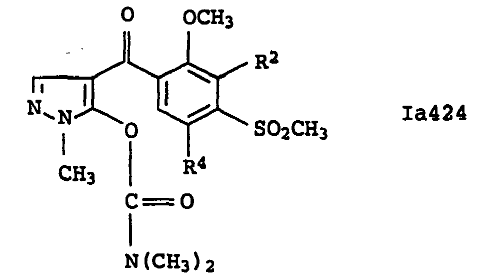
- Ebenso insbesondere außerordentlich bevorzugt sind die Verbindungen Ia425; insbesondere die Verbindungen Ia425.1-Ia425.164, die sich von den Verbindungen Ia1.1-Ia1.164 dadurch unterscheiden, daß R¹ für Methoxy, R⁶ für Ethyl und R⁷ für Dimethylaminocarbonyl stehen: 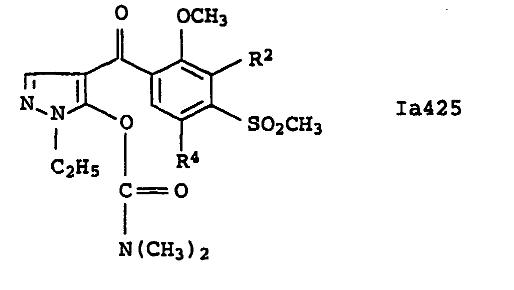
- Ebenso insbesondere außerordentlich bevorzugt sind die Verbindungen Ia426; insbesondere die Verbindungen Ia426.1-Ia426.164, die sich von den Verbindungen Ia1.1-Ia1.164 dadurch unterscheiden, daß R¹ für Methoxy, R⁷ für Dimethylaminocarbonyl und R⁸ für Methyl stehen: 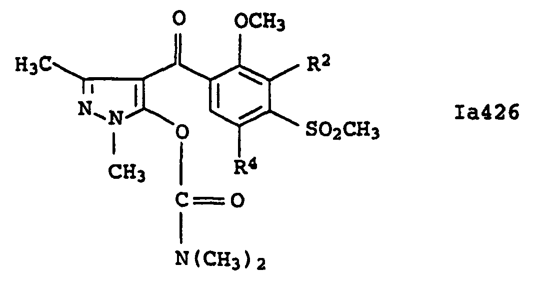
- Ebenso insbesondere außerordentlich bevorzugt sind die Verbindungen Ia427; insbesondere die Verbindungen Ia427.1-Ia427.164, die sich von den Verbindungen Ia1.1-Ia1.164 dadurch unterscheiden, daß R¹ für Methoxy und R⁷ für Methoxycarbonylmethyl stehen: 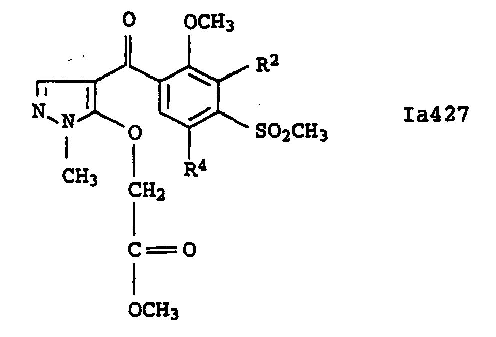
- Ebenso insbesondere außerordentlich bevorzugt sind die Verbindungen Ia428; insbesondere die Verbindungen Ia428.1-Ia428.164, die sich von den Verbindungen Ia1.1-Ia1.164 dadurch unterscheiden, daß R¹ für Methoxy, R⁶ für Ethyl und R⁷ für Methoxycarbonylmethyl stehen: 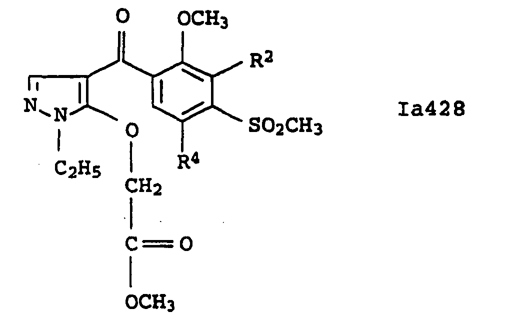
- Ebenso insbesondere außerordentlich bevorzugt sind die Verbindungen Ia429; insbesondere die Verbindungen Ia429.1-Ia429.164, die sich von den Verbindungen Ia1.1-Ia1.164 dadurch unterscheiden, daß R¹ für Methoxy, R⁷ für Methoxycarbonylmethyl und R⁸ für Methyl stehen: 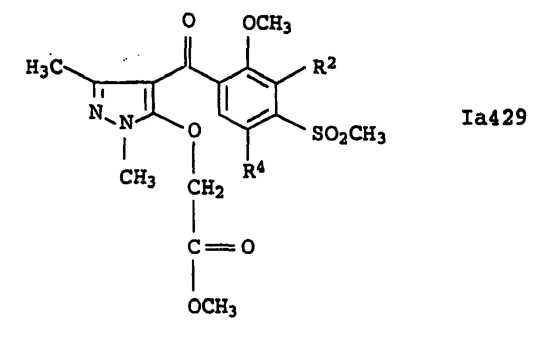
- Ebenso insbesondere außerordentlich bevorzugt sind die Verbindungen Ia430; insbesondere die Verbindungen Ia430.1-Ia430.164, die sich von den Verbindungen Ia1.1-Ia1.164 dadurch unterscheiden, daß R¹ für Methoxy und R⁷ für Dimethylaminocarbonylmethyl stehen: 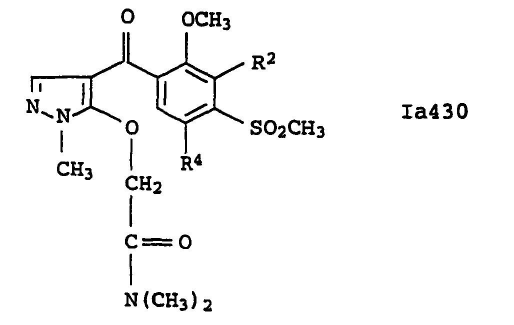
- Ebenso insbesondere außerordentlich bevorzugt sind die Verbindungen Ia431; insbesondere die Verbindungen Ia431.1-Ia431.164, die sich von den Verbindungen Ia1.1-Ia1.164 dadurch unterscheiden, daß R¹ für Methoxy, R⁶ für Ethyl und R⁷ für Dimethylaminocarbonylmethyl stehen: 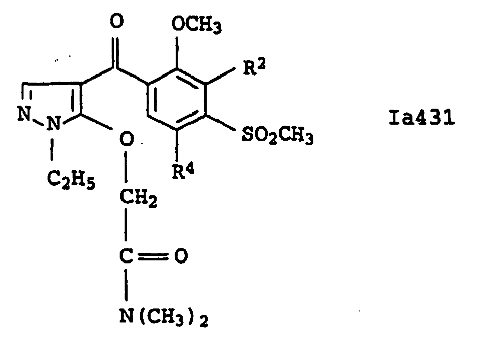
- Ebenso insbesondere außerordentlich bevorzugt sind die Verbindungen Ia432; insbesondere die Verbindungen Ia432.1-Ia432.164, die sich von den Verbindungen Ial.1-Ial.164 dadurch unterscheiden, daß R¹ für Methoxy, R⁷ für Dimethylaminocarbonylmethyl und R⁸ für Methyl stehen: 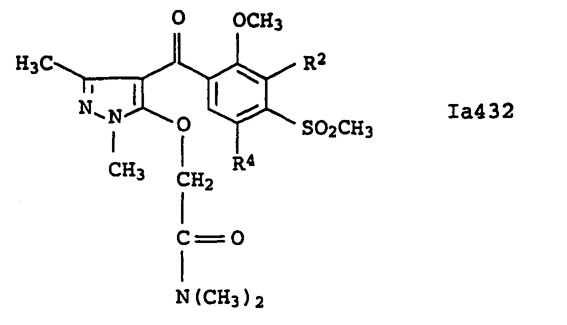
- Ebenso insbesondere außerordentlich bevorzugt sind die Verbindungen Ia433; insbesondere die Verbindungen Ia433.1-Ia433.164, die sich von den Verbindungen Ia1.1-Ia1.164 dadurch unterscheiden, daß R¹ für Methoxy und R⁷ für Phenylcarbonylmethyl stehen: 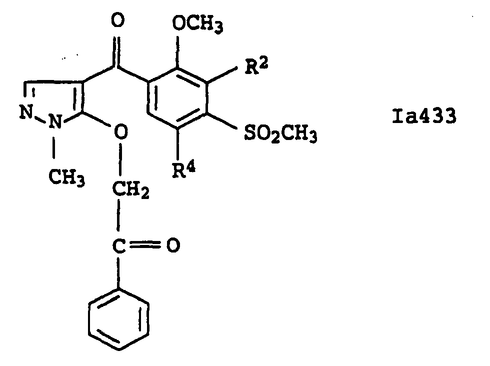
- Ebenso insbesondere außerordentlich bevorzugt sind die Verbindungen Ia434; insbesondere die Verbindungen Ia434.1-Ia434.164, die sich von den Verbindungen Ia1.1-Ia1.164 dadurch unterscheiden, daß R¹ für Methoxy, R⁶ für Ethyl und R⁷ für Phenylcarbonylmethyl stehen: 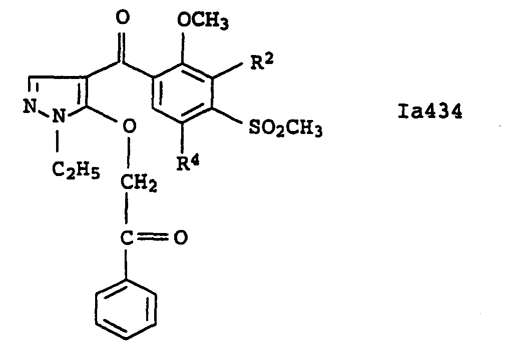
- Ebenso insbesondere außerordentlich bevorzugt sind die Verbindungen Ia435; insbesondere die Verbindungen Ia435.1-Ia435.164, die sich von den Verbindungen Ia1.1-Ia1.164 dadurch unterscheiden, daß R¹ für Methoxy, _{R}⁷ für Phenylcarbonylmethyl und R⁸ für Methyl stehen: 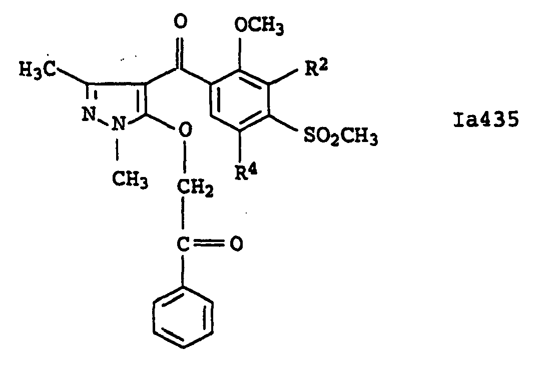
- Ebenso insbesondere außerordentlich bevorzugt sind die Verbindungen Ia436; insbesondere die Verbindungen Ia436.1-Ia436.164, die sich von den Verbindungen Ia1.1-Ia1.164 dadurch unterscheiden, daß R¹ für Methoxy und R⁷ für 4-Methylphenylcarbonylmethyl stehen: 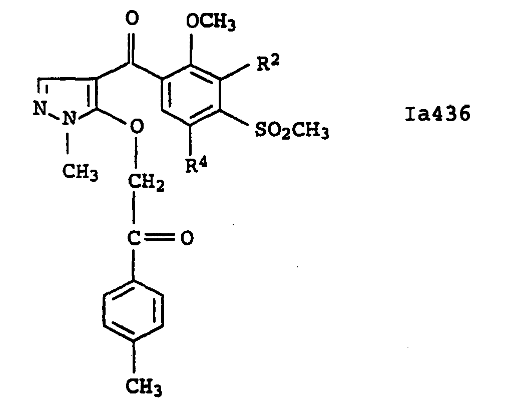
- Ebenso insbesondere außerordentlich bevorzugt sind die Verbindungen Ia437; insbesondere die Verbindungen Ia437.1-Ia437.164, die sich von den Verbindungen Ia1.1-Ia1.164 dadurch unterscheiden, daß R¹ für Methoxy, R⁶ für Ethyl und R⁷ für 4-Methylphenylcarbonylmethyl stehen: 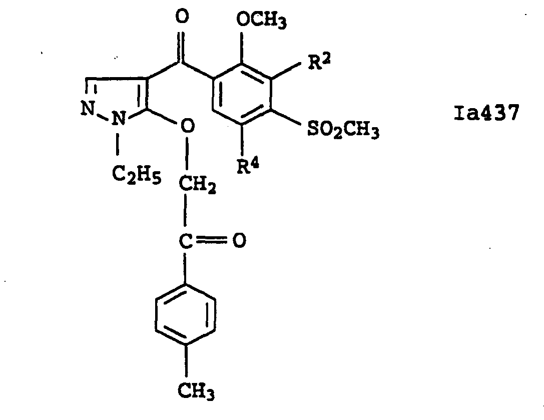
- Ebenso insbesondere außerordentlich bevorzugt sind die Verbindungen Ia438; insbesondere die Verbindungen Ia438.1-Ia438.164, die sich von den Verbindungen Ia1.1-Ia1.164 dadurch unterscheiden, daß R¹ für Methoxy, R⁷ für 4-Methylphenylcarbonylmethyl und R⁸ für Methyl stehen: 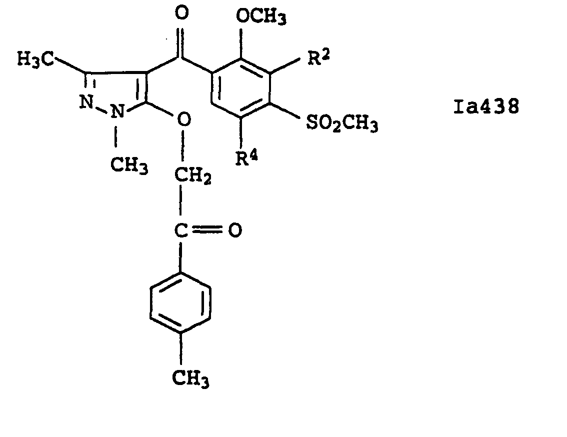
- Ebenso insbesondere außerordentlich bevorzugt sind die Verbindungen Ia439; insbesondere die Verbindungen Ia439.1-Ia439.164, die sich von den Verbindungen Ia1.1-Ia1.164 dadurch unterscheiden, daß R¹ für Methoxy und R⁷ für Phenylcarbonyl stehen: 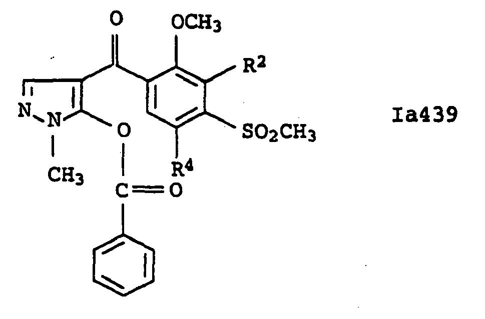
- Ebenso insbesondere außerordentlich bevorzugt sind die Verbindungen Ia440; insbesondere die Verbindungen Ia440.1-Ia440.164, die sich von den Verbindungen Ia1.1-Ia1.164 dadurch unterscheiden, daß R¹ für Methoxy, R⁶ für Ethyl und R⁷ für Phenylcarbonyl stehen: 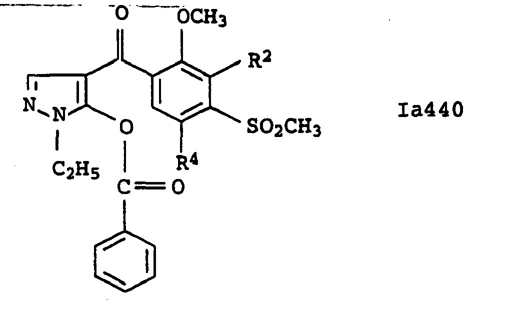
- Ebenso insbesondere außerordentlich bevorzugt sind die Verbindungen Ia441; insbesondere die Verbindungen Ia441.1-Ia441.164, die-sich von den Verbindungen Ia1.1-Ia1.164 dadurch unterscheiden, daß R¹ für Methoxy, R⁷ für Phenylcarbonyl und R⁸ für Methyl stehen: 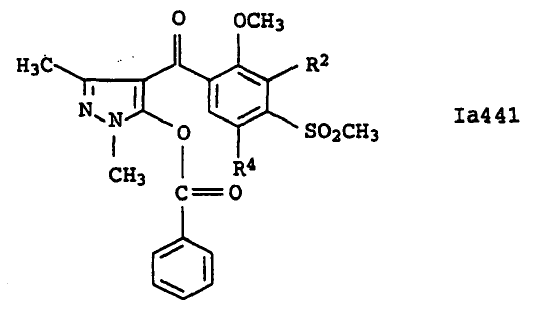
- Ebenso insbesondere außerordentlich bevorzugt sind die Verbindungen Ia442; insbesondere die Verbindungen Ia442.1-Ia442.164, die sich von den Verbindungen Ia1.1-Ia1.164 dadurch unterscheiden, daß R¹ für Methoxy und R⁷ für 4-Methylphenylcarbonyl stehen: 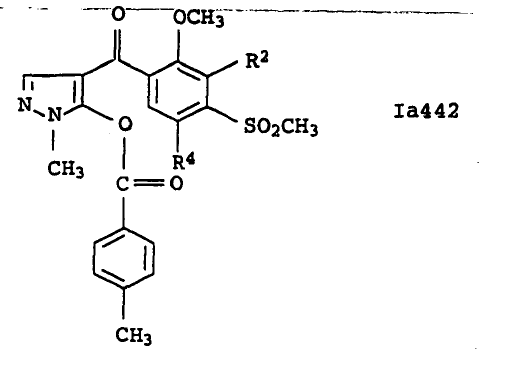
- Ebenso insbesondere außerordentlich bevorzugt sind die Verbindungen Ia443; insbesondere die Verbindungen Ia443.1-Ia443.164, die-sich von den Verbindungen Ia1.1-Ia1.164 dadurch unterscheiden, daß R¹ für Methoxy, R⁶ für Ethyl und R⁷ für 4-Methylphenylcarbonyl stehen: 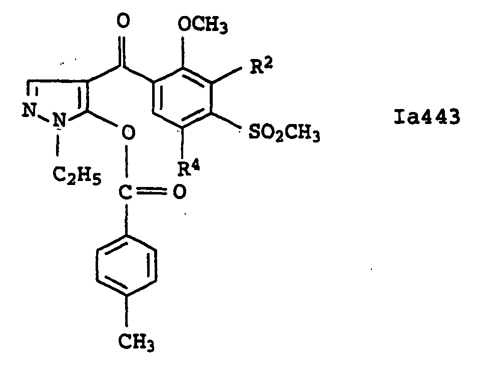
- Ebenso insbesondere außerordentlich bevorzugt sind die Verbindungen Ia444; insbesondere die Verbindungen Ia444.1-1a444.164, die sich von den Verbindungen Ia1.1-Ia1.164 dadurch unterscheiden, daß R¹ für Methoxy, R⁷ für 4-Methylphenylcarbonyl und R⁸ für Methyl stehen: 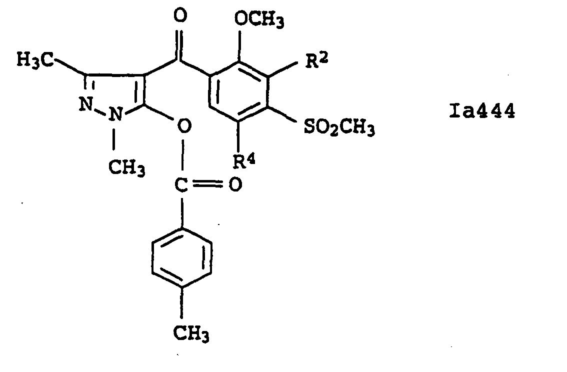
- Ebenso insbesondere außerordentlich bevorzugt sind die Verbindungen Ia445; insbesondere die Verbindungen Ia445.1-Ia445.164, die-sich von den Verbindungen Ia1.1-Ia1.164 dadurch unterscheiden, daß R¹ für Methoxy und R⁷ für Benzyl stehen: 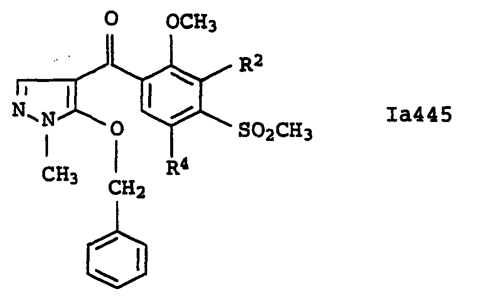
- Ebenso insbesondere außerordentlich bevorzugt sind die Verbindungen Ia446; insbesondere die Verbindungen Ia446.1-Ia446.164, die sich von den Verbindungen Ia1.1-Ia1.164 dadurch unterscheiden, daß R¹ für Methoxy, R⁶ für Ethyl und R⁷ für Benzyl stehen: 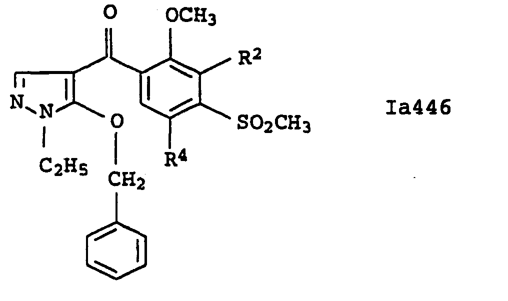
- Ebenso insbesondere außerordentlich bevorzugt sind die Verbindungen Ia447; insbesondere die Verbindungen Ia447.1-Ia447.164, die sich von den Verbindungen Ia1.1-Ia1.164 dadurch unterscheiden, daß R¹ für Methoxy, R⁷ für Benzyl und R⁸ für Methyl stehen: 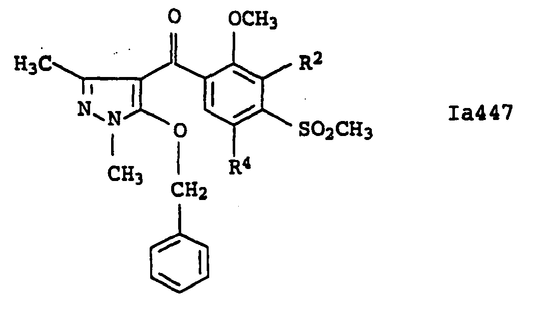
- Ebenso insbesondere außerordentlich bevorzugt sind die Verbindungen Ia448; insbesondere die Verbindungen Ia448.1-Ia448.164, die sich von den Verbindungen Ia1.1-Ia1.164 dadurch unterscheiden, daß R¹ für Methoxy und R⁷ für 4-Methylphenylmethyl stehen: 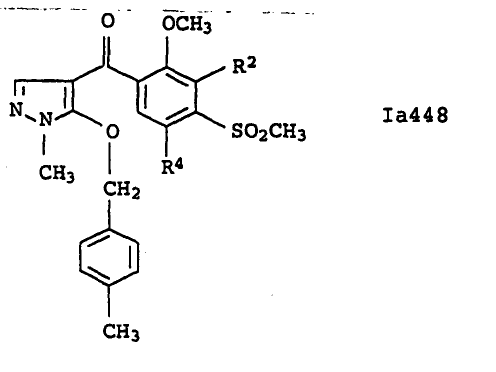
- Ebenso insbesondere außerordentlich bevorzugt sind die Verbindungen Ia449; insbesondere die Verbindungen Ia449.1-Ia449.164, die sich von den Verbindungen Ia1.1-Ia1.164 dadurch unterscheiden, daß R¹ für Methoxy, R⁶ für Ethyl und R⁷ für 4-Methylphenylmethyl stehen: 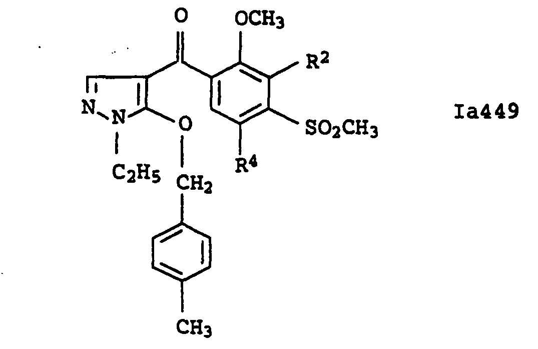
- Ebenso insbesondere außerordentlich bevorzugt sind die Verbindungen Ia447; insbesondere die Verbindungen Ia447.1-Ia447.164, die sich von den Verbindungen Ia1.1-Ia1.164 dadurch unterscheiden, daß R¹ für Methoxy, R⁷ für 4-Methylphenylmethyl und R⁸ für Methyl stehen: 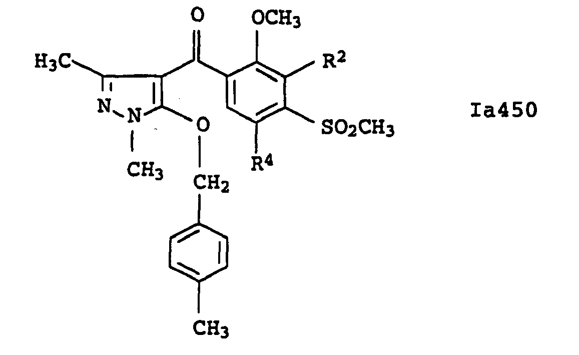
- Ebenso insbesondere außerordentlich bevorzugt sind die Verbindungen Ia451; insbesondere die Verbindungen 1a451.1-Ia451.164, die-sich von den Verbindungen Ia1.1-Ia1.164 dadurch unterscheiden, daß R¹ für Methoxy und R⁷ für 4-Chlorphenylmethyl stehen: 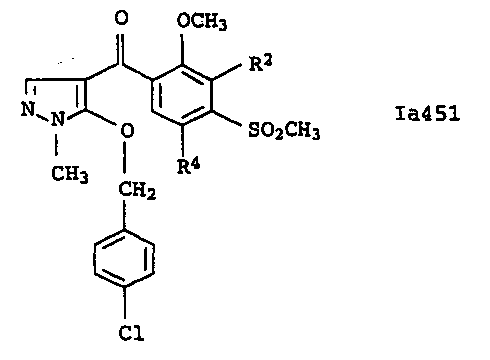
- Ebenso insbesondere außerordentlich bevorzugt sind die Verbindungen Ia452; insbesondere die Verbindungen Ia452.1-Ia452.164, die sich von den Verbindungen Ia1.1-Ia1.164 dadurch unterscheiden, daß R¹ für Methoxy, R⁶ für Ethyl und R⁷ für 4-Chlorphenylmethyl stehen: 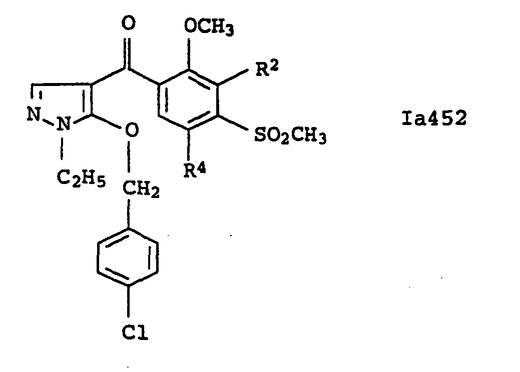
- Ebenso insbesondere außerordentlich bevorzugt sind die Verbindungen Ia453; insbesondere die Verbindungen Ia453.1-Ia453.164, die sich von den Verbindungen Ia1.1-Ia1.164 dadurch unterscheiden, daß R¹ für Methoxy, R⁷ für 4-Chlorphenylmethyl und R⁸ für Methyl stehen: 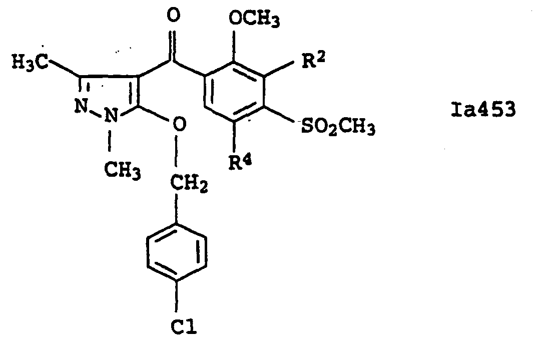
- Ebenso insbesondere außerordentlich bevorzugt sind die Verbindungen Ia454; insbesondere die Verbindungen Ia454.1-Ia454.164, die sich von den Verbindungen Ia1.1-Ia1.164 dadurch unterscheiden, daß R³ für Ethylsulfonyl steht: 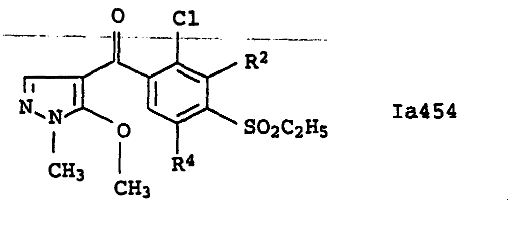
- Ebenso insbesondere außerordentlich bevorzugt sind die Verbindungen Ia455; insbesondere die Verbindungen Ia455.1-Ia455.164, die sich von den Verbindungen Ia1.1-Ia1.164 dadurch unterscheiden, daß R³ für Ethylsulfonyl und R⁶ für Ethyl stehen: 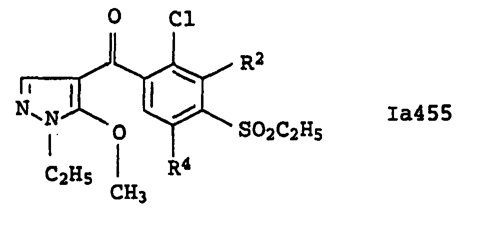
- Ebenso insbesondere außerordentlich bevorzugt sind die Verbindungen Ia456; insbesondere die Verbindungen Ia456.1-Ia456.164, die sich von den Verbindungen Ia1.1-Ia1.164 dadurch unterscheiden, daß R³ für Ethylsulfonyl und R⁸ für Methyl stehen: 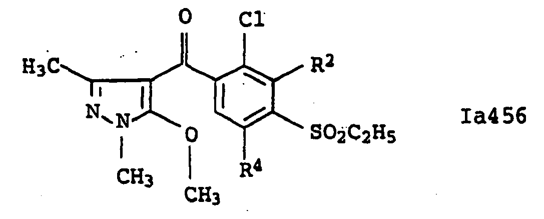
- Ebenso insbesondere außerordentlich bevorzugt sind die Verbindungen Ia457; insbesondere die Verbindungen Ia457.1-Ia457.164, die sich von den Verbindungen Ia1.1-Ia1.164 dadurch unterscheiden, daß R³ für Ethylsulfonyl und R⁷ für Ethyl stehen: 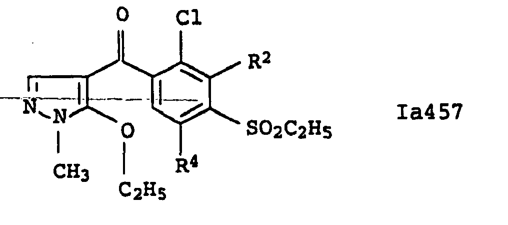
- Ebenso insbesondere außerordentlich bevorzugt sind die Verbindungen Ia458; insbesondere die Verbindungen Ia458.1-Ia458.164, die sich von den Verbindungen Ia1.1-Ia1.164 dadurch unterscheiden, daß R³ für Ethylsulfonyl, R⁶ und R⁷ für Ethyl stehen: 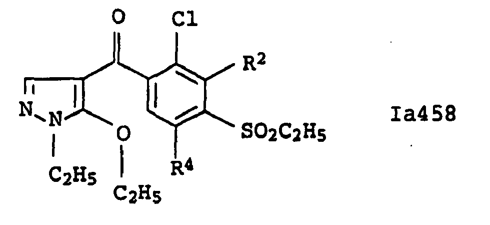
- Ebenso insbesondere außerordentlich bevorzugt sind die Verbindungen Ia459; insbesondere die Verbindungen Ia459.1-Ia459.164, die sich von den Verbindungen Ia1.1-Ia1.164 dadurch unterscheiden, daß R³ für Ethylsulfonyl, R⁷ für Ethyl und R⁸ für Methyl stehen: 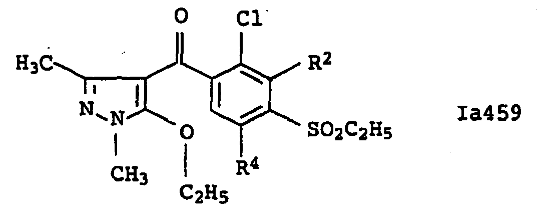
- Ebenso insbesondere außerordentlich bevorzugt sind die Verbindungen Ia460; insbesondere die Verbindungen Ia460.1-Ia460.164, die sich von den Verbindungen Ia1.1-Ia1.164 dadurch unterscheiden, daß R³ für Ethylsulfonyl und R⁷ für Allyl stehen: 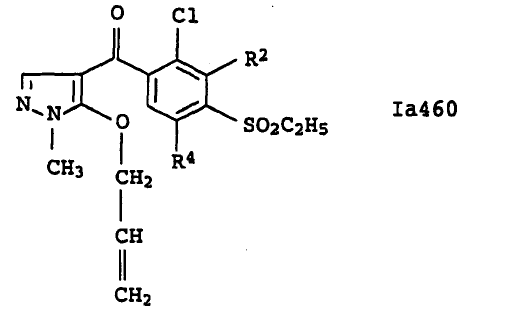
- Ebenso insbesondere außerordentlich bevorzugt sind die Verbindungen Ia461; insbesondere die Verbindungen Ia461.1-Ia461.164, die sich von den Verbindungen Ia1.1-Ia1.164 dadurch unterscheiden, daß R³ für Ethylsulfonyl, R⁶ und R⁷ für Allyl stehen: 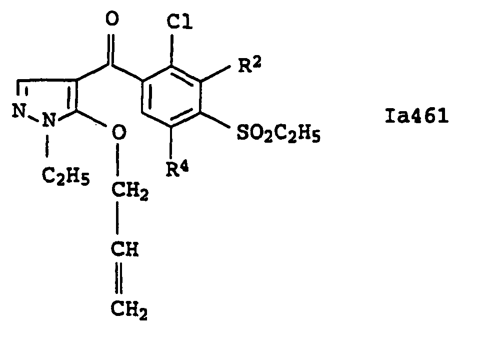
- Ebenso insbesondere außerordentlich bevorzugt sind die Verbindungen Ia462; insbesondere die Verbindungen Ia462.1-Ia462.164, die sich von den Verbindungen Ia1.1-Ia1.164 dadurch unterscheiden, daß R³ für Ethylsulfonyl, R⁷ für Allyl und R⁸ für Methyl stehen: 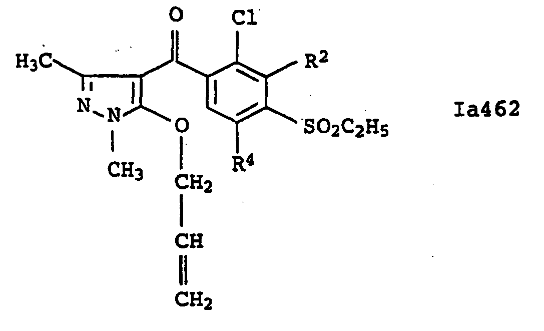
- Ebenso insbesondere außerordentlich bevorzugt sind die Verbindungen Ia463; insbesondere die Verbindungen Ia463.1-Ia463.164, die sich von den Verbindungen Ia1.1-Ia1.164 dadurch unterscheiden, daß R³ für Ethylsulfonyl und R⁷ für Methylcarbonyl stehen: 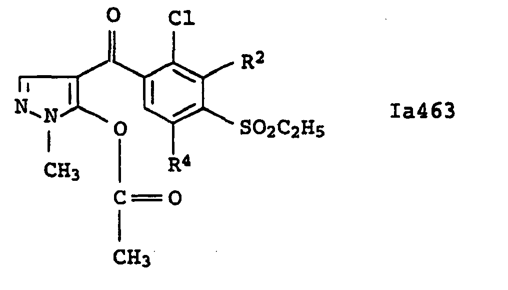
- Ebenso insbesondere außerordentlich bevorzugt sind die Verbindungen Ia464; insbesondere die Verbindungen Ia464.1-Ia464.164, die sich von den Verbindungen Ia1.1-Ia1.164 dadurch unterscheiden, daß R³ für Ethylsulfonyl, R⁶ für Ethyl und R⁷ für Methylcarbonyl stehen: 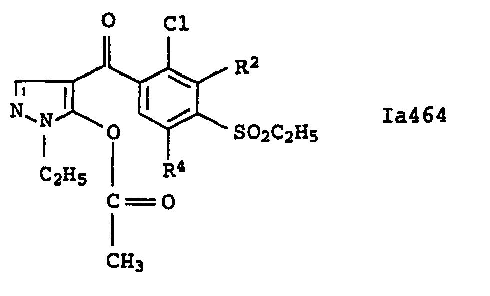
- Ebenso insbesondere außerordentlich bevorzugt sind die Verbindungen Ia465; insbesondere die Verbindungen Ia465.1-Ia465.164, die sich von den Verbindungen Ia1.1-Ia1.164 dadurch unterscheiden, daß R³ für Ethylsulfonyl, R⁷ für Methyl - carbonyl und R⁸ für Methyl stehen: 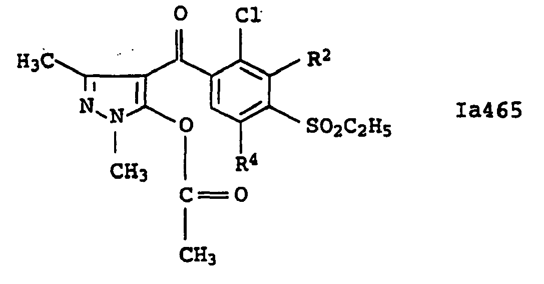
- Ebenso insbesondere außerordentlich bevorzugt sind die Verbindungen Ia466; insbesondere die Verbindungen Ia466.1-Ia466.164, die sich von den Verbindungen Ia1.1-Ia1.164 dadurch unterscheiden, daß R³ für Ethylsulfonyl und R⁷ für Methoxycarbonyl stehen: 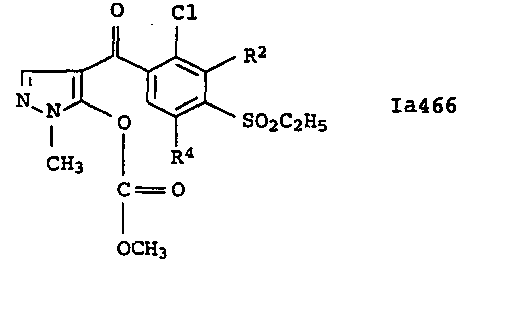
- Ebenso insbesondere außerordentlich bevorzugt sind die Verbindungen Ia467; insbesondere die Verbindungen Ia467.1-Ia467.164, die sich von den Verbindungen Ia1.1-Ia1.164 dadurch unterscheiden, daß R³ für Ethylsulfonyl, R⁶ für Ethyl und R⁷ für Methoxycarbonyl stehen: 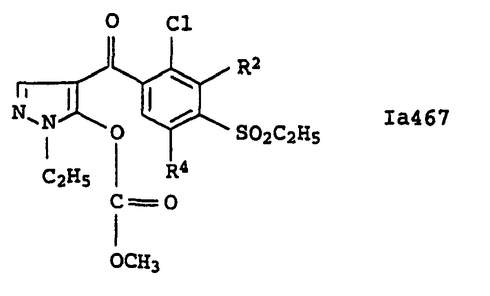
- Ebenso insbesondere außerordentlich bevorzugt sind die Verbindungen Ia468; insbesondere die Verbindungen Ia468.1-Ia468.164, die sich von den Verbindungen Ia1.1-Ia1.164 dadurch unterscheiden, daß R³ für Ethylsulfonyl, R⁷ für Methoxycarbonyl und R⁸ für Methyl stehen: 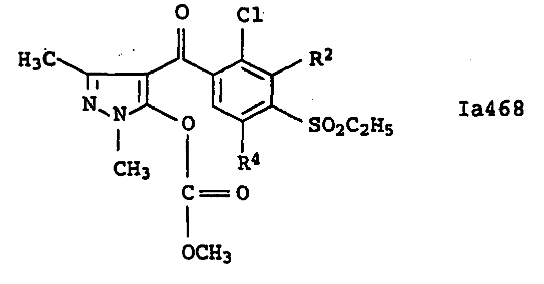
- Ebenso insbesondere außerordentlich bevorzugt sind die Verbindungen Ia469; insbesondere die Verbindungen Ia469.1-Ia469.164, die sich von den Verbindungen Ia1.1-Ia1.164 dadurch unterscheiden, daß R³ für Ethylsulfonyl und R⁷ für Dimethylaminocarbonyl stehen: 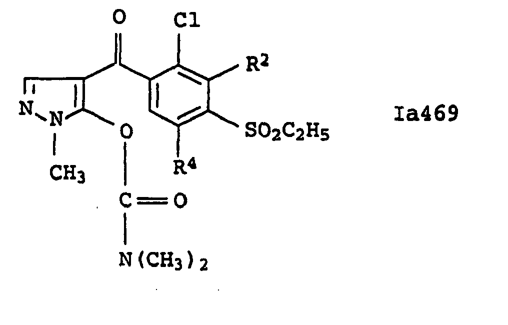
- Ebenso insbesondere außerordentlich bevorzugt sind die Verbindungen Ia470; insbesondere die Verbindungen Ia470.1-Ia470.164, die sich von den Verbindungen Ia1.1-Ia1.164 dadurch unterscheiden, daß R³ für Ethylsulfonyl, R⁶ für Ethyl und R⁷ für Dimethylaminocarbonyl stehen: 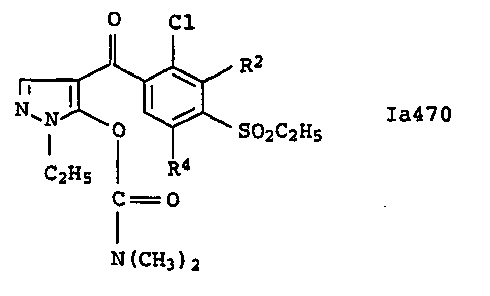
- Ebenso insbesondere außerordentlich bevorzugt sind die Verbindungen Ia471; insbesondere die Verbindungen Ia471.1-Ia471.164, die sich von den Verbindungen Ia1.1-Ia1.164 dadurch unterscheiden, daß R³ für Ethylsulfonyl, R⁷ für Dimethylaminocarbonyl und R⁸ für Methyl stehen: 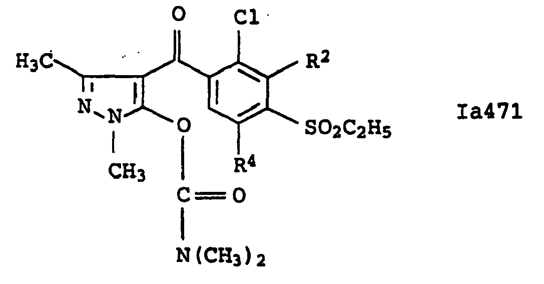
- Ebenso insbesondere außerordentlich bevorzugt sind die Verbindungen Ia472; insbesondere die Verbindungen Ia472.1-Ia472.164, die sich von den Verbindungen Ia1.1-Ia1.164 dadurch unterscheiden, daß R³ für Ethylsulfonyl und R⁷ für Methoxycarbonylmethyl stehen: 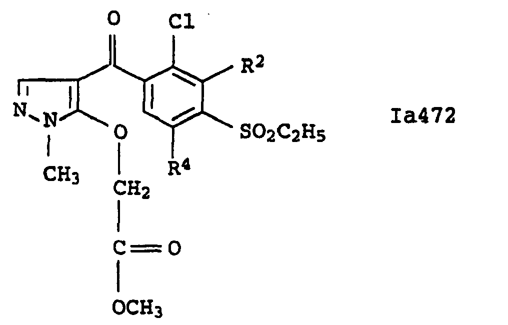
- Ebenso insbesondere außerordentlich bevorzugt sind die Verbindungen Ia473; insbesondere die Verbindungen Ia473.1-Ia473.164, die sich von den Verbindungen Ia1.1-Ia1.164 dadurch unterscheiden, daß R³ für Ethylsulfonyl, R⁶ für Ethyl und R⁷ für Methoxycarbonylmethyl stehen: 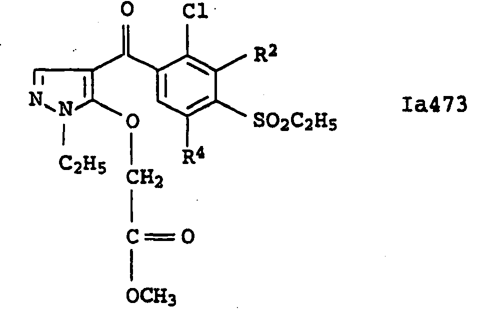
- Ebenso insbesondere außerordentlich bevorzugt sind die Verbindungen Ia474; insbesondere die Verbindungen Ia474.1-Ia474.164, die sich von den Verbindungen Ia1.1-Ia1.164 dadurch unterscheiden, daß R³ für Ethylsulfonyl, R⁷ für Methoxycarbonylmethyl und R⁸ für Methyl stehen: 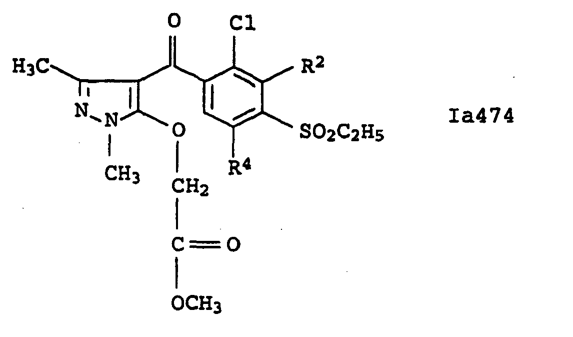
- Ebenso insbesondere außerordentlich bevorzugt sind die Verbindungen Ia475; insbesondere die Verbindungen 1a475.1-Ia475.164, die sich von den Verbindungen Ia1.1-Ia1.164 dadurch unterscheiden, daß R³ für Ethylsulfonyl und R⁷ für Dimethylaminocarbonylmethyl stehen: 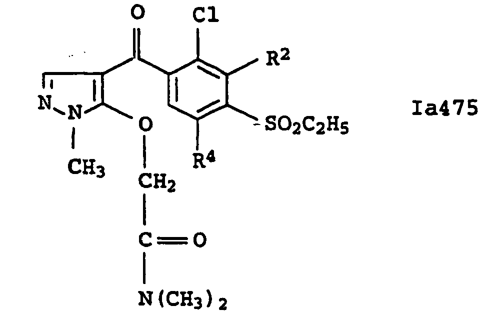
- Ebenso insbesondere außerordentlich bevorzugt sind die Verbindungen Ia476; insbesondere die Verbindungen Ia476.1-Ia476.164, die sich von den Verbindungen Ia1.1-Ia1.164 dadurch unterscheiden, daß R³ für Ethylsulfonyl, R⁶ für Ethyl und R⁷ für Dimethylaminocarbonylmethyl stehen: 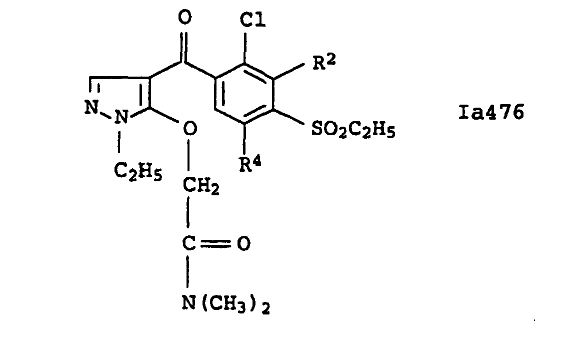
- Ebenso insbesondere außerordentlich bevorzugt sind die Verbindungen Ia477; insbesondere die Verbindungen Ia477.1-Ia477.164, die-sich von den Verbindungen Ia1.1-Ia1.164 dadurch unterscheiden, daß R³ für Ethylsulfonyl, R⁷ für Dimethylaminocarbonylmethyl und R⁸ für Methyl stehen: 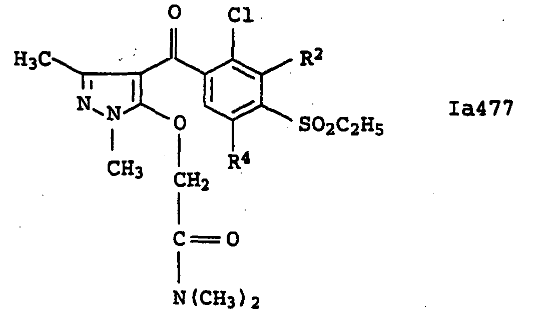
- Ebenso insbesondere außerordentlich bevorzugt sind die Verbindungen Ia478; insbesondere die Verbindungen Ia478.1-Ia478.164, die sich von den Verbindungen Ia1.1-Ia1.164 dadurch unterscheiden, daß R³ für Ethylsulfonyl und R⁷ für Phenylcarbonylmethyl stehen: 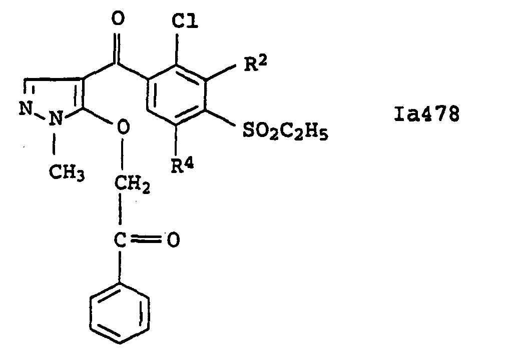
- Ebenso insbesondere außerordentlich bevorzugt sind die Verbindungen Ia479; insbesondere die Verbindungen Ia479.1-Ia479.164, die-sich von den Verbindungen Ia1.1-Ia1.164 dadurch unterscheiden, daß R³ für Ethylsulfonyl, R⁶ für Ethyl und R⁷ für Phenylcarbonylmethyl stehen: 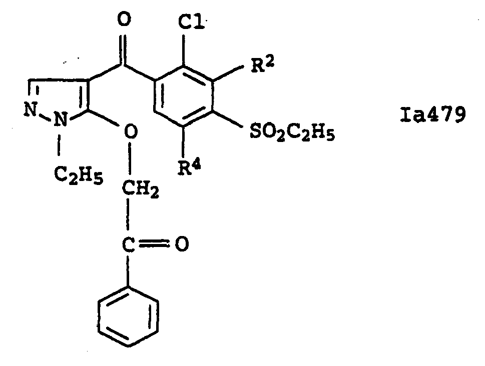
- Ebenso insbesondere außerordentlich bevorzugt sind die Verbindungen Ia480; insbesondere die Verbindungen Ia480.1-Ia480.164, die sich von den Verbindungen Ia1.1-Ia1.164 dadurch unterscheiden, daß R³ für Ethylsulfonyl, R⁷ für Phenylcarbonylmethyl und R⁸ für Methyl stehen: 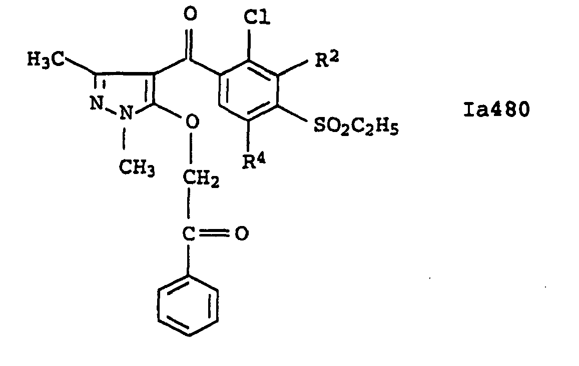
- Ebenso insbesondere außerordentlich bevorzugt sind die Verbindungen Ia481; insbesondere die Verbindungen Ia481.1-Ia481.164, die sich von den Verbindungen Ia1.1-Ia1.164 dadurch unterscheiden, daß R³ für Ethylsulfonyl und R⁷ für 4-Methylphenylcarbonylmethyl stehen: 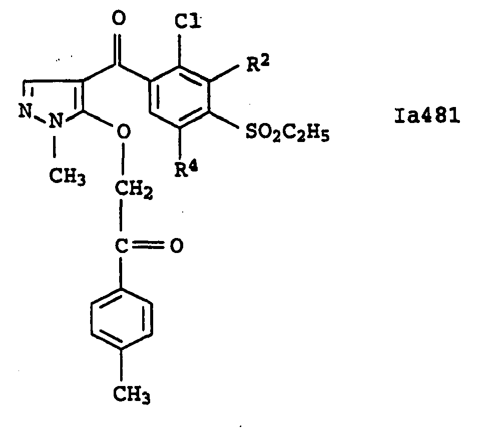
- Ebenso insbesondere außerordentlich bevorzugt sind die Verbindungen Ia482; insbesondere die Verbindungen Ia482.1-Ia482.164, die sich von den Verbindungen Ia1.1-Ia1.164 dadurch unterscheiden, daß R³ für Ethylsulfonyl, R⁶ für Ethyl und R⁷ für 4-Methylphenylcarbonylmethyl stehen: 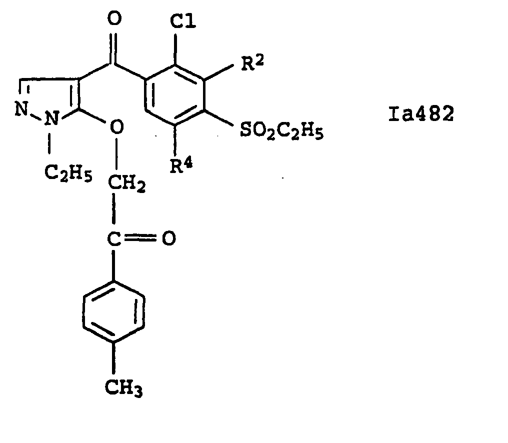
- Ebenso insbesondere außerordentlich bevorzugt sind die Verbindungen Ia483; insbesondere die Verbindungen Ia483.1-Ia483.164, die sich von den Verbindungen Ia1.1-Ia1.164 dadurch unterscheiden, daß R³ für Ethylsulfonyl, R⁷ für 4-Methylphenylcarbonylmethyl und R⁸ für Methyl stehen: 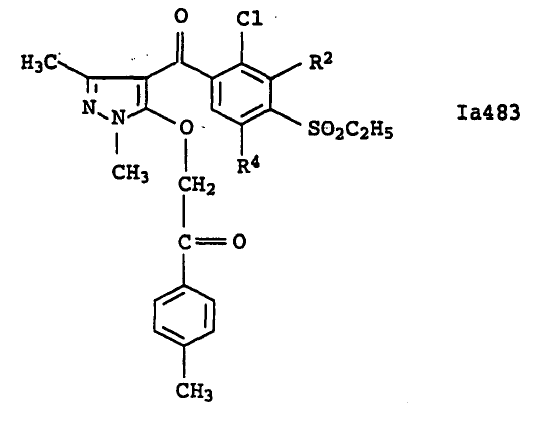
- Ebenso insbesondere außerordentlich bevorzugt sind die Verbindungen Ia484; insbesondere die Verbindungen Ia484.1-Ia484.164, die sich von den Verbindungen Ia1.1-Ia1.164 dadurch unterscheiden, daß R³ für Ethylsulfonyl und R⁷ für Phenylcarbonyl stehen: 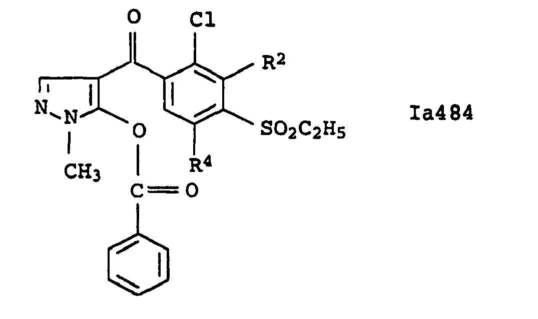
- Ebenso insbesondere außerordentlich bevorzugt sind die Verbindungen Ia485; insbesondere die Verbindungen Ia485.1-Ia485.164, die-sich von den Verbindungen Ia1.1-Ia1.164 dadurch unterscheiden, daß R³ für Ethylsulfonyl, R⁶ für Ethyl und R⁷ für Phenylcarbonyl stehen: 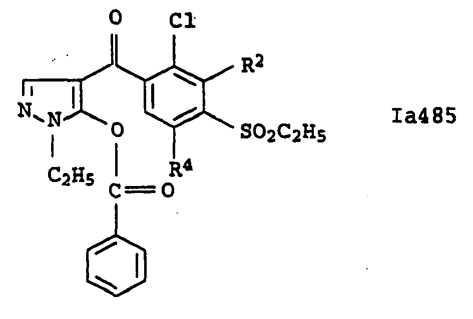
- Ebenso insbesondere außerordentlich bevorzugt sind die Verbindungen Ia486; insbesondere die Verbindungen Ia486.1-Ia486.164, die sich von den Verbindungen Ia1.1-Ia1.164 dadurch unterscheiden, daß R³ für Ethylsulfonyl, R⁷ für Phenylcarbonyl und R⁸ für Methyl stehen: 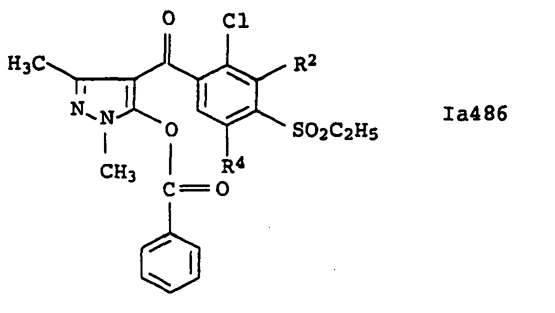
- Ebenso insbesondere außerordentlich bevorzugt sind die Verbindungen Ia487; insbesondere die Verbindungen Ia487.1-Ia487.164, die-sich von den Verbindungen Ia1.1-Ia1.164 dadurch unterscheiden, daß R³ für Ethylsulfonyl und R⁷ für 4-Methylphenylcarbonyl stehen: 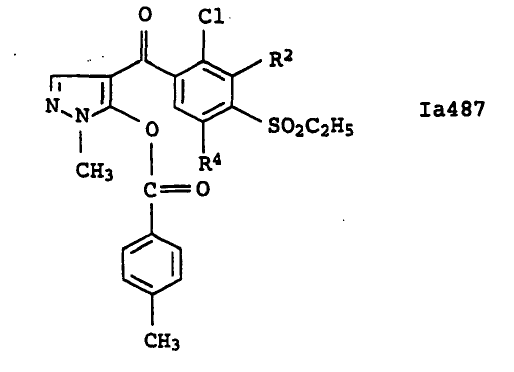
- Ebenso insbesondere außerordentlich bevorzugt sind die Verbindungen Ia488; insbesondere die Verbindungen Ia488.1-Ia488.164, die sich von den Verbindungen Ia1.1-Ia1.164 dadurch unterscheiden, daß R³ für Ethylsulfonyl, R⁶ für Ethyl und R⁷ für 4-Methylphenylcarbonyl stehen: 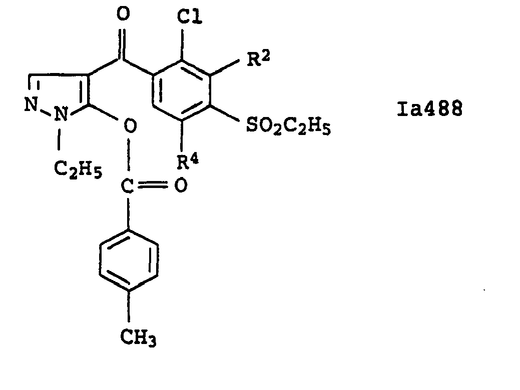
- Ebenso insbesondere außerordentlich bevorzugt sind die Verbindungen Ia489; insbesondere die Verbindungen Ia489.1-Ia489.164, die-sich von den Verbindungen Ia1.1-Ia1.164 dadurch unterscheiden, daß R³ für Ethylsulfonyl, R⁷ für 4-Methylphenylcarbonyl und R⁸ für Methyl stehen: 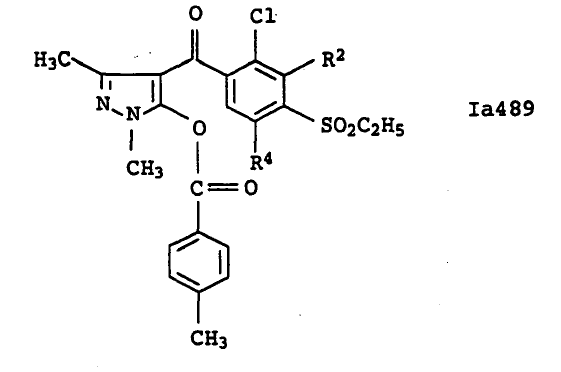
- Ebenso insbesondere außerordentlich bevorzugt sind die Verbindungen Ia490; insbesondere die Verbindungen Ia490.1-Ia490.164, die sich von den Verbindungen Ia1.1-Ia1.164 dadurch unterscheiden, daß R³ für Ethylsulfonyl und R⁷ für Benzyl stehen: 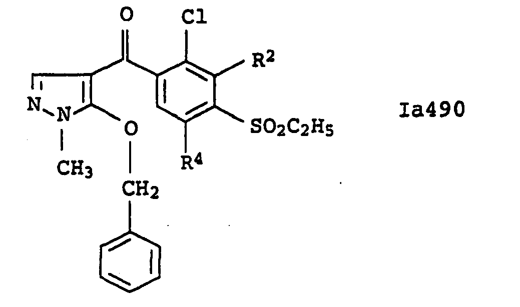
- Ebenso insbesondere außerordentlich bevorzugt sind die Verbindungen Ia491; insbesondere die Verbindungen Ia491.1-Ia491.164, die sich von den Verbindungen Ia1.1-Ia1.164 dadurch unterscheiden, daß R³ für Ethylsulfonyl, R⁶ für Ethyl und R⁷ für Benzyl stehen: 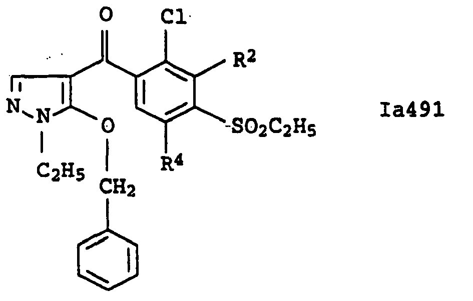
- Ebenso insbesondere außerordentlich bevorzugt sind die Verbindungen Ia492; insbesondere die Verbindungen Ia492.1-Ia492.164, die sich von den Verbindungen Ia1.1-Ia1.164 dadurch unterscheiden, daß R³ für Ethylsulfonyl, R⁷ für Benzyl und R⁸ für Methyl stehen: 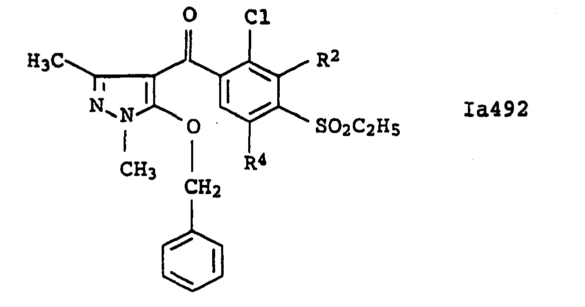
- Ebenso insbesondere außerordentlich bevorzugt sind die Verbindungen Ia493; insbesondere die Verbindungen Ia493.1-Ia493.164, die sich von den Verbindungen Ia1.1-Ia1.164 dadurch unterscheiden, daß R³ für Ethylsulfonyl und R⁷ für 4-Methylphenylmethyl stehen: 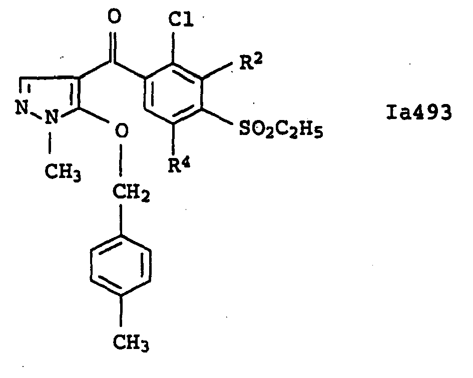
- Ebenso insbesondere außerordentlich bevorzugt sind die Verbindungen Ia494; insbesondere die Verbindungen Ia494.1-Ia494.164, die sich von den Verbindungen Ia1.1-Ia1.164 dadurch unterscheiden, daß R³ für Ethylsulfonyl, R⁶ für Ethyl und R⁷ für 4-Methylphenylmethyl stehen: 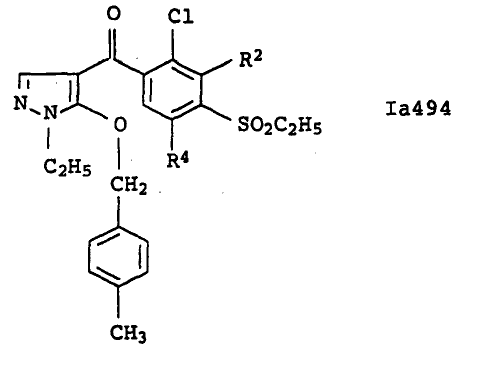
- Ebenso insbesondere außerordentlich bevorzugt sind die Verbindungen Ia495; insbesondere die Verbindungen Ia495.1-Ia495.164, die-sich von den Verbindungen Ia1.1-Ia1.164 dadurch unterscheiden, daß R³ für Ethylsulfonyl, R⁷ für 4-Methylphenylmethyl und R⁸ für Methyl stehen: 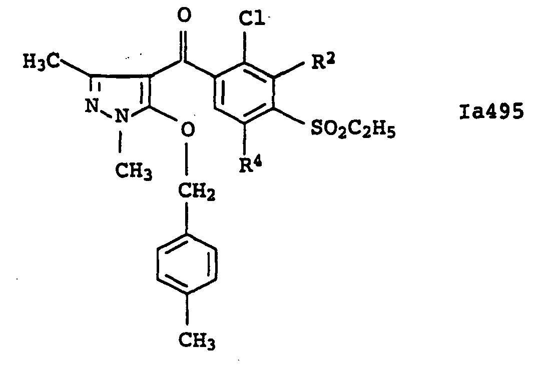
- Ebenso insbesondere außerordentlich bevorzugt sind die Verbindungen Ia496; insbesondere die Verbindungen Ia496.1-Ia496.164, die sich von den Verbindungen Ia1.1-Ia1.164 dadurch unterscheiden, daß R³ für Ethylsulfonyl und R⁷ für 4-Chlorphenylmethyl stehen: 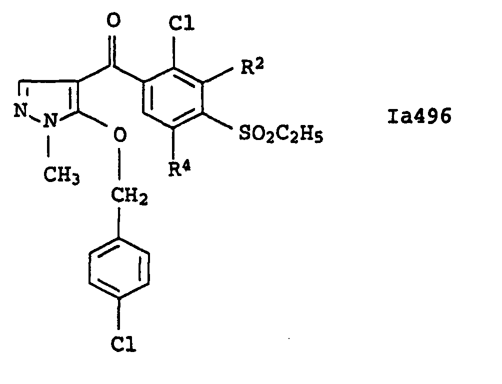
- Ebenso insbesondere außerordentlich bevorzugt sind die Verbindungen Ia497; insbesondere die Verbindungen Ia497.1-Ia497.164, die-sich von den Verbindungen Ia1.1-Ia1.164 dadurch unterscheiden, daß R³ für Ethylsulfonyl, R⁶ für Ethyl und R⁷ für 4-Chlorphenylmethyl stehen: 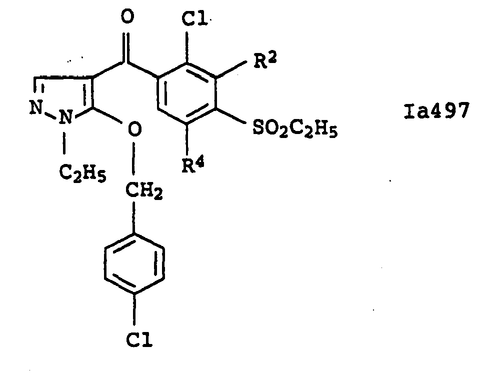
- Ebenso insbesondere außerordentlich bevorzugt sind die Verbindungen Ia498; insbesondere die Verbindungen Ia498.1-Ia498.164, die sich von den Verbindungen Ia1.1-Ia1.164 dadurch unterscheiden, daß R³ für Ethylsulfonyl, R⁷ für 4-Chlorphenylmethyl und R⁸ für Methyl stehen: 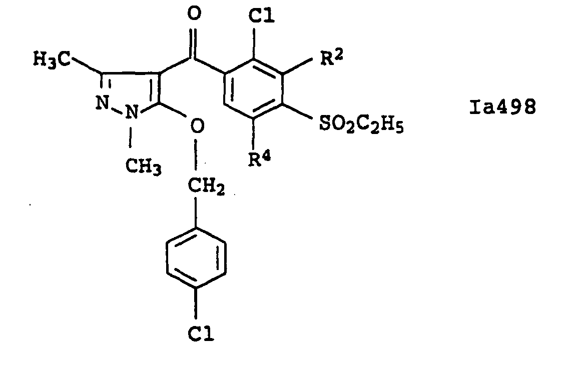
- Ebenso insbesondere außerordentlich bevorzugt sind die Verbindungen Ia499; insbesondere die Verbindungen Ia499.1-Ia499.164, die sich von den Verbindungen Ia1.1-Ia1.164 dadurch unterscheiden, daß R¹ für Trifluormethyl steht: 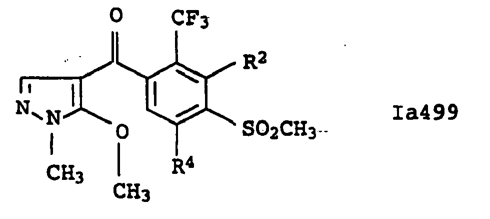
- Ebenso insbesondere außerordentlich bevorzugt sind die Verbindungen Ia500; insbesondere die Verbindungen Ia500.1-Ia500.164, die sich von den Verbindungen Ia1.1-Ia1.164 dadurch unterscheiden, daß R¹ für Trifluormethyl und R⁶ für Ethyl stehen: 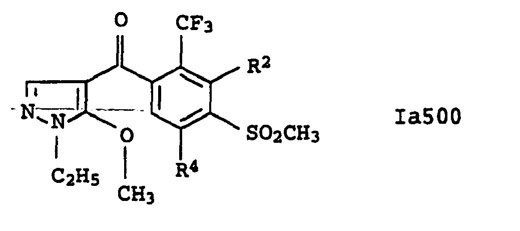
- Ebenso insbesondere außerordentlich bevorzugt sind die Verbindungen Ia501; insbesondere die Verbindungen Ia501.1-Ia501.164, die sich von den Verbindungen Ia1.1-Ia1.164 dadurch unterscheiden, daß R¹ für Trifluormethyl und R⁷ für Ethyl stehen: 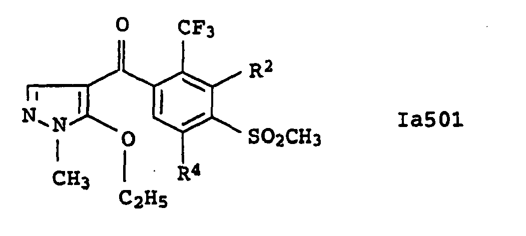
- Ebenso insbesondere außerordentlich bevorzugt sind die Verbindungen Ia502; insbesondere die Verbindungen Ia502.1-Ia502.164, die-sich von den Verbindungen Ia1.1-Ia1.164 dadurch unterscheiden, daß R¹ für Trifluormethyl, R⁶ und R⁷ für Ethyl stehen: 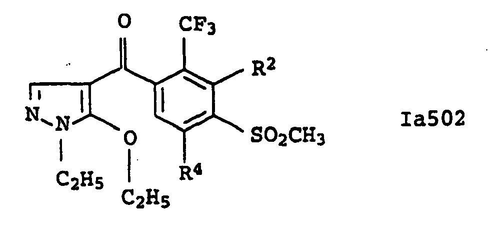
- Ebenso insbesondere außerordentlich bevorzugt sind die Verbindungen Ia503; insbesondere die Verbindungen Ia503.1-Ia503.164, die sich von den Verbindungen Ia1.1-Ia1.164 dadurch unterscheiden, daß R¹ für Trifluormethyl und R⁷ für Methylcarbonyl stehen: 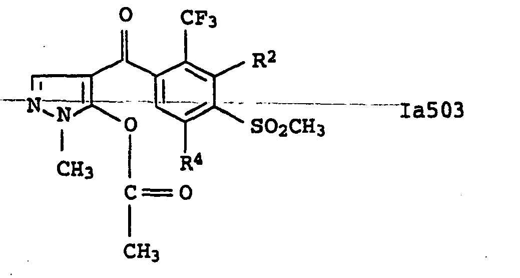
- Ebenso insbesondere außerordentlich bevorzugt sind die Verbindungen Ia504; insbesondere die Verbindungen Ia504.1-Ia504.164, die sich von den Verbindungen Ia1.1-Ia1.164 dadurch unterscheiden, daß R¹ für Trifluormethyl, R⁶ für Ethyl und R⁷ für Methylcarbonyl stehen: 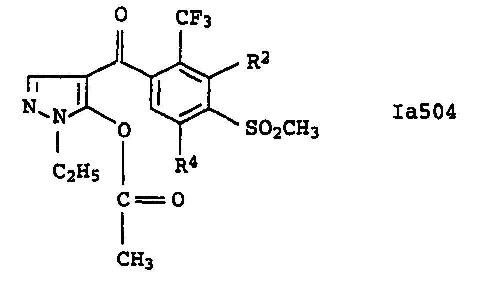
- Ebenso insbesondere außerordentlich bevorzugt sind die Verbindungen Ia505; insbesondere die Verbindungen Ia505.1-Ia505.164, die sich von den Verbindungen Ia1.1-Ia1.164 dadurch unterscheiden, daß R¹ für Trifluormethyl und R⁷ für Methoxycarbonyl stehen: 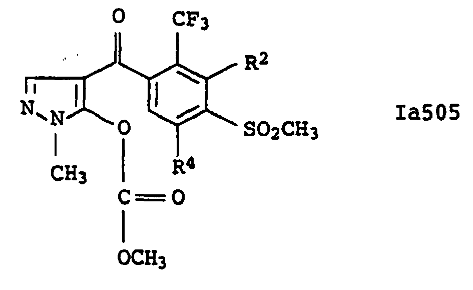
- Ebenso insbesondere außerordentlich bevorzugt sind die Verbindungen Ia506; insbesondere die Verbindungen Ia506.1-Ia506.164, die sich von den Verbindungen Ia1.1-Ia1.164 dadurch unterscheiden, daß R¹ für Trifluormethyl, R⁶ für Ethyl und R⁷ für Methoxycarbonyl stehen: 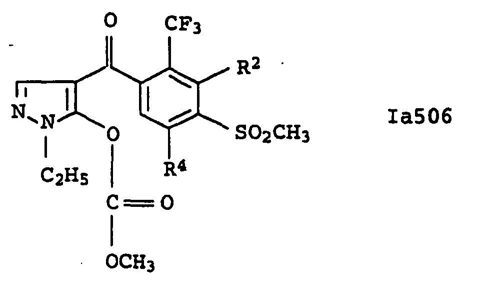
- Ebenso insbesondere außerordentlich bevorzugt sind die Verbindungen Ia507; insbesondere die Verbindungen Ia507.1-Ia507.164, die sich von den Verbindungen Ia1.1-Ia1.164 dadurch unterscheiden, daß R¹ für Trifluormethyl und R⁷ für Dimethylaminocarbonyl stehen: 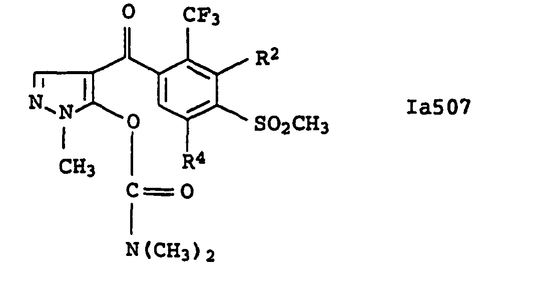
- Ebenso insbesondere außerordentlich bevorzugt sind die Verbindungen Ia508; insbesondere die Verbindungen Ia508.1-Ia508.164, die-sich von den Verbindungen Ia1.1-Ia1.164 dadurch unterscheiden, daß R¹ für Trifluormethyl, R⁶ für Ethyl und R⁷ für Dimethylaminocarbonyl stehen: 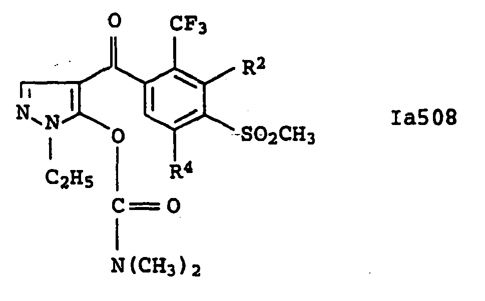
- Ebenso insbesondere außerordentlich bevorzugt sind die Verbindungen Ia509; insbesondere die Verbindungen Ia509.1-Ia509.164, die sich von den Verbindungen Ia1.1-Ia1.164 dadurch unterscheiden, daß R¹ für Trifluormethyl und R⁷ für Methoxycarbonylmethyl stehen: 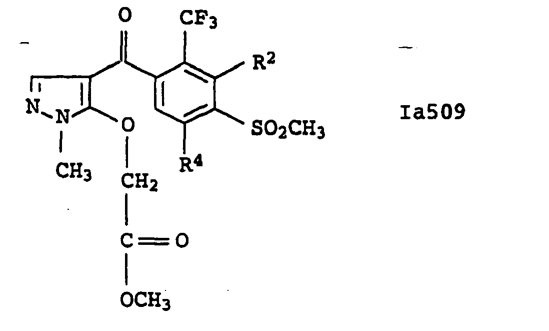
- Ebenso insbesondere außerordentlich bevorzugt sind die Verbindungen Ia510; insbesondere die Verbindungen Ia510.1-Ia510.164, die sich von den Verbindungen Ia1.1-Ia1.164 dadurch unterscheiden, daß R¹ für Trifluormethyl, R⁶ für Ethyl und R⁷ für Methoxycarbonylmethyl stehen: 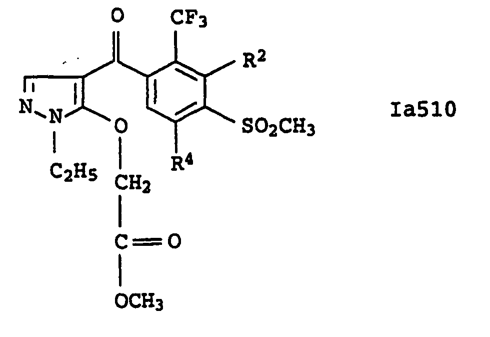
- Ebenso insbesondere außerordentlich bevorzugt sind die Verbindungen Ia511; insbesondere die Verbindungen Ia511.1-Ia511.164, die sich von den Verbindungen Ia1.1-Ia1.164 dadurch unterscheiden, daß R¹ für Trifluormethyl und R⁷ für Phenylcarbonylmethyl stehen: 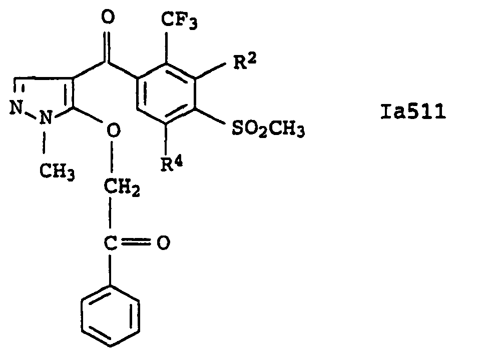
- Ebenso insbesondere außerordentlich bevorzugt sind die Verbindungen Ia512; insbesondere die Verbindungen Ia512.1-Ia512.164, die sich von den Verbindungen Ia1.1-Ia1.164 dadurch unterscheiden, daß R¹ für Trifluormethyl, R⁶ für Ethyl und R⁷ für Phenylcarbonylmethyl stehen: 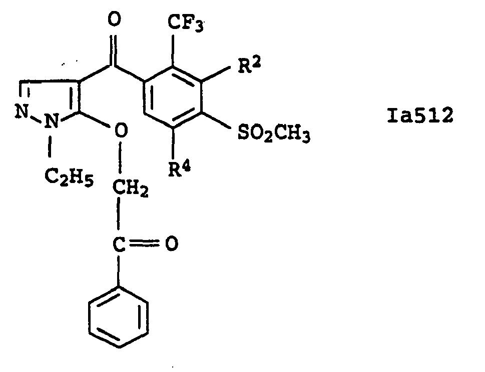
- Ebenso insbesondere außerordentlich bevorzugt sind die Verbindungen Ia513; insbesondere die Verbindungen Ia513.1-Ia513.164, die sich von den Verbindungen Ia1.1-Ia1.164 dadurch unterscheiden, daß R¹ für Trifluormethyl und R⁷ für Phenylcarbonyl stehen: 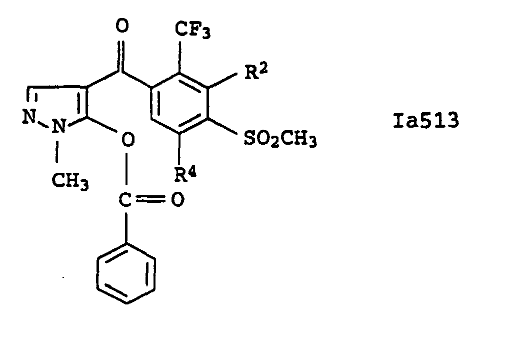
- Ebenso insbesondere außerordentlich bevorzugt sind die Verbindungen Ia514; insbesondere die Verbindungen Ia514.1-Ia514.164, die-sich von den Verbindungen Ia1.1-Ia1.164 dadurch unterscheiden, daß R¹ für Trifluormethyl, R⁶ für Ethyl und R⁷ für Phenylcarbonyl stehen: 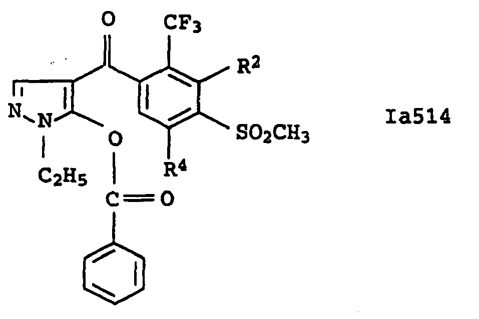
- Ebenso insbesondere außerordentlich bevorzugt sind die Verbindungen Ia515; insbesondere die Verbindungen Ia515.1-Ia515.164, die sich von den Verbindungen Ia1.1-Ia1.164 dadurch unterscheiden, daß R¹ für Trifluormethyl und R⁷ für Benzyl stehen: 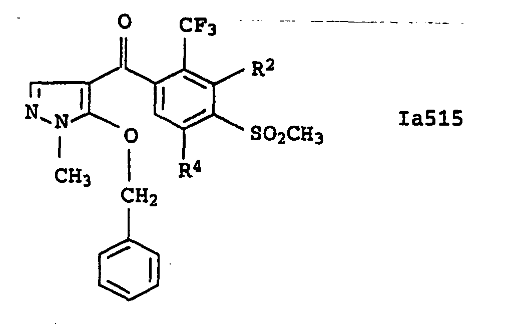
- Ebenso insbesondere außerordentlich bevorzugt sind die Verbindungen Ia516; insbesondere die Verbindungen Ia514.1-Ia514.164, die sich von den Verbindungen Ia1.1-Ia1.164 dadurch unterscheiden, daß R¹ für Trifluormethyl, R⁶ für Ethyl und R⁷ für Benzyl stehen: 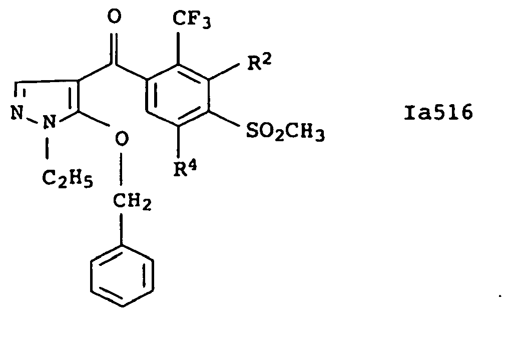
- Ebenso insbesondere außerordentlich bevorzugt sind die Verbindungen Ia553; insbesondere die Verbindungen Ia553.1-Ia553.164, die sich von den Verbindungen Ia1.1-Ia1.164 dadurch unterscheiden, daß R¹ für Methyl und R³ für Chlor stehen: 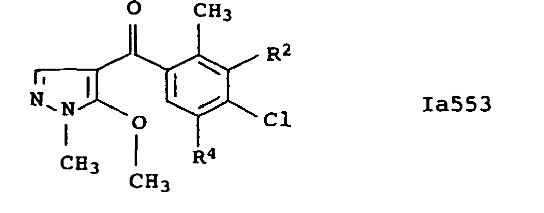
- Ebenso insbesondere außerordentlich bevorzugt sind die Verbindungen Ia554; insbesondere die Verbindungen Ia554.1-Ia554.164, die sich von den Verbindungen Ia1.1-Ia1.164 dadurch unterscheiden, daß R¹ für Methyl, R³ für Chlor und R⁶ für Ethyl stehen: 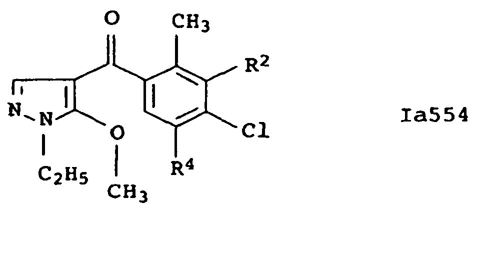
- Ebenso insbesondere außerordentlich bevorzugt sind die Verbindungen Ia555; insbesondere die Verbindungen Ia555.1-Ia555.164, die-sich von den Verbindungen Ia1.1-Ia1.164 dadurch unterscheiden, daß R¹ für Methyl, R³ für Chlor und R⁷ für Ethyl stehen: 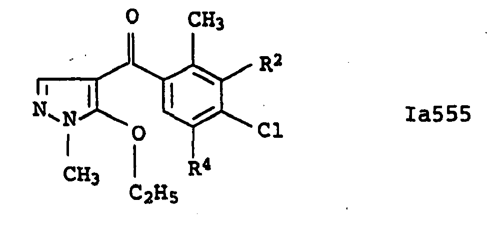
- Ebenso insbesondere außerordentlich bevorzugt sind die Verbindungen Ia556; insbesondere die Verbindungen Ia556.1-Ia556.164, die sich von den Verbindungen Ia1.1-Ia1.164 dadurch unterscheiden, daß R¹ für Methyl, R³ für Chlor, R⁶ und R⁷ für Ethyl stehen: 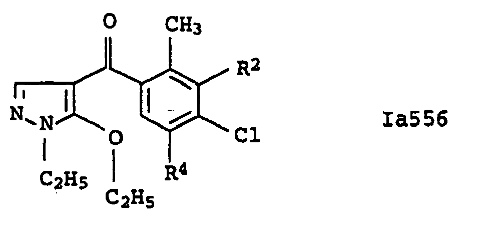
- Ebenso insbesondere außerordentlich bevorzugt sind die Verbindungen Ia557; insbesondere die Verbindungen Ia557.1-Ia557.164, die sich von den Verbindungen Ia1.1-Ia1.164 dadurch unterscheiden, daß R¹ für Methyl, R³ für Chlor und R⁷ für Methylcarbonyl stehen: 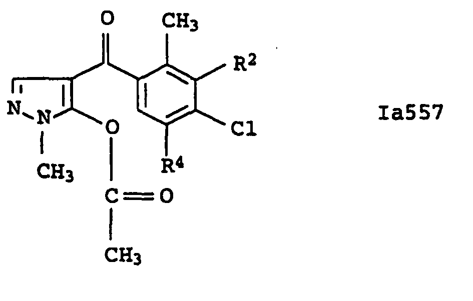
- Ebenso insbesondere außerordentlich bevorzugt sind die Verbindungen Ia558; insbesondere die Verbindungen Ia558.1-Ia558.164, die-sich von den Verbindungen Ia1.1-Ia1.164 dadurch unterscheiden, daß R¹ für Methyl, _{R}³ für Chlor, R⁶ für Ethyl und R⁷ für Methylcarbonyl stehen: 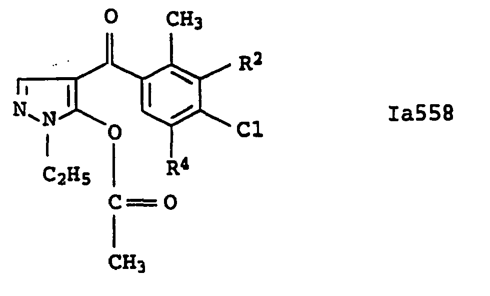
- Ebenso insbesondere außerordentlich bevorzugt sind die Verbindungen Ia559; insbesondere die Verbindungen Ia559.1-Ia559.164, die sich von den Verbindungen Ia1.1-Ia1.164 dadurch unterscheiden, daß R¹ für Methyl, R³ für Chlor und R⁷ für Methoxycarbonyl stehen: 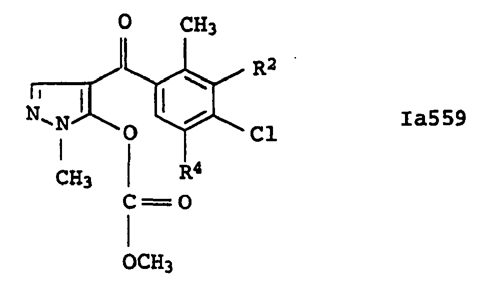
- Ebenso insbesondere außerordentlich bevorzugt sind die Verbindungen Ia560; insbesondere die Verbindungen Ia560.1-Ia560.164, die sich von den Verbindungen Ia1.1-Ia1.164 dadurch unterscheiden, daß R¹ für Methyl, R³ für Chlor, R⁶ für Ethyl und R⁷ für Methoxycarbonyl stehen: 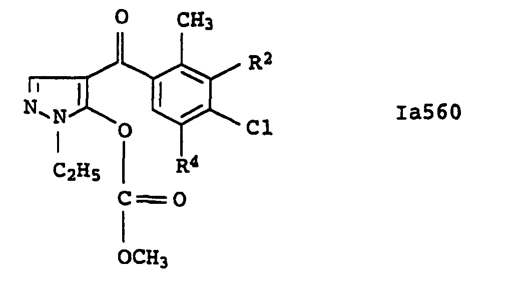
- Ebenso insbesondere außerordentlich bevorzugt sind die Verbindungen Ia561; insbesondere die Verbindungen Ia561.1-Ia561.164, die-sich von den Verbindungen Ia1.1-Ia1.164 dadurch unterscheiden, daß R¹ für Methyl, R³ für Chlor und R⁷ für Dimethylaminocarbonyl stehen: 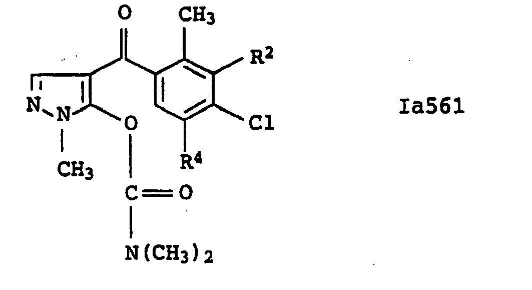
- Ebenso insbesondere außerordentlich bevorzugt sind die Verbindungen Ia562; insbesondere die Verbindungen Ia562.1-Ia562.164, die sich von den Verbindungen Ia1.1-Ia1.164 dadurch unterscheiden, daß R¹ für Methyl, R³ für Chlor, R⁶ für Ethyl und R⁷ für Dimethylaminocarbonyl stehen: 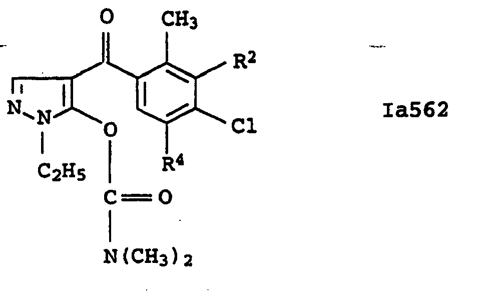
- Ebenso insbesondere außerordentlich bevorzugt sind die Verbindungen Ia563; insbesondere die Verbindungen Ia563.1-Ia563.164, die-sich von den Verbindungen Ia1.1-Ia1.164 dadurch unterscheiden, daß R¹ für Methyl, R³ für Chlor und R⁷ für Methoxycarbonylmethyl stehen: 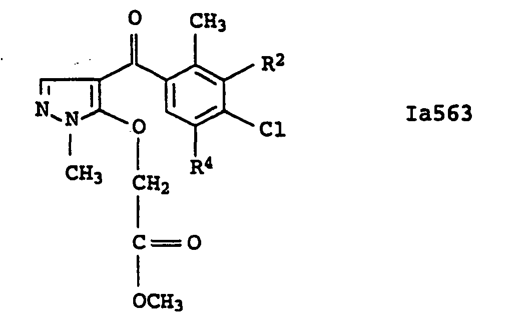
- Ebenso insbesondere außerordentlich bevorzugt sind die Verbindungen Ia564; insbesondere die Verbindungen Ia564.1-Ia564.164, die sich von den Verbindungen Ia1.1-Ia1.164 dadurch unterscheiden, daß R¹ für Methyl, R³ für Chlor, R⁶ für Ethyl und R⁷ für Methoxycarbonylmethyl stehen: 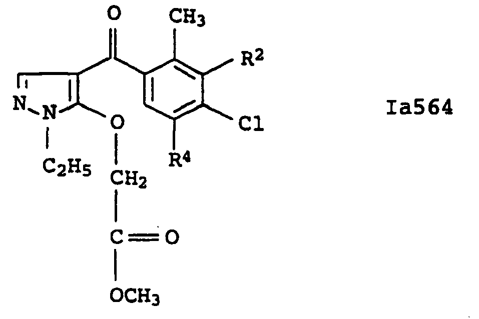
- Ebenso insbesondere außerordentlich bevorzugt sind die Verbindungen Ia565; insbesondere die Verbindungen Ia565.1-Ia565.164, die-sich von den Verbindungen Ia1.1-Ia1.164 dadurch unterscheiden, daß R¹ für Methyl, R³ für Chlor und R⁷ für Phenylcarbonylmethyl stehen: 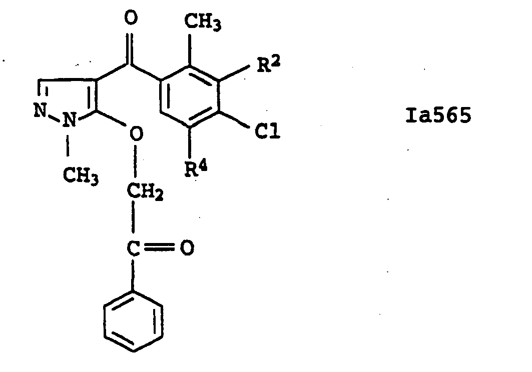
- Ebenso insbesondere außerordentlich bevorzugt sind die Verbindungen Ia566; insbesondere die Verbindungen Ia566.1-Ia566.164, die sich von den Verbindungen Ia1.1-Ia1.164 dadurch unterscheiden, daß R¹ für Methyl, R³ für Chlor, R⁶ für Ethyl und R⁷ für Phenylcarbonylmethyl stehen: 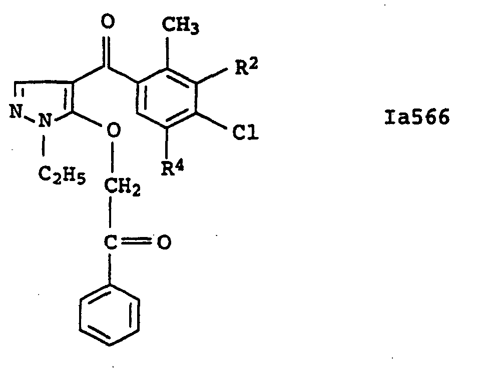
- Ebenso insbesondere außerordentlich bevorzugt sind die Verbindungen Ia567; insbesondere die Verbindungen Ia567.1-Ia567.164, die sich von den Verbindungen Ia1.1-Ia1.164 dadurch unterscheiden, daß R¹ für Methyl, R³ für Chlor und R⁷ für Phenylcarbonyl stehen: 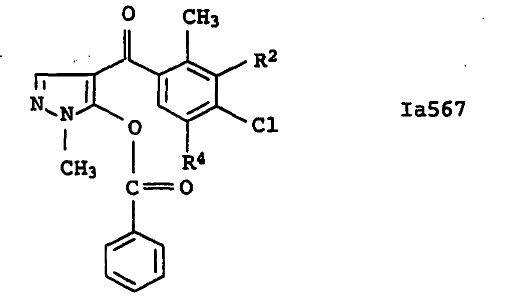
- Ebenso insbesondere außerordentlich bevorzugt sind die Verbindungen Ia568; insbesondere die Verbindungen Ia568.1-Ia568.164, die sich von den Verbindungen Ia1.1-Ia1.164 dadurch unterscheiden, daß R¹ für Methyl, R³ für Chlor, R⁶ für Ethyl und R⁷ für Phenylcarbonyl stehen: 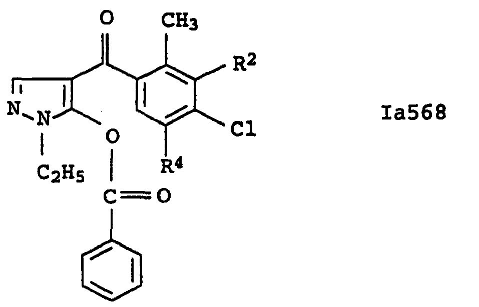
- Ebenso insbesondere außerordentlich bevorzugt sind die Verbindungen Ia569; insbesondere die Verbindungen Ia569.1-Ia569.164, die-sich von den Verbindungen Ia1.1-Ia1.164 dadurch unterscheiden, daß R¹ für Methyl, R³ für Chlor und R⁷ für Benzyl stehen: 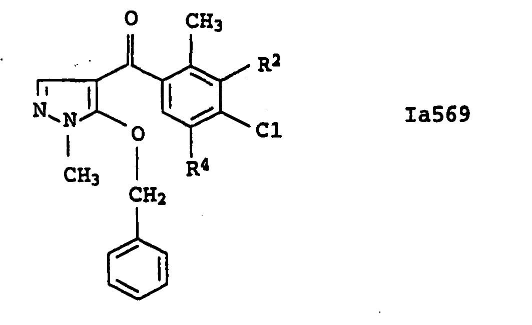
- Ebenso insbesondere außerordentlich bevorzugt sind die Verbindungen Ia570; insbesondere die Verbindungen Ia570.1-Ia570.164, die sich von den Verbindungen Ia1.1-Ia1.164 dadurch unterscheiden, daß R¹ für Methyl, R³ für Chlor, R⁶ für Ethyl und R⁷ für Benzyl stehen: 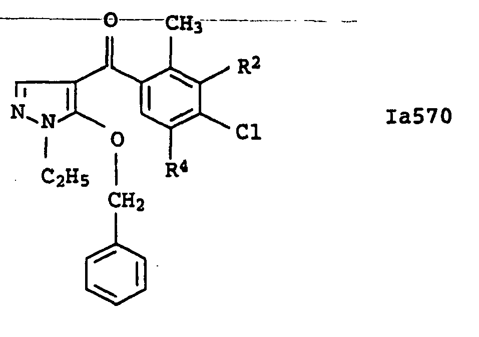
- Ebenso insbesondere außerordentlich bevorzugt sind die Verbindungen Ia571; insbesondere die Verbindungen Ia571.1-Ia571.164, die-sich von den Verbindungen Ia1.1-Ia1.164 dadurch unterscheiden, daß R³ für Methyl und R⁷ für Ethyl stehen: 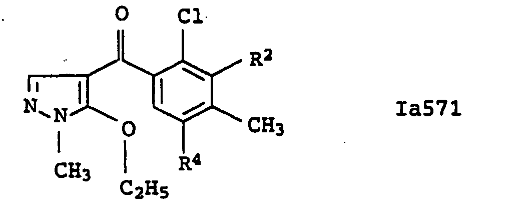
- Ebenso insbesondere außerordentlich bevorzugt sind die Verbindungen Ia572; insbesondere die Verbindungen Ia572.1-Ia572.164, die sich von den Verbindungen Ia1.1-Ia1.164 dadurch unterscheiden, daß R³ für Methyl, R⁶ und R⁷ für Ethyl stehen: 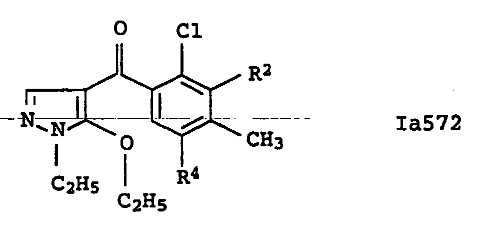
- Ebenso insbesondere außerordentlich bevorzugt sind die Verbindungen Ia573; insbesondere die Verbindungen Ia573.1-Ia573.164, die sich von den Verbindungen Ia1.1-Ia1.164 dadurch unterscheiden, daß R³ für Methyl und R⁷ für Methylcarbonyl stehen: 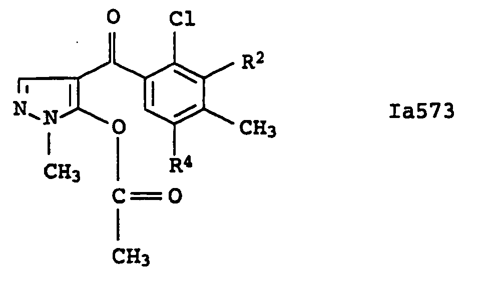
- Ebenso insbesondere außerordentlich bevorzugt sind die Verbindungen Ia574; insbesondere die Verbindungen Ia574.1-Ia574.164, die sich von den Verbindungen Ia1.1-Ia1.164 dadurch unterscheiden, daß R³ für Methyl, R⁶ für Ethyl und R⁷ für Methylcarbonyl stehen: 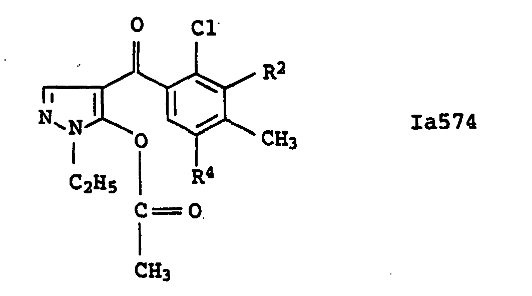
- Ebenso insbesondere außerordentlich bevorzugt sind die Verbindungen Ia575; insbesondere die Verbindungen Ia575.1-Ia575.164, die sich von den Verbindungen Ia1.1-Ia1.164 dadurch unterscheiden, daß R³ für Methyl und R⁷ für Dimethylaminocarbonyl stehen: 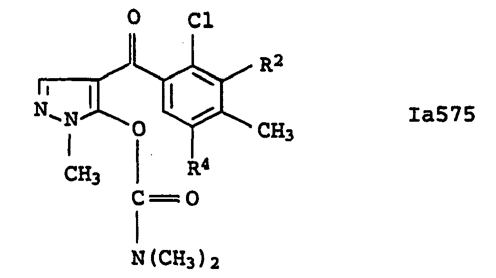
- Ebenso insbesondere außerordentlich bevorzugt sind die Verbindungen Ia576; insbesondere die Verbindungen Ia576.1-Ia576.164, die sich von den Verbindungen Ia1.1-Ia1.164 dadurch unterscheiden, daß R³ für Methyl, R⁶ für Ethyl und R⁷ für Dimethylaminocarbonyl stehen: 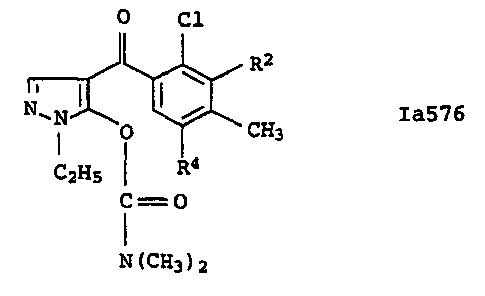
- Ebenso insbesondere außerordentlich bevorzugt sind die Verbindungen Ia577; insbesondere die Verbindungen Ia577.1-Ia577.164, die sich von den Verbindungen Ia1.1-Ia1.164 dadurch unterscheiden, daß R³ für Methyl und R⁷ für Phenylcarbonylmethyl stehen: 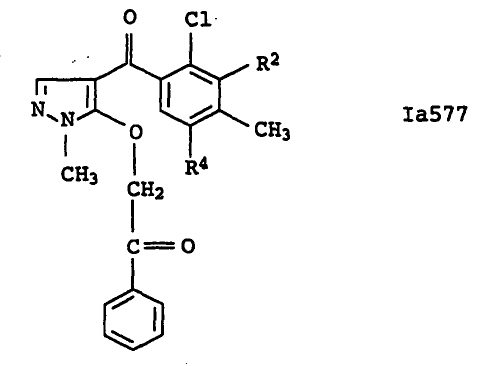
- Ebenso insbesondere außerordentlich bevorzugt sind die Verbindungen Ia578; insbesondere die Verbindungen Ia578.1-Ia578.164, die sich von den Verbindungen Ia1.1-Ia1.164 dadurch unterscheiden, daß R³ für Methyl, R⁶ für Ethyl und R⁷ für Phenylcarbonylmethyl stehen: 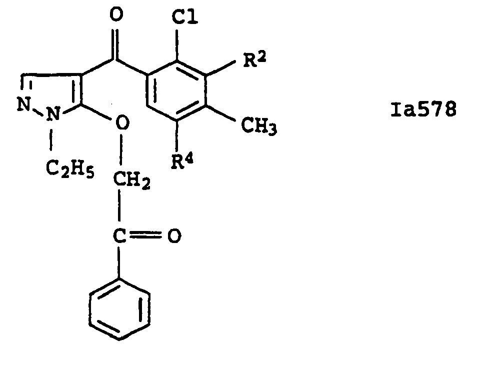
- Ebenso insbesondere außerordentlich bevorzugt sind die Verbindungen Ia579; insbesondere die Verbindungen Ia579.1-Ia579.164, die sich von den Verbindungen Ia1.1-Ia1.164 dadurch unterscheiden, daß R³ für Methyl und R⁷ für Benzyl stehen: 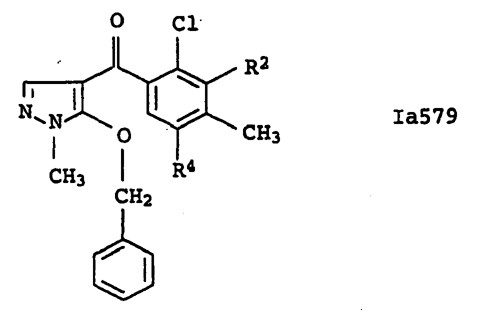
- Ebenso insbesondere außerordentlich bevorzugt sind die Verbindungen Ia580; insbesondere die Verbindungen Ia580.1-Ia580.164, die sich von den Verbindungen Ia1.1-Ia1.164 dadurch unterscheiden, daß R³ für Methyl, R⁶ für Ethyl und R⁷ für Benzyl stehen: 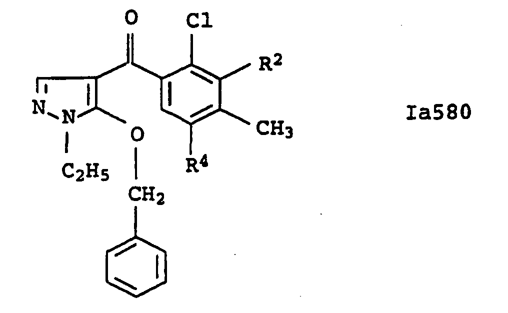
- Ebenso insbesondere außerordentlich bevorzugt sind die Verbindungen Ia581; insbesondere die Verbindungen Ia581.1-Ia581.164, die sich von den Verbindungen Ia1.1-Ia1.164 dadurch unterscheiden, daß R¹ und R⁴ für Methyl und R⁷ für Ethyl stehen: 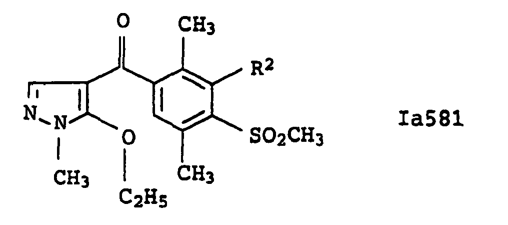
- Ebenso insbesondere außerordentlich bevorzugt sind die Verbindungen Ia582; insbesondere die Verbindungen Ia582.1-Ia582.164, die-sich von den Verbindungen Ia1.1-Ia1.164 dadurch unterscheiden, daß R¹ und R⁴ für Methyl, R⁶ und R⁷ für Ethyl stehen: 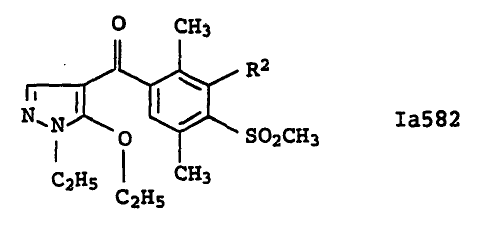
- Ebenso insbesondere außerordentlich bevorzugt sind die Verbindungen Ia583; insbesondere die Verbindungen Ia583.1-Ia583.164, die sich von den Verbindungen Ia1.1-Ia1.164 dadurch unterscheiden, daß R¹ und R⁴ für Methyl und R⁷ für Methylcarbonyl stehen: 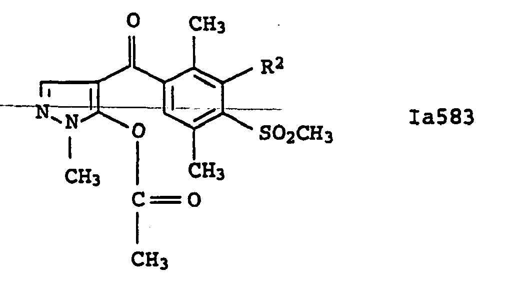
- Ebenso insbesondere außerordentlich bevorzugt sind die Verbindungen Ia584; insbesondere die Verbindungen Ia584.1-Ia584.164, die sich von den Verbindungen Ia1.1-Ia1.164 dadurch unterscheiden, daß R¹ und R⁴ für Methyl, R⁶ für Ethyl und R⁷ für Methylcarbonyl stehen: 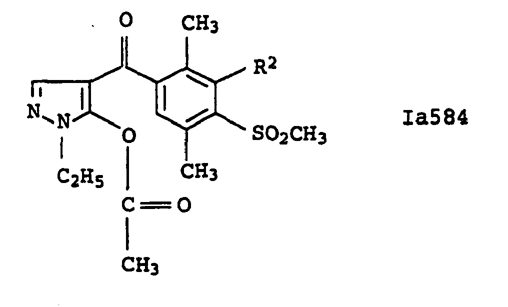
- Ebenso insbesondere außerordentlich bevorzugt sind die Verbindungen Ia585; insbesondere die Verbindungen Ia585.1-Ia585.164, die-sich von den Verbindungen Ia1.1-Ia1.164 dadurch unterscheiden, daß R¹ und R⁴ für Methyl und R⁷ für Dimethylaminocarbonyl stehen: 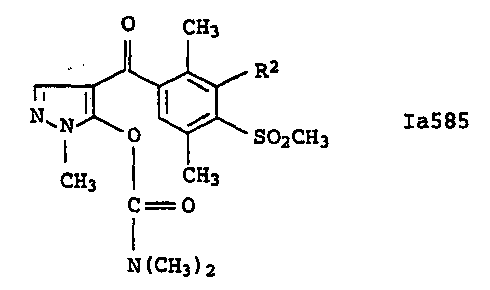
- Ebenso insbesondere außerordentlich bevorzugt sind die Verbindungen Ia586; insbesondere die Verbindungen Ia586.1-Ia586.164, die sich von den Verbindungen Ia1.1-Ia1.164 dadurch unterscheiden, daß R¹ und R⁴ für Methyl, R⁶ für Ethyl und R⁷ für Dimethylaminocarbonyl stehen: 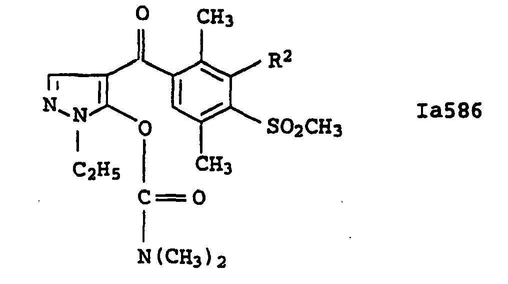
- Ebenso insbesondere außerordentlich bevorzugt sind die Verbindungen Ia587; insbesondere die Verbindungen Ia587.1-Ia587.164, die-sich von den Verbindungen Ia1.1-Ia1.164 dadurch unterscheiden, daß R¹ und R⁴ für Methyl und R⁷ für Phenylcarbonylmethyl stehen: 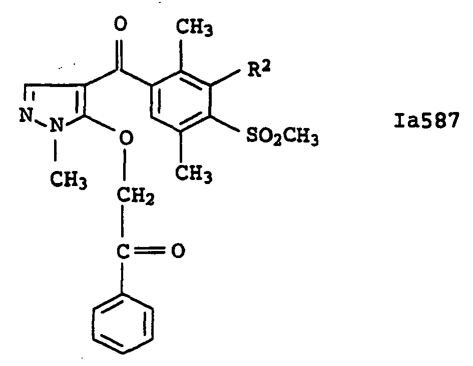
- Ebenso insbesondere außerordentlich bevorzugt sind die Verbindungen Ia588; insbesondere die Verbindungen Ia588.1-Ia588.164, die sich von den Verbindungen Ia1.1-Ia1.164 dadurch unterscheiden, daß R¹ und R⁴ für Methyl, R⁶ für Ethyl und R⁷ für Phenylcarbonylmethyl stehen: 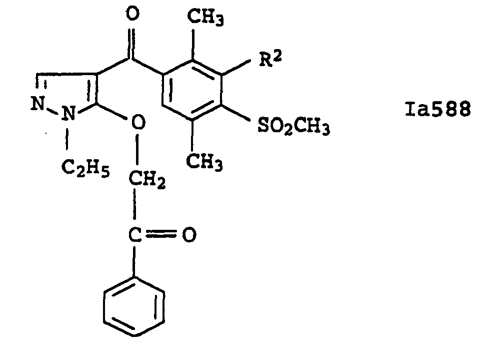
- Ebenso insbesondere außerordentlich bevorzugt sind die Verbindungen Ia589; insbesondere die Verbindungen Ia589.1-Ia589.164, die-sich von den Verbindungen Ia1.1-Ia1.164 dadurch unterscheiden, daß R¹ und R⁴ für Methyl und R⁷ für Benzyl stehen: 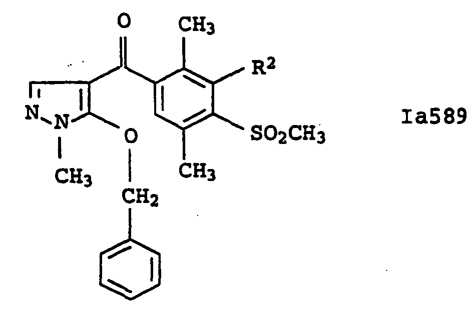
- Ebenso insbesondere außerordentlich bevorzugt sind die Verbindungen Ia590; insbesondere die Verbindungen Ia590.1-Ia590.164, die sich von den Verbindungen Ia1.1-Ia1.164 dadurch unterscheiden, daß R¹ und R⁴ für Methyl, R⁶ für Ethyl und R⁷ für Benzyl stehen: 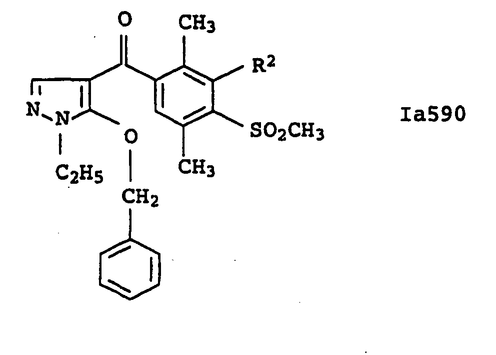
- Ebenso insbesondere außerordentlich bevorzugt sind die Verbindungen Ia591; insbesondere die Verbindungen Ia591.1-Ia591.164, die-sich von den Verbindungen Ia1.1-Ia1.164 dadurch unterscheiden, daß R³ für Trifluormethyl und R⁷ für Ethyl stehen: 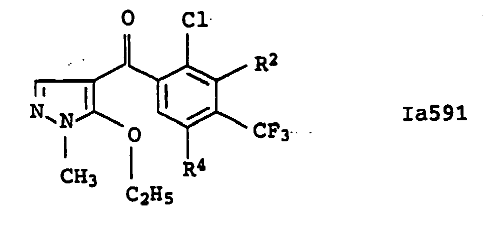
- Ebenso insbesondere außerordentlich bevorzugt sind die Verbindungen Ia592; insbesondere die Verbindungen Ia592.1-Ia592.164, die sich von den Verbindungen Ia1.1-Ia1.164 dadurch unterscheiden, daß R³ für Trifluormethyl, R⁶ und R⁷ für Ethyl stehen: 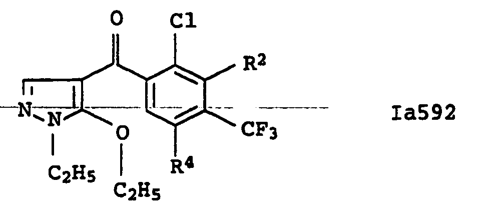
- Ebenso insbesondere außerordentlich bevorzugt sind die Verbindungen Ia593; insbesondere die Verbindungen Ia593.1-Ia593.164, die sich von den Verbindungen Ia1.1-Ia1.164 dadurch unterscheiden, daß R³ für Trifluormethyl und R⁷ für Methylcarbonyl stehen: 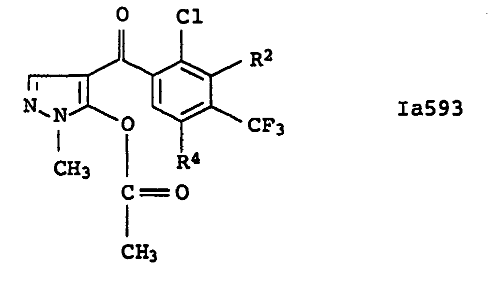
- Ebenso insbesondere außerordentlich bevorzugt sind die Verbindungen Ia594; insbesondere die Verbindungen Ia594.1-Ia594.164, die-sich von den Verbindungen Ia1.1-Ia1.164 dadurch unterscheiden, daß R³ für Trifluormethyl, R⁶ für Ethyl und R⁷ für Methylcarbonyl stehen: 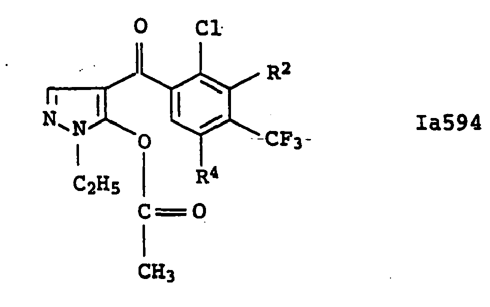
- Ebenso insbesondere außerordentlich bevorzugt sind die Verbindungen Ia595; insbesondere die Verbindungen 1a595.1-Ia595.164, die sich von den Verbindungen Ia1.1-Ia1.164 dadurch unterscheiden, daß R³ für Trifluormethyl und R⁷ für Dimethylaminocarbonyl stehen: 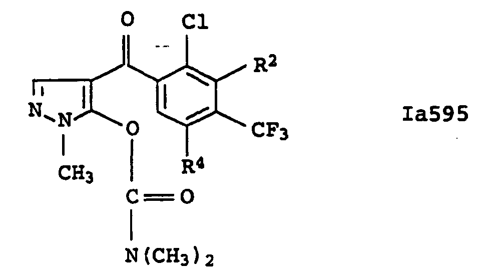
- Ebenso insbesondere außerordentlich bevorzugt sind die Verbindungen Ia596; insbesondere die Verbindungen Ia596.1-Ia596.164, die sich von den Verbindungen Ia1.1-Ia1.164 dadurch unterscheiden, daß R³ für Trifluormethyl, R⁶ für Ethyl und R⁷ für Dimethylaminocarbonyl stehen: 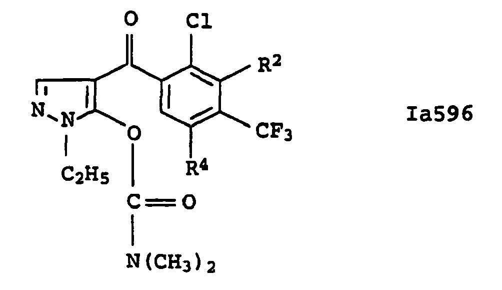
- Ebenso insbesondere außerordentlich bevorzugt sind die Verbindungen Ia597; insbesondere die Verbindungen Ia597.1-Ia597.164, die-sich von den Verbindungen Ia1.1-Ia1.164 dadurch unterscheiden, daß R³ für Trifluormethyl und R⁷ für Phenylcarbonylmethyl stehen: 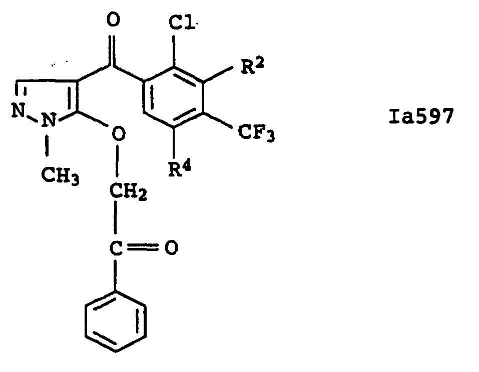
- Ebenso insbesondere außerordentlich bevorzugt sind die Verbindungen Ia598; insbesondere die Verbindungen Ia598.1-Ia598.164, die sich von den Verbindungen Ia1.1-Ia1.164 dadurch unterscheiden, daß R³ für Trifluormethyl, R⁶ für Ethyl und R⁷ für Phenylcarbonylmethyl stehen: 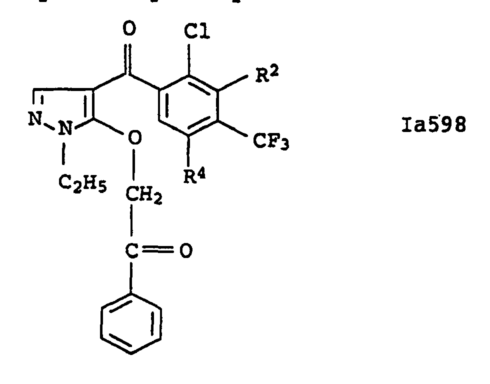
- Ebenso insbesondere außerordentlich bevorzugt sind die Verbindungen Ia599; insbesondere die Verbindungen Ia599.1-Ia599.164, die-sich von den Verbindungen Ia1.1-Ia1.164 dadurch unterscheiden, daß R³ für Trifluormethyl und R⁷ für Benzyl stehen: 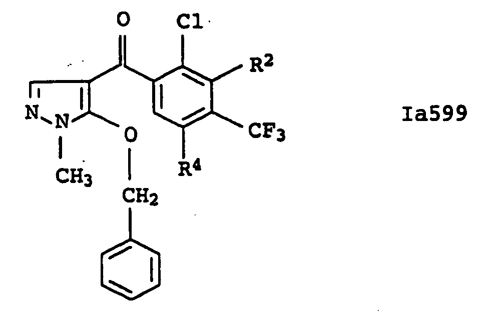
- Ebenso insbesondere außerordentlich bevorzugt sind die Verbindungen Ia600; insbesondere die Verbindungen Ia600.1-Ia600.164, die sich von den Verbindungen Ia1.1-Ia1.164 dadurch unterscheiden, daß R³ für Trifluormethyl, R⁶ für Ethyl und R⁷ für Benzyl stehen: 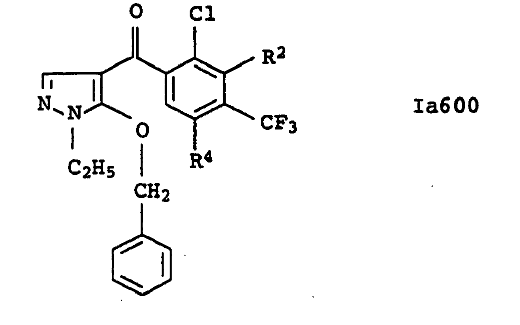
- Ebenso insbesondere außerordentlich bevorzugt sind die Verbindungen Ia601; insbesondere die Verbindungen Ia601.1-Ia601.164, die sich von den Verbindungen Ia1.1-Ia1.164 dadurch unterscheiden, daß R¹ für Nitro und R⁷ für Ethyl stehen: 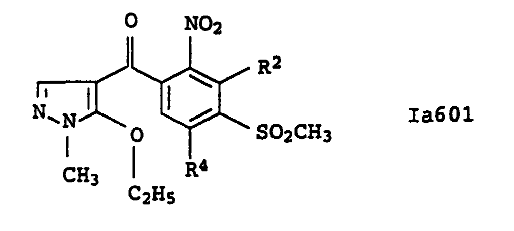
- Ebenso insbesondere außerordentlich bevorzugt sind die Verbindungen Ia602; insbesondere die Verbindungen Ia602.1-Ia602.164, die sich von den Verbindungen Ia1.1-Ia1.164 dadurch unterscheiden, daß R¹ für Nitro, R⁶ und R⁷ für Ethyl stehen: 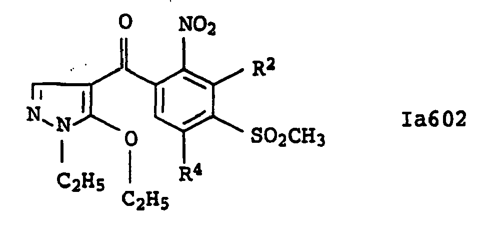
- Ebenso insbesondere außerordentlich bevorzugt sind die Verbindungen Ia603; insbesondere die Verbindungen Ia603.1-Ia603.164, die sich von den Verbindungen Ia1.1-Ia1.164 dadurch unterscheiden, daß R¹ für Nitro und R⁷ für Methylcarbonyl stehen: 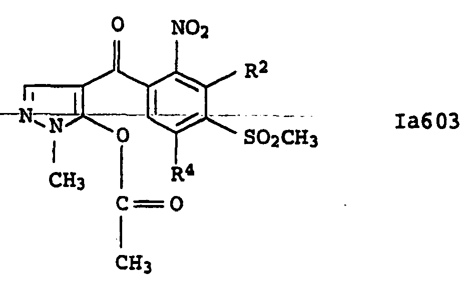
- Ebenso insbesondere außerordentlich bevorzugt sind die Verbindungen Ia604; insbesondere die Verbindungen Ia604.1-Ia604.164, die sich von den Verbindungen Ia1.1-Ia1.164 dadurch unterscheiden, daß R¹ für Nitro, R⁶ für Ethyl und R⁷ für Methylcarbonyl stehen: 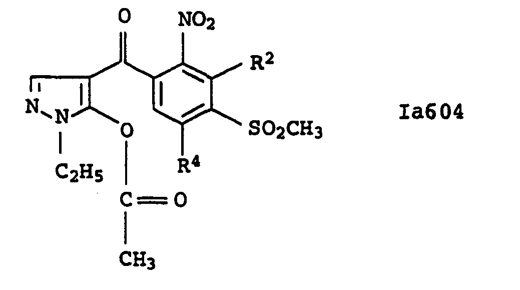
- Ebenso insbesondere außerordentlich bevorzugt sind die Verbindungen Ia605; insbesondere die Verbindungen Ia605.1-Ia605.164, die-sich von den Verbindungen Ia1.1-Ia1.164 dadurch unterscheiden, daß R¹ für Nitro und R⁷ für Dimethylaminocarbonyl stehen: 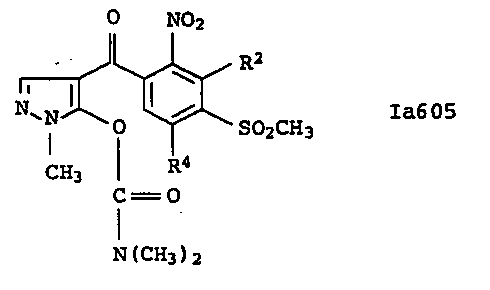
- Ebenso insbesondere außerordentlich bevorzugt sind die Verbindungen Ia606; insbesondere die Verbindungen Ia606.1-Ia606.164, die sich von den Verbindungen Ia1.1-Ia1.164 dadurch unterscheiden, daß R¹ für Nitro, R⁶ für Ethyl und R⁷ für Dimethylaminocarbonyl stehen: 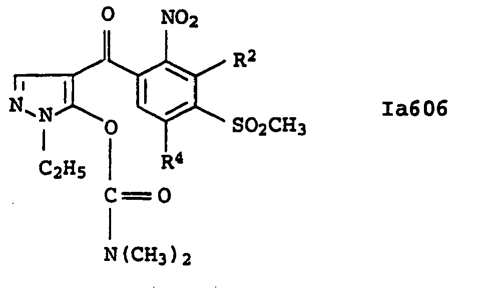
- Ebenso insbesondere außerordentlich bevorzugt sind die Verbindungen Ia607; insbesondere die Verbindungen Ia607.1-Ia607.164, die-sich von den Verbindungen Ia1.1-Ia1.164 dadurch unterscheiden, daß R¹ für Nitro und R⁷ für Phenylcarbonylmethyl stehen: 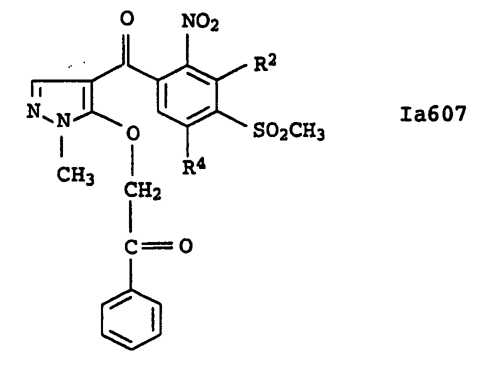
- Ebenso insbesondere außerordentlich bevorzugt sind die Verbindungen Ia608; insbesondere die Verbindungen Ia608.1-Ia608.164, die sich von den Verbindungen Ia1.1-Ia1.164 dadurch unterscheiden, daß R¹ für Nitro, R⁶ für Ethyl und R⁷ für Phenylcarbonylmethyl stehen: 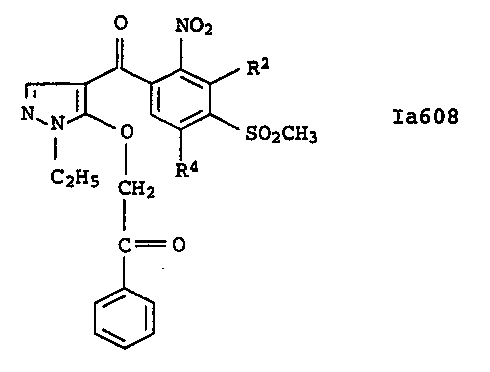
- Ebenso insbesondere außerordentlich bevorzugt sind die Verbindungen Ia609; insbesondere die Verbindungen Ia609.1-Ia609.164, die-sich von den Verbindungen Ia1.1-Ia1.164 dadurch unterscheiden, daß R¹ für Nitro und R⁷ für Benzyl stehen: 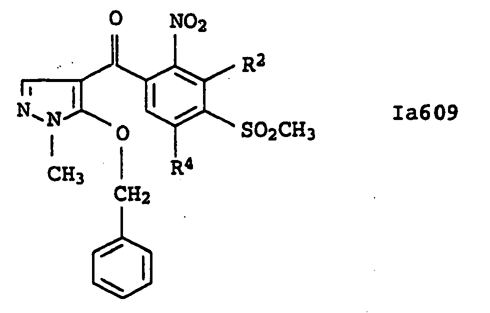
- Ebenso insbesondere außerordentlich bevorzugt sind die Verbindungen Ia610; insbesondere die Verbindungen Ia610.1-Ia610.164, die sich von den Verbindungen Ia1.1-Ia1.164 dadurch unterscheiden, daß R¹ für Nitro, R⁶ für Ethyl und R⁷ für Benzyl stehen: 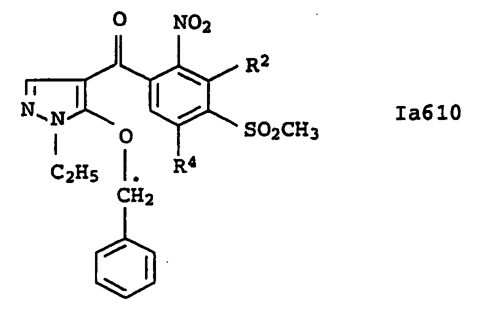
- Ebenso insbesondere außerordentlich bevorzugt sind die Verbindungen Ia611; insbesondere die Verbindungen Ia611.1-1a611.164, die sich von den Verbindungen Ia1.1-Ia1.164 dadurch unterscheiden, daß R¹ für Nitro, R³ für Chlor und R⁷ für Ethyl stehen: 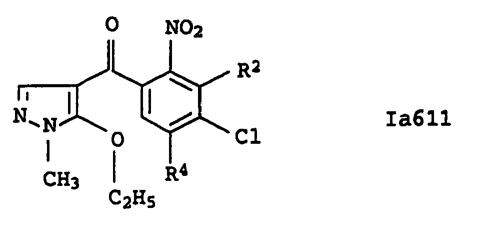
- Ebenso insbesondere außerordentlich bevorzugt sind die Verbindungen Ia612; insbesondere die Verbindungen Ia612.1-Ia612.164, die sich von den Verbindungen Ia1.1-Ia1.164 dadurch unterscheiden, daß R¹ für Nitro, R³ für Chlor, R⁶ und R⁷ für Ethyl stehen: 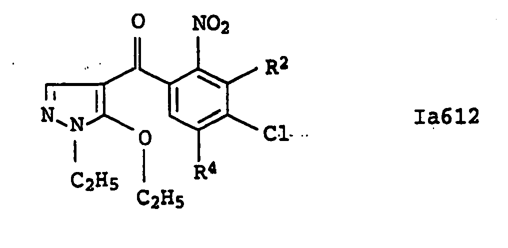
- Ebenso insbesondere außerordentlich bevorzugt sind die Verbindungen Ia613; insbesondere die Verbindungen Ia613.1-Ia613.164, die sich von den Verbindungen Ia1.1-Ia1.164 dadurch unterscheiden, daß R¹ für Nitro, R³ für Chlor und R⁷ für Methylcarbonyl stehen: 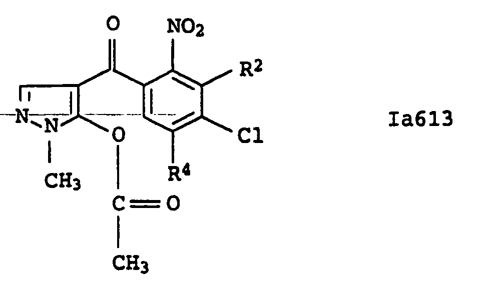
- Ebenso insbesondere außerordentlich bevorzugt sind die Verbindungen Ia614; insbesondere die Verbindungen Ia614.1-Ia614.164, die sich von den Verbindungen Ia1.1-Ia1.164 dadurch unterscheiden, daß R¹ für Nitro, _{R}³ für Chlor, R⁶ für Ethyl und R⁷ für Methylcarbonyl stehen: 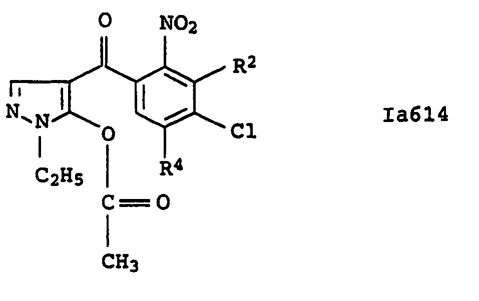
- Ebenso insbesondere außerordentlich bevorzugt sind die Verbindungen Ia615; insbesondere die Verbindungen Ia615.1-Ia615.164, die-sich von den Verbindungen Ia1.1-Ia1.164 dadurch unterscheiden, daß R¹ für Nitro, R³ für Chlor und R⁷ für Dimethylaminocarbonyl stehen: 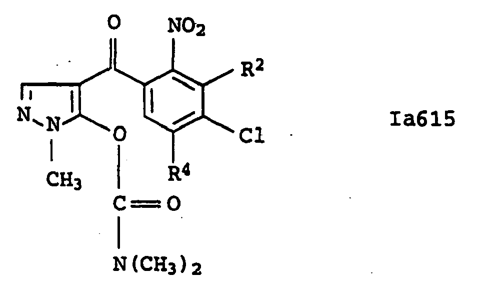
- Ebenso insbesondere außerordentlich bevorzugt sind die Verbindungen Ia616; insbesondere die Verbindungen Ia616.1-Ia616.164, die sich von den Verbindungen Ia1.1-Ia1.164 dadurch unterscheiden, daß R¹ für Nitro, R³ für Chlor, R⁶ für Ethyl und R⁷ für Dimethylaminocarbonyl stehen: 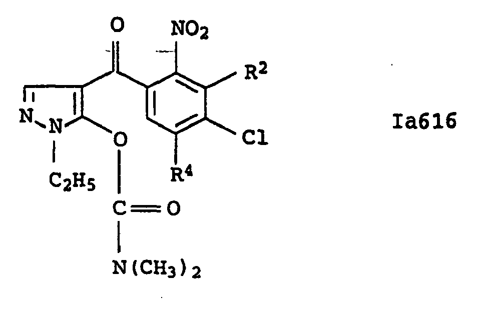
- Ebenso insbesondere außerordentlich bevorzugt sind die Verbindungen Ia617; insbesondere die Verbindungen Ia617.1-Ia617.164, die sich von den Verbindungen Ia1.1-Ia1.164 dadurch unterscheiden, daß R¹ für Nitro, R³ für Chlor und R⁷ für Phenylcarbonylmethyl stehen: 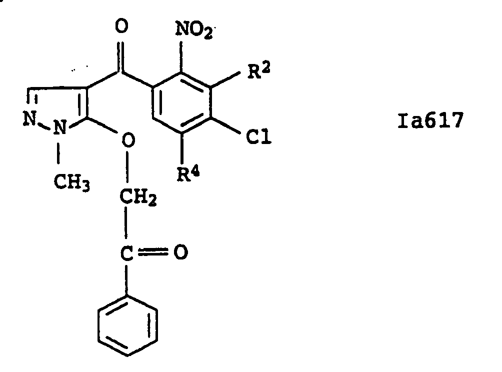
- Ebenso insbesondere außerordentlich bevorzugt sind die Verbindungen Ia618; insbesondere die Verbindungen Ia618.1-Ia618.164, die sich von den Verbindungen Ia1.1-Ia1.164 dadurch unterscheiden, daß R¹ für Nitro, R³ für Chlor, R⁶ für Ethyl und R⁷ für Phenylcarbonylmethyl stehen: 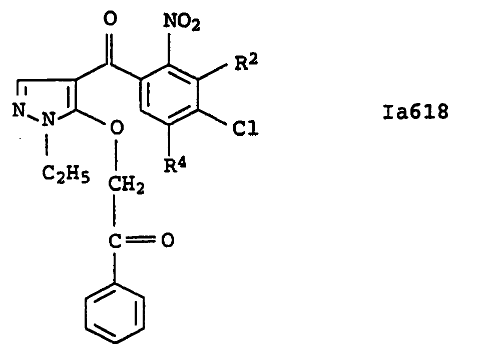
- Ebenso insbesondere außerordentlich bevorzugt sind die Verbindungen Ia619; insbesondere die Verbindungen Ia619.1-Ia619.164, die sich von den Verbindungen Ia1.1-Ia1.164 dadurch unterscheiden, daß R¹ für Nitro, R³ für Chlor und R⁷ für Benzyl stehen: 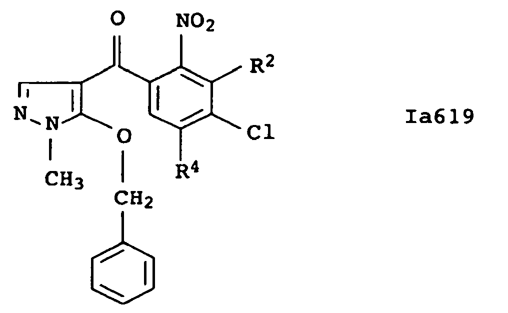
- Ebenso insbesondere außerordentlich bevorzugt sind die Verbindungen Ia620; insbesondere die Verbindungen Ia620.1-Ia620.164, die sich von den Verbindungen Ia1.1-Ia1.164 dadurch unterscheiden, daß R¹ für Nitro, R³ für Chlor, R⁶ für Ethyl und R⁷ für Benzyl stehen:

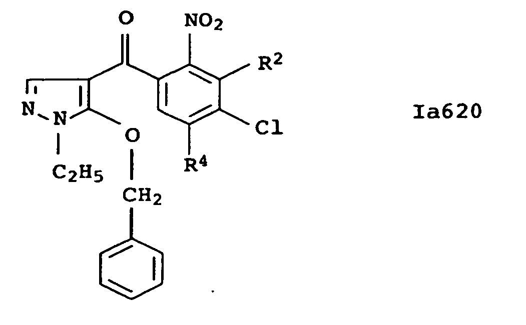

Weiterhin insbesondere bevorzugt sind die 4-(3-Heterocyclyl-1-benzoyl)pyrazole der Formel I, in der die Variablen folgende Bedeutungen haben:
- R¹: Halogen oder C₁-C₆-Alkyl;
insbesondere Chlor oder Methyl;
- R²: gegebenenfalls substituiertes 4,5-Dihydroisoxazol-3-yl, 4,5-Dihydrothiazol-2-yl, 1,3-Dithiolan-2-yl, Thiazol-2-yl, Isoxazol-3-yl, Isoxazol-4-yl, Isoxazol-5-yl;
insbesondere gegebenenfalls substituiertes 4,5-Dihydroisoxazol-3-yl, 4,5-Dihydrothiazol-2-yl, 1,3-Dithiolan-2-yl;
- R³: Nitro, Halogen oder C₁-C₆-Alkylsulfonyl; insbesondere Chlor oder C₁-C₄-Alkylsulfonyl;
- R⁴: Wasserstoff;
- R⁶: C₁-C₆-Alkyl;
insbesondere Methyl, Ethyl, Propyl, 2-Methylpropyl oder Butyl;
- R⁷: C₁-C₆-Alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, N-(C₁-C₆-Alkoxy-N-(C₁-C₆-alkyl)aminocarbonyl,
wobei die genannten Alkyl- und Alkoxyreste partiell oder vollständig halogeniert sein können und/oder eine bis drei der folgenden Gruppen tragen können:
Cyano, C₁-C₄-Alkoxycarbonyl
Phenyl-C₁-C₆-alkyl, Phenylcarbonyl-C₂-C₆-alkyl, Phenyl-C₂-C₆-alkenylcarbonyl oder Phenylcarbonyl, wobei der Phenylrest der 4 letztgenannten Substituenten partiell oder vollständig halogeniert sein kann und/oder einen bis drei der folgenden Reste tragen kann:
   Nitro, Cyano, C₁-C₄-Alkyl, C₁-C₄-Halogenalkyl, C₁-C₄-Alkoxy oder C₁-C₄-Halogenalkoxy;
- R⁸: Wasserstoff;
bedeuten;
sowie deren landwirtschaftlich brauchbaren Salze.

Die 4-(3-Heterocyclyl-1-benzoyl)pyrazole der Formel I sind auf verschiedene Art und Weise erhältlich, beispielsweise nach folgendem verfahren.

Umsetzung von 4-(3-Heterocyclyl-1-benzoyl)pyrazolen der Formel III mit einer Verbindung der Formel IV (Schema 1):

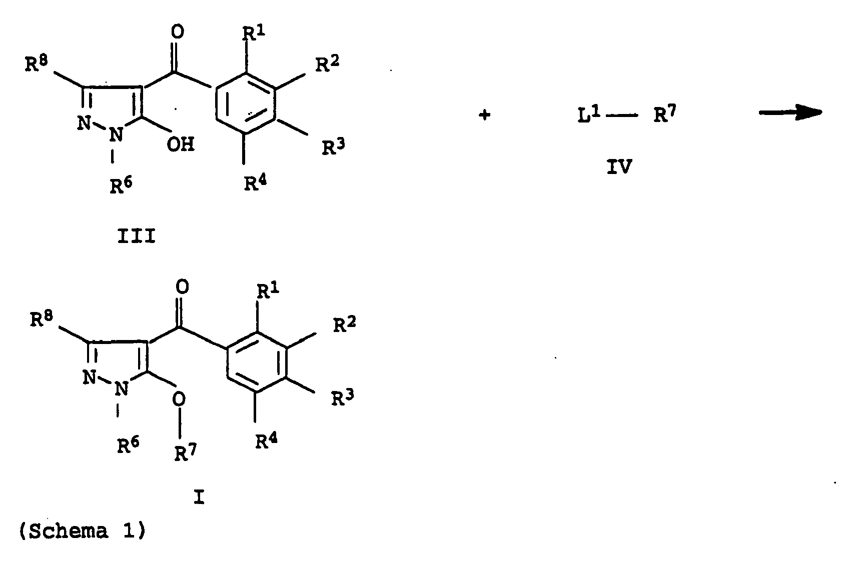

L¹ steht für eine nucleophil verdrängbare Abgangsgruppe, wie Halogen, z.B. Brom oder Chlor, Hetaryl, z.B. Imidazolyl oder Pyridyl, Carboxylat, z.B. Acetat oder Trifluoracetat oder Sulfonat, z.B. Mesylat oder Triflat etc.

Die Verbindungen der Formel IV können direkt eingesetzt werden, wie z.B. im Fall der Alkylhalogenide, Carbonsäurehalogenide, Sulfonsäurehalogenide, Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Base werden dabei zweckmäßigerweise in äquimolaren Mengen eingesetzt. Ein Überschuß der Base z.B. 1,5 bis 3 Moläquivalente, bezogen auf III, kann unter Umständen vorteilhaft sein.

Als Basen eignen sich tertiäre Alkylamine, wie Triethylamin, aromatische Amine, wie Pyridin, Alkalimetallcarbonate, z.B. Natriumcarbonat oder Kaliumcarbonat oder Alkalimetallhydride, z.B. Natriumhydrid. Bevorzugt verwendet werden Triethylamin oder Pyridin.

Als Lösungsmittel kommen z.B. chlorierte Kohlenwasserstoffe, wie Methylenchlorid oder 1,2-Dichlorethan, aromatische Kohlenwasserstoffe, z.B. Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die Benzoylpyrazole der Formel III sind bekannt oder können nach an sich bekannten Verfahren hergestellt werden (z.B. WO 96/26206 oder ältere deutsche Patentanmeldung DE-A 1970 1446)). Beispielsweise durch Umsetzung von Pyrazolen der Formel V mit einer aktivierten Benzoesäure VIα oder einer Benzoesäure VIß, die vorzugsweise in situ aktiviert wird, zu dem Acylierungsprodukt VII und anschließende Umlagerung.

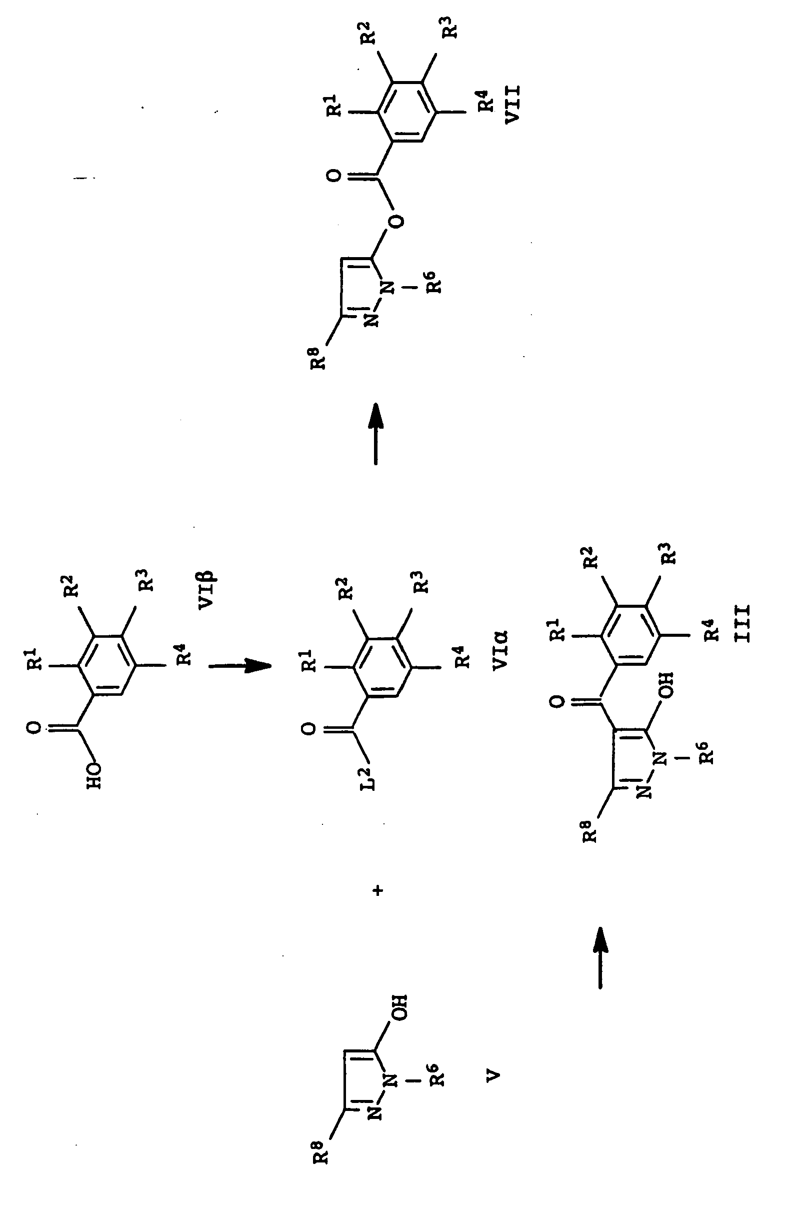

L² steht für eine nucleophil verdrängbare Abgangsgruppe, wie Halogen z.B Brom oder Chlor, Hetaryl, z.B. Imidazolyl oder Pyridyl, Carboxylat, z.B. Acetat oder Trifluoracetat etc.

Die aktivierte Benzoesäure VIα kann direkt eingesetzt werden, wie im Fall der Benzoylhalogenide oder in situ erzeugt werden, z.B. mit Dicyclohexylcarbodiimid, Triphenylphosphin/Azodicarbonsäureester, 2-Pyridindisulfid/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf V, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid oder 1,2-Dichlorethan, aromatische Kohlenwasserstoffe, wie Toluol, Xylol oder Chlorbenzol, Ether, wie Diethylether, Methyl-tert.-butylether, Tetrahydrofuran oder Dioxan, polare aprotische Lösungsmittel, wie Acetonitril, Dimethylformamid oder Dimethylsulfoxid oder Ester wie Essigsäureethylester oder Gemische hiervon verwendet werden.

Werden Benzoylhalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels kann der rohe Ester ohne weitere Reinigung zur Umlagerung eingesetzt werden.

Die Umlagerung der Ester zu den Verbindungen der Formel I erfolgt zweckmäßigerweise bei Temperaturen von 20 bis 100°C in einem Lösungsmittel und in Gegenwart einer Base sowie gegebenenfalls mit Hilfe einer Cyanoverbindung als Katalysator.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, aromatische Amine wie Pyridin oder Alkalicarbonate, wie Natriumcarbonat oder Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonat verwendet, vorzugsweise in doppelt äquimolaren Verhältnis in Bezug auf den Ester.

Als Cyanoverbindungen kommen anorganische Cyanide, wie Natriumcyanid oder Kaliumcyanid und organische Cyanoverbindungen, wie Acetoncyanhydrin oder Trimethylsilylcyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid oder Essigsäureethylester extrahiert. Der organische Extrakt kann mit 5-10%iger Alkalicarbonatlösung, z.B. Natriumcarbonat- oder Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt. (Beispiele für die Darstellung von Estern von Hydroxypyrazolen und für die Umlagerung der Ester sind z.B. in EP-A 282 944 und US 4 643 757 genannt).

Die Benzoylhalogenide der Formel VIα' (mit L²' = Cl, Br) können auf an sich bekannte Art und Weise durch Umsetzung der Benzoesäuren der Formel VIß mit Halogenierungsreagentien wie Thionylchlorid, Thionylbromid, Phosgen, Diphosgen, Triphosgen, Oxalylchlorid, Oxalylbromid hergestellt werden.

Die Benzoesäuren der Formel VIß können in bekannter Weise durch saure oder basische Hydrolyse aus den entsprechenden Estern der Formel VIγ (L³ = C₁-C₆-Alkoxy) hergestellt werden.

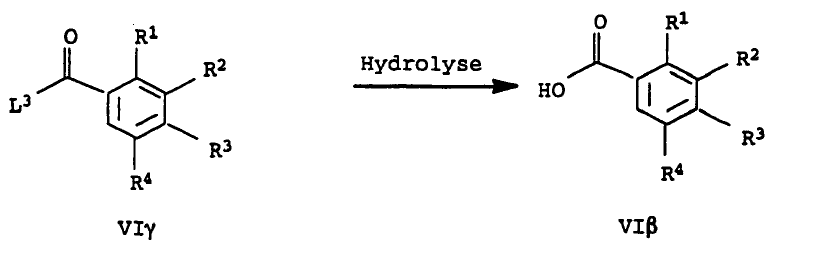

Ebenso können die Benzoesäuren der Formel VIβ durch Umsetzung von entsprechenden Brom- oder Iod-substituierten Verbindungen der Formel VIII, in Gegenwart eines Palladium-, Nickel-, Cobalt- oder Rhodium-Übergangsmetallkatalysators und einer Base mit Kohlenmonoxid und Wasser unter erhöhtem Druck erhalten werden.

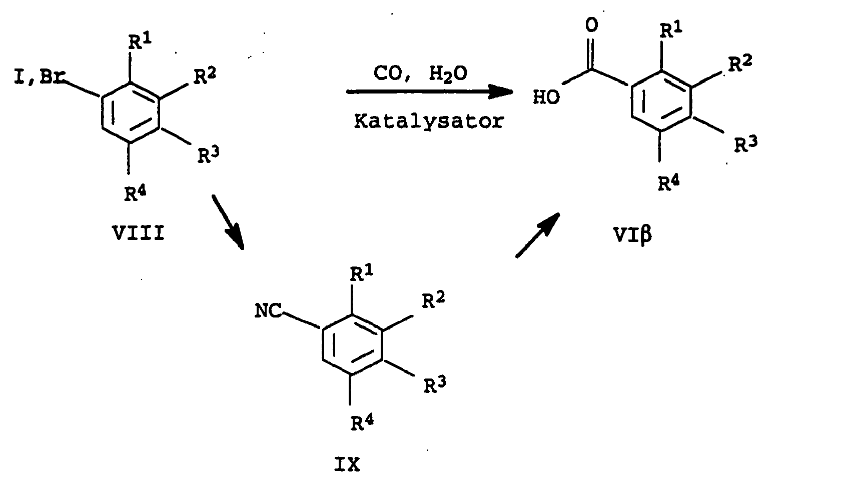

Weiterhin ist es möglich durch Rosenmund-von Braun-Reaktion Verbindungen der Formel VIII in die entsprechenden Nitrile der Formel IX zu überführen (vgl. z.B. Org. Synth. Bd III, 212 (1955)) und diese durch nachfolgende Verseifung in die Verbindungen der Formel VIß umzuwandeln.

Die Ester der Formel VIγ können durch Umsetzung von Arylhalogenverbindungen oder Arylsulfonaten der Formel X, wobei L⁴ für eine Ausgangsgruppe wie Brom, Iod, Triflat, Fluorsulfonyloxy etc. steht, mit Heterocyclyl-stannaten (Stille-Kupplungen), Heterocyclyl-Borverbindungen (Suzuki-Kupplungen) oder Heterocyclyl-Zinkverbindungen (Negishi-Reaktion) XI, wobei M entsprechend für Sn(C₁-C₄-Alkyl)₃, B(OH)₂, ZnHal (mit Hal = Chlor, Brom) etc. steht, auf an sich bekannte Art und Weise (vgl. z.B. Tetrahedron Lett. 27, 5269 (1986)) in Gegenwart eines Palladium- oder Nickel-Übergangsmetallkatalysator und gegebenenfalls einer Base erhalten werden. 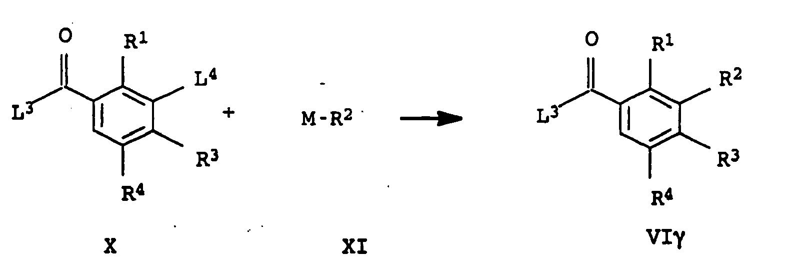 -(mit L⁴ = Br, J,
-OSO₂CF₃,
-OS0₂F)
mit M = Sn(C₁-C₄-Alkyl)₃,
B(OH)₂, ZnHal,
wobei Hal für Cl
oder Br steht)

Ebenso ist es möglich, Ester der Formel VIγ durch Aufbau des in 3-Position gebundenen Heterocycluses zu erhalten.

Beispielsweise können aus Amidoximen der Formel XII durch Kondensation mit Aldehyden oder Ketonen 1,2,4-Oxadiazolin-3-yl-Derivate hergestellt werden (vgl. z.B. Arch. Phar. 326, 383-389 (1993)).

Die Thioamide der Formel XIII sind geeignete Vorprodukte für 2-Thiazolinyl-Derivate (vgl. z.B. Tetrahedron 42, 1449-1460 (1986)). Es ist aber auch möglich, daraus 2-Thiazolyl- oder 5,6-Dihydro-4H-1,3-thiazin-2-yl-Derivate aufzubauen (vgl. z.B. Houben-Weyl, Methoden der organischen Chemie, 4. Auflage, Bd. E5, S. 1268 ff (1985)). Ebenso können daraus 1,2,4-Thiadiazol-5-yl-Derivate (vgl. z.B. J. Org. Chem. 45, 3750-3753 (1980) oder 1,3,4-Thiadiazol-2-yl-Derivate (J. Chem. Soc. Perkin Trans. I, 1987-1991 (1992)) hergestellt werden.

Aus den Carbonsäuren der Formel XIV sind 2-Oxazolinyl-, 2-Thiazolinyl- und 2-Imidazolinyl-Derivate zugänglich (vgl. z.B. Tetrahedron Let. 22, 4471-4474 (1981)).

Nach literaturbekannten Verfahren können aus Carbonsäurehalogeniden der Formel XV, wobei Hal für Halogen steht, insbesondere aus Carbonsäurechloriden, 1,3-Thiazol-5(4H)-thion-2-yl- (vgl. z.B. Helv. Chim. Acta, 69, 374-388 (1986)) und 5-Oxo-2-imidazolin-2-yl-Derivate (vgl. z.B. Heterocycles 29, 1185-1189 (1989)) hergestellt werden.

Auf an sich bekannte Art und Weise können aus den Carbonsäuren der Formel XIV bzw. den Carbonsäurehalogeniden der Formel XV 2-Oxazolyl-, 1,2,4-oxadiazol-5-yl- und 1,3,4-Oxadiazol-2-yl-Derivate (vgl. z.B. J. Heterocyclic Chem. 28, 17-28 (1991)) oder 2-Pyrrolyl-Derivate (vgl. z.B. Hetercycles 26, 3141-3151 (1987)) hergestellt werden.

Die Umwandlung von Oximen der Formel XVI in 4,5-Dihydro-isoxazol-3-yl- bzw. in Isoxazol-3-yl-Derivate kann in an sich bekannter Weise über die Zwischenstufe der Hydroxamsäurechloride erfolgen. Aus letzteren werden in situ Nitriloxide erzeugt, die mit Alkenen bzw. Alkinen zu den gewünschten Produkten abreagieren (vgl. z.B. Chem. Ber. 106, 3258-3274 (1973)). 1,3-Dipolare Cycloadditionen von Chlorsulfonylisocyanat an Nitriloxide liefern 1,2,4-Oxadiazolin-5-on-3-yl-Derivate (vgl. z.B. Heterocycles 27, 683-685 (1988)).

Die Aldehyde der Formel XVIII können über die Zwischenstufe der Semicarbazone in 2,4-Dihydro-1,2,4-triazol-3-on-5-yl-Derivate umgewandelt werden (vgl. z.B. J. Heterocyclic Chem., 23, 881-883 (1986)).

2-Imidazolinyl-Derivate sind aus Benzonitrilen der Formel XIX nach bekannten Methoden (vgl.z.B. J. Org. Chem. 52, 1017-1021 (1987)) herstellbar.

Ebenfalls aus den Benzonitrilen der Formel XIX sind nach bekannten Verfahren 1,2,4-Triazol-3-yl-Derivate herstellbar (vgl. z.B. J. Chem. Soc. 3461-3464 (1954)). Mittels 1,3-dipolarer Cycloaddition von Diazoalkanen, Nitriliminen bzw. Nitriloxiden mit Arylalkenen der Formel XX (wobei R* die Bedeutung eines unter den bei R² möglichen genannten Subsubstituenten hat) können 3-Pyrazolinyl- oder 4-Pyrazolinyl-Derivate bzw. 4,5-Dihydroisoxazol-4-yl oder 4,5-Dihydroisoxazol-5-yl-Derivate dargestellt werden.

Die Arylalkine XXI (wobei R* die Bedeutung eines unter den bei R² möglichen Subsubstituenten hat) können im Rahmen einer 1,3-dipolaren Cycloaddition, z.B. mit den oben genannten 1,3-Dipolen, zu Pyrazol-3-yl oder Pyrazol-4-yl, bzw. Isoxazol-4-yl oder Isoxazol-5-yl-Derivaten umgesetzt werden.

Die Aldehyde XVIII lassen sich durch Wittig-Reaktion mit Phosphonium-Salzen (Ph)₃P⁺CH₂COR*X⁻ (R* hat die Bedeutung eines unter R² genannten Subsubstituenten) auf an sich bekannte Art und Weise (J. March, "Advanced Organic Chemistry", 3. Auflage, S. 864 ff., Wiley-Interscience Publication, 1985) zu α,β-ungesättigten Ketonen XXII umsetzen. Durch Reaktion mit Hydroxylamin erhält man daraus die entsprechenden Oxime, die mittels oxidativer Cyclisierung in die 5-Isoxazolyl-Derivate übergeführt werden können (J. Am. Chem. Soc. 94 (1972) 9128).

Ebenso können die Aldehyde XVIII mit Alkoxymethylphosphoniumsalze auf an sich bekannte Weise (J. March, "Advanced Organic Chemistry", 3. Auflage, S. 864 ff., Wiley-Interscience Publication, 1985) zu den entsprechenden Endethern übergeführt werden. Spaltung dieser Enolether in Analogie zu literaturbekannten Verfahren führt den Acetaldehyd-Derivaten XXIII. Diese können durch Bromierung in α-Position zu den α-Bromacetaldehyd-Abkömmlingen umgesetzt werden (Tetrahydron Lett. 29 (1988) 5893), welche durch Cyclisierung mit Amiden, Thioamiden und Amidinen Oxazole, Thiazole und Imidazole liefern. Weiterhin können die Acetaldehyd-Derivate XXIII mit Dimethylformamidimethylacetal in entsprechende Enamine übergeführt werden, die dann mit Hydroxylaminen bzw. Hydrazinen zu Isoxazolen bzw. Pyrazolen übergeführt werden können.

Weiterhin können die Aldehyde XVIII mittels Aldolreaktion mit Ketonen in Hydroxyketon-Derivate übergeführt werden. Anschließende Oxidation führt zu 1,3-Diketonen, die mit Hydroxylamin, Hydrazinen oder Amidinen zu Isoxazolen, Pyrazolen oder Pyrimidinen übergeführt werden können.

Ebenso können die Aldehyde XVIII nach literaturgekannten Methoden (Houben-Weyl, "Methoden der organischen Chemie", 4. Auflage, Bd. E14b) in die entsprechenden Diazoverbindungen XXIV übergeführt werden. 1,3-dipolare Cycloaddition an Alkene bzw. Alkine und anschließende Isomerisierung führt zu Pyrazolinen bzw. Pyrazolen.

Die als Ausgangsverbindungen verwendeten Brom- oder Iod-substituierten Verbindungen der Formel VIII können in Analogie zu literaturbekannten Methode, z.B. durch Sandmeyer-Reaktion aus entsprechenden Anilinen erhalten werden, die ihrerseits durch Reduktion geeigneter Nitroverbindungen synthetisiert werden. Die Brom-substituierten Verbindungen der Formel VIII können außerdem durch direkte Bromierung geeigneter Edukte erhalten werden (vgl. Monatsh. Chem. 99, 815-822 (1968)).

Die Nitrile der Formel IX können wie oben beschrieben erhalten werden. Ebenso ist es möglich, diese aus entsprechenden Anilinen mittels Sandmeyer-Reaktion darzustellen.

Die Ausgangsverbindungen der Formel X sind bekannt (vgl. z.B. Coll. Czech. Chem. Commun. 40, 3009-3019 (1975)) oder können leicht durch geeignete Kombination bekannter Synthesen hergestellt werden.

Beispielsweise können die Sulfonate X (L⁴ = -OSO₂CF₃, -OSO₂F) aus den entsprechenden Phenolen, die ihrerseits bekannt sind (vgl. z.B. EP-A 195 247) oder nach bekannten Methoden hergestellt werden können, erhalten werden (vgl. z.B. Synthesis 1993, 735-762).

Die Halogenverbindungen X (L⁴ = C1, Br oder I) können beispielsweise durch Sandmeyer-Reaktion aus entsprechenden Anilinen der Formel XXV erhalten werden.

Die Amidoxime der Formel XII, die Thioamide der Formel XIII und die Carbonsäuren der Formel XIV können auf an sich bekannte Art und Weise aus den Nitrilen der Formel XIX dargestellt werden.

Weiterhin ist es möglich, die Carbonsäuren der Formel XIV aus den Aldehyden der Formel XVIII nach bekannten Verfahren herzustellen (vgl. z.B. J. March, Advanced Organic Chemistry, 3. Auflage, S. 629 ff, Wiley-Interscience Publication (1985)).

Die Carbonsäurehalogenide der Formel XV können in Analogie zu Standardverfahren aus den entsprechenden Carbonsäuren der Formel XIV erhalten werden.

Die Oxime der Formel XVI erhält man vorteilhaft dadurch, daß man in an sich bekannter Weise Aldehyde der Formel XVIII mit Hydroxylamin umsetzt (vgl. z.B. J. March, Advanced Organic Chemistry, 3. Aufl., S. 805-806, Wiley-Interscience Publication (1985)).

Die Aldehyde der Formel XVIII sind bekannt oder in Analogie zu bekannten Verfahren darstellbar. So können sie aus Methylverbindungen der Formel XXVI durch Bromierung, beispielsweise mit N-Bromsuccinimid oder 1,3-Dibrom-5,5-dimethylhydantoin, und anschließende Oxidation dargestellt werden (vgl. Synth. Commun. 22, 1967 - 1971 (1992)).

Die Umwandlung der Oxime der Formel XVI in Nitrile der Formel XIX kann ebenfalls nach an sich bekannten Verfahren erfolgen (vgl. z.B. J. March, Advanced Organic Chemistry, 3. Aufl., S. 931-932, Wiley-Interscience Publication (1985)).

Ausgehend von den Halogenverbindungen oder Sulfonaten der Formel X (L⁴ = Br, Cl, OSO₂CF₃, OSO₂F) lassen sich u.a. durch Heck-Reaktion mit Olefinen in Gegenwart eines Palladiumkatalysators Arylalkene der Formel XX darstellen (vgl. z.B. Heck, Palladium Reagents in Organic Synthesis, Academic Press, London 1985; Synthesis 1993, 735 - 762).

Die Arylalkine der Formel XXI können auf an sich bekannte Weise durch Umsetzung von Arylhalogenverbindungen oder Arylsulfonaten der Formel X mit substituierten Alkinen in Gegenwart eines Palladiums- oder Nickel-Übergangsmetallkatalysator hergestellt werden (z.B. Heterocycles 24, 31-32 (1986)). Alkine mit terminaler Wasserstoff-Funktion erhält man zweckmäßigerweise aus den entsprechenden Silylverbindungen (vgl. z.B. J. Org. Chem. 46, 2280-2286 (1981)).

Die als Ausgangsmaterialien verwendeten Verbindungen der Formel IV sind bekannt bzw. können nach literaturbekannten Verfahren hergestellt werden.

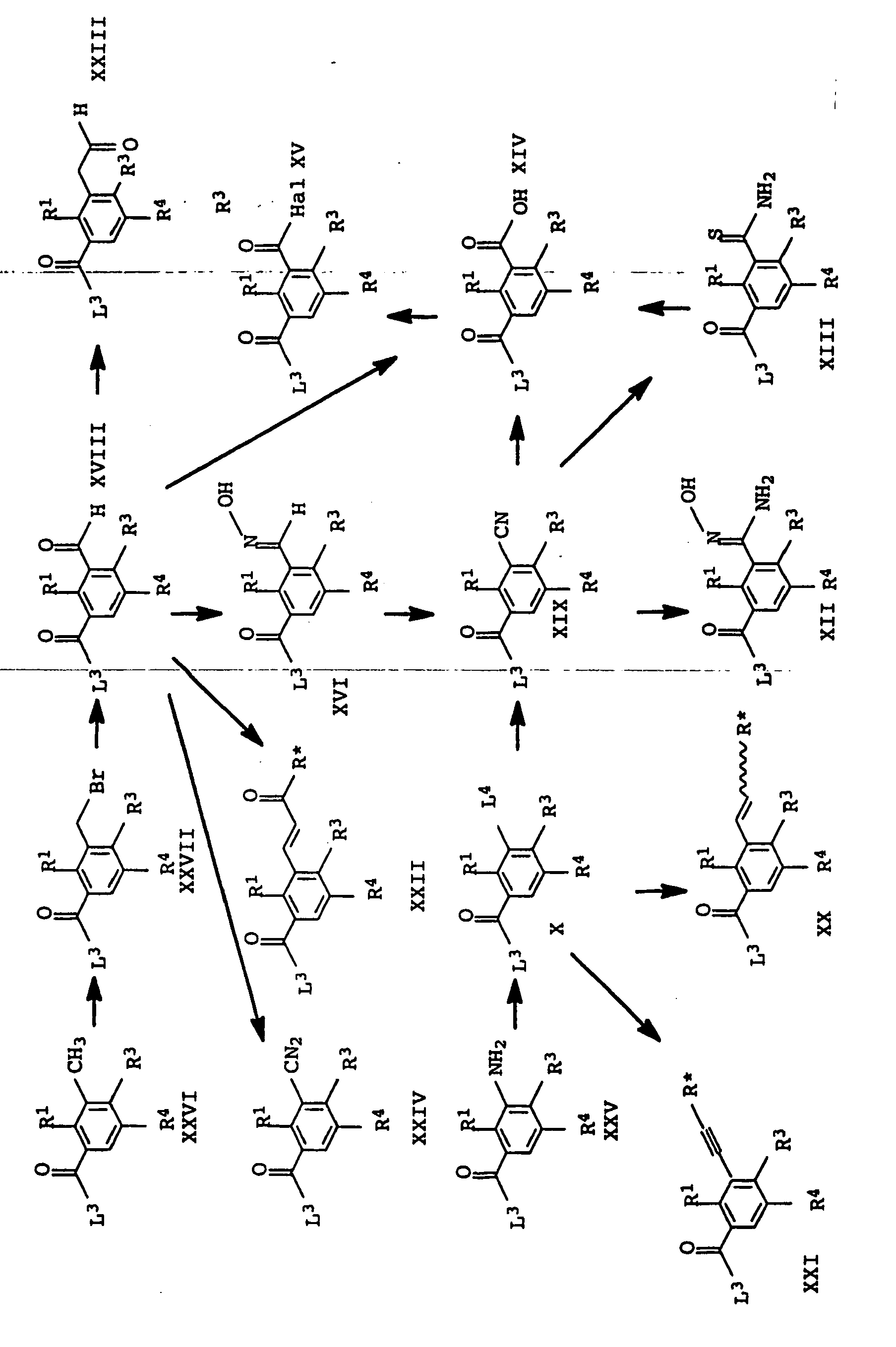

### Herstellungsbeispiele:

### 5-Benzyloxy-4-[2-chlor-3-4,5-dihydroisoxazol-3-yl)-4-methylsulfonyl-benzoyl]-1-ethyl-1H-pyrazol (Verbindung 2.45)

Zu einer Lösung von 6,00 g (15 mmol) 4-[2-Chlor-3-(4,5-dihydroisoxazol-3-yl)-4-methylsulfonyl-benzoyl]-1-ethyl-5-hydroxy-1H-pyrazol in 300 ml trockenem Dioxan wurden portionsweise 1,44 g (60 mmol) Natriumhydrid und 10,40 g (60 mmol) Benzylbromid gegeben. Nach 24 Stunden Rühren unter Rückfluß wurde das Lösungsmittel am Vakuum entfernt, der Rückstand in Dichlormethan aufgenommen und dreimal mit Wasser gewaschen. Die organische Phase wurde getrocknet, eingeengt und der Rückstand an Kieselgel chromatographiert (Eluent: n-Pentan/Essigsäureethylester). Man erhielt 1,5 g (20 % d.Th.) 5-Benzyloxy-4-[2-chlor-3-(4,5-dihydroisoxazol-3-yl)-4-methylsulfonyl-benzoyl]-1-ethyl-1H-pyrazol. (Fp.: 70-75°C)

In Tabelle 2 sind neben der voranstehenden Verbindung noch weitere 4-(3-Heterocyclyl-1-benzoyl)pyrazole der Formel I aufgeführt, die in analoger Weise hergestellt wurden oder herstellbar sind:

**Tabelle 2:**

| | | | | | | | | |
|---|---|---|---|---|---|---|---|---|
| 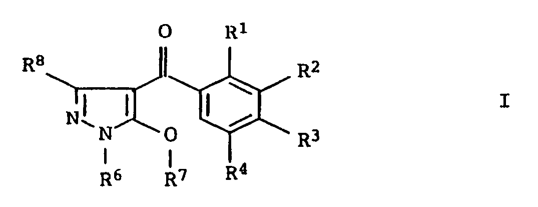 | | | | | | | | |

| Nr. | R¹ | R² | R³ | R⁴ | R⁶ | R⁷ | R⁸ | physikalische Daten Fp. [°C]; ¹H-NMR [δ in ppm] |
|---|---|---|---|---|---|---|---|---|
| 2.1 | Cl | 5,5-Dimethyl-4,5-dihydro-isoxazol-3-yl | SO₂CH₃ | H | n-C₃H₇ | C₆H₅CH₂ | H | 0,78(t); 1,50(s); 1,62(sext); 3,18(s); 3,36(s); 3,85(t); 5,51(s); 7,41(m); 7,54(s); 7,90(d); 8,14(d). |
| 2.2 | Cl | 5,5-Dimethyl-4,5-dihydro-isoxazol-3-yl | SO₂CH₃ | H | n-C₄H₉ | C₆H₅CH₂ | H | 0,80(t); 1,15(m); 1,48(s); 1,50(m); 3,15(s); 3,34(s); 3,84(t); 5,49(s); 7,42(m); 7,52(s); 7,88(d); 8,13(d). |
| 2 . 3 | Cl | 4,5-Dihydroisoxazol-3-yl | SO₂CH₃ | H | C₂H₅ | CH₃OCO | H | 68 - 75 |
| 2 . 4 | Cl | 4,5-Dihydroisoxazol-3-yl | SO₂CH₃ | H | CH₃ | C₂H₅CO | H | 65 - 70 |
| 2.5 | Cl | 4,5-Dihydroisoxazol-3-yl | SO₂CH₃ | H | C₂H₅ | C₂H₅CO | H | 1,28(t); 1,45(t); 2,69(q); 3,28(s); 3,43(t); 4,03(q); 4,61(t); 7,56(s); 7,62(d); 8,14(d). |
| 2 . 6 | Cl | 4,5-Dihydroisoxazol-3-yl | SO₂CH₃ | H | C₂H₅ | 4-Cl-C₆H₄CO | H | 105-108 |
| 2 . 7 | Cl | 4,5-Dihydroisoxazol-3-yl | SO₂CH₃ | H | C₂H₅ | C₆H₅CO | H | 207-209 |
| 2.8 | Cl | 4,5-Dihydroisoxazol-3-yl | SO₂CH₃ | H | C₂H₅ | CH₃CO | H | 67 - 73 |
| 2.9 | Cl | 5,5-Dirnelhyl-4,5-dihydroisoxazol-3-yl | SO₂CH₃ | H | CH₃ | C₆H₅CH₂ | H | 1,36(s); 3,02(s); 3,19(s); 3,43(s); 5,31(s); 7,28(m); 7,42(s); 7,76(d); 8,00(d). |
| 2.10 | Cl | 5,5-Dimethyl-4,5-dihydroisoxazol-3-yl | SO₂CH₃ | H | C₂H₅ | C₆H₅CH₂ | H | 1,12(t); 1,48(s); 3,15(s); 3,33(s); 3,89(q); 5,59(s); 7,41(m); 7,54(s); 7,89(d); 8,13(d). |
| 2.11 | Cl | 5,5-Dimethyl-4,5-dihydroisoxazol-3-yl | SO₂CH₃ | H | i-C₄H₉ | C₆H₅CH₂ | H | 0,78(d); 1,49(s); 2,01(m); 3,15(s); 3,35(s); 3,68(d); 5,49(s); 7,39(m); 7,52(s); 7,89(d); 8,13(d). |
| 2.12 | Cl | 4,5-Dihydroisoxazol-3-yl | SO₂CH₃ | H | CH₃ | i-C₃H₇ | H | 1,28(d); 3,33(s); 3,36(m); 3,69(s); 4,52(t); 4,98(m); 7,55(s); 7,86(d), 8,12(d). |
| 2.13 | Cl | 4,5-Dihydroisoxazol-3-yl | SO₂CH₃ | H | C₂H₅ | i-C₃H₇ | H | 1,30(d); 1,32(t); 3,35(s); 3,38(m); 4,15(q); 4,52(t); 5,08(m); 7,55(s); 7,90(d); 8,12(d). |
| 2.14 | Cl | 4,5-Dihydioisoxazol-3-yl | SO₂CH₃ | H | CH₃ | C₂H₅ | H | 83 - 88 |
| 2.15 | Cl | 4,5-Dihydroisoxazol-3-yl | SO₂CH₃ | H | C₂H₅ | C₂H₅ | H | 1,26(t); 1,32(t); 3,34(s); 3,37(t); 4,03(q); 4,41(q); 4,50(l); 7,56(s); 7,88(d); 8,11 (d). |
| 2.16 | Cl | 4,5-Dihydroisoxazol-3-yl | SO₂CH₃ | H | C₂H₅ | C₆H₅COCH₂ | H | 168 - 173 |
| 2.17 | Cl | 5-Ethyl-4,5-dihydroisoxazol-3-yl | SO₂CH₃ | H | C₂H₅ | C₂H₅ | H | 0,96(t); 1,28(t); 1,32(t); 1,72(m): 3.01(dd); 3,31(s); 3.38(dd); 4,05(q); 4,41(q); 4,75(m), 7,54(s); 7,86(d); 8,10(d). |
| 2.18 | Cl | 5-Ethyl-4,5-dihydroisoxazol-3-yl | SO₂CH₃ | H | C₂H₅ | i-C₃H₇ | H | 0,95(t); 1,30(d); 1,32(t); 1,75(m); 3,03(dd); 3,34(s); 3,45(dd); 4,00(q); 4,75(m); 5.07(m); 7,50(s); 7,88(d); 8,09(d). |
| 2.19 | Cl | 5-Ethyl-4,5-dihydroisoxazol-3-yl | SO₂CH₃ | H | CH₃ | C₂H₅ | H | 0,96(t); 1,22(l); 1,74(m); 3,00(dd); 3,32(s); 3,44(dd); 3,67(s); 4,40(q); 4,75(m); 7,56(s); 7,87(d); 8,10(d). |
| 2.20 | Cl | 5-Ethyl-4,5-dihydroisoxazol-3-yl | SO₂CH₃ | H | CH₃ | i-C₃H₇ | H | 0,96(t); 1,25(d); 1,73(m); 3.02(dd); 3,31(s); 3,41(dd); 3,67(s); 4,74(m); 4,98(m); 7,53(s); 7,88(d); 8,10(d). |
| 2.21 | Cl | 5,5-Diethyl-4,5-dihydroisoxazol-3-yl | SO₂CH₃ | H | CH₃ | C₂H₅ | H | 0,91(t); 1,24(t); 1,35(t); 3,11(s); 3,35(s); 3,67(s); 4,36(q); 7,54(s); 7,86(d); 8,10(d). |
| 2.22 | Cl | 5,5-Diethyl-4,5-dihydroisoxazol-3-yl | SO₂CH₃ | H | CH₃ | i-C₃H₇ | H | 1,91(t); 1,29(d); 1,35(d); 1,75(m); 3,12(s); 3,35(s); 3,65(s); 4,98(m); 7,50(s); 7,86(d); 8,10(d). |
| 2.23 | Cl | 5,5-Diethyl-4,5-dihydroisoxazol-3-yl | SO₂CH₃ | H | C₂H₅ ; | C₂H₅ | H | 0,94(t); 1,31(m); 1,76(m); 3,10(s); 3,32(s); 4,03(q); 4,40(q); 7,53(s); 7,85(d); 8,10(d). |
| 2.24 | Cl | 5,5-Diethyl-4,5-dihydroisoxazol-3-yl | SO₂CH₃ | H | C₂H₅ | i-C₃H₇ | H | 0,90(t); 1,32(m); 1,75(m); 3,12(s); 3,34(s); 4,00(q); 5,05(m); 5,20(m); 7,50(s); 7,86(d); 8,08(d). |
| 2.25 | Cl | (4,5-Dihydro-isoxazol-5-spiro-cyclopentan)-3-yl | SO₂CH₃ | H | CH₃ | C₂H₅ | H | 148-153 |
| 2.26 | Cl | (4,5-Dihydro-isoxazol-5-spiro-cyclopentan)-3-yl | SO₂CH₃ | H | C₂H₅ | C₂H₅ | H | 1,25 (t); 1,32 (t); 1,74 (m): 2,02 (m); 3,05 (s); 3,37 (s); 4,04 (q); 4,39 (q); 7,54 (s); 7,86 (d); 8,09 (d). |
| 2.27 | Cl | 2-Thiazolyl | SO₂CH₃ | H | C₂H₅ | CH₃CO | H | 173-174 |
| 2.28 | Cl | 2-Thiazolyl | SO₂CH₃ | H | C₂H₅ | C₆H₅CO | H | 158 |
| 2.29 | Cl | 2-Thiazolyl | SO₂CH₃ | H | C₂H₅ | C₆H₅CH=CHCO | H | 98 |
| 2.30 | Cl | 2-Thiazolyl | SO₂CH₃ | H | C₂H₅ | (3-Cl-C₆H₄)CO | H | 165 |
| 2.31 | Cl | 2-Thiazolyl | SO₂CH₃ | H | C₂H₅ | (CH₃)₂C=CHCO | H | 158-160 |
| 2.32 | Cl | 2-Thiazolyl | SO₂CH₃ | H | C₂H₅ | C₂H₅OCO | H | 151 |
| 2.33 | Cl | 2-Thiazolyl | SO₂CH₃ | H | C₂H₅ | C₂H₅CO | H | 141-144 |
| 2.34 | Cl | 2-Thiazolyl | SO₂CH₃ | H | C₂H₅ | n-C₃H₇CO | H | 1,09 (t); 1,46 (t); 1,81 (q); 2,63 (t); 3,27 (s); 4,05 (q); 7,62 (s); 7,66 (d); 7,71 (d); 7,98 (d); 8,25 (d). |
| 2.35 | Cl | 2-Thiazolyl | SO₂CH₃ | H | C₂H₅ | CH₃OCO | H | 91 |
| 2.36 | Cl | 2-Thiazolyl | SO₂CH₃ | H | C₂H₅ | (4-Cl-C₆H₄)CO | H | 148-149 |
| 2.37 | Cl | 2-Thiazolyl | SO₂CH₃ | H | C₂H₅ | C₆H₅CH₂ | H | 56 |
| 2.38 | Cl | 2-Thiazolyl | SO₂CH₃ | H | C₂H₅ | [(CH₃O)CH₃N]CO | H | 62 |
| 2.39 | Cl | 2-Thiazolyl | SO₂CH₃ | H | C₂H₅ | C₂H₅ | H | 145-147 |
| 2.40 | Cl | 1,3-Dithiolan-2-yl | Cl | H | C₂H₅ | C₂H₅CO | H | 52-54 |
| | | | | | | | | |
| | | | | | | | | |
| | | | | | | | | |
| 2.44 | Cl | 4,5-Dihydroisoxazol-3-yl | SO₂CH₃ | H | CH₃ | C₆H₅CH₂ | H | 195-200 |
| 2.45 | Cl | 4,5-Dihydroisoxazol-3-yl | SO₂CH₃ | H | C₂H₅ | C₆H₅CH₂ | H | 70-75 |
| 2.46 | Cl | 4,5-Dihydroisoxazol-3-yl | SO₂CH₃ | H | CH₃ | (CH₃)₂NCS | H | 85-95 |
| 2.47 | CH₃ | 4,5-Dihydrothiazol-2-yl | H | H | C₂H₅ | C₆H₅CH₂ | H | 1,29 (t); 2,46 (s); 3,49 (t); 3,91 (q); 4,53 (t); 5,57 (s); 7,31 (m); 7,59 (d). |
| 2.48 | Cl | 5-Methyl-4,5-dihydroisoxazol-3-yl | n-SO₂C₃H₇ | H | C₂H₅ | C₆H₅CH₂ | H | 0,94 (t); 1,12 (t); 1,39 (d); 1,61 (m); 2,98 (m); 3,42 (m); 3,87 (m); 4,98 (m); 5,49 (s); 7,41 (m); 7,59 (s); 7,90 (d); 8,10 (d) |
| 2.49 | Cl | 4,5-Dihydroisoxazol-3-yl | SO₂CH₃ | H | CH₃ | i-C₄H₉ | H | 94-96 |
| 2.50 | Cl | 4,5-Dihydroisoxazol-3-yl | SO₂CH₃ | H | C₂H₅ | i-C₄H₉ | H | 55-60 |
| 2.52 | Cl | 4,5-Dihydroisoxazol-3-yl | SO₂CH₃ | H | CH₃ | (C₂H₅)₂CH | H | 64-68 |
| 2.53 | Cl | 4,5-Dihydroisoxazol-3-yl | SO₂CH₃ | H | C₂H₅ | (C₂H₅)₂CH | H | 58-62 |
| 2.54 | Cl | 4,5-Dihydroisoxazol-3-yl | SO₂CH₃ | H | CH₃ | (CH₃)₂NCS | H | 85-90 |
| 2.55 | CI | 4,5-Dihydroisoxazol-3-yl | SO₂CH₃ | H | CH₃ | C₆H₅CH₂ | H | 195-200 |
| 2.56 | Cl | 4,5-Dihydroisoxazol-3-yl | SO₂CH₃ | H | C₂H₅ | C₆H₅CH₂ | H | 70-75 |
| 2.57 | Cl | 4,5-Dihydroisoxazol-3-yl | SO₂CH₃ | H | CH₃ | CH₃ | H | 78-92 |
| 2.58 | Cl | 4,5-Dihydroisoxazol-3-yl | SO₂CH₃ | H | C₂H₅ | CH₃ | H | 70-75 |
| 2.59 | Cl | 4,5-Dihydroisoxazol-3-yl | SO₂CH₃ | H | CH₃ | CH₃OCOCH₂ | H | 88-93 |
| 2.60 | Cl | 4,5-Dihydroisoxazol-3-yl | SO₂CH₃ | H | C₂H₅ | CH₃OCOCH₂ | H | 120-123 |
| | | | | | | | | |
| | | | | | | | | |
| | | | | | | | | |
| | | | | | | | | |
| 2.65 | Cl | 5-Methyl-4,5-dihydroisoxazol-3-yl | SO₂-n-C₃H₇ | H | C₂H₅ | C₆H₅CH₂ | H | |
| 2.66 | Cl | 5-Chlormethyl-4,5-dihydroisoxazol-3-yl | SO₂CH₃ | H | CH₃ | C₂H₅CO | H | 69-74 |
| 2.67 | Cl | 3-tert.Butyl-isoxazol-4-yl | Cl | H | CH₃ | C₂H₅CO | H | 1,3 (t); 1,4 (s); 2,6 (q); 3,7 (s); 6,4 (s); 7,4 (d); 7,5 (d); 7,7 (s) |
| | | | | | | | | |
| 2.69 | CH₃ | 4,5-Dihydroisoxazol-3-yl | SO₂CH₃ | H | C₂H₅ | i-C₃H₇ | H | 155-160 |
| 2.70 | Cl | 3-Methyl-isoxazol-5-yl | Cl | H | CH₃ | C₂H₅CO | H | |
| 2.71 | Cl | 2-Thiazolyl | SO₂CH₃ | H | C₂H₅ | C₆H₅CH(CH₃) | H | 1,26 (t); 1,82 (d); 3,26 (s); 3,89 (q); 6,20 (q); 7,35 (m); 7,58 (d); 7,71 (d); 7,99 (d); 8,23 (d) |
| 2.72 | Cl | 2-Thiazolyl | SO₂CH₃ | H | C₂H₅ | (4-CH₃-C₆H₄)CH₂ | H | 131-136 |
| 2.73 | Cl | 2-Thiazolyl | SO₂CH₃ | H | C₂H₅ | CH₂=CHCH₂ | H | 1,44 (t); 3,29 (s); 4,11 (q); 5,10 (d); 5,31 (d); 5,42 (d); 6,04 (m); 7,37 (s); 7,69 (d); 7,72 (d); 7,99 (d); 8,27 (d) |
| 2.74 | Cl | 2-Thiazolyl | SO₂CH₃ | H | C₂H₅ | CH≡CCH₂ | H | 1,46 (t); 2,57 (m); 3,29 (s); 4,15 (q); 5,32 (d); 7,39 (s); 7,70 (m); 7,99 (d); 8,29 (d) |
| 2.75 | Cl | 2-Thiazolyl | SO₂CH₃ | H | C₂H₅ | (4-CH₃-C₆H₄)COCH₂ | H | 1,52 (t); 2,41 (s); 3,23 (s); 4,32 (q); 6,16 (s); 7,27 (m); 7,60 (d); 7,70 (d); 7,82 (d); 7,98 (d); 8,21 (d) |
| 2.76 | Cl | 2-Thiazolyl | SO₂CH₃ | H | C₂H₅ | C₆H₅COCH₂ | H | 199-202 |
| | | | | | | | H | |
| | | | | | | | H | |
| 2.79 | Cl | 4,5-Dihydroisoxazol-3-yl | SO₂CH₃ | H | CH₃ | CF₃ | H | |
| 2.80 | Cl | 4,5-Dihydroisoxazol-3-yl | SO₂CH₃ | H | C₂H₅ | CF₃ | H | |
| 2.81 | Cl | 4,5-Dihydroisoxazol-3-yl | SO₂CH₃ | H | CH₃ | CHF₂ | H | |
| | | | | | | | H | |
| | | | | | | | H | |
| 2.84 | Cl | 4,5-Dihydroisoxazot-3-yl | SO₂CH₃ | H | CH₃ | CH₃ | H | 78-84 |
| 2.85 | Cl | 4,5-Dihydroisoxazol-3-yl | SO₂CH₃ | H | C₂H₅ | CH₃ | H | 112-116 |
| | | | | | | | H | |
| 2.87 | Cl | 5-Methyl-isoxazol-3-yl | SO₂CH₃ | H | CH₃ | i-C₃H₇ | H | 101-102 |
| 2.88 | Cl | 5-Methyl-isoxazol-3-yl | SO₂CH₃ | H | CH₃ | H₃COCOCH₂ | H | 92-96 |
| 2.89 | Cl | 4,5-Dimethyl-4,5-dihydroisoxazol-3-yl | SO₂CH₃ | H | CH₃ | H₃COCOCH₂ | H | 68-70 |
| | | | | | | | | |
| | | | | | | | | |
| 2.92 | Cl | 4,5-Dimethyl-4,5-dihydroisoxazol-3-yl | SO₂CH₃ | H | CH₃ | i-C₃H₇ | H | 141-144 |
| | | | | | | | | |
| 2.94 | Cl | 4,5-Dihydroisoxazol-3-yl | SO₂CH₃ | H | CH₃ | NCCH₂ | H | 96-99 |
| 2.95 | Cl | 4,5-Dihydroisoxazol-3-yl | SO₂CH₃ | H | C₂H₅ | NCCH₂ | H | 135-138 |
| 2.96 | Cl | 4,5-Dihydroisoxazol-3-yl | SO₂CH₃ | H | CH₃ | CH₂=CHCH₂ | H | 105-108 |
| 2.97 | Cl | 4,5-Dihydroisoxazol-3-yl | SO₂CH₃ | H | C₂H₅ | CH₂=CHCH₂ | H | 128-131 |
| | | | | | | | | |
| 2.99 | Cl | 4,5-Dihydroisoxazol-3-yl | SO₂CH₃ | H | C₂H₅ | n-C₃H₇ | H | 114-118 |
| 2.100 | Cl | 4,5-Dihydroisoxazol-3-yl | SO₂CH₃ | H | CH₃ | FCH₂CH₂CH₂ | H | 115-117 |
| 2.101 | Cl | 4,5-Dihydroisoxazol-3-yl | SO₂CH₃ | H | C₂H₅ | FCH₂CH₂CH₂ | H | 1,43 (t); 2,25 (m); 3,30 (s); 3,45 (t); 4,06 (q); 4,66 (m); 4,75 (t); 7,30 (s); 7,63 (d); 8,16 (d) |
| 2.102 | Cl | 4,5-Dihydroisoxazol-3-yl | SO₂CH₃ | H | CH₃ | s-C₄H₉ | H | 168-173 |
| 2.103 | Cl | 4,5-Dihydroisoxazol-3-yl | SO₂CH₃ | H | C₂H₅ | s-C₄H₉ | H | 165-170 |
| 2.104 | Cl | 4,5-Dihydroisoxazol-3-yl | SO₂CH₃ | H | CH₃ | HC≡CCH₂ | H | 164-168 |
| 2.105 | Cl | 4,5-Dihydroisoxazol-3-yl | SO₂CH₃ | H | C₂H₅ | HC≡CCH₂ | H | 122-125 |
| 2.106 | Cl | 4,5-Dihydroisoxazol-3-yl | SO₂CH₃ | H | CH₃ | H₃CCH=CHCH₂ | H | 145-147 |
| 2.107 | Cl | 4,5-Dihydroisoxazol-3-yl | SO₂CH₃ | H | C₂H₅ | CH₃CH=CHCH₂ | H | 123-126 |
| 2.108 | Cl | 4,5-Dihydroisoxazol-3-yl | SO₂CH₃ | H | CH₃ | n-C₃H₇ | H | 55-60 |
| 2.109 | Cl | 5-Methyl-isoxazol-3-yl | SO₂CH₃ | H | C₂H₅ | CH₃ | H | 1,30 (t); 2,50 (s); 3,30 (s); 4,05 (q); 4,13 (s); 6,53 (s); 7,52 (s); 7,93 (d); 8,16 (d) |
| 2.110 | Cl | 5-Methyl-isoxazol-3-yl | SO₂CH₃ | H | C₂H₅ | i-C₃H₇ | H | 70-73 |
| 2.111 | Cl | 4,5-Diethyl-isoxazol-3-yl | SO₂CH₃ | H | CH₃ | i-C₃H₇ | H | 0,93 (t); 1,25 (m); 2,23 (m); 2,83 (q); 3,65 (s); 4,95 (sept); 7,48 (s); 7,93 (d); 8,20 (d) |
| 2.112 | Cl | 5-t-Butyl-isoxazol-3-yl | SO₂CH₃ | H | C₂H₅ | i-C₃H₇ | H | 1,31-1,35 (m); 3,31 (s); 4,02 (q); 5,08 (sept); 6,52 (s); 7,49 (s); 7,90 (d); 8,16 (d) |
| 2.113 | Cl | 5-n-Propyl-isoxazot-3-yl | SO₂CH₃ | H | CH₃ | i-C₃H₇ | H | 0,93 (t); 1,30 (d); 1,74 (sext); 2,85 (t); 3,37 (s); 3,68 (s); 5,02 (sept); 6,56 (s); 7,53 (s); 7,90 (d); 8,18 (d) |

Nachfolgend sind die Synthesen einiger Ausgangsstoffe aufgeführt:
4-[2-Chlor-3-(4,5-dihydroisoxazol-3-yl)-4-methylsulfonylbenzoyl]-5-hydroxy-1-methyl-1H-pyrazol (Verbindung 3.21)
   Stufe a) 2-Chlor-3-methyl-a-methylthio-acetophenon
      Zu einer Suspension von 286 g (2,14 mol) Aluminiumtrichlorid in 420 ml 1,2-Dichlorethan wurde bei 15-20°C eine Lösung von 157 g (2 mol) Acetylchlorid in 420 mol 1,2-Dichlorethan getropft. Anschließend wurde eine Lösung von 346 g (2 mol) 2-Chlor-6-methylthio-toluol in 1 1 1,2-Dichlorethan zugetropft. Nach 12 Stunden Rühren wurde das Reaktionsgemisch in eine Mischung aus 3 1 Eis und 1 1 konz. Salzsäure gegossen. Es wurde mit Methylenchlorid extrahiert, die organische Phase mit Wasser gewaschen, mit Natriumsulfat getrocknet und eingeengt. Der Rückstand wurde im Vakuum destilliert. Man erhielt 256 g (60 % d.Th.) 2-Chlor-3-methyl-4-methylthio-acetophenon. (Fp.: 46°C)
   Stufe b) 2-Chlor-3-methyl-4-methylsulfonyl-acetophenon
      163,0 g (0,76 mol) 2-Chlor-3-methyl-4-methylthio-acetophenon wurden in 1,5 1 Eisessig gelöst, mit 18,6 g Natriumwolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser gewaschen und getrocknet. Man erhielt 164,0 g (88 % d.Th.) 2-Chlor-3-methyl-4-methylsulfonyl-acetophenon. (Fp.: 110-111°C)
   Stufe c) 2-Chlor-3-methyl-4-methylsulfonyl-benzoesäure
      82 g (0,33 mol) 2-Chlor-3-methyl-4-methylsulfonyl-acetophenon wurden in 700 ml Dioxan gelöst und bei Raumtemperatur mit 1 1 einer 12,5 %igen Natriumhypochloritlösung versetzt. Anschließend wurde 1 Stunde bei 80°C nachgerührt. Nach dem Abkühlen bildeten sich zwei Phasen, von denen die schwerere mit Wasser verdünnt und schwach angesäuert wurde. Der ausgefallene Feststoff wurde abgesaugt, mit Wasser nachgewaschen und getrocknet. Man erhielt 60 g (73 % d.Th) 2-Chlor-3-methyl-4-methylsulfonylbenzoesäure.
      (Fp.: 230-231°C)
   Stufe d) 2-Chlor-3-methyl-4-methylsulfonyl-benzoesäuremetylester
      100 g (0,4 mol) 2-Chlor-3-methyl-4-methylsulfonyl-benzoesäure wurden in 1 1 Methanol gelöst und bei Rückflußtemperatur 5 Stunden mit Chlorwasserstoff begast. Anschließend wurde eingeengt. Man erhielt 88,5 g (84 % d.Th.) 2-Chlor-3-methyl-4-methylsulfonyl-benzoesäuremethylester.
      (Fp.: 107-108°C)
   Stufe e) 3-Brommethyl-2-chlor-4-methylsulfonyl-benzoesäuremethylester
      82 g (0,31 mol) 2-Chlor-3-methyl-4-methylsulfonyl-benzoesäuremethylester werden in 2 1 Tetrachlormethan gelöst und unter Belichtung portionsweise mit 56 g (0,31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wurde mit Petrolether versetzt, der ausgefallene Feststoff abgesaugt und getrocknet. Man erhielt 74,5 g (70 % d.Th.) 3-Brommethyl-2-chlor-4-methylsulfonyl-benzoesäuremethylester.
      (Fp.: 74-75°C)
   Stufe f) 2-Chlor-3-formyl-4-methylsulfonyl-benzoesäuremethylester
      Eine Lösung von 41,0 g (0,12 mol) 3-Brommethyl-2-chlor-4-methylsulfonyl-benzoesäuremethylester in 250 ml Acetonitril wurde mit 42,1 g (0,36 mol) N-Methylmorpholin-N-oxid versetzt. Der Ansatz wurde 12 Stunden bei Raumtemperatur gerührt, anschließend eingeengt und der Rückstand in Essigsäureethylester aufgenommen. Die Lösung wurde mit Wasser gewaschen, getrocknet und eingeengt. Man erhielt 31,2 g (94 % d.Th.) 2-Chlor-3-formyl-4-methylsulfonyl-benzoesäuremethylester
      (Fp.: 98-105°C)
   Stufe g) 2-Chlor-3-hydroxyiminomethyl-4-methylsulfonyl-benzoesäure
      15,00 g (54 mmol) 2-Chlor-3-formyl-4-methylsulfonyl-benzoesäuremethylester und 4,20 g (60 mmol) Hydroxylamin-hydrochlorid wurden in 300 ml Methanol aufgenommen und eine Lösung von 3,18 g (30 mmol) Natriumcarbonat in 80 ml Wasser zugetropft. Nach 12 Stunden Rühren bei Raumtemperatur wurde das Methanol abdestilliert, der Rückstand mit Wasser verdünnt und mit Diethylether extrahiert. Nach Trocknen der organischen Phase wurde das Lösungsmittel entfernt. Man erhielt 14,40 g (91 % d.Th.) 2-Chlor-3-hydroxyiminomethyl-4-methylsulfonyl-benzoesäuremethylester.
      (Fp.: 126-128°C).
   Stufe h) 2-Chlor-3-(4,5-dihydroisoxazol-3-yl)-4-methylsulfonyl-benzoesäuremethylester (Verbindung 4.3)
      In eine Lösung von 158,0 g (0,54 mol) 2-Chlor-3-hydroxyiminomethyl-4-methylsulfonyl-benzoesäuremethylester und 1 1 Dichlormethan wurde bei 15-20°C 30 Minuten lang Ethylen eingeleitet. Nach Zugabe von 1,6 g Natriumacetat wurden 454 ml Natriumhydrochlorit-Lösung bei 10°C unter gleichzeitiger Ethylen-Einleitung zugetropft. Anschließend wurde für weitere 15 Minuten Ethylen bei 10°C eingeleitet. Nach 12 Stunden Rühren wurden die Phasen getrennt, die organische Phase mit Wasser gewaschen, getrocknet und eingeengt. Man erhielt 156,5 g (90 % d.Th.) 2-Chlor-(4,5-dihydroisoxazol-3-yl)-4-methylsulfonyl-benzoesäuremethylester.
      (¹H-NMR (δ in ppm) : 3,24 (s) ; 3, 42 (t) ; 3,99 (s) ; 4, 60 (t); 7, 96 (d) ; 8,10 (d)) .
   Stufe i) 2-Chlor-3-(4,5-dihydroisoxazol-3-yl)-4-methylsulfonylbenzoesäure (Verbindung 4.4)
      Zu einem Gemisch von 170,0 g (0,54 mol) 2-Chlor-3-(4,5-dihydroisoxazol-3-yl)-4-methylsulfonyl-benzoesäuremethylester und 1 1 Methanol wurde bei 40-45°C langsam eine Lösung von 32,8 g Natriumhydroxid gelöst in 330 ml Methanol getropft. Die Suspension wurde 5 Stunden bei 50°C gerührt. Nach Abdestillieren des Lösungsmittels nahm man den Rückstand in 1,5 1 Wasser auf und extrahierte die wäßrige Phase dreimal mit Essigsäureethylester. Die wäßrige Phase wurde mit Salzsäure angesäuert und dreimal mit Essigsäureethylester extrahiert. Die vereinigten organischen Phasen wurden anschließend mit Wasser neutral gewaschen, getrocknet und eingeengt. Man erhielt 148,8 g (91 % d.Th.) 2-Chlor-3-(4,5-dihydroisoxazol-3-yl)-4-methylsulfonyl-benzoesäure.
      (¹H-NMR (δ in ppm): 3,26 (s) ; 3,45 (t) ; 4,63 (t) ; 8, 15 (s); 8,53 (s, br)).
   Stufe j) 2-Chlor-3-(4,5-dihydroisoxazol-3-yl)-4-methylsulfonylbenzoylchlorid (Verbindung 4.5)
      Zu einer Lösung von 139,0 g 2-Chlor-3-(4,5-dihydroisoxazol-3-yl)-4-methylsulfonyl-benzoesäure, 1 ml Dimethylformamid und 1 1 trockenem Toluol wurden bei 50°C 74,8 g (0,63 mol) Thionylchlorid in 50 ml trockenem Toluol getropft. Nach 6 Stunden Erhitzen auf 110°C wurde das Lösungsmittel abdestilliert. Man erhielt 2-Chlor-3-(4,5-dihydroisoxazol-3-yl)-4-methylsulfonyl-benzoylchlorid in quantitativer Ausbeute.
      (¹H-NMR (δ in ppm): 3,25 (s) ; 3,46 (t) ; 4,62 (t); 8,21 (dd)).
   Stufe k) 4-[2-Chlor-3-(4,5-dihydroisoxazol-3-yl)-4-methylsulfonylbenzoyl]-5-hydroxy-1-methyl-1H-pyrazol (Verbindung 3.21)
      Zu einer Lösung von 12,74 g (0,13 mol) 5-Hydroxy-1-methyl-pyrazol und 300 ml wasserfreiem Dioxan wurden unter Schutzgasatmosphäre bei Raumtemperatur gleichzeitig 43,60 g (0,13 mol) 2-Chlor-3-(4,5-dihydroisoxazol-3-yl)-4-methylsulfonyl-benzoylchlorid in 375 ml wasserfreiem Dioxan und 13,56 g (0,134 mol) Triethylamin in 375 ml wasserfreiem Dioxan getropft. Nach 2 Stunden Rühren bei Raumtemperatur wurde das Reaktionsgemisch über Kieselgel abfiltriert und mit Dioxan nachgewaschen. Das Eluat wurde am Vakuum auf ca. 500 ml eingeengt und mit 17,94 g (0,13 mol) getrocknetem, fein gepulvertem Kaliumcarbonat versetzt. Nach 6 Stunden Erhitzen unter Rückfluß wurde das Lösungsmittel am Vakuum abdestilliert und der Rückstand in ca. 700 ml Wasser aufgenommen. Unlösliche Bestandteile wurden abfiltriert und der pH-Wert des Filtrats durch langsame Zugabe von 10 %iger Salzsäure auf pH = 2 - 3 eingestellt. Der sich bildende Niederschlag wurde abgesaugt. Man erhielt 46,16 g (92 % d. Th.)
      4-[2-Chlor-3-(4,5-dihydroisoxazol-3-yl)-4-methylsulfonylbenzoyl]-5-hydroxy-1-methyl-1H-pyrazol.
      (Fp. > 250°C)
4-[2-Chlor-3-(5-methyl-4,5-dihydroisoxazol-3-yl)-4-methylsulfonyl-benzoyl]-5-hydroxy-1-methyl-1H-pyrazol (Verbindung 3.5)
   Stufe a) 2-Chlor-3-(5-methyl-4,5-dihydroisoxazol-3-yl)-4-methylsulfonyl-benzoesäuremethylester (Verbindung 4.25)
      In eine Lösung von 15,0 g (52 mmol) 2-Chlor-3-hydroxyiminomethyl-4-methylsulfonyl-benzoesäuremethylester und 200 ml Dichlormethan wurde bei Raumtemperatur 30 Minuten lang Propen eingeleitet. Nach Zugabe von 1,6 g Natriumacetat wurden 42,8 ml Natriumhydrochlorit-Lösung bei Raumtemperatur unter gleichzeitiger Propen-Einleitung zugetropft. Anschließend wurde für weitere 15 Minuten Propen bei Raumtemperatur eingeleitet. Nach 3 Stunden Erhitzen unter Rückfluß wurde 12 Stunden bei Raumtemperatur gerührt, nochmals 5 Stunden unter Rückfluß Propen eingeleitet und wiederum 12 Stunden bei Raumtemperatur gerührt. Nach Trennung der Phasen wurde die organische Phase mit Wasser gewaschen, getrocknet und eingeengt. Man erhielt 15,5 g (89 % d.Th.) 2-Chlor-(5-methyl-4,5-dihydroisoxazol-3-yl)-4-methylsulfonyl-benzoesäuremethylester.
      (Fp.: 130-135°C).
   Stufe b) 2-Chlor-3-(5-methyl-4,5-dihydroisoxazol-3-yl)-4-methylsulfonyl-benzoesäure (Verbindung 4.26)
      Zu einem Gemisch von 15,00 g (45 mmol) 2-Chlor-3-(5-methyl-4,5-dihydroisoxazol-3-yl)-4-methylsulfonyl-benzoesäuremethylester und 200 ml Methanol wurde langsam eine Lösung von 3,52 g (88 mmol) Natriumhydroxid gelöst in 100 ml Methanol getropft. Die Suspension wurde 48 Stunden bei Raumtemperatur gerührt. Nach Abdestillieren des Lösungsmittels nahm man den Rückstand in Wasser auf und wusch die wäßrige Phase dreimal mit Essigsäureethylester. Die wäßrige Phase wurde mit Salzsäure angesäuert und dreimal mit Essigsäureethylester extrahiert. Die vereinigten organischen Phasen wurden anschließend mit Wasser neutral gewaschen, getrocknet und eingeengt. Man erhielt 13,20 g (92 % d.Th.) 2-Chlor-3-(5-methyl-4,5-dihydroisoxazol-3-yl)-4-methylsulfonyl-benzoesäure.
      (Fp.: 173-178°C).
   Stufe c) 2-Chlor-3-(5-methyl-4,5-dihydroisoxazol-3-yl)-4-methylsulfonyl-benzoylehlorid (Verbindung 4.39)
      Zu einer Lösung von 13,0 g (41 mmol) 2-Chlor-3-(5-methyl-4,5-dihydroisoxazol-3-yl)-4-methylsulfonylbenzoesäure, 1 ml Dimethylformamid und 250 ml trockenem Toluol wurden bei Raumtemperatur 5,7 g (51 mmol) Thionylchlorid getropft. Anschließend wurde bis zur vollständigen Umsetzung unter Rückfluß erhitzt. Nach Abkühlen wurde das Lösungsmittel abdestilliert. Man erhielt 14,2 g 2-Chlor-3-(5-methyl-4,5-dihydroisoxazol-3-yl)-4-methylbenzoylchlorid in quantitativer Ausbeute.
   Stufe d) 4-[2-Chlor-3-(5-methyl-4,5-dihydroisoxazol-3-yl)-4-methylsulfonyl-benzoyl]-5-hydroxy-1-methyl-1H-pyrazol (Verbindung 3.5)
      Zu einer Lösung von 1,20 g (12 mmol) 5-Hydroxy-1-methylpyrazol in 30 ml Dioxan wurden bei Raumtemperatur zuerst 4,00 g (12 mmol) 2-Chlor-3-(5-methyl-4,5-dihydroisoxazol-3-yl)-4-methylsulfonyl-benzoylchlorid in 50 ml Dioxan und anschließend-1,20 g (12,2 mmol) Triethylamin in 30 ml Dioxan zugetropft. Nach zwölf Stunden Rühren wurde das Reaktionsgemisch über Kieselgel abfiltriert, das Filtrat mit 0,50 g (3,6 mmol) Kaliumcarbonat versetzt und zwölf Stunden unter Rückfluß erhitzt. Nach weiteren zwölf Stunden Rühren bei Raumtemperatur wurde eine Spatelspitze Kaliumcarbonat zugegeben und wiederum unter Rückfluß erhitzt. Nach Abkühlen wurde das Lösungsmittel im Vakuum abdestilliert, der Rückstand in Wasser aufgenommen, mit Essigsäureethylester gewaschen, mit 10 %iger Salzsäure ein pH-Wert von 1-2 eingestellt und mit Essigsäureethylester mehrmals extrahiert. Die vereinigten organischen Phasen wurden mit Wasser gewaschen, getrocknet und das Lösungsmittel entfernt. Der Rückstand wurde in kaltem Essigsäureethylester digeriert. Man erhielt 1,60 g (34 % d.Th.) 4-[2-Chlor-3-(5-methyl-4,5-dihydroisoxazol-3-yl)-4-methylsulfonyl-benzoyl]-5-hydroxy-1-methyl-1H-pyrazol.
      (Fp.: 230-235°C)
4-[2-Chlor-3-(4,4-dimethyl-4,5-dihydrooxazol-2-yl)-4-methylsulfonyl-benzoyl]-5-hydroxy-1-methyl-1H-pyrazol (Verbindung 3.29)
   Stufe a) 2-Chlor-3-hydroxycarbonyl-4-methylsulfonyl-benzoesäuremethylester
      Zu einer Lösung von 115,3 g (0,42 mol) 2-Chlor-3-formyl-4-methylsulfonyl-benzoesäuremethylester und 2000 ml Acetonitril wurden bei 5°C nacheinander 13,8 g (0,11 mol) Natriumhydrogenphosphatmonohydrat in 170 ml Wasser, 49,3 g (0,43 mol) 30 %ige Wasserstoffperoxidlösung und 66,2 g (0,59 mol) 80 %ige wäßrige Natriumchloritlösung gegeben. Die Reaktionslösung wurde anschließend 1 Stunde bei 5°C und 12 Stunden bei Raumtemperatur gerührt. Dann wurde mit 10 %iger Salzsäure auf pH = 1 eingestellt und 1500 ml wäßrige 40 %ige Natriumhydrogensulfit-Lösung zugegeben. Nach 1 Stunde Rühren bei Raumtemperatur wurde die wäßrige Phase dreimal mit Essigsäureethylester extrahiert. Die vereinigten organischen Phasen wurden mit Natriumhydrogensulfit-Lösung gewaschen und getrocknet. Nach Abdestillation des Lösungsmittels erhielt man 102,0 g 2-Chlor-3-hydroxycarbonyl-4-methylsulfonyl-benzoesäuremethylester.
      (¹H-NMR (δ in ppm): 3,34 (s); 3,93 (s); 8,08 (s); 14,50 (s, br.).)
   Stufe b) 2-Chlor-3-chlorcarbonyl-4-methylsulfonyl-benzoesäuremethylester
      Zu einer Lösung von 6,0 g (0,021 mol) 2-Chlor-3-hydroxycarbonyl-4-methylsulfonyl-benzoesäuremethylester und 50 ml trockenem Toluol wurden 2 Tropfen Dimethylformamid und 11,9 g (0,1 mol) Thionylchlorid gegeben. Die Lösung wurde 4 Stunden unter Rückfluß erhitzt. Nach Entfernen des Lösungsmittels am Vakuum erhielt man 6,2 g 2-Chlor-3-chlorcarbonyl-4-methylsulfonyl-benzoesäuremethylester. (¹H-NMR (δ in ppm): 3,21 (s); 4,02 (s); 8,02 (d); 8,07 (d).)
   Stufe c) 2-Chlor-3-(1-hydroxy-2,2-dimethyleth-2-ylaminocarbonyl)-4-methylsulfonyl-benzoesäuremethylester
      Zu einer Lösung von 4,54 g (50 mmol) 2,2-Dimethylethanolamin in 40 ml Dichlormethan wurde bei 0-5°C eine Lösung von 7,80 g (25 mmol) 2-Chlor-3-chlorcarbonyl-4-methylsulfonyl-benzoesäuremethylester getropft. Nach 6 Stunden Rühren bei Raumtemperatur wurde die Reaktionslösung dreimal mit Wasser extrahiert, getrocknet und eingeengt. Man erhielt 8,20 g (80 % d.Th.) 2-Chlor-3-(1-hydroxy-2,2-dimethyleth-2-ylaminocarbonyl)-4-methylsulfonyl-benzoesäuremethylester.
      (Fp.: 70-72°C).
   Stufe d) 2-Chlor-3-(1-chlor-2,2-dimethyleth-2-ylamirocarbonyl)-4-methylsulfonyl-benzoesäuremethylester
      Ein Gemisch aus 6,9 g (20 mmol) 2-Chlor-3-(1-hydroxy-2,2-dimethyleth-2-ylaminocarbonyl)-4-methylsulfonylbenzoesäuremethylester und 5 ml Thionylchlorid wurde 6 Stunden bei Raumtemperatur gerührt. Die Lösung wurde mit 50 ml Dichlormethan verdünnt und anschließend eingeengt. Der Rückstand wurde in 20 ml Dichlormethan gelöst. Durch Zugabe von Cyclohexan bildete sich ein kristalliner Niederschlag, der abgesaugt und getrocknet wurde. Man erhielt 6,4 g (88 % d.Th.) 2-Chlor-3-(1-chlor-2,2-dimethyleth-2-ylaminocarbonyl)-4-methylsulfonyl-benzoesäuremethylester.
   Stufe e) 2-Chlor-3- (4,4-dimethyl-4,5-dihydrooxazol-2-yl) -4-methylsulfonyl-benzoesäure (Verbindung 4.38)
      Eine Lösung von 5,82 g (15 mmol) 2-Chlor-3-(1-chlor-2,2-dimethyleth-2-ylaminocarbonyl)-4-methylsulfonyl-benzoesäuremethylester und 0,81 g (20 mmol) Natriumhydroxid in 80 ml Methanol rührte 8 Stunden bei Raumtemperatur. Nach Abdestillieren des Lösungsmittels wurde der Rückstand in Wasser aufgenommen und dreimal mit Essigsäureethylester gewaschen. Die wäßrige Phase wird mit Salzsäure angesäuert und dreimal mit Essigsäureethylester extrahiert. Nach dem Trocknen der organischen Phase entfernte man das Lösungsmittel am Vakuum. Man erhielt 3,10 g (56 % d.Th.) 2-Chlor-3-(4,4-dimethyl-4,5-dihydrooxazol-2-yl)-4-methylsulfonyl-benzoesäure.
      (¹H-NMR (δ in ppm): 1,34 (s); 3,40 (s); 4,13 (s); 8,07 (s) ; 13,95 (s, br)).
   Stufe f) 2-Chlor-3-(1-chlor-2,2-dimethyleth-2-ylaminocarbonyl)-4-methylsulfonyl-benzoylchlorid
      Eine Lösung von 3,00 g (9 mmol) 2-Chlor-3-(4,4-dimethyl-4,5-dihydrooxazol-2-yl) -4-methylsulfonyl-benzoesäure, 1,43 g Thionylchlorid und 1 Tropfen Dimethylformamid in 80 ml trockenem Toluol wurde 3 Stunden unter Rückfluß erhitzt. Nach dem Abkühlen wurde das Lösungsmittel am Vakuum abdestilliert. Man erhielt 3,43 g (86 % d.Th.) 2-Chlor-3-(1-chlor-2,2-dimethyleth-2-ylaminocarbonyl)-4-methylsulfonyl-benzoylchlorid.
   Stufe g) 4-[2-Chlor-3-(4,4-dimethyl-4,5-dihydrooxazol-2-yl)-4-methylsulfonyl-benzoyl]-5-hydroxy-1-methyl-1H-pyrazol (Verbindung 3.29)
      Zu einem Gemisch aus 0,42 g (4,3 mmol) 5-Hydroxy-1-methylpyrazol in 10 ml Dioxan wurden bei 15°C 1,65 g (4,3 mmol) 2-Chlor-3-(1-chlor-2,2-dimethyleth-2-ylaminocarbonyl)-4-methylsulfonyl-benzoylchlorid in 25 ml Dioxan und 0,45 g (4,5 mmol) Triethylamin in 10 ml Dioxan getropft. Nach vier Stunden Rühren bei Raumtemperatur wurde über Kieselgel abfiltriert und mit Dioxan nachgewaschen. Die vereinigten Filtrate wurden auf 60 ml eingeengt und mit 1,24 g (9 mmol) fein gepulvertem Kaliumcarbonat versetzt. Nach fünf Stunden Erhitzen unter Rückfluß wurde abgekühlt, das Lösungsmittel am Vakuum entfernt, der Rückstand in Wasser aufgenommen, unlösliche Bestandteile abfiltriert, mit 10 %iger Salzsäure angesäuert und mehrmals mit Essigsäureethylester extrahiert. Die vereinigten organischen Phasen wurden anschließend mit Wasser gewaschen, getrocknet und eingeengt. Man erhielt 1,2 g (68 % d.Th.) 4-[2-Chlor-3-(4,4-dimethyl-4,5-dihydro-oxazol-2-yl)-4-methylsulfonyl-benzoyl]-5-hydroxy-1-methyl-1H-pyrazol.
      (Fp.: 132-135°C)
2,4-Dichlor-3-(2-oxoeth-1-yl)-benzoesäuremethylester
   Stufe a) 2,4-Dichlor-3-(2-methoxy-ethen-1-yl)-benzoesäuremethylester
   Zu 14,0 g (60 mmol) 2,4-Dichlor-3-formylbenzoesäure-methylester und 39,2 g (114 mmol) (Methoxymethyl)triphenylphosphoniumchlorid in 500 ml Tetrahydrofuran wurden bei 0-5°C 10,1 g (90 mmol) Kalium-t-butylat in 100 ml Tetrahydrofuran getropft. Nach 1 Stunde Rühren wurde das Reaktionsgemisch mit Wasser verdünnt, mit Methyl-t.-butylether extrahiert und mit Diethylether verrührt. Anschließend die unlöslichen Bestandteile abgetrennt, das Filtrat eingeengt und der Rückstand an Kieselgel (Eluent: Cyclohexan : Essigsäureethylester = 9:1) chromatographiert. Man erhielt 12,2 g (78 % der Theorie) 2,4-Dichlor-3-(2-methoxy-ethan-1-yl)-benzoesäureethylester.
   Stufe b) 2,4-Dichlor-3-(2-oxo-eth-1-yl)-benzoesäuremethylester
      Zu 2,6 g (10 mmol) 2,4-Dichlor-3-(2-oxo-eth-1-yl)-benzoesäuremethylester in 80 ml Dioxan wurden 6 ml 85 %ige Phosphorsäure und 6 ml H₂O getropft. Nach 12 Stunden Erhitzen unter Rückfluß wurde 12 Stunden bei Raumtemperatur gerührt, dann das Lösungsmittel entfernt, der Rückstand in Essigsäureethylester aufgenommen, mit 10 %iger Natriumhydrogencarbonatlösung gewaschen, getrocknet und eingeengt. Der Rückstand wurde an Kieselgel (Eluent: Cyclohexan : Essigsäureethylester = 9:1) chromatographiert. Man erhielt 1,4 g (57 % der Theorie) 2,4-Dichlor-3-(2-oxo-eth-1-yl)benzoesäuremethylester.
4-[2,4-Dichlor-3-(3-methyl-isoxazol-5-yl)benzoyl]-5-hydroxy-1-methyl-1H-pyrazol (Verbindung 3.52)
   Stufe a) 2.4-Dichlor-3-(2-oxo-but-3-en-4-yl)benzoesäuremethylester
      Zu 53,2 g (150 mmol) (2-Oxopropyl)triphenylphosphonium-chlorid in 300 ml Tetrahydrofuran wurden bei Raumtemperatur zuerst 14,0 g (125 mmol) Kalium-t-butylat und nach 30 Minuten 23,3 g (100 mmol) 2,4-Dichlor-3-formyl-benzoesäuremethylester in 200 ml Tetrahydrofuran gegeben. Nach 4,5 Stunden Rühren bei Raumtemperatur wurden 400 ml Wasser zugegeben, die organische Phase abgetrennt und die wäßrige Phase mit Methyl-t-butylether extrahiert. Die vereinigten organischen Phasen wurden getrocknet, eingeengt und der resultierende Rückstand an Kieselgel chromatographiert (Eluent: Cyclohexan : Essigsäureethylester = 9:1). Man erhielt 24,0 g (88 % der Theorie) 2,4-Dichlor-3-(2-oxo-but-3-en-4-yl)benzoesäuremethylester.
   Stufe b) 2,4-Dichlor-(2-hydroxyimino-but-3-en-4-yl)benzoesäuremethylester
      Zu 5,0 g (18,3 mmol) 2,4-Dichlor-3-(2-oxo-but-3-en-4-yl)-benzoesäuremethylester in 160 ml Ethanol wurden 1,8 g (25,9 mmol) Hydroxylamin-Hydrochlorid und 1,5 g (11,0 mmol) Kaliumcarbonat gegeben und das Reaktionsgemisch mit Wasser versetzt, bis eine klare Lösung entstand. Nach 3 Stunden Erhitzen unter Rückfluß wurde abgekühlt, das Reaktionsgemisch in 400 ml Wasser aufgenommen und mit Essigsäureethylester extrahiert. Die vereinigten organischen Phasen wurden getrocknet und das Lösungsmittel entfernt. Man erhielt 2,4-Dichlor-(2-hydroxyimino-but-3-en-4-yl)benzoesäuremethylester in quantitativer Ausbeute.
   Stufe c) 2,4-Dichlor-3-(3-methylisoxazol-5-yl)benzoesäuremethylester
      Zu 4,0 g (13,9 mmol) 2,4-Dichlor-(2-hydroxyimino-but-3-en-4-yl)benzoesäuremethylester in 100 ml Tetrahydrofuran wurden 4,7 g (55,6 mmol) Natriumhydrogencarbonat in 50 ml Wasser zugegeben. Unter Ausschluß von Licht wurden dann 7,9 g (47,8 mmol) Kaliumiodid und 3,7 g (14,6 mmol) Iod in 50 ml Wasser zugegeben und 4 Stunden unter Rückfluß erhitzt. Nach Abkühlen wurden portionsweise 100 ml 24 %ige Natriumpyrosulfit-Lösung zugegeben, mit Diethylether extrahiert und eingeengt. Der Rückstand wurde anschließend an Kieselgel chromatographiert (Eluent: Cyclohexan : Essigsäureethylester = 9:1). Man erhielt 2,4 g (60 % der Theorie) 2,4-Dichlor-3-(3-methylisoxazol-5-yl)-benzoesäuremethylester.
   Stufe d) 2,4-Dichlor-3-(3-methylisoxazol-5-yl)benzoesäure
      Zu 2,3 g (8,0 mmol) 2,4-Dichlor-3-(3-methylisoxazol-5-yl)benzoesäuremethylester in einem Gemisch von 50 ml Methanol und 50 ml Tetrahydrofuran wurden 0,35 g (8,8 mmol) Natriumhydroxid in 35 ml Wasser gegeben. Nach 12 Stunden Rühren bei Raumtemperatur wurde das Lösungsmittel entfernt und der Rückstand in Essigsäureethylester/Wasser aufgenommen. Nach Phasentrennung wurde die organische Phase abgetrennt und die wäßrige Phase mit Essigsäureethylester gewaschen. Nach Ansäuern der verbleibenden wäßrigen Phase wurde diese mit Essigsäureethylester extrahiert,; die resultierende organische Phase getrocknet und eingeengt. Man erhielt 2,1 g (96 % der Theorie) 2,4-Dichlor-3-(3-methylisoxazol-5-yl)benzoesäure.
   Stufe e) 2,4-Dichlor-3-(3-methylisoxazol-5-yl)benzoylchlorid
      Zu 2,0 g (7,35 mmol) 2,4-Dichlor-3-(3-methylisoxazol-5-yl)benzoesäure in 50 ml Toluol wurden 1 Tropfen Dimethylformamid und 1,1 g (9,5 mmol) Thionylchlorid gegeben. Nach 2 Stunden Erhitzten unter Rückfluß wurde abgekühlt und das Lösungsmittel entfernt.
   Stufe f) 4-[2,4-Dichlor-3-(3-methylisoxazol-5-yl)benzoyl]-5-hydroxy-1-methyl-1H-pyrazol
      Zu 0,7 g (7,35 mmol) B-Hydroxy-1-methyl-1H-pyrazol in 30 ml Dimethoxyethan und 2,0 g (14,7 mmol) Kaliumcarbonat wurden bei 0 bis 5°C das oben erhaltene 2,4-Dichlor-3-(3-methylisoxazol-5-yl)benzoylchlorid (Stufe e) in 40 ml Dimethoxyethan gegeben. Nach 3,5 Stunden Rühren bei Raumtemperatur wurde 1 Stunde unter Rückfluß erhitzt und 12 Stunden bei Raumtemperatur gerührt. Der sich bildende Niederschlag wurde abgesaugt und in 50 ml Wasser eingetragen. Nach Ansäuern auf pH = 1 wird der Feststoff abgesaugt und getrocknet.
      Ebenso wurde das erstgenannte Filtrat in 400 ml Wasser aufgenommen, mit Methyl-t-butylether gewaschen, auf pH 3 gestellt und mit Methylenchlorid extrahiert. Die vereinigten organischen Phasen wurden getrocknet und eingeengt. Man erhielt 1,9 g (73 % der Theorie) 4-[2,4-Dichlor-3-(3-methylisoxazol-5-yl)benzoyl]-5-hydroxy-1-methyl-1H-pyrazol.
      (Fp.: 143-144°C)

In den nachfolgenden Tabellen 3 bzw. 4 sind neben den voranstehend beschriebenen Verbindungen weitere Verbindungen der Formel III bzw. Benzoesäurederivate der Formel VI aufgeführt, die in analoger Weise hergestellt wurden oder herstellbar sind:

**Tabelle 3:**

| | | | | | | | |
|---|---|---|---|---|---|---|---|
| 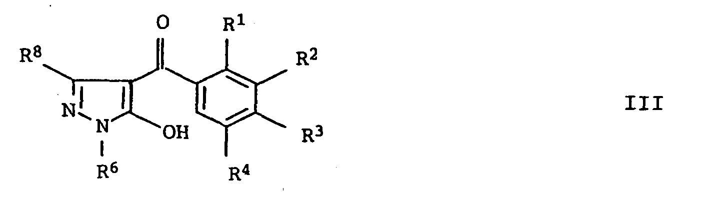 | | | | | | | |

| Nr. | R¹ | R² | R³ | R⁴ | R⁶ | R⁸ | physikalische Daten Fp. [°C]; ¹H-NMR [δ in ppm] |
|---|---|---|---|---|---|---|---|
| 3.1 | Cl | 4,5-Dihydroisoxazol-3-yl | Cl | H | n-C₄H₉ | H | 116-117 |
| 3.2 | Cl | 4,5-Dihydroisoxazol-3-yl | Cl | H | i-C₄H₉ | H | 148-151 |
| 3.3 | Cl | 5-Ethoxycarbonyl-4,5-dihydroisoxazol-3-yl | SO₂CH₃ | H | CH₃ | H | 70-75 |
| 3.4 | Cl | 5-Ethoxycarbonyl-4,5-dihydroisoxazol-3-yl | SO₂CH₃ | H | C₂H₅ | H | 65-70 |
| 3.5 | Cl | 5-Methyl-4,5-dihydroisoxazol-3-yl | SO₂CH₃ | H | CH₃ | H | 230-235 |
| 3.6 | Cl | 5-Methyl-4,5-diliydroisoxazol-3-yl | SO₂CH₃ | H | C₂H₅ | H | 210-215 |
| 3.7 | Cl | 5-Melhyl-4,5-dihydroisoxazol-3-yl | SO₂CH₃ | H | n-C₃H₇ | H | 95 - 100 |
| 3.8 | Cl | 5,5-Dimethyl-4,5-dihydroisoxazol-3-yl | SO₂CH₃ | H | CH₃ | H | 220 - 225 |
| 3.9 | Cl | 5,5-Dimethyl-4,5-dihydroisoxazol-3-yl | SO₂CH₃ | H | C₂H₅ | H | 82 - 86 |
| 3.10 | Cl | 5,5-Dimethyl-4,5-dihydro-isoxazol-3-yl | SO₂CH₃ | H | n-C₃H₇ | H | 70-75 |
| 3.11 | Cl | 5,5-Dimethyl-4,5-dihydro-isoxazol-3-yl | SO₂CH₃ | H | n-C₄H₉ | H | 68-73 |
| 3.12 | Cl | 5,5-Dimethyl-4,5-dihydro-isoxazol-3-yl | SO₂CH₃ | H | i-C₄H₉ | H | 45 - 50 |
| 3.13 | Cl | 5-Ethyl-4,5-dihydroisoxazol-3-yl | SO₂CH₃ | H | CH₃ | H | 220 - 225 |
| 3.14 | Cl | 5-Ethyl-4,5-dihydroisoxazol-3-yl | SO₂CH₃ | H | C₂H₅ | H | 170 - 175 |
| 3.15 | Cl | 4,5-Dihydroisoxazol-3-yl | SO₂CH₃ | H | n-C₃H₇ | H | 65 - 70' |
| 3.16 | Cl | 4,5-Dihydroisoxazol-3-yl | SO₂CH₃ | H | n-C₄H₉ | H | 55 - 60 |
| 3.17 | Cl | 4,5-Dihydroisoxazol-3-yl | SO₂CH₃ | H | i-C₄H₉ | H | 58 - 63 |
| 3.18 | Cl | 4,5-Dihydroisoxazol-3-yl | Cl | H | n-C₃H₇ | H | 119 - 121 |
| 3.19 | Cl | 4,5-Dihydroisoxawl-3-yl | Cl | H | CH₃ | CH₃ | 115 - 117 |
| 3.20 | Cl | 4,5-Dihydroisoxazol-3-yl | NO₂ | H | C₂H₅ | H | 217 - 218 |
| 3.21 | Cl | 4,5-Dihydroisoxazol-3-yl | SO₂CH₃ | H | CH₃ | H | >250 |
| 3.22 | Cl | 4,5-Dihydroisoxazol-3-yl | Cl | H | C₂H₅ | H | 125 - 128 |
| 3.23 | Cl | 5,5-Dimethyl-4,5-dihydro-isoxazol-3-yl | SO₂C₂H₅ | H | CH₃ | H | > 200 |
| 3.24 | Cl | 5-Methyl-4,5-dihydroisoxazol-3-yl | SO₂C₂H₅ | H | CH₃ | H | 220 - 223 |
| 3.25 | Cl | 5-Methyl-4,5-dihydroisoxazol-3-yl | SO₂C₂H₅ | H | C₂H₅ | H | > 230 |
| 3.26 | Cl | 5-Methyl-4,5-dihydroisox-azol-3-yl | SO₂-n-C₃H₇ | H | CH₃ | H | 1,12(t); 1,53(d); 1,76(quin); 3,18(dd); 3,38(t); 3,55(dd); 3,73(s); 5,04(m); 5,55(s,br.); 7,37(s); 7,68(d); 8,13(d). |
| 3.27 | Cl | 5-Methyl-4,5-dihydroisexazol-3-yl | SO₂-n-C₃H₇ | H | C₂H₅ | H | 1,07(t); 1,50(m); 1,78(quin); 3,07(dd); 3,39(t); 3,55(dd); 4,12(t); 5,08(m); 7,38(s); 7,69(d); 8,11(d). |
| 3.28 | Cl | 4,5-Dihydrooxazol-2-yl | SO₂CH₃ | H | CH₃ | H | |
| 3.29 a) | Cl | 4,4-Dimethyl-4,5-dihydrooxazol-2-yl | SO₂CH₃ | H | CH₃ | H | 1,33(s); 3,40(s); 4,17(s); 7,43(s); 7,79(d); 8,04(d). |
| 3.30 a) | Cl | 4,4-Dimethyl-4,5-dihydrooxazol-2-yl | SO₂CH₃ | H | C₂H₅ | H | 1,27(t); 1,36(s); 3,41(q); 4,01(q); 4,18(s); 7,47(s); 7,83(d); 8,07(d). |
| | | | | | | | |
| | | | | | | | |
| 3.33 | Cl | 4,5-Dihydro-isoxazol-5-spiro-cyclopentan-3-yl | SO₂CH₃ | H | CH₃ | H | 230-235 |
| 3.34 | Cl | 4,5-Dihydro-isoxazol-5-spiro-cyclopentan-3-yl | SO₂CH₃ | H | C₂H₅ | H | 190-195 |
| | | | | | | | |
| 3.36 | Cl | 5,5-Dimethyl-4,5-dihydroisoxazol-3-yl | SO₂C₂H₅ | H | CH₃ | H | > 230 |
| 3.37 | Cl | 5,5-Dimethyl-4,5-dihydroisoxazol-3-yl | SO₂C₂H₅ | H | C₂H₅ | H | 198-200 |
| 3.38 | Cl | 4,5-Dihydroisoxazol-3-yl | SO₂C₂H₅ | H | CH₃ | H | 215-218 |
| 3.39 | Cl | 4,5-Dihydroisoxazol-3-yl | SO₂C₂H₅ | H | C₂H₅ | H | 213-215 |
| 3.40 | Cl | 4,5-Dihydroisoxazol-3-yl | SO₂-n-C₃H₇ | H | CH₃ | H | 186-190 |
| 3.41 | Cl | 4,5-Dihydroisoxazol-3-yl | SO₂-n-C₃H₇ | H | C₂H₅ | H | 84-86 |
| | | | | | | | |
| 3.43 | Cl | 5,5-Diethyl-4,5-dihydro-isoxazol-3-yl | SO₂CH₃ | H | CH₃ | H | 70-75 |
| 3.44 | Cl | 5,5-Diethyl-4,5-dihydro-isoxazol-3-yl | SO₂CH₃ | H | C₂H₅ | H | 50-55 |
| | | | | | | | |
| 3.46 | Cl | 1,3-Dithiolan-2-yl | Cl | H | C₂H₅ | H | 1,46 (t); 3,28 (m); 3,67 (m); 4,10 (q); 6,80 (s); 7,24 (d); 7,36 (5); 7,36 (d) |
| | | | | | | | |
| | | | | | | | |
| | | | | | | | |
| | | | | | | | |
| | | | | | | | |
| 3.52 | Cl | 3-Methyl-isoxazol-5-yl | Cl | H | CH₃ | H | 143-144 |
| 3.53 | Cl | 3-Methyl-isoxazol-5-yl | SO₂CH₃ | H | CH₃ | H | 102-108 |
| | | | | | | | |
| 3.55 | Cl | 1,3-Dithiolan-2-yl | Cl | H | CH₃ | H | 64-67 |
| 3.56 | Cl | 4-tert.-Butyl-thiazol-2-yl | SO₂CH₃ | H | C₂H₅ | H | 1,40 (s); 1,48 (t); 3,28 (s); 4,09 (q); 7,27 (s); 7,40 (s); 7,71 (d); 8,28 (d) |
| 3.57 | Cl | 2-Thiazolyl | Cl | H | C₂H₅ | H | 1,39 (t); 3,96 (q); 7,39 (m); 7,60 (d); 8.,01 (d) |
| 3.58 | Cl | 3-tert.-Butyl-isoxazol-4-yl | Cl | H | CH₃ | H | 1,4 (s); 3,7 (s); 6,4 (s); 7,4 (s); 7,5 (d); 7,6 (d) |
| | | | | | | | |
| | | | | | | | |
| 3.61 | Cl | 5-Methoxyethyl-isoxazol-3-yl | SO₂CH₃ | H | CH₃ | H | 80-85 |
| 3.62 | Cl | 5-Methoxyethyl-isoxazol-3-yl | SO₂CH₃ | H | C₂H₅ | H | 170-175 |
| 3.63 | Cl | 4,5-Dimethyl-isoxazol-3-yl | SO₂CH₃ | H | CH₃ | H | 95-100 |
| 3.64 | Cl | 5-(n-Propyl)-isoxazol-3-yl | SO₂CH₃ | H | CH₃ | H | 65-70 |
| 3.65 | Cl | 5-(t-Butyl)-isoxazol-3-yl | SO₂CH₃ | H | CH₃ | H | 225-230 |
| 3.66 | Cl | 5-(t-Butyl)-isoxazol-3-yl | SO₂CH₃ | H | C₂H₅ | H | 70-75' |
| 3.67 | Cl | 4,5-Diethyl-isoxazol-3-yl | SO₂CH₃ | H | CH₃ | H | 125-130 |
| 3.68 | Cl | 4,5-Dielhyl-isoxazol-3-yl | SO₂CH₃ | H | C₂H₅ | H | 70-75 |
| 3.69 | Cl | 5-(n-Propyl)-isoxazol-3-yl | SO₂CH₃ | H | C₂H₅ | H | 60-65 |
| 3.70 | Cl | 4,5-Dimethyl-isoxazol-3-yl | SO₂CH₃ | H | C₂H₅ | H | 75-80 |
| 3.71 | Cl | 5-Chlormethyl-isoxazol-3-yl | SO₂CH₃ | H | C₂H₅ | H | 55-60 |
| 3.72 | Cl | 5-Ethoxy-isoxazol-3-yl | SO₂CH₃ | H | CH₃ | H | 80-85 |
| 3.73 | Cl | 5-Ethoxy-isoxazol-3-yl | SO₂CH₃ | H | CH₃ | H | 70-75 |
| 3.74 | Cl | 5-Methyl-isoxazol-3-yl | SO₂CH₃ | H | C₂H₅ | H | 232-234 |
| 3.75 | Cl | 5-(2-Mcthylpropyl)-isoxazol-3-yl | SO₂CH₃ | H | C₂H₅ | H | 0,95 (d); 1,28 (t); 2,04 (m); 2,73 (d); 3,31 (s); 3,91 (q); 6,54 (s); 7,50 (s); 7,82 (d); 8,14 (d) |
| 3.76 | Cl | 5-(2-Methylpropyl)-isoxazol-3-yl | SO₂CH₃ | H | CH₃ | H | 0,94 (d); 2,03 (m); 2,73 (d); 3,30 (s); 3,52 (s); 6,52 (s); 7,47 (s); 7,81 (d); 8,13 (d) |
| 3.77 | Cl | 5-Methyl-isoxazol-3-yl | SO₂CH₃ | H | CH₃ | H | 190-194 |

| | | | | | | | |
|---|---|---|---|---|---|---|---|
| ^{a)} Hergestellt aus 2-Chlor-3-(1'-chlor-2',2'-dimethylethylaminocarbonyl)-4-methylsulfonyl-benzoylchlorid mit zwei Äquivalenten Kaliumcarbonat | | | | | | | |

**Tabelle 4:**

| | | | | | | |
|---|---|---|---|---|---|---|
| 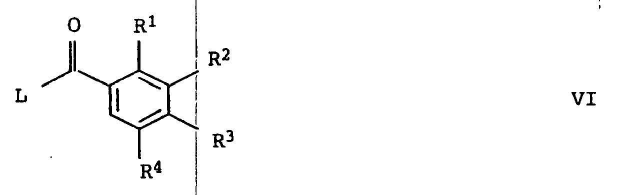 | | | | | | |

| Nr. | R¹ | R² | R³ | R⁴ | L | physikalische Daten Fp. [°C]; ¹H=NMR [δ in ppm], |
|---|---|---|---|---|---|---|
| 4.1 | Cl | 4,5-Dihydroisoxazol-3-yl | Cl | H | OCH₃ | 3,29 (t); 3,91 (s); 4,58 (t); 7,46 (d); 7,83 (d). |
| 4.2 | Cl | 4,5-Dihydroisoxazol-3-yl | Cl | H | OH | 3,28 (t); 4,60 (t); 7,02 (s, br); 7,46 (d); 7,98 (d). |
| 4.3 | Cl | 4,5-Dihydroisoxazol-3-yl | SO₂CH₃ | H | OCH₃ | 3,24 (s); 3,42 (t); 3,99 (s); 4,60 (t); 7,96 (d); 8,10 (d). |
| 4.4 | Cl | 4,5-Dihydroisoxazol-3-yl | SO₂CH₃ | H | OH | 3,26 (s); 3,45 (t); 4,63 (t); 8,15 (s); 8,53 (s, br). |
| 4.5 | Cl | 4,5-Dihydroisoxazol-3-yl | SO₂CH₃ | H | Cl | 3,25 (s); 3,46 (t); 4,62 (t); 8,21 (dd). |
| 4.6 | Cl | 4,4-Dimethyl-4,5-dihydrooxazol-2-yl | Cl | H | OH | 1,31 (s); 4,16 (s); 7,69 (d); 7,90 (d); 13,8 (s, br). |
| 4.7 | Cl | 5,5-Dimethyl-4,5-dihydroisoxazol-3-yl | SO₂C₂H₅ | H | OCH₃ | 1,25 (t); 1,57 (s); 3,21 (s); 3,42 (q); 3,99 (s); 7,94 (d); 8,07 (d). |
| 4.8 | Cl | 5,5-Dimelhyl-4,5-dihydroisoxazol-3-yl | SO₂C₂H₅ | H | OH | 1,13 (t); 1,47 (s); 3,15 (s); 3,43 (q); 8,06 (s); 13,8 (s, br). |
| 4.9 | Cl | 4,5-Dihydroisoxazol-3-yl | SO₂C₂H₅ | H | OCH₃ | 1,28 (t); 3,41 (m); 4,02 (s); 4,62 (t); 7,95 (d); 8,06 (d). |
| 4.10 | Cl | 4,5-Dihydroisoxazol-3-yl | SO₂C₂H₅ | H | OH | 137-140 |
| 4.11 | Cl | 5-Methyl-4,5-dihydroisoxazol-3-yl | SO₂C₂H₅ | H | OCH₃ | 1,26 (t); 1,53 (d); 3,06 (dd); 3,42 (q); 3,49 (dd); 5,05 (m); 7,95 (d); 8,07 (d). |
| 4.12 | Cl | 5-Methyl-4,5-dihydroisoxazol-3-yl | SO₂C₂H₅ | H | OH | Fp. [°C], 1H-NMR [δ in ppm] 140-143 |
| 4.13 | Cl | 4,5-Dihydrooxazol-2-yl | SO₂CH₃ | H | OCH₃ | 3,30 (s); 3,98 (s); 4,11 (t); 4,55 (t); 7,97 (d); 8,08 (d). |
| 4.14 | Cl | 4,5-Dihydrooxazol-2-yl | SO₂CH₃ | H | OH | 3,38 (s); 4,00 (t); 4,46 (t); 8,08 (s). |
| 4.15 | Cl | 4,5-Dihydroisoxazol-3-yl | SO₂CH₃ | H | OH | 3,30 (s); 3,35 (t); 4,15 (s, br); 4,50 (t); 8,05 (s). |
| 4.16 | Cl | 5,5-Dimethyl-4,5-dihydroisoxazol-3-yl | SO₂-n-C₃H₇ | H | OCH₃ | 0,95 (t); 1,47 (s); 1,58 (quin); 3,12 (s); 3,31 (s); 3,43 (t); 3,93 (s); 8,09 (dd). |
| 4.17 | Cl | 5,5-Dimethyl-4,5-dihydroisoxazol-3-yl | SO₂-n-C₃H₇ | H | OH | 0,93 (t); 1,47 (s); 1,58 (quin); 3,15 (s); 3,42 (t); 8,05(s). |
| 4.18 | Cl | 4,5-Dihydroisoxazol-3-yl | SO₂-n-C₃H₇ OCH₃ | H | OCH₃ | 0,92 (t); 1,55 (quin); 3,39 (m); 3,93 (s); 4,50 (t); 8,08 (dd). |
| 4.19 | Cl | 4,5-Dihydroisoxazol-3-yl | SO₂-n-C₃H₇ | H | OH | 148-150 |
| 4.20 | Cl | 5-Methyl-4,5-dihydroisoxazol-3-yl | SO₂-n-C₃H₇ | H | OCH₃ | 0,93 (t); 1,49 (d); 1,58 (quin); 2,94 (dd); 3,42 (m); 3,93 (s); 4,97 (m); 8,10 (dd). |
| 4.21 | Cl | 5-Methyl-4,5-dihydroisoxazol-3-yl | SO₂-n-C₃H₇ | H | OH | 0,94 (t); 1,39 (d); 1,58 (quin); 2,96 (dd); 3,50 (m); 4,95 (m); 8,05 (s). |
| | | | | | | |
| 4.23 | Cl | 5-Ethoxycarbonyl-4,5-dihydroisoxazol-3-yl | SO₂CH₃ | H | OCH₃ | 118-121 |
| 4.24 | Cl | 5-Ethoxycarbonyl-4,5-dihydroisoxazol-3-yl | SO₂CH₃ | H | OH | |
| 4.25 | Cl | 5-Methyl-4,5-dihydroisoxazol-3-yl | SO₂CH₃ | H | OCH₃ | 130-135 |
| 4.26 | Cl | 5-Methyl-4,5-dihydroisoxazol-3-yl | SO₂CH₃ | H | OH | 173-178 |
| 4.27 | Cl | 5,5-Dimethyl-4,5-dihydroisoxazol-3-yl | SO₂CH₃ | H | OCH₃ | 1,57 (s); 3,18 (s); 3,27 (s); 4,01 (s); 7,97 (d); 8,12 (d). |
| 4.28 | Cl | 5,5-Dimethyl-4,5-dihydroisoxazol-3-yl | SO₂CH₃ | H | OH | 1,48 (s); 3,15 (s); 3,34 (s); 8,08 (dd). |
| 4.29 | Cl | 5-Ethyl-4,5-dihydroisoxazol-3-yl | SO₂CH₃ | H | OCH₃ | 0,97 (t); 1,72 (m); 3,10 (dd); 3,32 (s); 3,37 (dd); 4,72 (m); 8,08 (dd). |
| | | | | | | |
| | | | | | | |
| | | | | | | |
| | | | | | | |
| 4.34 | Cl | 4,5-Dihydro-isoxazol-5-spiro-cyclpentan-3-yl | SO₂CH₃ | H | OCH₃ | 1,78 (m); 2,24 (m); 3,27 (s); 3,36 (s); 3,98 (s); 7,94 (d); 8,12 (d). |
| 4.35 | Cl | 4,5-Dihydro-isoxazol-5-spiro-cyclpentan-3-yl | S02CH3 | H | OH | 1,76 (m); 2,05 (m); 3,30 (s); 3,33 (s); 8,09 (dd) |
| 4.36 | Cl | 5,5-Diethyl-4,5-dihydroisoxazol-3-yl | SO₂CH₃ | H | OCH₃ | 1,00 (t); 1,85 (m); 3,13 (s); 3,27 (s); 3,98 (s); 7,94 d); 8,11 (d). |
| 4.37 | Cl | 5,5-Diethyl-4,5-dihydroisoxazol-3-yl | SO₂CH₃ | H | OH | 0,91(t); 1,76 (m); 3,12 (s); 3,33 (s); 8,07 (dd); 13,75 (s, br). |
| 4.38 | Cl | 4,4-Dimethyl-4,5-dihydrooxazol-2-yl | SO₂CH₃ | H | OH | 1,34 (s); 3,40 (s); 4,13 (s); 8,07 (s); 13,95 (s, br). |
| 4.39 | Cl | 5-Methyl-4,5-dihydroisoxazol-3-yl | SO₂CH₃ | H | Cl | |
| | | | | | | |
| | | | | | | |
| 4.42 | Cl | 1,3-Dithiolan-2-yl | Cl | H | OCH₃ | 3,44 (m); 3,68 (m); 3,93 (s); 6,80 (s); 7,38 (d); 7,52(d) |
| 4.43 | Cl | 1,3-Dithiolan-2-yl | Cl | H | OH | 195-198 |
| | | | | | | |
| | | | | | | |
| 4.46 | Cl | 5-(cyclo-Propyl)-isoxazol-3-yl | SO₂CH₃ | H | OH | 1,14 (4H); 2,12 (1H); 3,19 (3H); 6,12 (1H); 8,07 (1H); 8,16 (1H) |
| 4.47 | Cl | 5-Trifluormelhyl-isoxazol-3-yl | SO₂CH₃ | H | OCH₃ | 3,22 (3H); 4,01 (3H); 6,92 (1H); 8,07 (1H); 8,20 (1H) |
| | | | | | | |
| 4.49 | Cl | 5-(n-Propyl)-isoxazol-3-yl | SO₂CH₃ | H | OCH₃ | 1,05 (3H); 1,83 (2H); 2,86 (2H); 3,23 (3H); 3,96 (3H); 6,20 (1H); 7,96 (1H); 8,20 (1H) |
| 4.50 | Cl | 5-(n-Propyl)-isoxazol-3-yl | SO₂CH₃ | H | OH | 0,95 (3H), 1,72 (2H); 2,82 (2H); 3,30 (3H); 6,54 (1H); 8,09 (1H); 8,15 (1H) |
| 4.51 | Cl | 5-(t-Butyl)-isoxazol-3-yl | SO₂CH₃ | H | OCH₃ | 115-120 |
| 4.52 | Cl | 5-(t-Butyl)-isoxazol-3-yl | SO₂CH₃ | H | OH | 160-165 |
| | | | | | | |
| | | | | | | |
| | | | | | | |
| 4.56 | Cl | Oxazol-2-yl | SO₂CH₃ | H | OCH₃ | 177 |
| 4.57 | Cl | 5-Methoxymethyl-isoxazol-3-yl | SO₂CH₃ | H | OCH₃ | 3,21 (3H); 3,47 (3H); 3,98 (3H); 4,69 (2H); 6,49 (1H); 7,99 (1H); 8,20 (1H) |
| 4.58 | Cl | 5-Methoxymethyl-isoxazol-3-yl | SO₂CH₃ | H | OH | 3,30 (3H); 3,37 (3H); 4,66 (2H); 6,83 (1H); 8,13 (1H); 8,18 (1H) |
| | | | | | | |
| | | | | | | |
| | | | | | | |
| 4.62 | Cl | 5-Methyl-isoxazol-3-yl | SO₂CH₃ | H | OCH₃ | 127 |
| 4.63 | Cl | 5-Methyl-isoxazol-3-yl | SO₂CH₃ | H | OH | 148-150 |
| 4.64 | Cl | 4,5-Dimethyl-isoxazol-3-yl | SO₂CH₃ | H | OCH₃ | 120-125 |
| 4.65 | Cl | 4,5-Dimethyl-isoxazol-3-yl | SO₂CH₃ | H | OH | 85-90 |
| 4.66 | Cl | 4,5-Diethyl-isoxazol-3-yl | SO₂CH₃ | H | OCH₃ | 90-95 |
| 4.67 | Cl | 4,5-Diethyl-isoxazol-3-yl | SO₂CH₃ | H | OH | 0,90 (3H);1,26 (3H); 2,20 (2H); 2,83 (2H); 3,30 (3H); 8,12 (1H); 8,18 (1H) |
| 4.68 | Cl | 5-(cyclo-Propyl)-isoxazol-3-yl | SO₂CH₃ | H | OCH₃ | 1,10 (4H); 2,15 (1H); 3,22 (3H); 3,98 (3H); 6,12 (1H); 7,95 (1H); 8,18 (1H) |
| | | | | | | |
| | | | | | | |
| 4.71 | Cl | 4,5-Dihydrothiazol-2-yl | SO₂CH₃ | H | OH | 3,31 (s); 3,60 (t); 4,38 (t); 8,04 (d); 8,10 (d) |
| | | | | | | |
| | | | | | | |
| 4.74 | Cl | 5-(2-Methylpropyl)-isoxazol-3-yl | SO₂CH₃ | H | OCH₃ | 1,01 (d); 2,13 (sept); 2,75 (d); 3,24 (s); 3,99 (s); 6,19 (d); 7,97 (d); 8,20 (d) |
| 4.75 | Cl | 5-(2-Methylpropyl)-isoxazol-3-yl | SO₂CH₃ | H | OH | 0,96 (d); 2,03 (sept); 2,74 (d); 3,29 (s); 6,53 (s); 8,08 (d); 8,13 (d) |
| | | | | | | |
| 4.77 | Cl | 5-Chlormethyl-isoxazot-3-yl | SO₂CH₃ | H | OH | 3,30(s); 3,36 (s); 4,66 (s); 5,06 (s); 6,82 (s); 6,93 (s); 8,10 (d); 8,16 (d) |
| 4.78 | Cl | 5-Ethoxy-isoxazol-3-yl | SO₂CH₃ | H | OH | 1,39 (t); 3,33 (s); 4,33 (q); 5,95 (s); 8,07 (d); 8,16 (d) |
| | | | | | | |
| 4.81 | Cl | 5-Ethoxysoxazol-3-yl | SO₂CH₃ | H | OCH₃ | 1,50 (t); 3.28 (s); 3.98 (s); 4.36 (q); 5,43 (s); 7,95 (d); 8,16 (d) |
| 4.82 | Cl | 5-Chlormethylisoxazol-3-yl | SO₂CH₃ | H | OCH₃ | 115-120 |

Die 4-(3-Heterocyclyl-1-benzoyl)pyrazole der Formel I und deren landwirtschaftlich brauchbaren Salze eignen sich - sowohl als Isomerengemische als auch in Form der reinen Isomeren - als Herbizide. Die herbiziden Mittel, die Verbindungen der Formel I enthalten, bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel.enthalten eine herbizid wirksame Menge mindestens einer Verbindung der Formel I oder eines landwirtschaftlich brauchbaren Salzes von I und für die Formulierung von Pflanzenschutzmitteln übliche Hilfsmittel.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline und deren Derivate, alkylierte Benzole oder deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 4-(3-Heterocyclyl-1-benzoyl)pyrazole als solche oder in einem Ö1 oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Im allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:
I. 20 Gewichtsteile der Verbindung Nr. 2.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile der Verbindung Nr. 2.6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichts-teilen Wasser erhält man eine wäßrige Dispersion, die 0,02 Gew.-% des Wirkstoffs enthält.
III. 20 Gewichtsteile des Wirkstoffs Nr. 2.18 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile des Wirkstoffs Nr. 2.27 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20000 Gewichtsteilen Wasser erhält man eine Spritzbrühe, die 0,1 Gew.-% des Wirkstoffs enthält.
V. 3 Gewichtsteile des Wirkstoffs Nr. 2.36 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.37 werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil des Wirkstoffs Nr. 2.45 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil des Wirkstoffs Nr. 2.48 wird in einer Mischung gelöst, die aus 80 Gewichtsteilen Cyclohexanon und 20 Gewichtsteilen Wettol^{®} EM 31 (= nichtionischer Emulgator auf der Basis von ethoxyliertem Rizinusöl) besteht. Man erhält ein stabiles Emulsionskonzentrat.

Die Applikation der Verbindungen der Formel I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Verbindung der Formel I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 4-(3-Heterocyclyl-1-benzoyl)pyrazole der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Amino-phosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen der Formel I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

### Anwendungsbeispiele

Die herbizide Wirkung der 4-(3-Heterocyclyl-1-benzoyl)pyrazole der Formel I ließ sich durch die folgenden Gewächshausversuche zeigen:
Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 62,5 bzw. 31,3 g/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Amaranthus retroflexus | Zurückgekrümmter Fuchsschwanz | redroot pigweed |
| Echinochloa crus-galli | Hühnerhirse | barnyard grass |
| Setaria faberii | Borstenhirse | giant foxtail |

Bei Aufwandmengen von 62,5 bzw. 31,3 g/ha zeigte die Verbindung 2.37 (Tabelle 2) im Nachauflauf eine sehr gute Wirkung gegen die oben genannten mono- und dicotylen Schadpflanzen.

## Patentansprüche

1. 4-(3-Heterocyclyl-1-benzoyl)pyrazole der Formel I 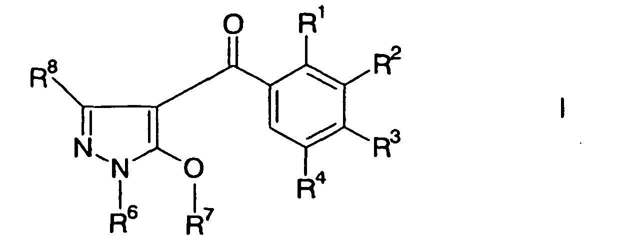 in der die Variablen folgende Bedeutungen haben:
R¹, R³ Nitro, Halogen, Cyano, C₁-C₆-Alkyl, C₁-C₆-Halogen-alkyl, C₁-C₆-Alkoxy, C₁-C₆-Halogenalkoxy, C₁-C₆-Alkylthio, C₁-C₆-Halogenalkylthio, C₁-C₆-Alkylsulfinyl, C₁-C₆-Halogenalkylsulfinyl, C₁-C₆-Alkylsulfonyl oder C₁-C₆-Halogenalkylsulfonyl;
R² für einen unsubstituierten oder durch einen oder zwei Reste aus folgender Gruppe:
C₁-C₄-Alkyl, C₃-C₆-Cycloalkyl, C₁-C₄-Halogenalkyl, C₁-C₄-Alkoxy-C₁-C₄-alkyl, C₁-C₄-Alkoxycarbonyl, C₁-C₄-Alkoxy oder C₃-C₆-Spirocycloalkan; substituierten 5-gliedrigen, C-verknüpften Heterocyclyl-Rest, enthaltend zwei gleiche oder verschiedene Heteroatome, ausgewählt aus folgender Gruppe: Sauerstoff, Schwefel oder Stickstoff;
R⁴ Wasserstoff, Halogen oder C₁-C₆-Alkyl;
R⁶ C₁-C₆-Alkyl;
R⁷ C₁-C₆-Alkyl, C₃-C₆-Alkenyl, C₃-C₆-Alkinyl, C₁-C₆-Alkylcarbonyl, C₂-C₆-Alkenylcarbonyl, C₁-C₆-Alkoxycarbonyl, Di-(C₁-C₆-alkyl)-aminocarbonyl, N-(C₁-C₆-Alkoxy)-N-(C₁-C₆-alkyl)-aminocarbonyl oderDi-(C₁-C₆-alkyl)-aminothiocarbonyl, wobei die genannten Alkyl- und Alkoxyreste partiell oder vollständig halogeniert sein können und/oder eine bis drei der folgenden Gruppen tragen können:
Cyano, C₁-C₄-Alkoxy, C₁-C₄-Alkoxycarbonyl, C₁-C₄-Alkoxy-C₁-C₄-alkoxycarbonyl, Di-(C₁-C₄-alkyl)-amino-C₁-C₄-alkoxycarbonyl, C₁-C₄-Alkylaminocarbonyl, Di-(C₁-C₄-alkyl)-aminocarbonyl oder C₁-C₄-Alkylcarbonyloxy;
Phenyl-C₁-C₆-alkyl, Phenylcarbonyl-C₁-C₆-alkyl, Phenylcarbonyl oder Phenyl-C₂-C₆-alkenylcarbonyl wobei der Phenyl-Rest der 4 letztgenannten Substituenten partiell oder vollständig halogeniert sein kann und/oder einen bis drei der folgenden Reste tragen kann:
Nitro, Cyano, C₁-C₄-Alkyl, C₁-C₄-Halogenalkyl, C₁-C₄-Alkoxy oder C₁-C₄-Halogenalkoxy;
R⁸ Wasserstoff, C₁-C₆-Alkyl oder C₁-C₆-Halogenalkyl;
sowie deren landwirtschaftlich brauchbaren Salze.

2. 4-(3-Heterocyclyl-1-benzoyl)pyrazole der Formel I nach Anspruch 1, wobei R⁴ Wasserstoff bedeutet.

3. Verfahren zur Herstellung von 4-(3-Heterocyclyl-1-benzoyl)pyrazolen der Formel I gemäß Anspruch 1, **dadurch gekennzeichnet, daß** man ein Benzoylderivat der Formel III, wobei die Variablen R¹ bis R⁴, R⁶ und R⁸ die unter Anspruch 1 genannten Bedeutungen haben, 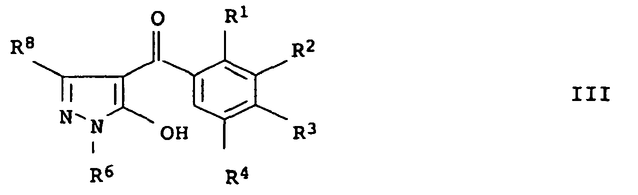 mit einer Verbindung der Formel IV
L¹-R⁷ IV
in der R⁷ die unter Anspruch 1 genannte Bedeutung hat und L¹ für eine nucleophil verdrängbare Abgangsgruppe steht, umsetzt.

4. Mittel, enthaltend eine herbizid wirksame Menge mindestens eines 4-(3-Heterocyclyl-1-benzoyl)pyrazols der Formel I oder eines landwirtschaftlich brauchbaren Salzes von I gemäß den Ansprüchen 1 bis 2, und für die Formulierung von Pflanzenschutzmitteln übliche Hilfsmittel.

5. Verfahren zur Herstellung von Mitteln gemäß Anspruch 4, **dadurch gekennzeichnet, daß** man eine herbizid wirksame Menge mindestens eines 4-(3-Heterocyclyl-1-benzoyl)pyrazols der Formel I oder eines landwirtschaftlich brauchbaren Salzes von I gemäß den Ansprüchen 1 bis 2 und für die Formulierung von Pflanzenschutzmitteln übliche Hilfsmittel mischt.

6. Verfahren zur Bekämpfung von unerwünschtem Pflanzenwuchs, **dadurch gekennzeichnet, daß** man eine herbizid wirksame Menge mindestens eines 4-(3-Heterocyclyl-1-benzoyl)pyrazols der Formel I oder eines landwirtschaftlich brauchbaren Salzes von I gemäß den Ansprüchen 1 bis 2, auf Pflanzen, deren Lebensraum und/oder auf Samen einwirken läßt.

7. Verwendung von 4-(3-Heterocyclyl-1-benzoyl)pyrazolen der Formel I oder deren landwirtschaftlich brauchbaren Salze gemäß den Ansprüchen 1 bis 2 als Herbizide.

## Claims

1. A 4-(3-heterocyclyl-1-benzoyl)pyrazole of the formula I 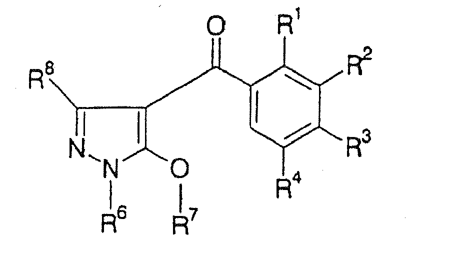 where:
R¹ and R³ are nitro, halogen, cyano, C₁-C₆-alkyl, C₁-C₆-haloalkyl, C₁-C₆-alkoxy, C₁-C₆-haloalkoxy, C₁-C₆-alkylthio, C₁-C₆-haloalkylthio, C₁-C₆-alkylsulfinyl, C₁-C₆-haloalkylsulfinyl, C₁-C₆-alkylsulfonyl or C₁-C₆-haloalkylsulfonyl;
R² is a 5-membered heterocyclyl radical which is attached via carbon, contains two identical or different heteroatoms selected from the following group: oxygen, sulfur or nitrogen, and which is unsubstituted or substituted by one or two radicals from the following group:
C₁-C₄-alkyl, C₃-C₆-cycloalkyl, C₁-C₄-haloalkyl, C₁-C₄-alkoxy-C₁-C₄-alkyl, C₁-C₄-alkoxycarbonyl, C₁-C₄-alkoxy or C₃-C₆-spirocycloalkane;
R⁴ is hydrogen, halogen or C₁-C₆-alkyl;
R⁶ is C₁-C₆-alkyl;
R⁷ is C₁-C₆-alkyl, C₃-C₆-alkenyl, C₃-C₆-alkynyl, C₁-C₆-alkylcarbonyl, C₂-C₆-alkenylcarbonyl, C₁-C₆-alkoxycarbonyl, di(C₁-C₆-alkyl)-aminocarbonyl, N-(C₁-C₆-alkoxy)-N-(C₁-C₆-alkyl)aminocarbonyl or di(C₁-C₆-alkyl)aminothiocarbonyl, where the alkyl and alkoxy radicals mentioned may be partially or fully halogenated and/or may carry one to three of the following groups:
cyano, C₁-C₄-alkoxy, C₁-C₄-alkoxycarbonyl, C₁-C₄-alkoxy-C₁-C₄-alkoxycarbonyl, di(C₁-C₄-alkyl)amino-C₁-C₄-alkoxycarbonyl, C₁-C₄-alkylaminocarbonyl, di(C₁-C₄-alkyl)aminocarbonyl, or C₁-C₄-alkylcarbonyloxy;
is phenyl-C₁-C₆-alkyl, phenylcarbonyl-C₁-C₆-alkyl, phenylcarbonyl or phenyl-C₂-C₆-alkenylcarbonyl where the phenyl radical of the last 4 substituents may be partially or fully halogenated and/or may carry one to three of the following radicals: nitro, cyano, C₁-C₄-alkyl, C₁-C₄-haloalkyl, C₁-C₄-alkoxy or C₁-C₄-haloalkoxy;
R⁸ is hydrogen, C₁-C₆-alkyl or C₁-C₆-haloalkyl;
and agriculturally useful salts thereof.

2. The 4-(3-heterocyclyl-1-benzoyl)pyrazole of the formula I according to claim 1 where R⁴ is hydrogen.

3. A process for preparing 4-(3-heterocyclyl-1-benzoyl)pyrazoles of the formula I as claimed in claim 1, which comprises reacting a benzoyl derivative of the formula III, where the variables R¹ to R⁴, R⁶ and R⁸ are each as defined under claim 1, 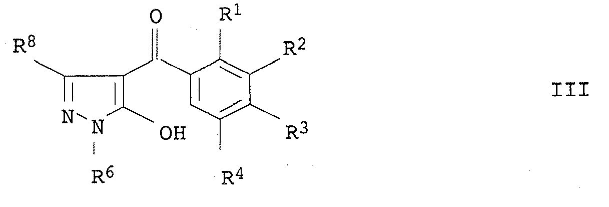 with a compound of the formula IV
L¹-R⁷ IV
in which R⁷ is as defined under claim 1 and L¹ is a nucleophilically displaceable leaving group.

4. A composition, which comprises a herbicidally effective amount of at least one 4-(3-heterocyclyl-1-benzoyl)pyrazole of the formula I or an agriculturally useful salt of I as claimed in claim 1 or 2, and auxiliaries which are customarily used for formulating crop protection agents.

5. A process for preparing compositions as claimed in claim 13, which comprises mixing a herbicidally effective amount of at least one 4-(3-heterocyclyl-1-benzoyl)pyrazole of the formula I or an agriculturally useful salt of I according to claim 1 or 2 and auxiliaries which are customarily used for formulating crop protection agents.

6. A method for controlling undesirable vegetation, which comprises allowing a herbicidally effective amount of at least one 4-(3-heterocyclyl-1-benzoyl)pyrazole of the formula I or an agriculturally useful salt of I as claimed in claim 1 or 2 to act on plants, their habitat and/or on seeds.

7. The use of the 4-(3-heterocyclyl-1-benzoyl)pyrazoles of the formula I or agriculturally useful salts thereof according to in claim 1 or 2 as herbicides.

## Revendications

1. 4-(3-Hétérocyclyl-1-benzoyl)pyrazoles de la formule I : 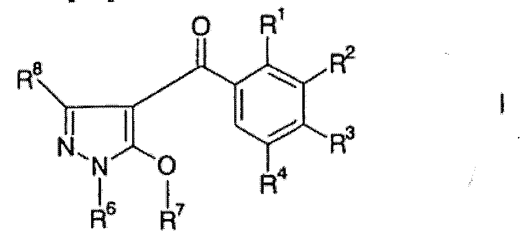 dans laquelle les variables ont les significations suivantes :
R¹, R³ représentent un halogène ou un groupe nitro, cyano, alkyle en C₁-C₆, halogénoalkyle en C₁-C₆, alcoxy en C₁-C₆, halogénoalcoxy en C₁-C₆, alkylthio en C₁-C₆, halogénoalkylthio en C₁-C₆, alkylsulfinyle en C₁-C₆, halogénoalkylsulfinyle en C₁-C₆, alkylsulfonyle en C₁-C₆ ou halogénoalkylsulfonyle en C₁-C₆,
R² représente un radical hétérocyclyle pentagonal à liaison C qui est non substitué ou substitué par un ou deux radicaux du groupe suivant :
alkyle en C₁-C₄, cycloalkyle en C₃-C₆, halogénoalkyle en C₁-C₄, C₁-C₄-alcoxy-C₁-C₄-alkyle, C₁-C₄-alcoxycarbonyle, alcoxy en C₁-C₄ ou spirocycloalcane en C₃-C₆,
et qui contient deux hétéroatomes identiques ou différents choisis parmi le groupe suivant :
oxygène, soufre ou azote,
R⁴ représente de l'hydrogène, un halogène ou un groupe alkyle en C₁-C₆,
R⁶ représente un groupe alkyle en C₁-C₆,
R⁷ représente un groupe alkyle en C₁-C₆, alcényle en C₃-C₆, alcynyle en C₃-C₆, alkylcarbonyle en C₁-C₆, alcénylcarbonyle en C₂-C₆, alcoxycarbonyle en C₁-C₆, di-(C₁-C₆-alkyl)-aminocarbonyle, N- (C₁-C₆-alcoxy) -N- (C₁-C₆-alkyl)-aminocarbonyle ou di-(C₁-C₆-alkyl)-aminothiocarbonyle, les radicaux alkyle et alcoxy cités pouvant être partiellement ou totalement halogénés et/ou porter un à trois des groupes suivants :
cyano, alcoxy en C₁-C₄, alcoxycarbonyle en C₁-C₄, C₁-C₄-alcoxy-C₁-C₄-alcoxycarbonyle, di-(C₁-C₄-alkyl) -amino-C₁-C₄-alcoxycarbonyle, C₁-C₄-alkylaminocarbonyle, di-(C₁-C₄-alkyl)-aminocarbonyle ou C₁-C₄-alkylcarbonyloxy, phényl-C₁-C₆-alkyle, phénylcarbonyl-C₁-C₆-alkyle, phénylcarbonyle ou phényl-C₂-C₆-alcénylcarbonyle, où le radical phényle des quatre substituants cités en dernier peut être partiellement ou totalement halogéné et/ou porter un à trois des radicaux suivants :
nitro, cyano, alkyle en C₁-C₄, halogénoalkyle en C₁-C₄, alcoxy en C₁-C₄ ou halogénoalcoxy en C₁-C₄,
R⁸ représente de l'hydrogène ou un groupe alkyle en C₁-C₆ ou halogénoalkyle en C₁-C₆,
ainsi que leurs sels utilisables en agriculture.

2. 4-(3-Hétérocyclyl-1-benzoyl)pyrazoles de la formule I suivant la revendication 1, dans lesquels R⁴ représente de l'hydrogène.

3. Procédé de préparation de 4-(3-hétérocyclyl-1-benzoyl)pyrazoles de la formule I suivant la revendication 1, **caractérisé en ce qu'**on fait réagir un dérivé benzoyle de la formule III, 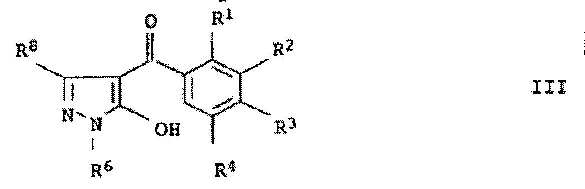 où les variables R¹ à R⁴, R⁶ et R⁸ ont les significations données dans la revendication 1,
avec un composé de la formule IV :
L¹-R⁷ IV
dans laquelle R⁷ a la signification donnée dans la revendication 1 et L¹ représente un groupe partant déplaçable par voie nucléophile.

4. Produit contenant une quantité efficace du point de vue herbicide d'au moins un 4-(3-hétérocyclyl-1-benzoyl)pyrazole de la formule I ou d'un sel utilisable en agriculture de I suivant les revendications 1 et 2 et des adjuvants courants pour la formulation de produits phytosanitaires.

5. Procédé de préparation de produits suivant la revendication 4, **caractérisé en ce qu'**on mélange une quantité efficace du point de vue herbicide d'au moins un 4-(3-hétérocyclyl-1-benzoyl)pyrazole de la formule I ou d'un sel utilisable en agriculture de I suivant les revendications 1 et 2 et des adjuvants courants pour la formulation de produits phytosanitaires.

6. Procédé de lutte contre la croissance de plantes non désirées, **caractérisé en ce qu'**on fait agir une quantité efficace du point de vue herbicide d'au moins un 4-(3-hétérocyclyl-1-benzoyl)pyrazole de la formule I ou d'un sel utilisable en agriculture de I suivant les revendications 1 et 2, sur des plantes, leur environnement et/ou des semences.

7. Utilisation de 4-(3-hétérocyclyl-1-benzoyl)pyrazoles de la formule I ou de leurs sels utilisables en agriculture suivant les revendications 1 et 2, comme herbicides.
